(19) Europäisches Patentamt
European Patent Office
Office européen des brevets

(11) EP 4 190 784 A1

(12) EUROPEAN PATENT APPLICATION
published in accordance with Art. 153(4) EPC

(43) Date of publication:
**07.06.2023 Bulletin 2023/23**

(21) Application number: **21849647.9**

(22) Date of filing: **30.07.2021**

(51) International Patent Classification (IPC):
***C07D 487/04*** *(2006.01)* ***A61K 31/495*** *(2006.01)*
***A61K 31/4985*** *(2006.01)* ***A61P 25/22*** *(2006.01)*

(52) Cooperative Patent Classification (CPC):
**A61K 31/495; A61K 31/4985; A61P 25/22; C07D 487/04;** Y02P 20/55

(86) International application number:
**PCT/CN2021/109577**

(87) International publication number:
**WO 2022/022680 (03.02.2022 Gazette 2022/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.07.2020 CN 202010753723**
**04.12.2020 CN 202011408236**

(71) Applicants:
- **Shanghai Hansoh Biomedical Co., Ltd.**
  **Shanghai 201203 (CN)**
- **Jiangsu Hansoh Pharmaceutical Group Co., Ltd.**
  **Lianyungang, Jiangsu 222047 (CN)**

(72) Inventors:
- **SU, Yidong**
  **Shanghai 201203 (CN)**
- **WANG, Feng**
  **Shanghai 201203 (CN)**
- **LI, Kailong**
  **Shanghai 201203 (CN)**
- **ZHANG, Wu**
  **Shanghai 201203 (CN)**
- **BAO, Rudi**
  **Shanghai 201203 (CN)**

(74) Representative: **Cabinet Beau de Loménie**
**158, rue de l'Université**
**75340 Paris Cedex 07 (FR)**

# (54) NITROGEN-CONTAINING FUSED RING DERIVATIVE INHIBITOR, PREPARATION METHOD THEREFOR AND USE THEREOF

(57) A nitrogen-containing fused ring derivative inhibitor, a preparation method therefor and the use thereof. The present invention particularly relates to a compound as shown in general formula (I), a preparation method therefor, a pharmaceutical composition thereof, and the use thereof as an NK inhibitor in the treatment of depression, anxiety, schizophrenia, sex hormone-dependent diseases and other related diseases. The various substituents in general formula (I) have the same definition as that in the description.

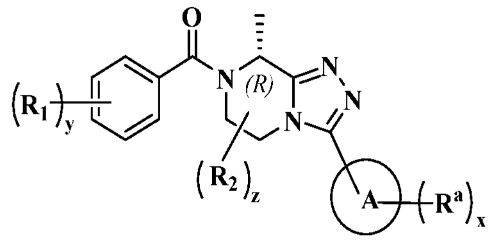

(I)

EP 4 190 784 A1

## Description

### FIELD OF THE INVENTION

The present invention belongs to the field of drug synthesis, and specifically relates to a nitrogen-containing fused ring derivative inhibitor, a preparation method therefor and a use thereof.

### BACKGROUND OF THE INVENTION

Neurokinins (NKs) include substance P (SP), neurokinin A and neurokinin B, which correspond to three types of receptors, neurokinin 1 receptor (NK1R), neurokinin 2 receptor (NK2R) and neurokinin 3 receptor (NK3R), respectively. These three types of receptors are all G protein-coupled receptors, among which NK1R is the most widely distributed. NK1R is mainly distributed in both the central nervous system and the peripheral nervous system, NK2R is mainly distributed in the peripheral nervous system, and NK3R is mainly distributed in the central nervous system. At present, NK receptor inhibitors have been used to treat menopausal hot flashes, depression, schizophrenia and the like. In particular, NK3R is closely related to hot flashes and other symptoms of menopausal syndrome, and NK3R inhibitors have been shown to have a good effect on ameliorating menopausal hot flashes.

Menopausal hot flashes refer to the symptoms such as hot flashes and sweating that often occur in menopausal people, which is a prominent feature of menopausal syndrome. Menopausal hot flashes are caused by vasomotor dysfunction due to decreased estrogen levels in the body. When estrogen decreases in the body, the brain mistakes it for hyperthermia. Thus, the brain sends signals to the heart to pump more blood, and to the sweat glands to release more sweat, accompanied by sweating, palpitations, vertigo and the like. More than three-quarters of women experience hot flashes during menopause. This symptom may last for more than 1 year in 80% of patients, and may last for about 5 years after menopause in some patients. At present, the treatment for menopausal hot flashes is mainly hormone replacement therapy, but this therapy has a relatively high risk factor, as it easily causes breast cancer, stroke, coronary heart disease, dementia and the like. The oral drug paroxetine (which belongs to SSRIs drugs and treats depression) is the only approved small molecule drug for treating menopausal hot flashes, yet it also has the problem of side effects and is approved only in the United States. Therefore, there is a clinical need to develop safer and more effective medicaments for treating menopausal syndrome.

NK receptor inhibitor compounds have been reported in the international application WO2014154895. However, most of the compounds therein have a Ki of above 20 nM in the in vitro binding experiments of NK1R/NK2R/NK3R, and most have an inhibitory effect on NK3R with $IC_{50}$ of above 30 nM in cell function experiments. It is reported in CN103906750 that most of the compounds therein have a Ki of above 20 nM in the in vitro binding experiments of NK1R/NK2R/NK3R, and most have an inhibitory effect on NK3R with $IC_{50}$ of above 20 nM in cell function experiments. It is reported in CN105229008B that most of the compounds therein have a Ki of above 30 nM in the in vitro binding experiments of NK3R. It is reported in CN102906093B that most of the compounds therein have a Ki of above 500 nM in the in vitro binding experiments of NK1R/NK2R/NK3R.

Therefore, there is an urgent need to develop highly active NK receptor inhibitors for ameliorating the symptom of menopausal hot flashes in order to meet the huge market demand.

### SUMMARY OF THE INVENTION

The objective of the present invention is to provide a compound of formula (I), a stereoisomer thereof or a pharmaceutically acceptable salt thereof, wherein the structure of the compound of formula (I) is as follows:

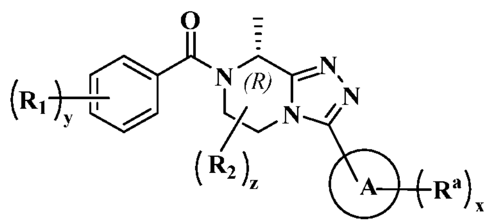

(I)

wherein:

ring A is selected from the group consisting of cycloalkyl, heterocyclyl, aryl and heteroaryl;

$R^a$ is selected from the group consisting of hydrogen, deuterium, alkyl, deuterated alkyl, haloalkyl, hydroxyalkyl, alkoxy, alkylthio, haloalkoxy, halogen, amino, nitro, hydroxy, cyano, alkenyl, alkynyl, cycloalkyl, heterocyclyl, aryl, heteroaryl, $-(CH_2)_n-$, $-(CH_2)_nR_{aa}$, $-(CH_2)_nOR_{aa}$, $-(CH_2)_nSR_{aa}$, $-(CH_2)_nC(O)R_{aa}$, $-(CH_2)_nC(O)OR_{aa}$, $-(CH_2)_nS(O)_mR_{aa}$, $-(CH_2)_nNR_{aa}R_{bb}$, $-(CH_2)_nC(O)NR_{aa}R_{bb}$, $-(CH_2)_nNR_{aa}C(O)R_{bb}$ and $-(CH_2)_nNR_{aa}S(O)_mR_{bb}$, the amino, alkyl, deuterated alkyl, haloalkyl, hydroxyalkyl, alkoxy, alkylthio, haloalkoxy, alkenyl, alkynyl, cycloalkyl, heterocyclyl, aryl and heteroaryl may optionally be further substituted;

$R_1$ is selected from the group consisting of hydrogen, deuterium, alkyl, deuterated alkyl, haloalkyl, hydroxyalkyl, alkoxy, alkylthio, haloalkoxy, halogen, amino, nitro, hydroxy, cyano, alkenyl, alkynyl, cycloalkyl, heterocyclyl, aryl and heteroaryl, the amino, alkyl, deuterated alkyl, haloalkyl, hydroxyalkyl, alkoxy, alkylthio, haloalkoxy, alkenyl, alkynyl, cycloalkyl, heterocyclyl, aryl and heteroaryl may optionally be further substituted;

$R_2$ is selected from the group consisting of hydrogen, deuterium, alkyl, deuterated alkyl, haloalkyl, hydroxyalkyl, alkoxy, alkylthio, haloalkoxy, halogen, amino, nitro, hydroxy, cyano, oxo, alkenyl, alkynyl, cycloalkyl, heterocyclyl, aryl and heteroaryl, the amino, alkyl, deuterated alkyl, haloalkyl, hydroxyalkyl, alkoxy, alkylthio, haloalkoxy, alkenyl, alkynyl, cycloalkyl, heterocyclyl, aryl and heteroaryl may optionally be further substituted;

$R_{aa}$ and $R_{bb}$ are each independently selected from the group consisting of hydrogen, deuterium, alkyl, deuterated alkyl, haloalkyl, hydroxyalkyl, alkoxy, alkylthio, haloalkoxy, halogen, amino, nitro, hydroxy, cyano, alkenyl, alkynyl, cycloalkyl, heterocyclyl, aryl and heteroaryl, the amino, alkyl, deuterated alkyl, haloalkyl, hydroxyalkyl, alkoxy, alkylthio, haloalkoxy, alkenyl, alkynyl, cycloalkyl, heterocyclyl, aryl and heteroaryl may optionally be further substituted;

x is 0, 1, 2, 3, 4, 5 or 6;

y is 0, 1, 2, 3, 4 or 5;

z is 0, 1, 2, 3, 4 or 5;

m is 0, 1 or 2; and

n is 0, 1 or 2.

In a further preferred embodiment of the present invention, in the compound of formula (I), the stereoisomer thereof or the pharmaceutically acceptable salt thereof, at least one deuterium atom is present, but (R)-(4-fluorophenyl)-(8-methyl-3-(3-(methyl-d3)-1,2,4-thiadiazol-5-yl)-5,6-dihydro-[1, 2,4]triazolo[4,3-a]pyrazin-7(8H)-yl)methanone is excluded.

In a further preferred embodiment of the present invention, ring A is selected from the group consisting of $C_{3-8}$ cycloalkyl, 3-12 membered heterocyclyl, $C_{6-14}$ aryl and 5-14 membered heteroaryl; preferably 5-10 membered heteroaryl containing 1-4 atoms selected from the group consisting of N, O and S; more preferably five membered heteroaryl, five membered fused five membered heteroaryl, or five membered fused six membered heteroaryl containing 1-4 atoms selected from the group consisting of N, O and S; further preferably

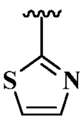 , 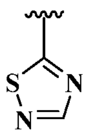 , 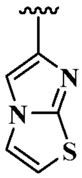 , 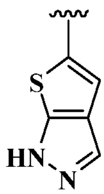 , 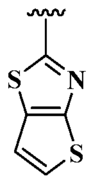 , 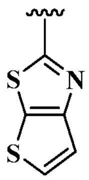 , 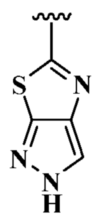 , 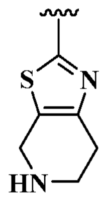 , 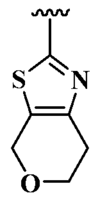 , 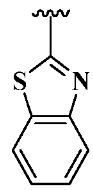 ,

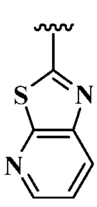 , 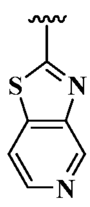 , 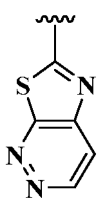 , 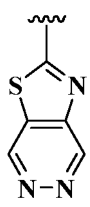 , 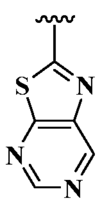 , 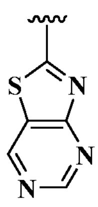 , 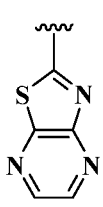 , 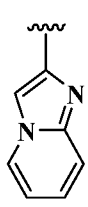 , 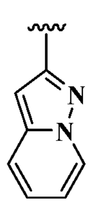 ,

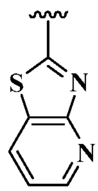 or 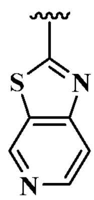 .

In a further preferred embodiment of the present invention, in the compound of formula (I), the stereoisomer thereof or the pharmaceutically acceptable salt thereof,

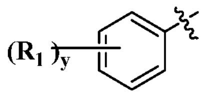

is selected from the group consisting of the following groups:

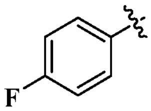, 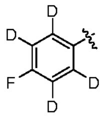, 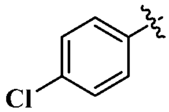, 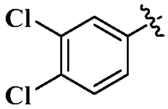, 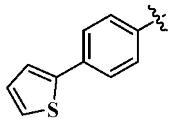, 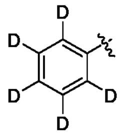,

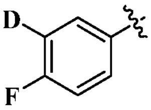, 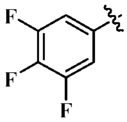, 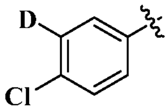, and 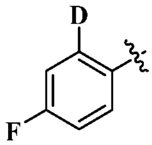.

In a further preferred embodiment of the present invention, in the compound of formula (I), the stereoisomer thereof or the pharmaceutically acceptable salt thereof,

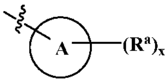

is selected from the group consisting of the following groups:

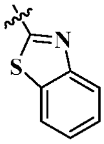,

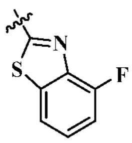, 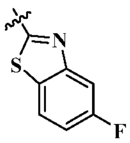, 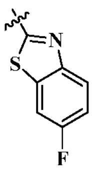, 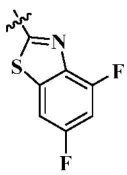, 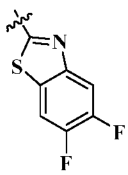, 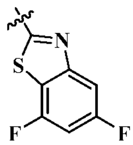, 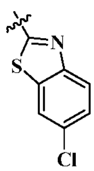, 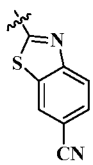,

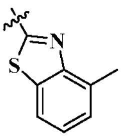, 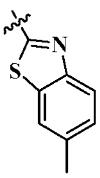, 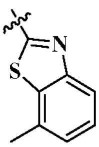, 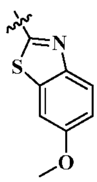, 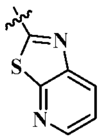, 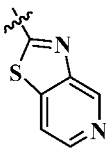, 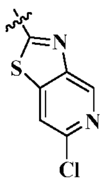, 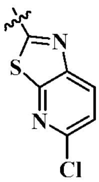,

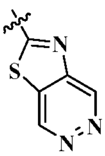, 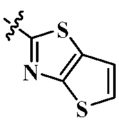, 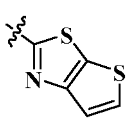, 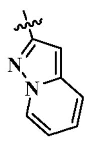, 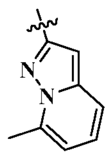, 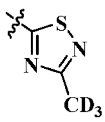, 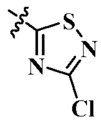, 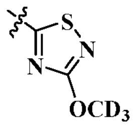,

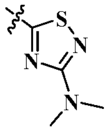, 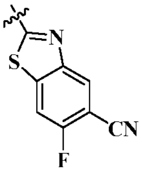, 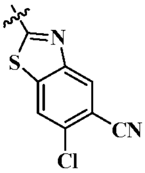, 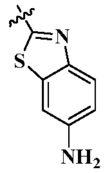, 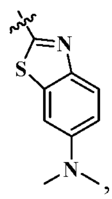, 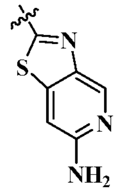, 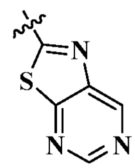, 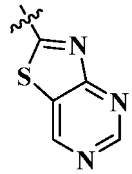,

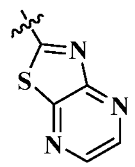, 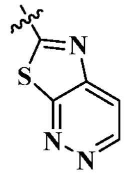, 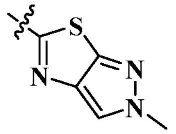, 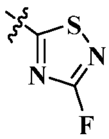, 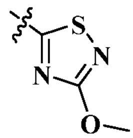, 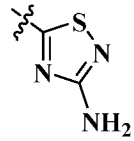, 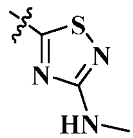,

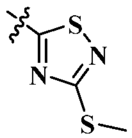, 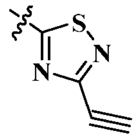, 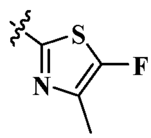, 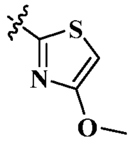, 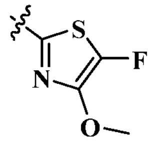, 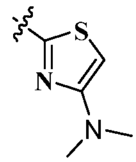, 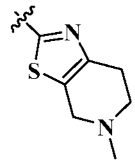,

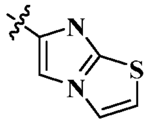, 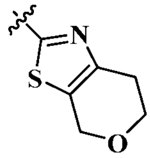, 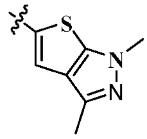, 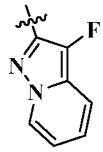, 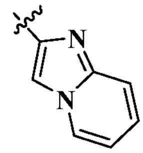, 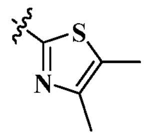, 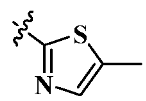,

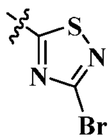, 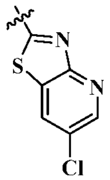, 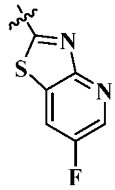, 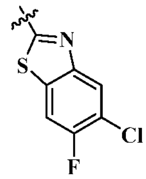, 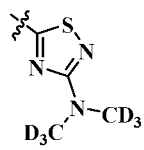, 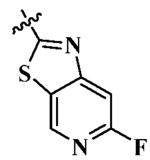, 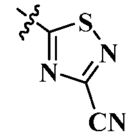,

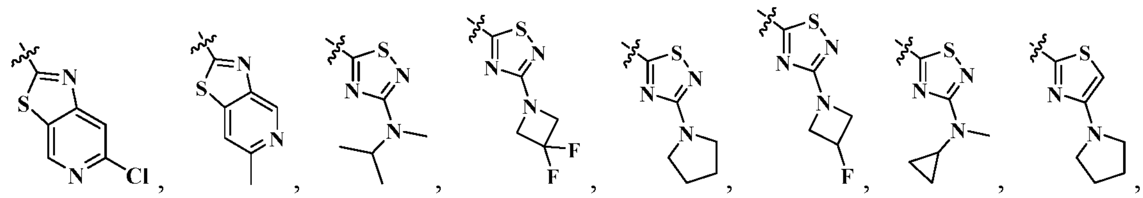
, , , , , , , ,

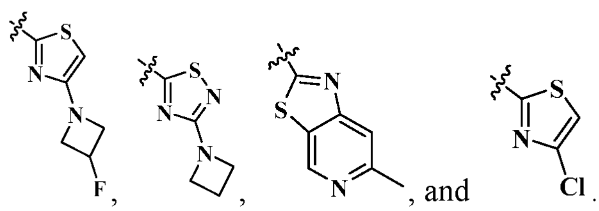
, , , and .

In a further preferred embodiment of the present invention, $R^a$ is selected from the group consisting of hydrogen, deuterium, halogen, amino, hydroxy, cyano, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-8}$ cycloalkyl, 3-12 membered heterocyclyl, $C_{6-14}$ aryl, 5-14 membered heteroaryl, $-(CH_2)_n-$, $-(CH_2)_nR_{aa}$, $-(CH_2)_nOR_{aa}$, $-(CH_2)_nSR_{aa}$, $-(CH_2)_nC(O)R_{aa}$, $-(CH_2)_nC(O)OR_{aa}$, $-(CH_2)_nS(O)_mR_{aa}$, $-(CH_2)_nNR_{aa}R_{bb}$, $-(CH_2)_nC(O)NR_{aa}R_{bb}$, $-(CH_2)_nNR_{aa}C(O)R_{bb}$ and $-(CH_2)_nNR_{aa}S(O)_mR_{bb}$, the $C_{1-6}$

alkyl, $C_{2-6}$ alkenyl, $C_{2-6}$ alkynyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-8}$ cycloalkyl, 3-12 membered heterocyclyl, $C_{6-14}$ aryl and 5-14 membered heteroaryl are each optionally further substituted by one or more substituents of deuterium, halogen, amino, hydroxy, cyano, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-8}$ cycloalkyl, 3-12 membered heterocyclyl, $C_{6-14}$ aryl and 5-14 membered heteroaryl;

preferably hydrogen, deuterium, halogen, cyano, amino, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ deuterated alkoxy, $-(CH_2)_{n1}OR_{aa}$, $-(CH_2)_{n1}SR_{aa}$ or $-(CH_2)_{n1}NR_{aa}R_{bb}$;
more preferably hydrogen, deuterium, fluorine, chlorine, bromine, cyano, amino, methyl, ethyl, isopropyl, deuterated methyl, ethynyl, $-OCH_3$, $-OCD_3$, $-NHCH_3$, $-N(CH_3)_2$ or $-SCH_3$.

In a further preferred embodiment of the present invention, $R_1$ is selected from the group consisting of hydrogen, deuterium, halogen, amino, hydroxy, cyano, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-8}$ cycloalkyl, 3-12 membered heterocyclyl, $C_{6-14}$ aryl and 5-14 membered heteroaryl, the $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-8}$ cycloalkyl, 3-12 membered heterocyclyl, $C_{6-14}$ aryl and 5-14 membered heteroaryl are each optionally further substituted by one or more substituents of deuterium, halogen, amino, hydroxy, cyano, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-8}$ cycloalkyl, 3-12 membered heterocyclyl, $C_{6-14}$ aryl and 5-14 membered heteroaryl;

preferably hydrogen, deuterium, halogen or 5-6 membered heteroaryl;
more preferably hydrogen, deuterium, fluorine, chlorine, bromine or thienyl.

In a further preferred embodiment of the present invention, $R_2$ is selected from the group consisting of hydrogen, deuterium, halogen, amino, hydroxy, cyano, oxo, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-8}$ cycloalkyl, 3-12 membered heterocyclyl, $C_{6-14}$ aryl and 5-14 membered heteroaryl.

In a further preferred embodiment of the present invention, $R_{aa}$ and $R_{bb}$ are each independently selected from the group consisting of hydrogen, deuterium, halogen, amino, hydroxy, cyano, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-8}$ cycloalkyl, 3-12 membered heterocyclyl, $C_{6-14}$ aryl and 5-14 membered heteroaryl, the $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-8}$ cycloalkyl, 3-12 membered heterocyclyl, $C_{6-14}$ aryl and 5-14 membered heteroaryl are each optionally further substituted by one or more substituents of deuterium, halogen, amino, hydroxy, cyano, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-8}$ cycloalkyl, 3-12 membered heterocyclyl, $C_{6-14}$ aryl and 5-14 membered heteroaryl.

In a further preferred embodiment of the present invention, ring A is selected from the group consisting of

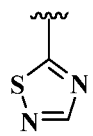 and 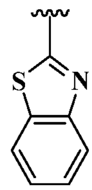 ;

$R^a$ is selected from the group consisting of hydrogen, deuterium, halogen, cyano, amino, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ deuterated alkoxy, $-(CH_2)_{n1}OR_{aa}$, $-(CH_2)_{n1}SR_{aa}$ and $-(CH_2)_{n1}NR_{aa}R_{bb}$;
preferably hydrogen, deuterium, fluorine, chlorine, cyano, amino, methyl, deuterated methyl, ethynyl, azetidinyl, tetrahydropyrrolyl, $-OCH_3$, $-OCD_3$, $-NHCH_3$, $-N(CH_3)_2$, $-SCH_3$, $-N(CD_3)_2$, $-N(CH_3)CH(CH_3)_2$,

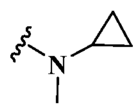 , 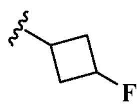

or

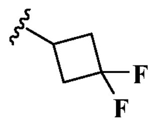

In a further preferred embodiment of the present invention, ring A is

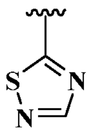

$R^a$ is halogen, preferably fluorine, chlorine or bromine, more preferably chlorine.

The present invention further provides a compound of formula (II), a stereoisomer thereof or a pharmaceutically acceptable salt thereof, wherein the structure of the compound of formula (II) is as follows:

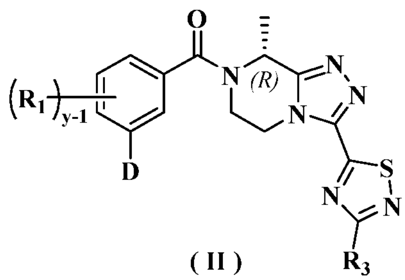

( II )

wherein:

$R_1$ is selected from the group consisting of hydrogen, deuterium and halogen; preferably hydrogen, deuterium, fluorine, chlorine or bromine;

$R_3$ is selected from the group consisting of hydrogen, deuterium, halogen, $C_{1-6}$ alkyl, $C_{1-6}$ deuterated alkyl, 3-6 membered heterocyclyl; the $C_{1-6}$ alkyl, $C_{1-6}$ deuterated alkyl and 3-6 membered heterocyclyl are each optionally further substituted by one or more substituents of deuterium and halogen;

preferably hydrogen, deuterium, fluorine, chlorine, bromine, methyl, deuterated methyl, azetidinyl, tetrahydropyrrolyl,

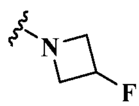 or 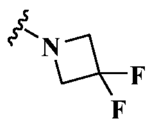 ;

y is 1, 2, 3, 4 or 5.

The present invention further provides a method for preparing the compound of formula (I) or the stereoisomer thereof or the pharmaceutically acceptable salt thereof, the method comprises the following steps of:

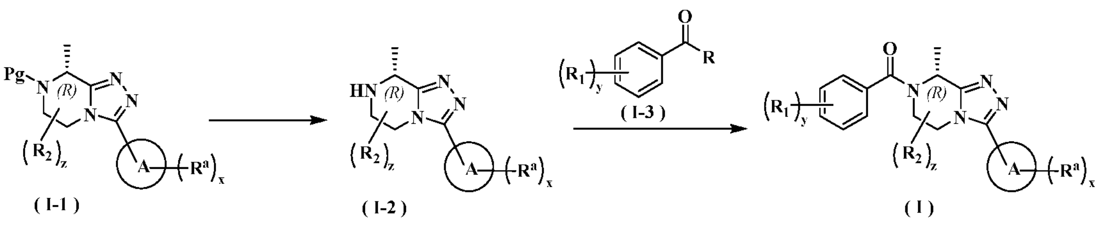

deprotecting a compound of formula (I-1) to obtain a compound of formula (I-2) or a stereoisomer thereof and a

pharmaceutically acceptable salt thereof; then, subjecting the compound of formula (I-2) and a compound of formula (I-3) to a condensation reaction to obtain the compound of formula (I) or the stereoisomer thereof or the pharmaceutically acceptable salt thereof;

wherein,

Pg is an amino protecting group; preferably allyloxycarbonyl, trifluoroacetyl, 2,4-dimethoxybenzyl, nitrobenzenesulfonyl, triphenylmethyl, fluorenylmethyloxycarbonyl, p-toluenesulfonyl, formate, acetyl, benzyloxycarbonyl, tert-butoxycarbonyl, benzyl or p-methoxyphenyl; more preferably 2,4-dimethoxybenzyl;

R is selected from the group consisting of halogen, hydroxy and -C(O)OR$_A$, preferably fluorine, chlorine, bromine, iodine or hydroxy; more preferably chlorine or hydroxy;

R$_A$ is C$_{1-6}$ alkyl.

The present invention further provides a method for preparing the compound of formula (II) or the stereoisomer thereof or the pharmaceutically acceptable salt thereof, the method comprises the following steps of:

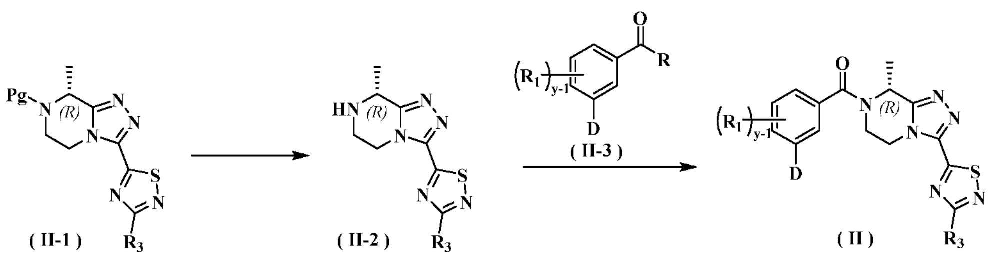

deprotecting a compound of formula (II-1) to obtain a compound of formula (II-2) or a stereoisomer thereof and a pharmaceutically acceptable salt thereof; then, subjecting the compound of formula (II-2) and a compound of formula (II-3) to a condensation reaction to obtain the compound of formula (II) or the stereoisomer thereof or the pharmaceutically acceptable salt thereof;

wherein,

Pg is an amino protecting group; preferably allyloxycarbonyl, trifluoroacetyl, 2,4-dimethoxybenzyl, nitrobenzenesulfonyl, triphenylmethyl, fluorenylmethyloxycarbonyl, p-toluenesulfonyl, formate, acetyl, benzyloxycarbonyl, tert-butoxycarbonyl, benzyl or p-methoxyphenyl; more preferably 2,4-dimethoxybenzyl;

R is selected from the group consisting of halogen, hydroxy and -C(O)OR$_A$, preferably fluorine, chlorine, bromine, iodine or hydroxy; more preferably chlorine or hydroxy;

R$_A$ is C$_{1-6}$ alkyl.

The present invention also provides a pharmaceutical composition comprising a therapeutically effective amount of the compound of each general formula, the stereoisomer thereof or the pharmaceutically acceptable salt thereof as described above, and one or more pharmaceutically acceptable carriers or excipients.

The present invention also provides a preferred embodiment that further relates to use of the compound of each general formula, the stereoisomer thereof or the pharmaceutically acceptable salt thereof as described above, or the pharmaceutical composition as described above, in the preparation of a NK inhibitor-related medicament, particularly in the preparation of a NK3 inhibitor-related medicament.

The present invention also provides a preferred embodiment that further relates to use of the compound of each general formula, the stereoisomer thereof or the pharmaceutically acceptable salt thereof as described above, or the pharmaceutical composition as described above, in the preparation of a medicament for treating and/or preventing psychotic disorder, cognitive disorder, Parkinson's disease, Alzheimer's disease, attention-deficit hyperactivity disorder, pain, convulsion, obesity, inflammatory disease, vomiting, preeclampsia, airway-related disease, reproductive disorder, sex hormone-dependent disease or gynecological disease-related disease.

The present invention also provides a preferred embodiment that further relates to use of the compound of each general formula, the stereoisomer thereof or the pharmaceutically acceptable salt thereof as described above, or the pharmaceutical composition as described above, in the preparation of a medicament for treating and/or preventing menopausal syndrome-related disease, wherein the menopausal syndrome includes symptoms of hot flashes, sweating, palpitations, vertigo and obesity.

The present invention further relates to the compound of each general formula, the stereoisomer thereof or the pharmaceutically acceptable salt thereof as described above, or the pharmaceutical composition comprising the same, for use as a medicament for treating and/or preventing psychotic disorder, cognitive disorder, Parkinson's disease, Alzheimer's disease, attention-deficit hyperactivity disorder, pain, convulsion, obesity, inflammatory disease, vomiting,

preeclampsia, airway-related disease, reproductive disorder, sex hormone-dependent disease or gynecological disease-related disease.

The present invention also relates to a method for treating and/or preventing psychotic disorder, cognitive disorder, Parkinson's disease, Alzheimer's disease, attention-deficit hyperactivity disorder, pain, convulsion, obesity, inflammatory disease, vomiting, preeclampsia, airway-related disease, reproductive disorder, sex hormone-dependent disease or gynecological disease-related disease, the method comprises administering a therapeutically effective amount of the compound of the present invention or a pharmaceutically acceptable salt, ester, prodrug, solvate, hydrate or derivative thereof, to a mammal.

In some embodiments, the method involves treatments such as treating and/or preventing psychotic disorder, cognitive disorder, Parkinson's disease, Alzheimer's disease, attention-deficit hyperactivity disorder, pain, convulsion, obesity, inflammatory disease, vomiting, preeclampsia, airway-related disease, reproductive disorder, sex hormone-dependent disease or gynecological disease-related disease.

The treatment method provided herein comprises administering a therapeutically effective amount of the compound of the present invention to a subject. In one embodiment, the present invention provides a method for treating related diseases including menopausal hot flashes in a mammal. The method comprises administering a therapeutically effective amount of the compound of the present invention, or a pharmaceutically acceptable salt, ester, prodrug, solvate, hydrate or derivative thereof, to the mammal.

## DEFINITIONS

Unless otherwise stated, the terms used in the specification and claims have the meanings described below.

The term "alkyl" refers to a saturated aliphatic hydrocarbon group, which is a straight or branched chain group comprising 1 to 20 carbon atoms, preferably an alkyl having 1 to 8 carbon atoms, more preferably an alkyl having 1 to 6 carbon atoms, most preferably an alkyl having 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof. More preferably, the alkyl group is a lower alkyl having 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl group can be substituted or unsubstituted. When substituted, the substituent group(s) can be substituted at any available connection point. The substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl. The alkyl of the present invention is preferably selected from the group consisting of methyl, ethyl, isopropyl, tert-butyl, haloalkyl, deuterated alkyl, alkoxy-substituted alkyl and hydroxy-substituted alkyl.

The term "alkylene" refers to an alkyl with one hydrogen being further substituted, for example, "methylene" refers to $-CH_2-$, "ethylene" refers to $-(CH_2)_2-$, "propylene" refers to $-(CH_2)_3-$, "butylene" refers to $-(CH_2)_4-$ and the like. The term "alkenyl" refers to an alkyl as defined above consisting of at least two carbon atoms and at least one carbon-carbon double bond, for example, ethenyl, 1-propenyl, 2-propenyl, 1-, 2- or 3-butenyl and the like. The alkenyl group can be substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio and heterocyclylthio.

The term "cycloalkyl" refers to a saturated or partially unsaturated monocyclic or polycyclic hydrocarbon substituent group having 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes cycloalkyl having a spiro ring, fused ring or bridged ring. The cycloalkyl is preferably cyclopropyl, cyclobutyl, cyclohexyl, cyclopentyl and cycloheptyl.

The term "spiro cycloalkyl" refers to a 5 to 20 membered polycyclic group with individual rings connected through

one shared carbon atom (called a spiro atom), wherein the rings can contain one or more double bonds, but none of the rings has a completely conjugated π-electron system. The spiro cycloalkyl is preferably 6 to 14 membered spiro cycloalkyl, and more preferably 7 to 10 membered spiro cycloalkyl. According to the number of the spiro atoms shared between the rings, the spiro cycloalkyl can be divided into mono-spiro cycloalkyl, di-spiro cycloalkyl, or poly-spiro cycloalkyl, and the spiro cycloalkyl is preferably a mono-spiro cycloalkyl or di-spiro cycloalkyl, and more preferably 4-membered/4-membered, 4-membered/5-membered, 4-membered/6-membered, 5-membered/5-membered, or 5-membered/6-membered mono-spiro cycloalkyl. Non-limiting examples of spiro cycloalkyl include:

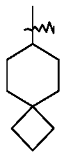 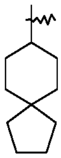 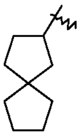 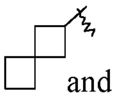 and 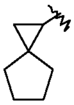 ;

spiro cycloalkyl also includes mono-spiro cycloalkyl in which a cycloalkyl and a heterocyclyl share a spiro atom, non-limiting examples of which include:

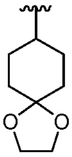 and 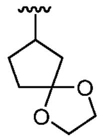 .

The term "fused cycloalkyl" refers to a 5 to 20 membered all-carbon polycyclic group, wherein each ring in the system shares an adjacent pair of carbon atoms with another ring, wherein one or more rings can contain one or more double bonds, but none of the rings has a completely conjugated π-electron system. The fused cycloalkyl is preferably 6 to 14 membered fused cycloalkyl, and more preferably 7 to 10 membered fused cycloalkyl. According to the number of membered rings, the fused cycloalkyl can be divided into bicyclic, tricyclic, tetracyclic or polycyclic fused cycloalkyl, and the fused cycloalkyl is preferably bicyclic or tricyclic fused cycloalkyl, and more preferably 5-membered/5-membered, or 5-membered/6-membered bicyclic fused cycloalkyl. Non-limiting examples of fused cycloalkyl include:

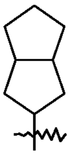 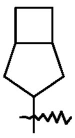 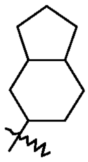 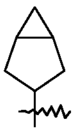 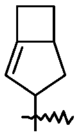 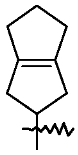 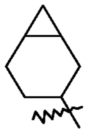 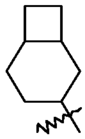 and 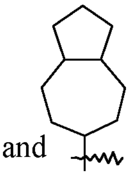 .

The term "bridged cycloalkyl" refers to a 5 to 20 membered all-carbon polycyclic group, wherein every two rings in the system share two disconnected carbon atoms, wherein the rings can have one or more double bonds, but none of the rings has a completely conjugated π-electron system. The bridged cycloalkyl is preferably 6 to 14 membered bridged cycloalkyl, and more preferably 7 to 10 membered bridged cycloalkyl. According to the number of membered rings, the bridged cycloalkyl can be divided into bicyclic, tricyclic, tetracyclic or polycyclic bridged cycloalkyl, and the bridged cycloalkyl is preferably bicyclic, tricyclic or tetracyclic bridged cycloalkyl, and more preferably bicyclic or tricyclic bridged cycloalkyl. Non-limiting examples of bridged cycloalkyl include:

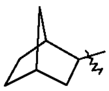 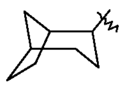 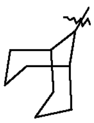 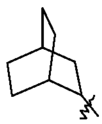 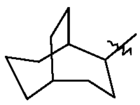

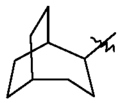 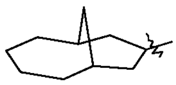 and 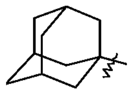 .

The cycloalkyl ring can be fused to the ring of aryl, heteroaryl or heterocyclyl, wherein the ring bound to the parent structure is cycloalkyl. Non-limiting examples include indanyl, tetrahydronaphthyl, benzocycloheptyl and the like. The cycloalkyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "heterocyclyl" refers to a 3 to 20 membered saturated or partially unsaturated monocyclic or polycyclic hydrocarbon group, wherein one or more ring atoms are heteroatoms selected from the group consisting of N, O and $S(O)_m$ (wherein m is an integer of 0 to 2), but excluding -O-O-, -O-S- or -S-S- in the ring, with the remaining ring atoms being carbon atoms. Preferably, the heterocyclyl has 3 to 12 ring atoms wherein 1 to 4 atoms are heteroatoms; more preferably, 3 to 8 ring atoms; and most preferably 3 to 8 ring atoms. Non-limiting examples of monocyclic heterocyclyl include azetidinyl, oxetanyl, tetrahydropyranyl, azepanyl, tetrahydropyrrolyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl and the like, and preferably azetidinyl, tetrahydropyrrolyl, oxetanyl, tetrahydrofuranyl, tetrahydropyranyl, azepanyl, piperidinyl and piperazinyl. Polycyclic heterocyclyl includes a heterocyclyl having a spiro ring, fused ring or bridged ring; wherein the involved heterocyclyl having a spiro ring, fused ring or bridged ring is optionally connected to other group(s) through single bond, or further fused to other cycloalkyl, heterocyclyl, aryl and heteroaryl through any two or more atoms in the ring.

The term "spiro heterocyclyl" refers to a 5 to 20 membered polycyclic heterocyclyl group with individual rings connected through one shared atom (called a spiro atom), wherein one or more ring atoms are heteroatoms selected from the group consisting of N, O and $S(O)_m$ (wherein m is an integer of 0 to 2), with the remaining ring atoms being carbon atoms, where the rings can contain one or more double bonds, but none of the rings has a completely conjugated $\pi$-electron system. The spiro heterocyclyl is preferably 6 to 14 membered spiro heterocyclyl, and more preferably 7 to 10 membered spiro heterocyclyl. According to the number of the spiro atoms shared between the rings, the spiro heterocyclyl can be divided into mono-spiro heterocyclyl, di-spiro heterocyclyl, or poly-spiro heterocyclyl, and the spiro heterocyclyl is preferably mono-spiro heterocyclyl or di-spiro heterocyclyl, and more preferably 4-membered/4-membered, 4-membered/5-membered, 4-membered/6-membered, 5-membered/5-membered, or 5-membered/6-membered mono-spiro heterocyclyl. Non-limiting examples of spiro heterocyclyl include:

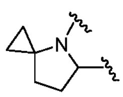 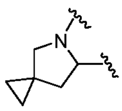 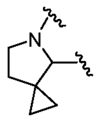 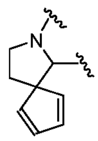 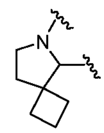 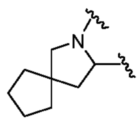

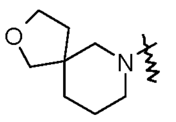 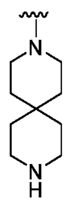 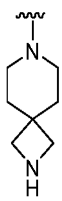 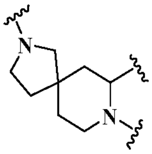 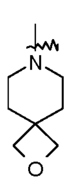 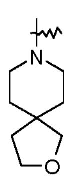 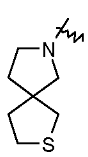 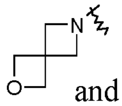 and 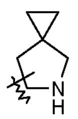 .

The term "fused heterocyclyl" refers to a 5 to 20 membered polycyclic heterocyclyl group, wherein each ring in the system shares an adjacent pair of atoms with another ring, wherein one or more rings can contain one or more double bonds, but none of the rings has a completely conjugated $\pi$-electron system, and wherein one or more ring atoms are heteroatoms selected from the group consisting of N, O and $S(O)_m$ (wherein m is an integer of 0 to 2), with the remaining ring atoms being carbon atoms. The fused heterocyclyl is preferably 6 to 14 membered fused heterocyclyl, and more preferably 7 to 10 membered fused heterocyclyl. According to the number of membered rings, the fused heterocyclyl can be divided into bicyclic, tricyclic, tetracyclic or polycyclic fused heterocyclyl, and the fused heterocyclyl is preferably bicyclic or tricyclic fused heterocyclyl, and more preferably 5-membered/5-membered or 5-membered/6-membered bicyclic fused heterocyclyl. Non-limiting examples of fused heterocyclyl include:

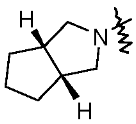 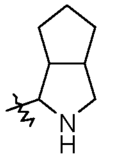 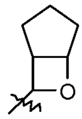 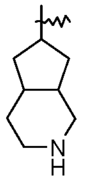 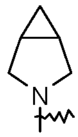 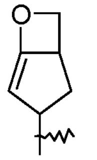 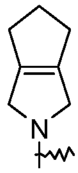 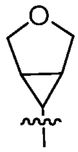 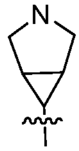

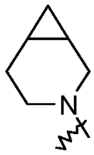 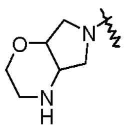 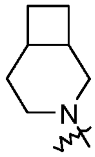 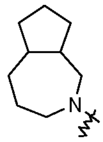 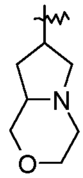 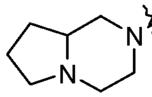 and 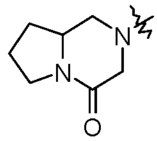 .

The term "bridged heterocyclyl" refers to a 5 to 14 membered polycyclic heterocyclyl group, wherein every two rings in the system share two disconnected atoms, wherein the rings can have one or more double bonds, but none of the rings has a completely conjugated $\pi$-electron system, and wherein one or more ring atoms are heteroatoms selected from the group consisting of N, O and S(O)m (wherein m is an integer of 0 to 2), with the remaining ring atoms being carbon atoms. The bridged heterocyclyl is preferably 6 to 14 membered bridged heterocyclyl, and more preferably 7 to 10 membered bridged heterocyclyl. According to the number of membered rings, the bridged heterocyclyl can be divided into bicyclic, tricyclic, tetracyclic or polycyclic bridged heterocyclyl, and the bridged heterocyclyl is preferably bicyclic, tricyclic or tetracyclic bridged heterocyclyl, and more preferably bicyclic or tricyclic bridged heterocyclyl. Non-limiting examples of bridged heterocyclyl include:

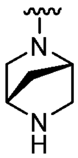 , 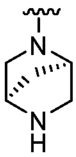 , 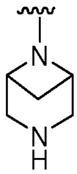 , 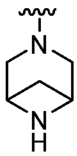 , 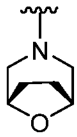 , 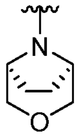 , 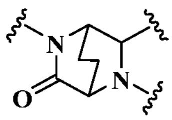 , 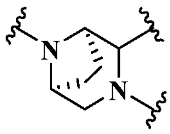 ,

and

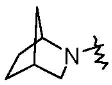 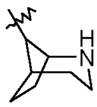 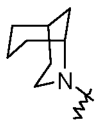 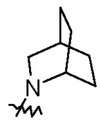

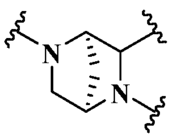 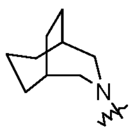 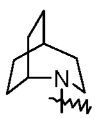 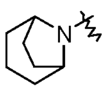 or 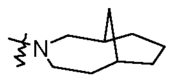 .

The heterocyclyl ring can be fused to the ring of aryl, heteroaryl or cycloalkyl, wherein the ring bound to the parent structure is heterocyclyl. Non-limiting examples thereof include:

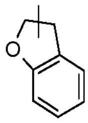 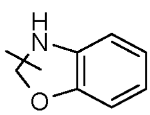 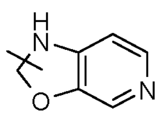 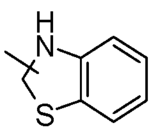 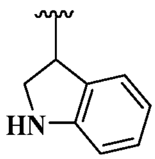 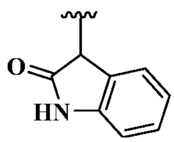 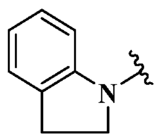

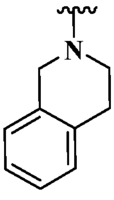 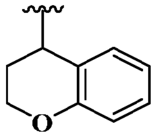 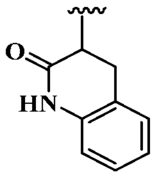 and 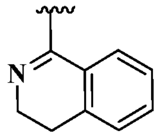 .

The heterocyclyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "aryl" refers to a 6 to 14 membered all-carbon monocyclic ring or polycyclic fused ring (i.e. each ring in the system shares an adjacent pair of carbon atoms with another ring in the system) having a conjugated $\pi$-electron system, preferably 6 to 10 membered aryl, for example, phenyl and naphthyl. The aryl is more preferably phenyl. The aryl ring can be fused to the ring of heteroaryl, heterocyclyl or cycloalkyl, wherein the ring bound to the parent structure is aryl ring. Non-limiting examples thereof include:

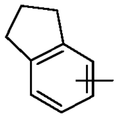 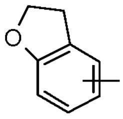 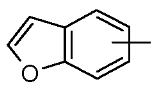 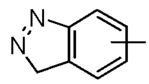 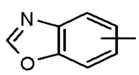 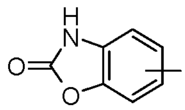 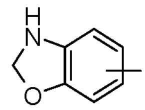

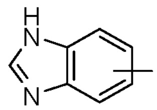 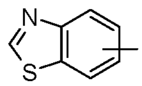 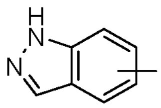 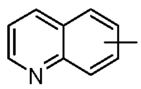 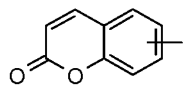 and 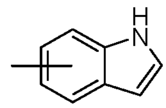 .

The aryl can be substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "heteroaryl" refers to a 5 to 14 membered heteroaromatic system having 1 to 4 heteroatoms selected from the group consisting of O, S and N. The heteroaryl is preferably 5 to 10 membered heteroaryl, more preferably 5 or 6 membered heteroaryl, for example imidazolyl, furyl, thienyl, thiazolyl, pyrazolyl, oxazolyl, pyrrolyl, triazolyl, tetrazolyl, pyridyl, pyridazinyl, pyrimidinyl, thiadiazolyl, pyrazinyl, oxadiazolyl and the like; preferably triazolyl, pyridyl, thienyl, thiazolyl, pyridazinyl, pyrimidinyl, pyrazinyl, pyrazolyl, imidazolyl, thiazolyl, thiadiazolyl; and more preferably triazolyl, thienyl, thiazolyl, thiadiazolyl, pyrazolyl, imidazolyl, pyridyl, pyridazinyl, pyrimidinyl, pyrazinyl. The heteroaryl ring can be fused to the ring of aryl, heterocyclyl or cycloalkyl, wherein the ring bound to the parent structure is heteroaryl ring. Non-limiting examples thereof include:

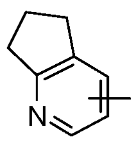 , 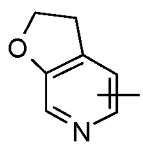 , 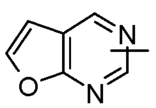 , 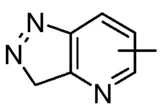 , 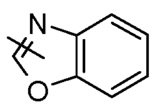 , 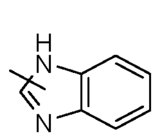 ,

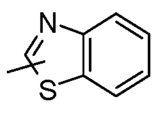 , 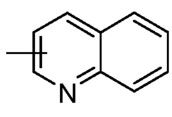 , 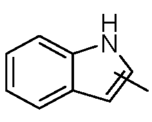 , 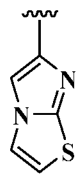 , 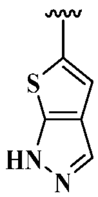 , 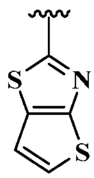 , 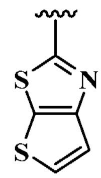 ,

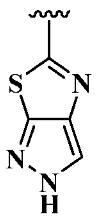, 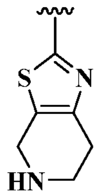, 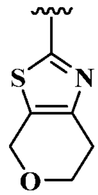, 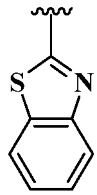, 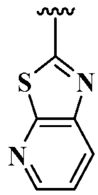, 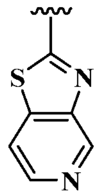, 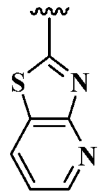, 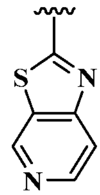, 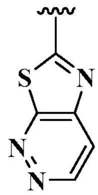,

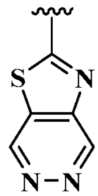, 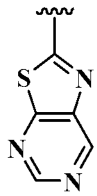, 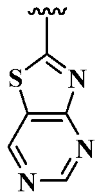, 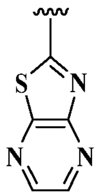, 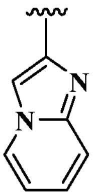 and 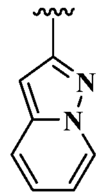.

The heteroaryl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "alkoxy" refers to an -O-(alkyl) or an -O-(unsubstituted cycloalkyl) group, wherein the alkyl is as defined above. Non-limiting examples of alkoxy include methoxy, ethoxy, propoxy, butoxy, cyclopropyloxy, cyclobutyloxy, cyclopentyloxy, cyclohexyloxy. The alkoxy can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The "haloalkyl" refers to an alkyl group substituted by one or more halogens, wherein the alkyl is as defined above.

The "haloalkoxy" refers to an alkoxy group substituted by one or more halogens, wherein the alkoxy is as defined above.

The "hydroxyalkyl" refers to an alkyl group substituted by hydroxy(s), wherein the alkyl is as defined above.

The "alkenyl" refers to a chain alkenyl, also known as alkene group, wherein the alkenyl can be further substituted by other related group(s), for example alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy or alkoxycarbonyl.

The "alkynyl" refers to (CH≡C-), wherein the alkynyl can be further substituted by other related group(s), for example alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy or alkoxycarbonyl.

The "hydroxy" refers to an -OH group.

The "halogen" refers to fluorine, chlorine, bromine or iodine.

The "amino" refers to $-NH_2$.

The "cyano" refers to -CN.

The "nitro" refers to $-NO_2$.

The "carboxy" refers to -C(O)OH.

The "THF" refers to tetrahydrofuran.

The "EtOAc" refers to ethyl acetate.

The "MeOH" refers to methanol.

The "DMF" refers to N,N-dimethylformamide.

The "DIPEA" refers to diisopropylethylamine.

The "TFA" refers to trifluoroacetic acid.

The "MeCN" refers to acetonitrile.

The "DMA" refers to N,N-dimethylacetamide.

The "$Et_2O$" refers to diethyl ether.

The "DCE" refers to 1,2 dichloroethane.

The "DIPEA" refers to N,N-diisopropylethylamine.

The "NBS" refers to N-bromosuccinimide.

The "NIS" refers to N-iodosuccinimide.

The "Cbz-Cl" refers to benzyl chloroformate.

The "$Pd_2(dba)_3$" refers to tris(dibenzylideneacetone)dipalladium.

The "Dppf' refers to 1,1'-bis(diphenylphosphino)ferrocene.

The "HATU" refers to 2-(7-azabenzotriazol-1-yl)-N,N,N',N'-tetramethyluronium hexafluorophosphate.

The "KHMDS" refers to potassium hexamethyldisilazide.

The "LiHMDS" refers to lithium bis(trimethylsilyl)amide.

The "MeLi" refers to methyl lithium.

The "n-BuLi" refers to n-butyl lithium.

The "$NaBH(OAc)_3$" refers to sodium triacetoxyborohydride.

Different expressions such as "X is selected from the group consisting of A, B, or C", "X is selected from the group consisting of A, B and C", "X is A, B or C", "X is A, B and C" and the like, express the same meaning, that is, X can be any one or more of A, B and C.

The hydrogen described in the present invention can all be substituted by its isotope deuterium, and any hydrogen in a compound involved in the examples of the present invention can also be substituted by deuterium.

"Optional" or "optionally" means that the event or circumstance described subsequently can, but need not, occur, and such a description includes the situation in which the event or circumstance does or does not occur. For example, "the heterocyclyl optionally substituted by an alkyl" means that an alkyl group can be, but need not be, present, and such a description includes the situation of the heterocyclyl being substituted by an alkyl and the heterocyclyl being not substituted by an alkyl.

"Substituted" refers to one or more hydrogen atoms in a group, preferably up to 5, and more preferably 1 to 3 hydrogen atoms, independently substituted by a corresponding number of substituents. It goes without saying that the substituents only exist in their possible chemical position. The person skilled in the art is able to determine whether the substitution is possible or impossible by experiments or theory without paying excessive efforts. For example, the combination of amino or hydroxy having free hydrogen and carbon atoms having unsaturated bonds (such as olefinic) may be unstable.

A "pharmaceutical composition" refers to a mixture of one or more of the compounds according to the present invention or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to show biological activity.

A "pharmaceutically acceptable salt" refers to a salt of the compound of the present invention, which is safe and effective in mammals and has the desired biological activity.

**DETAILED DESCRIPTION OF THE INVENTION**

The present disclosure will be further described with reference to the following examples, but these examples should not be considered as limiting the scope of the present disclosure.

EXAMPLES

The structures of the compounds of the present invention were identified by nuclear magnetic resonance (NMR) and/or liquid chromatography-mass spectrometry (LC-MS). NMR shifts ($\delta$) are given in $10^{-6}$ (ppm). NMR was determined by a Bruker AVANCE-400 spectrometer. The solvents for determination were deuterated dimethyl sulfoxide (DMSO-$d_6$), deuterated methanol ($CD_3OD$) and deuterated chloroform ($CDCl_3$), and the internal standard was tetramethylsilane (TMS).

Liquid chromatography-mass spectrometry (LC-MS) was determined on an Agilent 1200 Infinity Series mass spectrometer. High performance liquid chromatography (HPLC) was determined on an Agilent 1200DAD high pressure liquid chromatograph (Sunfire C18 150×4.6 mm chromatographic column) and Waters 2695-2996 high pressure liquid chromatograph (Gimini C18 150×4.6 mm chromatographic column).

Yantai Huanghai HSGF254 or Qingdao GF254 silica gel plate was used as the thin-layer silica gel chromatography (TLC) plate. The dimension of the silica gel plate used in TLC was 0.15 mm to 0.2 mm, and the dimension of the silica gel plate used in product purification was 0.4 mm to 0.5 mm. Yantai Huanghai 200 to 300 mesh silica gel was generally used as a carrier for column chromatography.

The starting materials in the examples of the present invention are known and commercially available, or can be synthesized by or according to methods known in the art.

Unless otherwise specified, all the reactions of the present invention were carried out under continuous magnetic stirring under dry nitrogen or argon atmosphere, the solvent was a dry solvent, and the reaction temperature is given in degrees Celsius.

**Example 1**

**(R)-(3-(Benzo [d] thiazol-2-yl)-8-methyl-5,6-dihydro- [1,2,4] triazolo [4,3-a] pyrazi n-7(8H)-yl)(4-fluorophenyl)methanone**

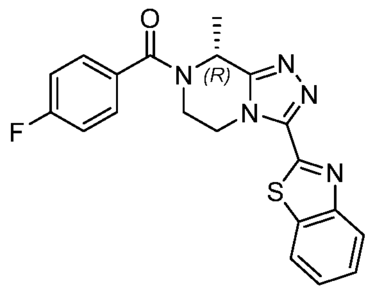

**Step 1: Preparation of (R)-1-(2,4-dimethoxybenzyl)-5-ethoxy-6-methyl-1,2,3,6-tetrahydropyrazine**

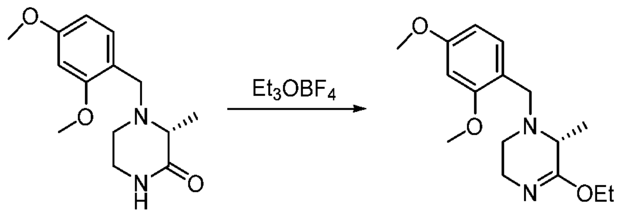


(R)-4-(2,4-Dimethoxybenzyl)-3-methylpiperazin-2-one (0.20 g, 0.38 mmol) was dissolved in dichloroethane (1 mL) and cooled to 0°C, and triethyloxidanium tetrafluoroborate (4.5 mL, 4.5 mmol) was added. The reaction solution was stirred at room temperature for 3 hours. Water (10 mL) was added, and the reaction solution was stirred for half an hour until the precipitation of solids, and then filtered. The aqueous phase was extracted with dichloromethane (20 mL×2). The organic phases were combined, dried over anhydrous sodium sulfate, filtered and concentrated to obtain (R)-1-(2,4-dimethoxybenzyl)-5-ethoxy-6-methyl-1,2,3,6-tetrahydropyrazine (0.2 g), which was used directly in the next step.

MS m/z (ESI): 293.2 $[M+H]^+$.

**Step 2: Preparation of benzo[d]thiazole-2-carbohydrazide**

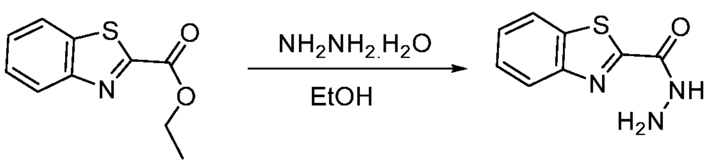


Ethyl benzo[d]thiazole-2-carboxylate (300 mg, 1.45 mmol) was dissolved in anhydrous alcohol (10 mL), and 85% hydrazine hydrate (102 mg, 1.74 mmol) was added. The reaction solution was stirred at room temperature for 4 hours, filtered and dried to obtain benzo[d]thiazole-2-carbohydrazide (270 mg, yield: 96%).

MS m/z (ESI): 194.2 $[M+H]^+$.

**Step 3: Preparation of (R)-2-(7-(2,4-dimethoxybenzyl)-8-methyl-5,6,7,8-tetrahydro-[1,2,4]triazolo[4,3-a]p yrazin-3-yl)benzo[d]thiazole**

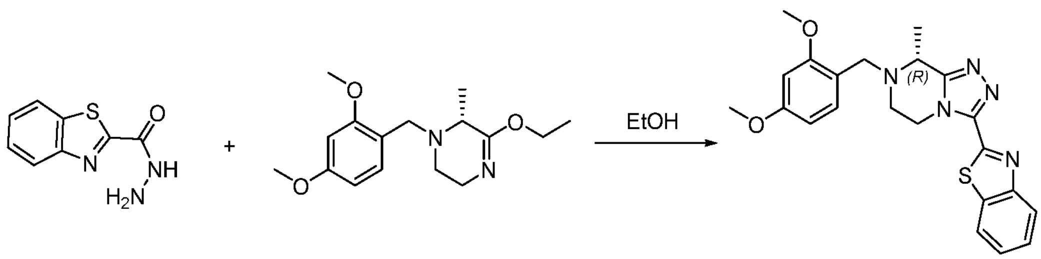


(R)-1-(2,4-Dimethoxybenzyl)-5-ethoxy-6-methyl-1,2,3,6-tetrahydropyrazine (200 mg, 0.68 mmol) was dissolved in ethanol (10 mL), and benzo[d]thiazole-2-carbohydrazide (120 mg, 0.62 mmol) was added. The reaction solution was stirred at 80°C overnight, and subjected to rotary evaporation until dry. The crude product was separated by column chromatography (petroleum ether/ethyl acetate: 5/1~1/2) to obtain (R)-2-(7-(2,4-dimethoxybenzyl)-8-methyl-5,6,7,8-tetrahydro-[1,2,4]triazolo[4,3-a]pyraz in-3-yl)benzo[d]thiazole (100 mg, yield: 38%).

MS m/z (ESI): 422.2 $[M+H]^+$.

**Step 4: Preparation of (R)-2-(8-methyl-5,6,7,8-tetrahydro-[1,2,4]triazolo[4,3-a]pyrazin-3-yl)benzo[d]thiaz ole**

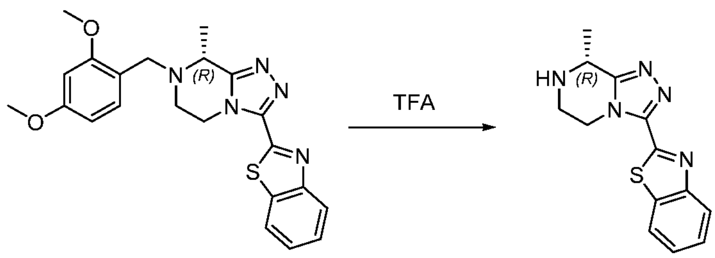


(R)-2-(7-(2,4-Dimethoxybenzyl)-8-methyl-5,6,7,8-tetrahydro-[1,2,4]triazolo[4,3-a] pyrazin-3-yl)benzo[d]thiazole (100 mg, 0.24 mmol) was dissolved in dichloromethane (2 mL), and trifluoroacetic acid (1 mL) was added. The reaction solution was stirred at room temperature for 1 hour. TLC showed that the reaction was complete. Water (10 mL) was added, and the reaction solution was stirred for 5 minutes and filtered. A 3 M solution of sodium hydroxide was added to the filtrate until the pH of the aqueous phase was >14. The filtrate was extracted with dichloromethane (10 mL×3). The organic phases were combined, washed with saturated brine (10 mL), dried over anhydrous sodium sulfate and concentrated under reduced pressure to obtain (R)-2-(8-methyl-5,6,7,8-tetrahydro-[1,2,4]triazolo[4,3-a]pyrazin-3-yl)benzo[d]thiazole (40 mg, yield: 62%).

MS m/z (ESI): 272.2 $[M+H]^+$.

**Step 5: Preparation of (R)-(3-(benzo[d]thiazol-2-yl)-8-methyl-5,6-dihydro-[1,2,4]triazolo[4,3-a]pyrazin-7( 8H)-yl)(4-fluorophenyl)methanone**

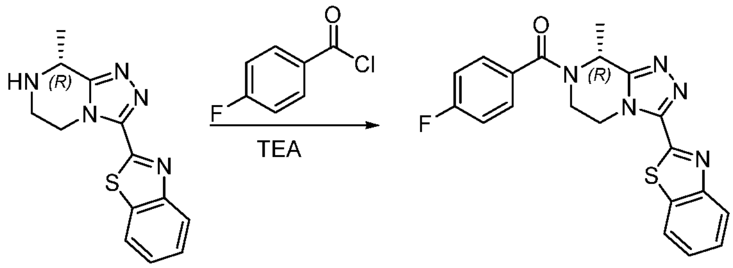


(R)-2-(8-Methyl-5,6,7,8-tetrahydro-[1,2,4]triazolo[4,3-a]pyrazin-3-yl)benzo[d]thia zole (40 mg, 0.15 mmol) was dissolved in dichloromethane (2 mL). Triethylamine (23 mg, 0.23 mmol) was added, and then 4-fluorobenzoyl chloride (28 mg, 0.18 mmol) was added. The reaction solution was stirred at room temperature for 1 hour, washed with water (10 mL) and extracted with dichloromethane (10 mL× 2). The organic phases were combined, washed with saturated brine (10 mL), dried over anhydrous sodium sulfate and concentrated under reduced pressure to obtain a crude product, which was purified by preparative high performance liquid chromatography to obtain (R)-(3-(benzo[d]thiazol-2-yl)-8-methyl-5,6-dihydro-[1,2,4]triazolo[4,3-a]pyrazin-7(8H) -yl)(4-fluorophenyl)methanone (25 mg, yield: 42%).

MS m/z (ESI): 394.2 $[M+H]^+$.

$^1H$ NMR (400 MHz, Chloroform-d) δ 8.06 (d, J = 8.1 Hz, 1H), 7.98 (d, J = 7.9 Hz, 1H), 7.57 -7.45 (m, 4H), 7.27 -7.16 (m, 2H), 5.78 (br s, 1H), 5.12 (d, J = 14.6 Hz, 1H), 4.84 -4.55 (m, 1H), 4.42 -4.35 (m, 1H), 3.60 -3.54 (m, 1H), 1.77 (d, J = 6.9 Hz, 3H).

**Example 2**

**(R)-(3-(Benzo[d]thiazol-2-yl)-8-methyl-5,6-dihydro-[1,2,4]triazolo[4,3-a]pyrazi n-7(8H)-yl)(4-fluorophenyl-2,3,5,6-d4)methanone**

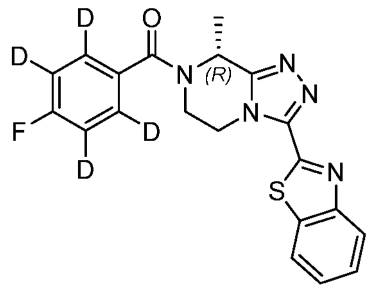

**Step 1: Preparation of 4-fluoro-2,3,5,6-d4 benzoic acid**

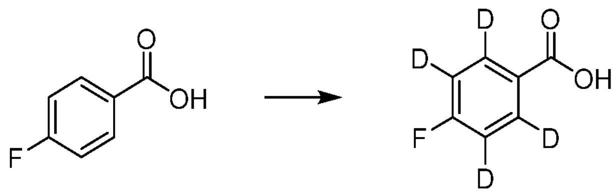

10% Pd/C (0.73 g, water content 50% w/w) was added to a solution of p-fluorobenzoic acid (1.8 g, 12.9 mmol) in isopropanol (50 mL) and deuterium water (100 mL) under a nitrogen atmosphere. The reaction solution was stirred at 100°C for 3 days, cooled and extracted with ethyl acetate (30 mL×3). The organic phase was dried over anhydrous sodium sulfate and concentrated under reduced pressure to obtain the target product 4-fluoro-2,3,5,6-d4 benzoic acid (1.5 g, yield: 81%).

MS m/z (ESI): 143.0$[M-H]^-$

**Step 2: Preparation of (R)-(3-(benzo[d]thiazol-2-yl)-8-methyl-5,6-dihydro-[1,2,4]triazolo[4,3-a]pyrazin-7( 8H)-yl)(4-fluorophenyl-2,3,5,6-d4)methanone**

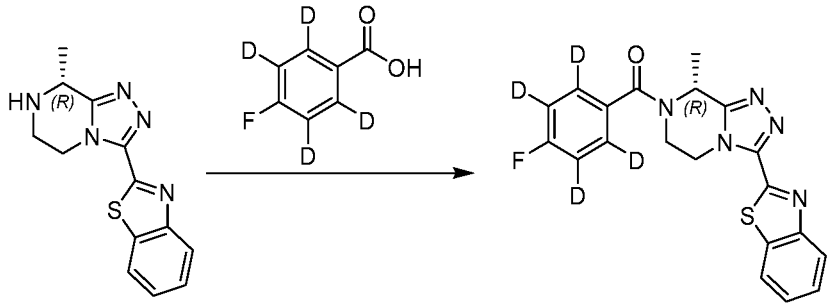

2-(7-Azabenzotriazol-1-yl)-N,N,N',N'-tetramethyluronium hexafluorophosphate (32 mg, 84 μmol) was added to a solution of (R)-2-(8-methyl-5,6,7,8-tetrahydro-[1,2,4]triazolo[4,3-a]pyrazin-3-yl)benzo[d]thiazole (15 mg, 55 μmol), 4-fluoro-2,3,5,6-d4 benzoic acid (10 mg, 68 μmol) and N,N-diisopropylethylamine (22 mg, 169 μmol) in N,N-dimethylformamide (1.5 ml). The reaction solution was stirred at room temperature for 16 hours. The crude product was subjected directly to Prep-HPLC separation to obtain the product (R)-(3-(benzo[d]thiazol-2-yl)-8-methyl-5,6-dihydro-[1,2,4]triazolo[4,3-a]pyrazin-7(8H) -yl)(4-fluorophenyl-2,3,5,6-d4)methanone (11 mg, yield: 50%).

MS m/z (ESI): 398.1[M+H]$^+$.

$^1$H NMR (400 MHz, Chloroform-d) δ 8.13-7.92 (m, 2H), 7.62-7.43 (m, 2H), 5.91-5.70 (m, 1H), 5.24-5.01 (m, 1H), 4.81-4.52 (m, 1H), 4.50-4.26 (m, 1H), 3.72-3.41 (m, 1H), 1.93-1.82 (m, 3H).

**Example 3**

**(R)-(3-(Benzo[d]thiazol-2-yl)-8-methyl-5,6-dihydro-[1,2,4]triazolo[4,3-a]pyrazi n-7(8H)-yl)(4-chlorophenyl)methanone**

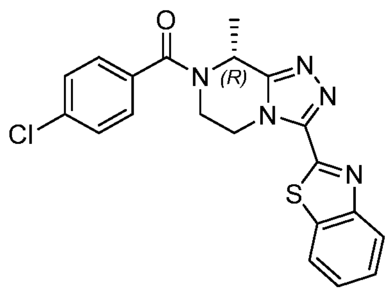

The preparation method of Example **3** referred to Step 5 of Example **1.**

MS m/z (ESI): 410.2 [M+H]$^+$.

$^1$H NMR (400 MHz, Chloroform-d) δ 8.06 (d, J = 8.1 Hz, 1H), 7.98 (d, J = 8.0 Hz, 1H), 7.58-7.40 (m, 6H), 5.93-5.73 (m, 1H), 5.13 (d, J = 13.6 Hz, 1H), 4.74-4.49 (m, 1H), 4.43-4.34 (m, 1H), 3.63-3.52 (m, 1H), 1.78 (d, J = 6.8 Hz, 3H).

**Example 4**

**(R)-(3-(Benzo[d]thiazol-2-yl)-8-methyl-5,6-dihydro-[1,2,4]triazolo[4,3-a]pyrazi n-7(8H)-yl)(3,4-dichlorophenyl)methanone**

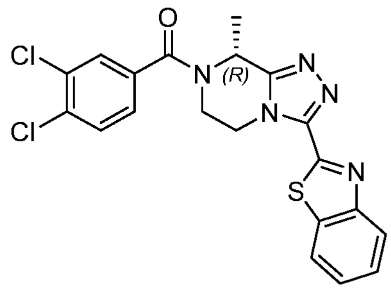

The preparation method of Example **4** referred to Step 5 of Example **1.**

MS m/z (ESI): 444.2 [M+H]$^+$.

$^1$H NMR (400 MHz, Chloroform-d) δ 8.07 (d, J = 8.0 Hz, 1H), 7.98 (d, J = 7.9 Hz, 1H), 7.62-7.47 (m, 4H), 7.33 (d, J = 8.2 Hz, 1H), 5.83 (br s, 1H), 5.16 (d, J = 13.6 Hz, 1H), 4.81-4.49 (m, 1H), 4.45-4.35 (m, 1H), 3.66-3.57 (m, 1H), 1.80 (d, J = 6.8 Hz, 3H).

**Example 5**

**(R)-(3-(Benzo[d]thiazol-2-yl)-8-methyl-5,6-dihydro-[1,2,4]triazolo [4,3-a]pyrazi n-7(8H)-yl)(4-(thien-2-yl)phenyl)methanone**

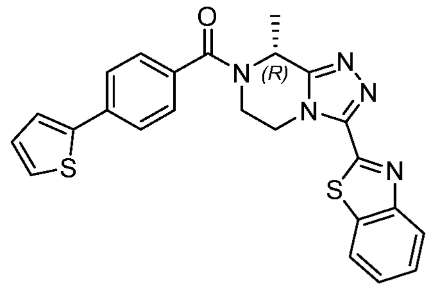

The preparation method of Example **5** referred to Step 5 of Example **1.**

MS m/z (ESI): 458.2 [M+H]$^+$.

$^1$H NMR (400 MHz, Chloroform-d) δ 8.08 (d, J = 8.0 Hz, 1H), 7.99 (d, J = 7.8 Hz, 1H), 7.72 (d, J = 7.6 Hz, 2H), 7.61-7.46 (m, 4H), 7.40 (s, 1H), 7.36 (d, J = 5.1 Hz, 1H), 7.13 (s, 1H), 6.02-5.84 (m, 1H), 5.17 (d, J = 13.4 Hz, 1H), 4.78-4.62 (m, 1H), 4.51-4.40 (m, 1H), 3.69-3.56 (m, 1H), 1.86 (d, J = 6.5 Hz, 3H).

**Example 6**

**(R)-(3-(Benzo[d]thiazol-2-yl)-8-methyl-5,6-dihydro-[1,2,4]triazolo[4,3-a]pyrazi n-7(8H)-yl)(phenyl-d5)methanone**

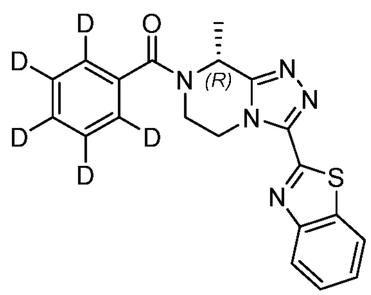

The preparation method of Example **6** referred to Step 5 of Example **1.**
MS m/z (ESI): 381.2 $[M+H]^+$.
$^1H$ NMR (400 MHz, $CDCl_3$) δ 8.04-7.94 (m, 2H), 7.55-7.47 (m, 2H), 5.84-5.79 (m, 1H), 5.13-5.10 (m, 1H), 4.46-4.39 (m, 2H), 3.59-3.55 (m, 1H), 1.80-1.78 (m, 3H).

**Example 7**

**(R)-(3-(4-Fluorobenzo[d]thiazol-2-yl)-8-methyl-5,6-dihydro-[1,2,4]triazolo [4,3-a] pyrazin-7 (8H)-yl)(4-fluorophenyl)methanone**

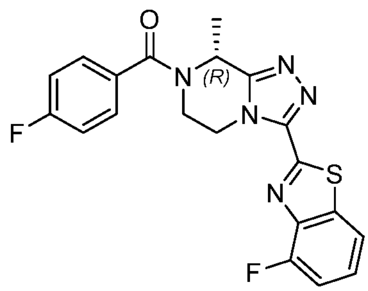

The preparation method of Example **7** referred to Example **1.**
MS m/z (ESI): 412.2 $[M+H]^+$.
$^1H$ NMR (400 MHz, $CDCl_3$) δ 7.75-7.73 (m, 1H), 7.52-7.43 (m, 3H), 7.22-7.16 (m, 3H), 5.86-5.80 (m, 1H), 5.15-5.12 (m, 1H), 4.65-4.39 (m, 2H), 3.60-3.55 (m, 1H), 1.78-1.75 (m, 3H).

**Example 8**

**(R)-(4-Chlorophenyl)(3-(4-fluorobenzo[d]thiazol-2-yl)-8-methyl-5,6-dihydro-[ 1,2,4]triazolo[4,3-a]pyrazin-7(8H)-yl)methanone**

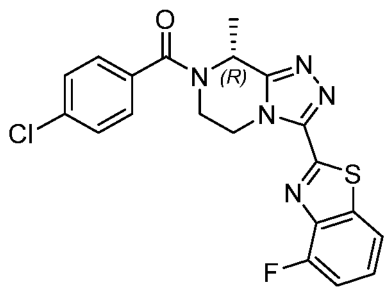

The preparation method of Example **8** referred to Example **1.**
MS m/z (ESI): 428.2 $[M+H]^+$.
$^1H$ NMR (400 MHz, $CDCl_3$) δ 7.77-7.74 (m, 1H), 7.50-7.40 (m, 4H), 7.25-7.18 (m, 2H), 5.86-5.80 (m, 1H), 5.14-5.11 (m, 1H), 4.61-4.38 (m, 2H), 3.60-3.56 (m, 1H), 1.77 (d, J = 8.0 Hz, 3H).

**Example 9**

**(R)-(3-(5-Fluorobenzo[d]thiazol-2-yl)-8-methyl-5,6-dihydro-[1,2,4]triazolo [4,3-a]pyrazin-7(8H)-yl)(4-fluorophenyl)methanone**

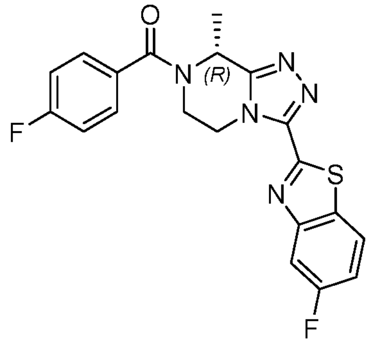

The preparation method of Example **9** referred to Example **1.**

MS m/z (ESI): 412.2 $[M+H]^+$.

$^1$H NMR (400 MHz, Chloroform-d) δ 7.96-7.84 (m, 1H), 7.73 (d, J = 9.2 Hz, 1H), 7.56-7.43 (m, 2H), 7.33-7.29 (m, 1H), 7.20-7.16 (m, 2H), 5.93-5.69 (m, 1H), 5.08 (d, J = 13.7 Hz, 1H), 4.76-4.48 (m, 1H), 4.41-4.30 (m, 1H), 3.66-3.49 (m, 1H), 1.78 (d, J = 6.8 Hz, 3H).

**Example 10**

(**R)-(4-Chlorophenyl)(3-(5-fluorobenzo[d]thiazol-2-yl)-8-methyl-5,6-dihydro-[ 1,2,4]triazolo[4,3-a]pyrazin-7(8H)-yl)methanone**

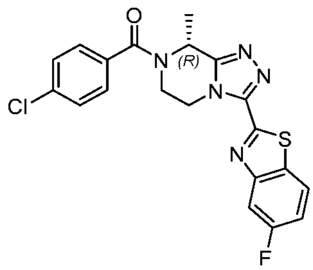

The preparation method of Example **10** referred to Example **1.**

MS m/z (ESI): 428.2 $[M+H]^+$.

$^1$H NMR (400 MHz, Chloroform-d) δ 7.95-7.87 (m, 1H), 7.73 (d, J = 9.0 Hz, 1H), 7.48-7.42 (m, 4H), 7.31-7.29 (m, 1H), 5.95-5.65 (m, 1H), 5.09 (d, J = 14.3 Hz, 1H), 4.73-4.48 (m, 1H), 4.40-4.33 (m, 1H), 3.64-3.48 (m, 1H), 1.78 (d, J = 6.4 Hz, 3H).

**Example 11**

**(R)-(3,4-Dichlorophenyl)(3-(5-fluorobenzo[d]thiazol-2-yl)-8-methyl-5,6-dihydr o-[1,2,4]triazolo[4,3-a]pyrazin-7(8H)-yl)methanone**

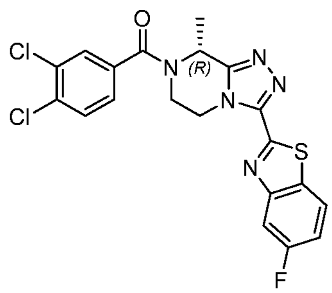

The preparation method of Example **11** referred to Example **1.**

MS m/z (ESI): 462.0 $[M+H]^+$.

$^1H$ NMR (400 MHz, Chloroform-d) δ 7.95-7.88 (m, 1H), 7.74 (d, J = 9.2 Hz, 1H), 7.58 (d, J = 9.6 Hz, 2H), 7.36-7.27 (m, 2H), 5.94-5.77 (m, 1H), 5.12 (d, J = 13.7 Hz, 1H), 4.65-4.49 (m, 1H), 4.42-4.33 (m, 1H), 3.67-3.54 (m, 1H), 1.79 (d, J = 6.7 Hz, 3H).

**Example 12**

**(R)-(3-(6-Fluorobenzo[d]thiazol-2-yl)-8-methyl-5,6-dihydro-[1,2,4]triazolo[4,3-a]pyrazin-7(8H)-yl)(4-fluorophenyl)methanone**

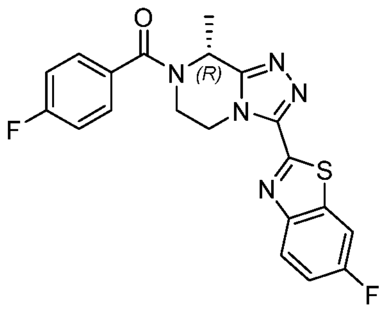

**Step 1: Preparation of ethyl 2-((4-fluorophenyl)amino)-2-oxoacetate**

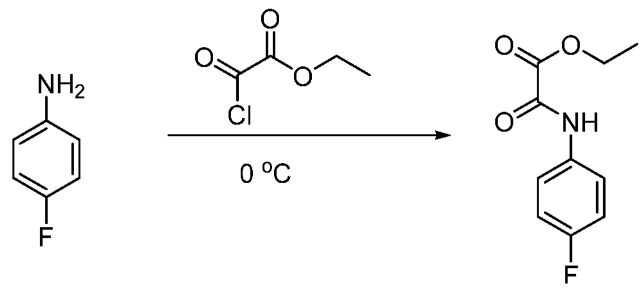


4-Fluoroaniline (5.0 g, 45 mmol) was dissolved in dichloromethane (50 mL) and cooled to 0°C. Triethylamine (9.1 g, 90 mmol) was added, and then ethyl 2-chloro-2-oxoacetate (7.4 g, 55 mmol) was added dropwise. The reaction solution was stirred at room temperature for one hour. Water (200 mL) was added, and the aqueous phase was extract with dichloromethane (100 mL×2). The organic phases were combined, dried over anhydrous sodium sulfate, filtered and concentrated to obtain ethyl 2-((4-fluorophenyl)amino)-2-oxoacetate (9 g, 95% crude), which was used directly in the next step.

MS m/z (ESI): 211.2 $[M+H]^+$.

**Step 2: Preparation of ethyl 2-((4-fluorophenyl)amino)-2-thioxoacetate**

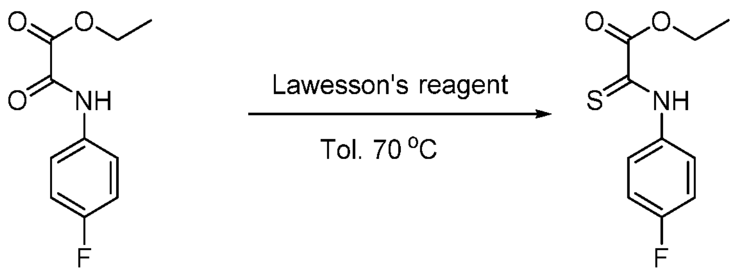


Ethyl 2-((4-fluorophenyl)amino)-2-oxoacetate (5.0 g, 24 mmol) was dissolved in toluene (50 mL), and Lawesson's reagent (5.74 g, 14 mmol) was added. The reaction solution was stirred at 70°C for 2 hours. The solvent was dried by rotary evaporation, and the crude product was separated by column chromatography (PE/EA = 10/1 ~ 1/1) to obtain ethyl 2-((4-fluorophenyl)amino)-2-thioxoacetate (4.0 g, yield: 100%) as a brown solid.
MS m/z (ESI): 228.2 $[M+H]^+$.

**Step 3: Preparation of 6-fluorobenzo[d]thiazole-2-carboxylic acid**

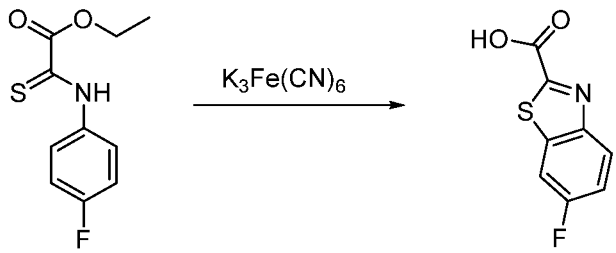


Ethyl 2-((4-fluorophenyl)amino)-2-thioxoacetate (1.0 g, 4.4 mmol) was dissolved in 2 M aqueous sodium hydroxide solution (10 mL), stirred for half an hour and cooled to 0°C. A solution of potassium ferricyanate (4.3 g, 13.2 mol) in water (10 mL) was added, and the reaction solution was stirred at room temperature for 2 hours. 2 M HCl was added to adjust the pH to 1-2, and solids were precipitated. The reaction solution was filtered, and the solids were dried to obtain a mixture of 6-fluorobenzo[d]thiazole-2-carboxylic acid and decarboxylation product (0.8 g, yield: 95%) as a white solid.
MS m/z (ESI): 196.2 $[M-H]^-$.

**Step 4: Preparation of methyl 6-fluorobenzo[d]thiazole-2-carboxylate**

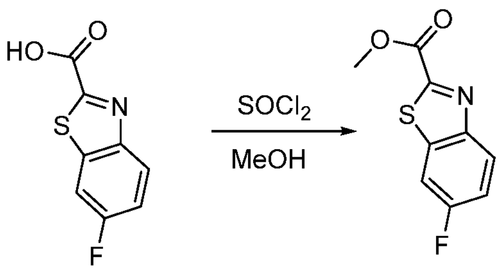


The mixture of 6-fluorobenzo[d]thiazole-2-carboxylic acid and decarboxylation product (0.8 g, 4.1 mmol) was dissolved in methanol (30 mL) and cooled to 0°C. Thionyl chloride (2.4 g, 20.3 mmol) was added, and the reaction solution was stirred at 60°C for 2 hours. The solvent was dried by rotatory evaporation, and a saturated sodium bicarbonate solution was added to adjust the pH to ~8. The aqueous phase was extracted with ethyl acetate (20 mL×2). The organic phase was dried over anhydrous sodium sulfate, filtered and concentrated. The crude product was separated by column chromatography (PE/EA = 100/1 ~ 3/1) to obtain methyl 6-fluorobenzo[d]thiazole-2-carboxylate (0.24 g, yield: 28%) as a brown solid.

MS m/z (ESI): 212.2 $[M+H]^+$.

**Step 5: Preparation of 6-fluorobenzo[d]thiazole-2-carbohydrazide**

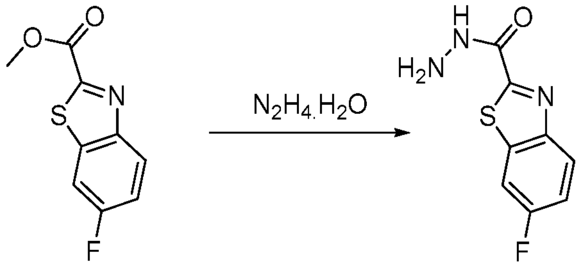


Methyl 6-fluorobenzo[d]thiazole-2-carboxylate (0.24 g, 1.1 mmol) was dissolved in anhydrous methanol (5 mL), and 85% hydrazine hydrate (0.13 g, 2.3 mmol) was added. The reaction solution was stirred at 60°C for 1 hour until the precipitation of solids, and then filtered. The solids were dried to obtain 6-fluorobenzo[d]thiazole-2-carbohydrazide (0.17 g, yield: 71%) as a white solid.

MS m/z (ESI): 212.2 $[M+H]^+$.

**Step 6: Preparation of (R)-1-(2,4-dimethoxybenzyl)-5-ethoxy-6-methyl-1,2,3,6-tetrahydropyrazine**

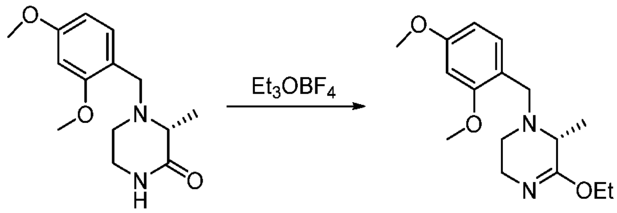


(R)-4-(2,4-Dimethoxybenzyl)-3-methylpiperazin-2-one (0.3 g, 1.1 mmol) was dissolved in dichloroethane (8 mL) and cooled to 0°C. Triethyloxidanium tetrafluoroborate (0.86 g, 4.6 mmol) was added, and the reaction solution was stirred at room temperature for 2 hours. TLC (DCM/MeOH = 10/1) showed that the reaction was complete. The reaction was quenched with a 2 M cold aqueous sodium hydroxide solution (30 mL). The aqueous phase was extracted with dichloromethane (30 mL×2). The organic phases were combined, dried over anhydrous sodium sulfate, filtered and concentrated to obtain (R)-1-(2,4-dimethoxybenzyl)-5-ethoxy-6-methyl-1,2,3,6-tetrahydropyrazine (0.3 g, 100% crude), which was used directly in the next step.

**Step 7: Preparation of (R)-2-(7-(2,4-dimethoxybenzyl)-8-methyl-5,6,7,8-tetrahydro-[1,2,4]triazolo[4,3-a]p yrazin-3-yl)-6-fluorobenzo[d]thiazole**

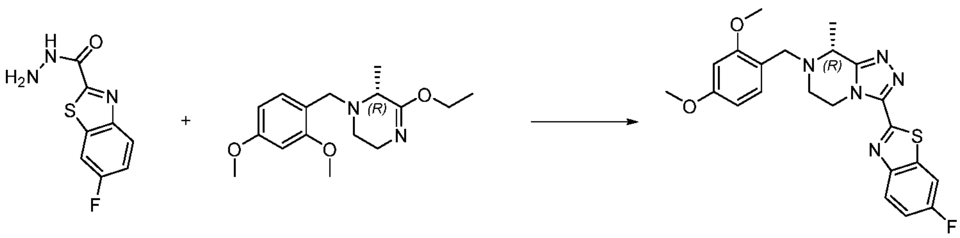

(R)-1-(2,4-Dimethoxybenzyl)-5-ethoxy-6-(methoxymethyl)-1,2,3,6-tetrahydropyra zine (300 mg, 1.0 mmol) was dissolved in ethanol (20 mL), and 6-fluorobenzo[d]thiazole-2-carbohydrazide (173 mg, 0.83 mmol) was added. The

reaction solution was stirred at 80°C overnight and subjected to rotatory evaporation until dry. The crude product was separated by column chromatography (PE/EA = 10/1 ~1/3) to obtain (R)-2-(7-(2,4-dimethoxybenzyl)-8-methyl-5,6,7,8-tetrahydro-[1,2,4]triazolo[4,3-a]pyraz in-3-yl)-6-fluorobenzo[d]thiazole (150 mg, yield: 42%) as a colorless colloid.

MS m/z (ESI): 440.2 [M+H]+.

**Step 8: Preparation of (R)-6-fluoro-2-(8-methyl-5,6,7,8-tetrahydro- [1,2,4] triazolo [4,3-a] pyrazin-3-yl)benz o[d]thiazole**

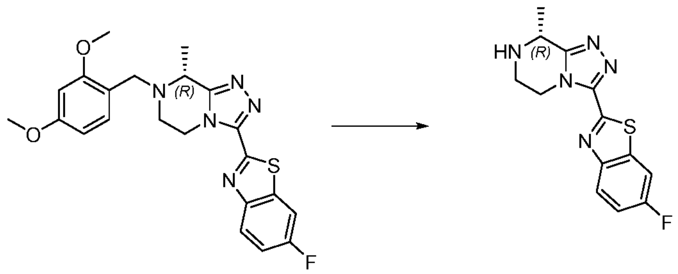

(R)-2-(7-(2,4-Dimethoxybenzyl)-8-methyl-5,6,7,8-tetrahydro-[1,2,4]triazolo[4,3-a] pyrazin-3-yl)-6-fluorobenzo[d]thiazole (150 mg, 0.3 mmol) was dissolved in dichloromethane (10 mL), and trifluoroacetic acid (3 mL) was added. The reaction solution was stirred at room temperature for 1 hour. Water (10 mL) was added, and the reaction solution was stirred for half an hour until the precipitation of solids, and then filtered. A 4 M sodium hydroxide solution was added to the filtrate to adjust the pH to >14, and the filtrate was extracted with dichloromethane (20 mL×2). The organic phase was dried over anhydrous sodium sulfate, filtered and concentrated to obtain (R)-6-fluoro-2-(8-methyl-5,6,7,8-tetrahydro-[1,2,4]triazolo[4,3-a]pyrazin-3-yl)benzo[d]t hiazole (60 mg, yield: 61%).

MS m/z (ESI): 290.2 [M+H]+.

**Step 9: Preparation of (R)-(3-(6-fluorobenzo[d]thiazol-2-yl)-8-methyl-5,6-dihydro-[1,2,4]triazolo[4,3-a]py razin-7(8H)-yl)(4-fluorophenyl)methanone**

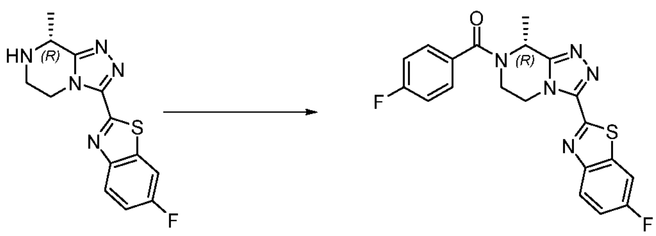

In accordance with Step 5 of Example 1, (R)-(3-(6-fluorobenzo[d]thiazol-2-yl)-8-methyl-5,6-dihydro-[1,2,4]triazolo[4,3-a]pyrazi n-7(8H)-yl)(4-fluorophenyl)methanone (19.5 mg, yield: 69%) was obtained as a white solid.

MS m/z (ESI): 412.2 [M+H]+.

$^1$H NMR (400 MHz, $CDCl_3$) δ 8.02-7.98 (m, 1H), 7.66-7.64 (m, 1H), 7.52-7.49 (m, 2H), 7.30-7.26 (m, 1H), 7.20-7.16 (m, 2H), 5.83-5.76 (m, 1H), 5.12-5.04 (m, 1H), 4.61-4.38 (m, 2H), 3.61-3.56 (m, 1H), 1.80-1.76 (m, 3H).

**Example 13**

**(R)-(4-Chlorophenyl)(3-(6-fluorobenzo[d]thiazol-2-yl)-8-methyl-5,6-dihydro-[ 1,2,4]triazolo[4,3-a]pyrazin-7(8H)-yl)methanone**

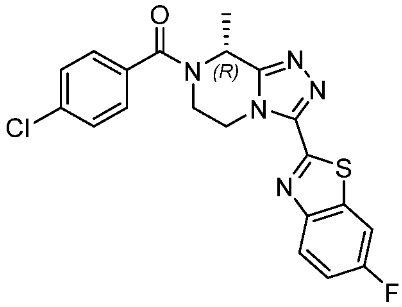

The preparation of Example **13** referred to Step 5 of Example **1.**

MS m/z (ESI): 428.2 $[M+H]^+$.

$^1H$ NMR (400 MHz, $CDCl_3$) $\delta$ 8.01-7.98 (m, 1H), 7.64 (d, J = 8.0 Hz, 1H), 7.48-7.42 (m, 4H), 7.29-7.27 (m, 1H), 5.85-5.76 (m, 1H), 5.08-5.05 (m, 1H), 4.58-4.32 (m, 2H), 3.59-3.55 (m, 1H), 1.76 (d, J = 8.0 Hz, 3H).

**Example 14**

**(R)-(3,4-Dichlorophenyl)(3-(6-fluorobenzo[d]thiazol-2-yl)-8-methyl-5,6-dihydr o-[1,2,4]triazolo[4,3-a]pyrazin-7(8H)-yl)methanone**

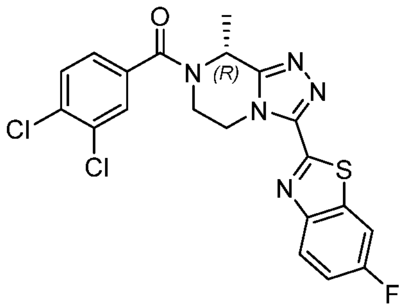

The preparation of Example **14** referred to Step 5 of Example **1.**

MS m/z (ESI): 462.2 $[M+H]^+$.

$^1H$ NMR (400 MHz, $CDCl_3$) $\delta$ 7.99-7.97 (m, 1H), 7.64-7.60 (m, 3H), 7.33-7.27 (m, 2H), 5.82-5.75 (m, 1H), 5.12-5.05 (m, 1H), 4.63-4.36 (m, 2H), 3.62-3.57 (m, 1H), 1.78-1.76 (m, 3H).

**Example 15**

**(R)-(3-(4,6-Difluorobenzo[d]thiazol-2-yl)-8-methyl-5,6-dihydro-[1,2,4]triazolo[ 4,3-a]pyrazin-7(8H)-yl)(4-fluorophenyl)methanone**

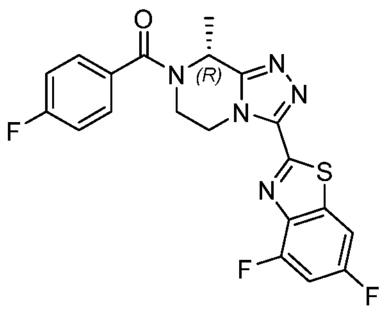

The preparation of Example **15** referred to Example **12.**

MS m/z (ESI): 430.2 [M+H]+.

$^1$H NMR (400 MHz, $CDCl_3$) δ 7.52-7.46 (m, 3H), 7.20-7.16 (m, 2H), 7.08-7.04 (m, 1H), 5.85-5.80 (m, 1H), 5.09-5.05 (m, 1H), 4.61-4.37 (m, 2H), 3.62-3.55 (m, 1H), 1.78-1.76 (m, 3H).

**Example 16**

**(R)-(4-Chlorophenyl)(3-(4,6-difluorobenzo[d]thiazol-2-yl)-8-methyl-5,6-dihydr o-[1,2,4]triazolo[4,3-a]pyrazin-7(8H)-yl)methanone**

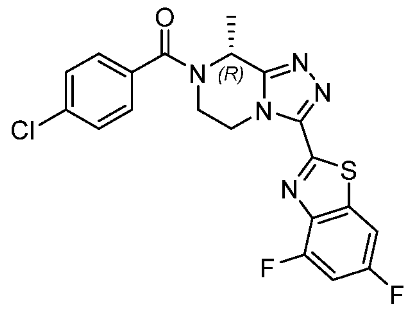

The preparation of Example **16** referred to Step 5 of Example **1.**

MS m/z (ESI): 446.2 [M+H]+.

$^1$H NMR (400 MHz, $CDCl_3$) δ 7.48-7.41 (m, 5H), 7.08-7.04 (m, 1H), 5.84-5.80 (m, 1H), 5.10-5.07 (m, 1H), 4.66-4.36 (m, 2H), 3.61-3.54 (m, 1H), 1.77 (d, J = 4.0 Hz, 3H).

**Example 17**

(**R)-(3-(5,6-Difluorobenzo[d]thiazol-2-yl)-8-methyl-5,6-dihydro-[1,2,4]triazolo[ 4,3-a]pyrazin-7(8H)-yl)(4-fluorophenyl)methanone**

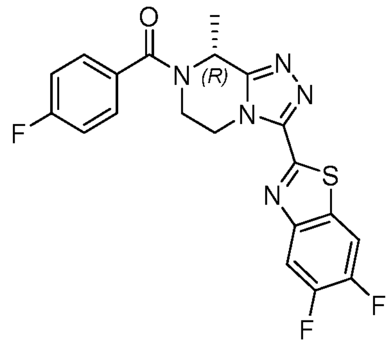

**Step 1: Ethyl 2-oxo-2-((2,4,5-trifluorophenyl)amino)acetate**

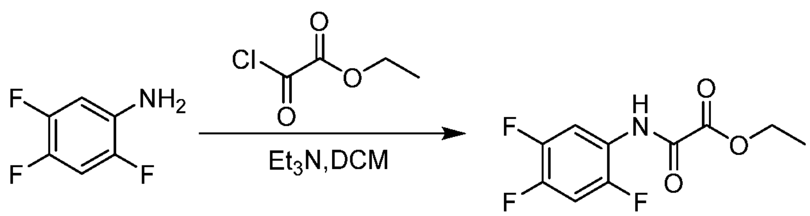

2,4,5-Trifluoroaniline (6.6 g, 44.87 mmol) and triethylamine (13.62 g, 134.60 mmol, 18.77 mL) were dissolved in dichloromethane (100 mL), and ethyl 2-chloro-2-oxoacetate (7.35 g, 53.84 mmol) was added under a nitrogen atmosphere. The mixture was stirred at 0°C for 20 minutes. The reaction was quenched with saturated brine (100 mL). The mixture was separated into two phases. The organic phase was washed with saturated brine (100 mL×3), dried over anhydrous sodium sulfate and concentrated under reduced pressure to obtain the target product ethyl 2-oxo-2-((2,4,5-trifluorophenyl)amino)acetate (10.4 g, yield: 93.78%) as a light yellow solid, which was used directly in the next step.
MS m/z (ESI): 248.0[M+H]$^+$.

**Step 2: Ethyl 2-thioxo-2-((2,4,5-trifluorophenyl)amino)acetate**

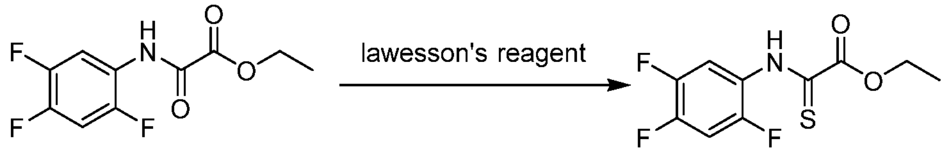


Ethyl 2-oxo-2-((2,4,5-trifluorophenyl)amino)acetate (10.4 g, 42.08 mmol) was dissolved in xylene (150 mL), and Lawesson's reagent (10.21 g, 25.25 mmol) was added under a nitrogen atmosphere. The mixture was stirred at 110°C for 1 hour. LCMS showed the formation of the reaction product, and the reaction solution was used directly in the next step.
MS m/z (ESI): 264.0[M+H]$^+$.

**Step 3: Ethyl 5,6-difluorobenzo[d]thiazole-2-carboxylate**

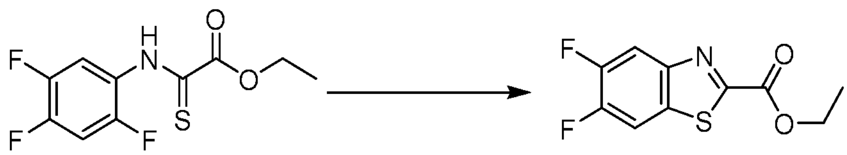

The reaction solution of the previous step was cooled, and cesium carbonate (24.75 g, 75.98 mmol) was added. The reaction solution was heated to 160°C for 3 hours under a nitrogen atmosphere. The reaction solution was cooled to room temperature, and the reaction was quenched with saturated brine (100 mL). Then ethyl acetate (100 ml) was added, and stirring was continued for 10 minutes. The mixture was filtered, and the liquid phase was extracted with ethyl acetate (75 mL×3). The organic phases were combined, washed with saturated brine (50 mL×2), dried over anhydrous sodium sulfate and concentrated under reduced pressure. The residue was purified by silica gel flash chromatography (elution: petroleum ether: ethyl acetate = 5:1) to obtain the target product ethyl 5,6-difluorobenzo[d]thiazole-2-carboxylate (3 g, yield: 32.47%).
MS m/z (ESI): 244.0[M+H]$^+$.

**Step 4: 5,6-Difluorobenzo[d]thiazole-2-carbohydrazide**

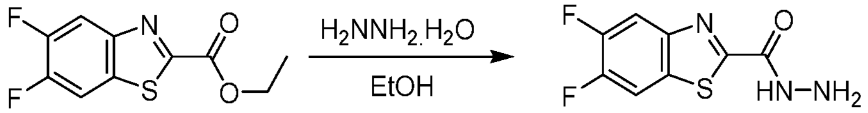


Ethyl 5,6-difluorobenzo[d]thiazole-2-carboxylate (0.58 g, 2.38 mmol) was dissolved in ethanol (10 mL), and hydrazine hydrate (210.31 mg, 3.58 mmol, 85% aqueous solution) was added. The mixture was stirred at 25°C for 1 hour. The reaction solution was filtered to obtain the solid crude product 5,6-difluorobenzo[d]thiazole-2-carbohydrazide (0.375 g, yield: 68.61%), which was used directly in the next step.
MS m/z (ESI): 230.0[M+H]$^+$.

**Step 5: (SR)-4-[(2,4-Dimethoxyphenyl)methyl]-6-ethoxy-5-methyl-3,5-dihydro-2H-pyrazin e**

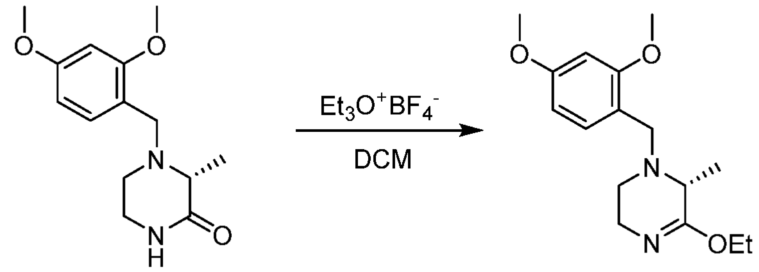


(3R)-4-[(2,4-Dimethoxyphenyl)methyl]-3-methyl-piperazin-2-one (0.6 g, 2.27 mmol) was dissolved in dichloromethane (7 mL), and triethyloxonium tetrafluoroborate (1.08 g, 5.67 mmol) was added at 0°C in three batches with an interval of 10 minutes. The mixture was stirred at 25°C for 3 hours. The reaction solution was added to a cold aqueous sodium hydroxide solution (2 M, 10 ml). The mixture was separated into two phases, and the aqueous phase was extracted with dichloromethane (10 mL×2). The organic phases were combined, dried over anhydrous sodium sulfate and concentrated under reduced pressure to obtain the target product (5R)-4-[(2,4-dimethoxyphenyl)methyl]-6-ethoxy-5-methyl-3,5-dihydro-2H-pyrazine (0.65 g) as a colorless oil. The crude product was used directly in the next step.
MS m/z (ESI): 293.2[M+H]$^+$.

**Step 6: (R)-2-(7-(2,4-Dimethoxybenzyl)-8-methyl-5,6,7,8-tetrahydro-[1,2,4]triazolo[4,3-a]p yrazin-3-yl)-5,6-difluorobenzo[d]thiazole**

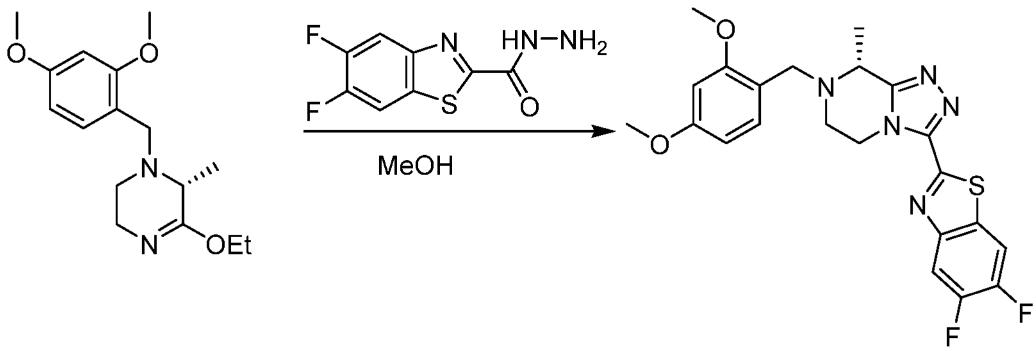


(5R)-4-[(2,4-Dimethoxyphenyl)methyl]-6-ethoxy-5-methyl-3,5-dihydro-2H-pyrazi ne (0.66 g, 2.26 mmol) and 5,6-difluorobenzo[d]thiazole-2-carbohydrazide (0.375 g, 1.64 mmol) were dissolved in MeOH (10 mL). The mixture was stirred at 80°C for 14 hours under a nitrogen atmosphere. The reaction solution was concentrated under reduced pressure. The residue was purified by silica gel flash chromatography (elution: dichloromethane: methanol=100:0 to 95:5) to obtain the target product (R)-2-(7-(2,4-dimethoxybenzyl)-8-methyl-5,6,7,8-tetrahydro-[1,2,4]triazolo[4,3-a]pyraz in-3-yl)-5,6-difluorobenzo[d]thiazole (0.46 g, yield: 45%) as a light yellow solid.
MS m/z (ESI): 458.1[M+H]$^+$.

**Step 7: (R)-5,6-Difluoro-2-(8-methyl-5,6,7,8-tetrahydro-[1,2,4]triazolo[4,3-a]pyrazin-3-yl)b enzo[d]thiazole**

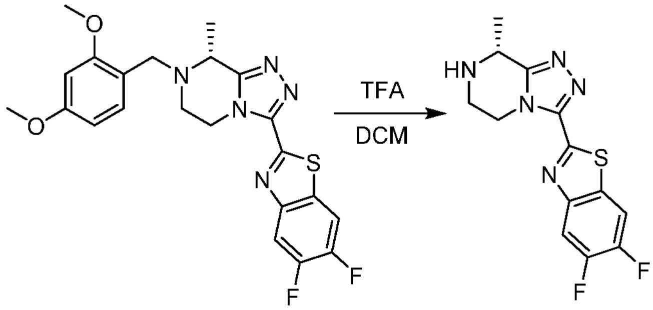


(R)-2-(7-(2,4-Dimethoxybenzyl)-8-methyl-5,6,7,8-tetrahydro-[1,2,4]triazolo[4,3-a] pyrazin-3-yl)-5,6-difluorobenzo[d]thiazole (110 mg, 0.24 mmol) was dissolved in dichloromethane (1.5 mL) and trifluoroacetic acid (3 mL) was added. The mixture was stirred at 25°C for 1 hour. The reaction solution was evaporated under reduced pressure until dry, and then water (7 ml) was added. The mixture was stirred at room temperature for 10 minutes and then filtered to remove solids. A 2 N aqueous sodium hydroxide solution was added to the liquid phase to adjust the pH to 13, and then the liquid phase was extracted with dichloromethane (10 mL×2). The organic phases were combined, dried over anhydrous sodium sulfate and concentrated under reduced pressure to obtain the product (R)-5,6-difluoro-2-(8-methyl-5,6,7,8-tetrahydro-[1,2,4]triazolo[4,3-a]pyrazin-3-yl)benz o[d]thiazole (46 mg, yield: 62%).

MS m/z (ESI): 308.0$[M+H]^+$.

**Step 8: (R)-(3-(5,6-Difluorobenzo[d]thiazol-2-yl)-8-methyl-5,6-dihydro-[1,2,4]triazolo[4,3-a ]pyrazin-7(8H)-yl)(4-fluorophenyl)methanone**

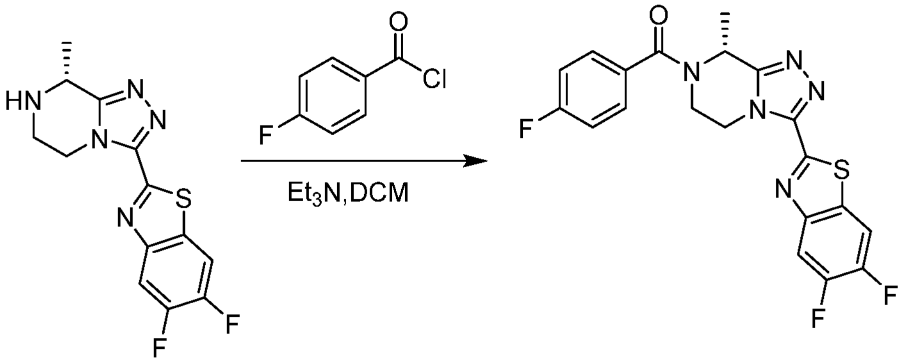


**(R)-5,6-Difluoro-2-(8-methyl-5,6,7,8-tetrahydro-[1,2,4]triazolo[4,3-a]pyrazin-3-yl)** benzo[d]thiazole (46 mg, 0.15 mmol) and triethylamine (45 mg, 0.45 mmol) were dissolved in dichloromethane (5 mL), and a p-fluorobenzoyl chloride solution (36 mg, 0.22 mmol) was added. The mixture was stirred at 25°C for 20 minutes. The reaction solution was evaporated until dry, and the crude product was separated by Prep-HPLC to obtain the product (31 mg, yield: 48%).

MS m/z (ESI): 430.1$[M+H]^+$.

$^1$H NMR (400 MHz, Chloroform-d) δ 7.88-7.79 (m, 1H), 7.79-7.70 (m, 1H), 7.55-7.46 (m, 2H), 7.22-7.13 (m, 2H), 5.94-5.63 (m, 1H), 5.13-4.98 (m, 1H), 4.84-4.44 (m, 1H), 4.44-4.25 (m, 1H), 3.66-3.46 (m, 1H), 1.82-1.71 (m, 3H).

**Example 18**

**(R)-(3-(5,7-Difluorobenzo[d]thiazol-2-yl)-8-methyl-5,6-dihydro-[1,2,4]triazolo[ 4,3-a]pyrazin-7(8H)-yl)(4-fluorophenyl)methanone**

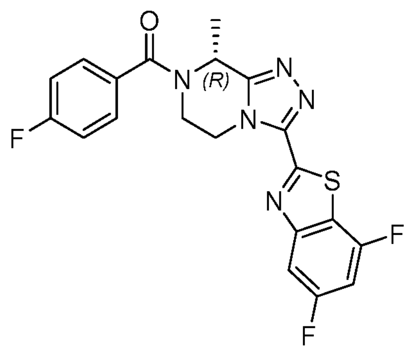

The preparation of Example **18** referred to Example **17.**

MS m/z (ESI): 429.8 $[M+H]^+$.

$^1H$ NMR (400 MHz, Chloroform-d) δ 7.62-7.55 (m, 1H), 7.53-7.46 (m, 2H), 7.18 (t, 2H), 7.04 (td, J = 9.1, 2.1 Hz, 1H), 5.96-5.64 (m, 1H), 5.05 (d, J = 13.6 Hz, 1H), 4.80-4.48 (m, 1H), 4.44-4.32 (m, 1H), 3.70-3.52 (m, 1H), 1.79 (d, J = 6.8 Hz, 3H).

## Example 19

**(R)-(3-(6-Chlorobenzo[d]thiazol-2-yl)-8-methyl-5,6-dihydro-[1,2,4]triazolo[4,3 -a]pyrazin-7(8H)-yl)(4-fluorophenyl)methanone**

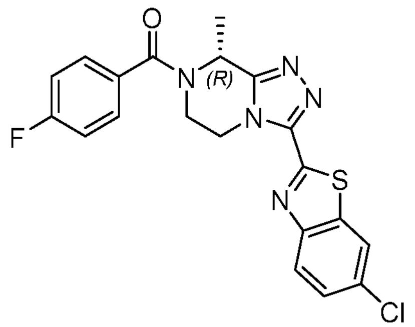

**Step 1: Preparation of (R)-1-(2,4-dimethoxybenzyl)-5-ethoxy-6-methyl-1,2,3,6-tetrahydropyrazine**

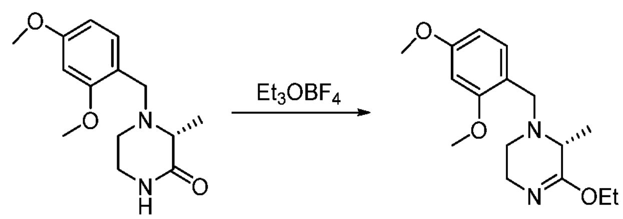


(R)-4-(2,4-Dimethoxybenzyl)-3-methylpiperazin-2-one (0.20 g, 0.38 mmol) was dissolved in dichloroethane (1 mL), cooled to 0°C, and triethyloxidanium tetrafluoroborate (4.5 mL, 4.5 mmol) was added. The reaction solution was stirred at room temperature for 3 hours, and water (10 mL) was added. The reaction solution was stirred for half an hour until the precipitation of solids, and then filtered. The aqueous phase was extracted with dichloromethane (20 mL×2). The organic phases were combined, dried over anhydrous sodium sulfate, filtered and concentrated to obtain (R)-1-(2,4-dimethoxybenzyl)-5-ethoxy-6-methyl-1,2,3,6-tetrahydropyrazine (0.2 g), which was used directly in the next step.

MS m/z (ESI): 293.2 $[M+H]^+$.

**Step 2: Preparation of 6-chlorobenzo[d]thiazole-2-carbonitrile**

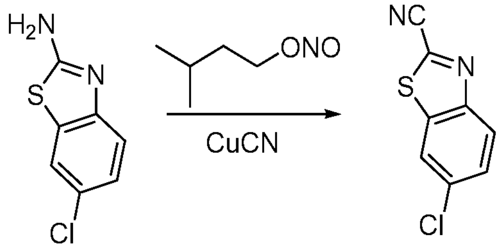


6-Chlorobenzo[d]thiazol-2-amine (5g, 27 mmol) and cuprous cyanide (2.9 g, 32.5 mmol) were added to acetonitrile (60 mL), and then isoamyl nitrite (4.8 g, 41 mmol) was added. The reaction solution was kept at 60°C for 3 hours and then cooled to room temperature and filtered. The filter cake was washed with ethyl acetate. The resulting solution was concentrated under reduced pressure and purified by column chromatography (petroleum ether: ethyl acetate = 20:1-10:1) to obtain the product 6-chlorobenzo[d]thiazole-2-carbonitrile (1 g, yield: 19%).

$^1$H NMR (400 MHz, Chloroform-d) δ 8.15 (d, J = 8.6 Hz, 1H), 7.98 (s, 1H), 7.63 (d, J = 8.9 Hz, 1H).

**Step 3: Preparation of methyl 6-chlorobenzo[d]thiazole-2-carboxylate**

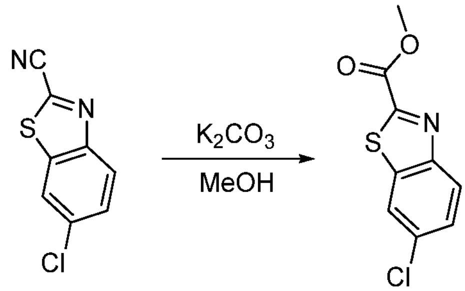


6-Chlorobenzo[d]thiazole-2-carbonitrile (1 g, 5.2 mmol) was dissolved in methanol (20 mL), and potassium carbonate (2.2 g, 15.6 mmol) was added. The reaction solution was kept at room temperature for 16 hours, concentrated under reduced pressure concentration and purified by column chromatography (petroleum ether: ethyl acetate = 20:1-10:1) to obtain the product methyl 6-chlorobenzo[d]thiazole-2-carboxylate (380 mg, yield: 32%).

$^1$H NMR (400 MHz, Chloroform-d) δ 8.03 (d, J = 8.9 Hz, 1H), 7.93 (s, 1H), 7.52 (d, J = 8.8 Hz, 1H), 4.05 (s, 3H).

**Step 4: Preparation of 6-chlorobenzo[d]thiazole-2-carbohydrazide**

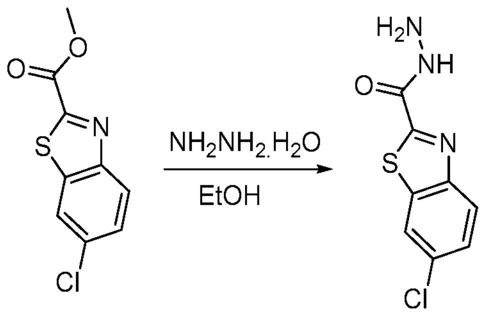


Methyl 6-chlorobenzo[d]thiazole-2-carboxylate (380 mg, 1.7 mmol) was dissolved in ethanol (10 mL), and 85% hydrazine hydrate (118 mg, 2 mmol) was added. The reaction solution was kept at room temperature for 3 hours, filtered

and dried to obtain the product 6-chlorobenzo[d]thiazole-2-carbohydrazide (300 mg, yield: 78%).

MS m/z (ESI): 227.8 $[M+H]^+$.

**Step 5: Preparation of (R)-6-chloro-2-(7-(2,4-dimethoxybenzyl)-8-methyl-5,6,7,8-tetrahydro-[1,2,4]triazol o[4,3-a]pyrazin-3-yl)benzo[d]thiazole**

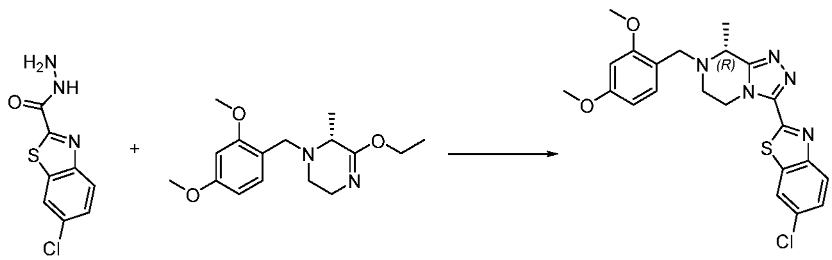

6-Chlorobenzo[d]thiazole-2-carbohydrazide (150 mg, 0.66 mmol) and (R)-1-(2,4-dimethoxybenzyl)-5-ethoxy-6-methyl-1,2,3,6-tetrahydropyrazine (200 mg, 0.68 mmol) were added to ethanol (10 mL). The reaction solution was kept at 80°C for 16 hours, concentrated under reduced pressure concentration and purified by column chromatography (petroleum ether: ethyl acetate = 1:1:1:1-1:2-2) to obtain the product (R)-6-chloro-2-(7-(2,4-dimethoxybenzyl)-8-methyl-5,6,7,8-tetrahydro-[1,2,4]triazolo[4, 3-a]pyrazin-3-yl)benzo[d]thiazole (150 mg, yield: 48%).

MS m/z (ESI): 455.8 $[M+H]^+$.

**Step 6: Preparation of (R)-6-chloro-2-(8-methyl-5,6,7,8-tetrahydro-[1,2,4]triazolo[4,3-a]pyrazin-3-yl)benz o[d]thiazole**

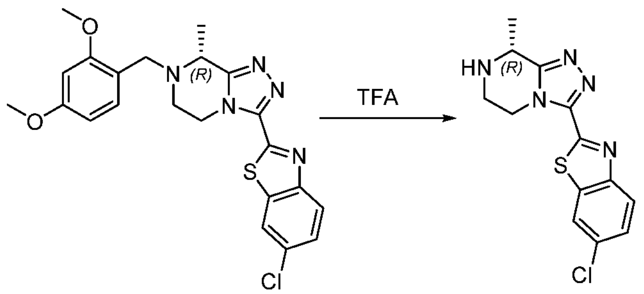


(R)-6-Chloro-2-(7-(2,4-dimethoxybenzyl)-8-methyl-5,6,7,8-tetrahydro-[1,2,4]triaz olo[4,3-a]pyrazin-3-yl)benzo[d]thiazole (150 mg, 0.33 mmol) was dissolved in dichloromethane (2 mL), and trifluoroacetic acid (1 mL) was added. The reaction solution was kept at room temperature for 1 hour, and water (5 mL) was added. The reaction solution was stirred for 5 minutes and filtered. The pH of the filtrate was adjusted to >7, and the filtrate was extracted with DCM (5 mL×2). The organic phases were combined, washed with saturated brine (10 mL), dried over anhydrous sodium sulfate and concentrated under reduced pressure to obtain the product (R)-6-chloro-2-(8-methyl-5,6,7,8-tetrahydro-[1,2,4]triazolo[4,3-a]pyrazin-3-yl)benzo[d] thiazole (70 mg, yield: 70%).

MS m/z (ESI): 306.2 $[M+H]^+$.

**Step 7: Preparation of (R)-(3-(6-chlorobenzo [d] thiazol-2-yl)-8-methyl-5,6-dihydro- [1,2,4] triazolo [4,3-a] py razin-7(8H)-yl)(4-fluorophenyl)methanone**

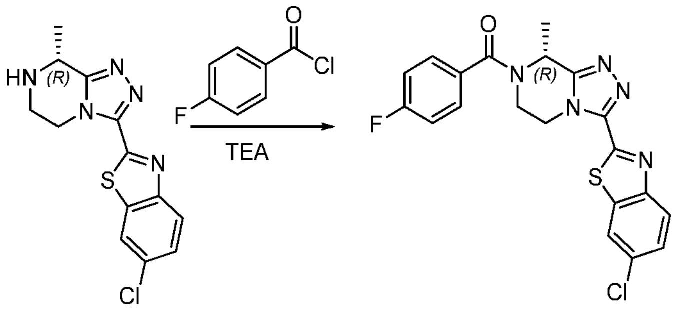


(R)-6-Chloro-2-(8-methyl-5,6,7,8-tetrahydro-[1,2,4]triazolo[4,3-a]pyrazin-3-yl)ben zo[d]thiazole (30 mg, 0.1 mmol) was dissolved in dichloromethane (1 mL), and triethylamine (15 mg, 0.15 mmol) and p-fluorobenzoyl chloride (19 mg, 0.12 mmol) were added. The reaction solution was kept at room temperature for 1 hour, subjected to rotatory evaporation to remove the solvent, and purified by pre-HPLC to obtain the product (R)-(3-(6-chlorobenzo[d]thiazol-2-yl)-8-methyl-5,6-dihydro-[1,2,4]triazolo[4,3-a]pyrazi n-7(8H)-yl)(4-fluorophenyl)methanone (7.9 mg, yield: 19%).

MS m/z (ESI): 427.8 $[M+H]^+$.

$^1H$ NMR (400 MHz, Chloroform-d) $\delta$ 7.99-7.91 (m, 2H), 7.55-7.45 (m, 3H), 7.18 (t, J = 8.0 Hz, 2H), 5.90-5.64 (m, 1H), 5.07 (d, J = 12.3 Hz, 1H), 4.82-4.57 (m, 1H), 4.42-4.31 (m, 1H), 3.61-3.52 (m, 1H), 1.77 (d, J = 6.7 Hz, 3H).

**Example 20**

**(R)-(3-(6-Chlorobenzo[d]thiazol-2-yl)-8-methyl-5,6-dihydro-[1,2,4]triazolo[4,3 -a]pyrazin-7(8H)-yl)(4-chlorophenyl)methanone**

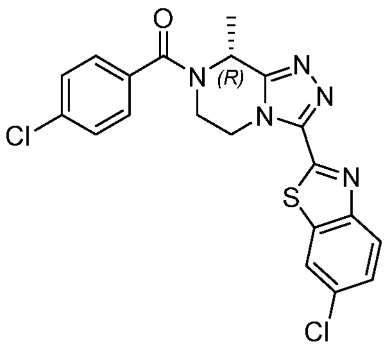

The preparation of Example **20** referred to Step 5 of Example **1.**

MS m/z (ESI): 444.0 $[M+H]^+$.

**Example 21**

**(R)-2-(7-(4-Fluorobenzoyl)-8-methyl-5,6,7,8-tetrahydro-[1,2,4]triazolo[4,3-a ]pyrazin-3-yl)benzo[d]thiazol-6-carbonitrile**

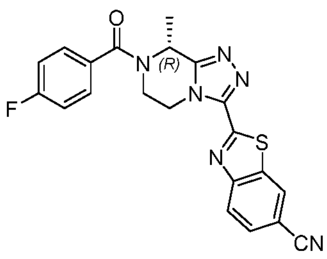

**Step 1: Preparation of (R)-1-(2,4-dimethoxybenzyl)-5-ethoxy-6-methyl-1,2,3,6-tetrahydropyrazine**

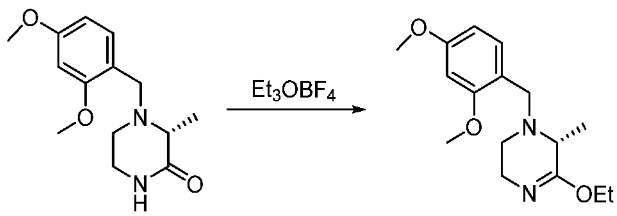


(R)-4-(2,4-Dimethoxybenzyl)-3-methylpiperazin-2-one (0.5 g, 1.1 mmol) was dissolved in dichloromethane (10 mL), cooled to 0°C, and triethyloxidanium tetrafluoroborate (1.44 g, 7.6 mmol) was added. The reaction solution was stirred at room temperature for 2 hours. TLC (DCM/MeOH = 10/1) showed that the reaction was complete. The reaction was quenched with a 2 M cold aqueous sodium hydroxide solution (30 mL), and the aqueous phase was extracted with dichloromethane (30 mL×2). The organic phases were combined, dried over anhydrous sodium sulfate, filtered and concentrated to obtain (R)-1-(2,4-dimethoxybenzyl)-5-ethoxy-6-methyl-1,2,3,6-tetrahydropyrazine (0.5 g, 90% crude), which was used directly in the next step.

**Step 2: Preparation of 6-bromobenzo[d]thiazole-2-carbohydrazide**

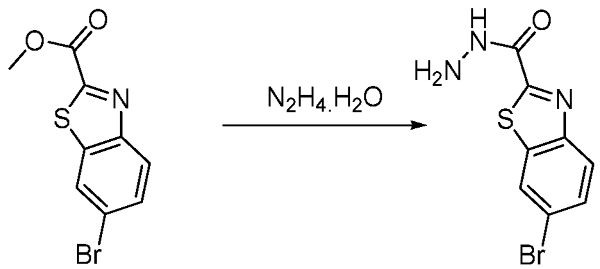


Methyl 6-bromobenzo[d]thiazole-2-carboxylate (0.2 g, 0.7 mmol) was dissolved in anhydrous methanol (5 mL), and 85% hydrazine hydrate (78 mg, 0.8 mmol) was added. The reaction solution was stirred at 60°C for 1 hour until the precipitation of solids, and subjected to rotatory evaporation to remove the solvent to obtain 6-bromobenzo[d]thiazole-2-carbohydrazide (0.2 g, yield: 100%) as a white solid.

**Step 3: Preparation of (R)-6-bromo-2-(7-(2,4-dimethoxybenzyl)-8-methyl-5,6,7,8-tetrahydro-[1,2,4]triazol o[4,3-a]pyrazin-3-yl)benzo[d]thiazole**

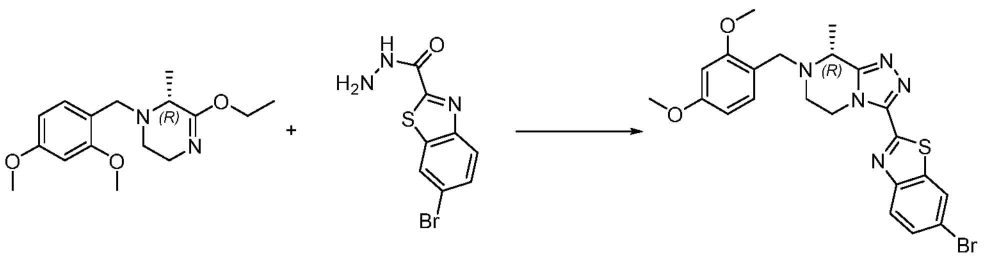

(R)-1-(2,4-Dimethoxybenzyl)-5-ethoxy-6-(methoxymethyl)-1,2,3,6-tetrahydropyra zine (500 mg, 1.7 mmol) was dissolved in methanol (20 mL), and 6-bromobenzo[d]thiazole-2-carbohydrazide (200 mg, 0.74 mmol) was added. The reaction solution was stirred at 65°C overnight and subjected to rotatory evaporation until dry. The crude product was separated by column chromatography (PE/EA = 10/1 ~1/3) to obtain (R)-6-bromo-2-(7-(2,4-dimethoxybenzyl)-8-methyl-5,6,7,8-tetrahydro-[1,2,4]triazolo[4, 3-a]pyrazin-3-yl)benzo[d]thiazole (200 mg, yield: 54%) as a colorless colloid.

MS m/z (ESI): 502.2/500.2 $[M+H]^+$.

**Step 4: Preparation of (R)-2-(7-(2,4-dimethoxybenzyl)-8-methyl-5,6,7,8-tetrahydro-[1,2,4]triazolo[4,3-a]p yrazin-3-yl)benzo[d]thiazol-6-carbonitrile**

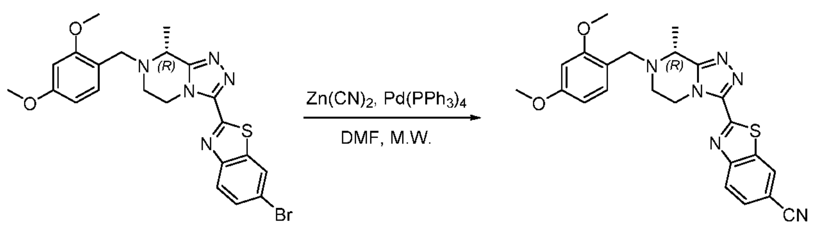


(R)-6-Bromo-2-(7-(2,4-dimethoxybenzyl)-8-methyl-5,6,7,8-tetrahydro-[1,2,4]triaz olo[4,3-a]pyrazin-3-yl)benzo[d]thiazole (100 mg, 0.3 mmol) was dissolved in N,N-dimethylformamide (3 mL), and zinc cyanide (94 mg, 0.8 mmol) and tetrakis(triphenylphosphine) palladium (23 mg, 0.02 mmol) was added. The reaction system was purged with nitrogen (×3), and the reaction solution was stirred in microwave at 100°C for 1 hour. Water (20 mL) was added, and the reaction solution was extracted with ethyl acetate (20 mL). The organic phase was dried over anhydrous sodium sulfate, filtered and concentrated. The crude product was separated by prep-TLC to obtain (R)-2-(7-(2,4-dimethoxybenzyl)-8-methyl-5,6,7,8-tetrahydro-[1,2,4]triazolo[4,3-a]pyraz in-3-yl)benzo[d]thiazole-6-carbonitrile (90 mg, yield: 99%) as a colorless colloid.

MS m/z (ESI): 447.2 $[M+H]^+$.

**Step 5: Preparation of (R)-2-(8-methyl-5,6,7,8-tetrahydro-[1,2,4]triazolo[4,3-a]pyrazin-3-yl)benzo[d]thiaz ole-6-carbonitrile**

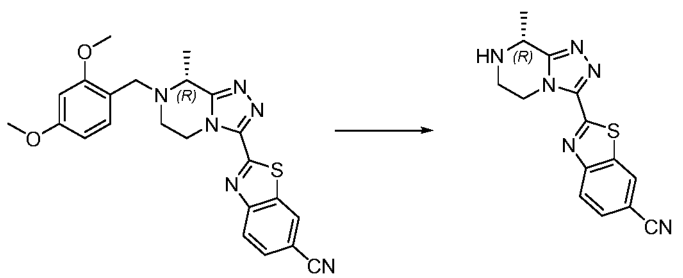

(R)-2-(7-(2,4-Dimethoxybenzyl)-8-methyl-5,6,7,8-tetrahydro-[1,2,4]triazolo[4,3-a] pyrazin-3-yl)benzo[d]thiazole-6-carbonitrile (90 mg, 0.2 mmol) was dissolved in dichloromethane (10 mL), and trifluoroacetic acid (3 mL) was added. The reaction solution was stirred at room temperature for 1 hour. Water (10 mL) was added, and the reaction

solution was stirred for half an hour until the precipitation of solids, and then filtered. A 4 M sodium hydroxide solution was added to adjust the pH of the filtrate to >14, and the filtrate was extracted with dichloromethane (20 mL×2). The organic phase was dried over anhydrous sodium sulfate, filtered and concentrated to obtain (R)-2-(8-methyl-5,6,7,8-tetrahydro-[1,2,4]triazolo[4,3-a]pyrazin-3-yl)benzo[d]thiazol-6-carbonitrile (60 mg, yield: 99%).

MS m/z (ESI): 297.2 $[M+H]^+$.

**Step 6: Preparation of (R)-2-(7-(4-fluorobenzoyl)-8-methyl-5,6,7,8-tetrahydro-[1,2,4]triazolo[4,3-a]pyrazi n-3-yl)benzo[d]thiazole-6-carbonitrile**

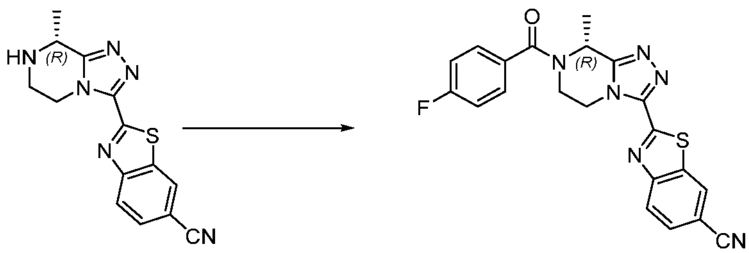

In accordance with Step 5 of Example 1, (R)-2-(7-(4-fluorobenzoyl)-8-methyl-5,6,7,8-tetrahydro-[1,2,4]triazolo[4,3-a]pyrazin-3-yl)benzo[d]thiazole-6-carbonitrile (49 mg, yield: 58%) was obtained as a white solid.

MS m/z (ESI): 419.2 $[M+H]^+$.

$^1$H NMR (400 MHz, $CDCl_3$) δ 8.32 (s, 1H), 8.13 (d, J = 8.0 Hz, 1H), 7.79 (d, J = 8.0 Hz, 1H), 7.52-7.48 (m, 2H), 7.20-7.16 (m, 2H), 5.80-5.76 (m, 1H), 5.10-5.06 (m, 1H), 4.64-4.36 (m, 2H), 3.61-3.55 (m, 1H), 1.78 (d, J = 8.0 Hz, 3H).

**Example 22**

**(R)-(4-Fluorophenyl)(8-methyl-3-(4-methylbenzo[d]thiazol-2-yl)-5,6-dihydro-[ 1,2,4]triazolo[4,3-a]pyrazin-7(8H)-yl)methanone**

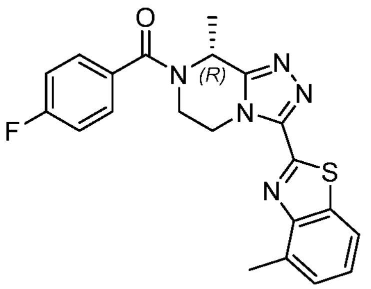

The preparation of Example **22** referred to Example **17.**

MS m/z (ESI): 407.8 $[M+H]^+$.

$^1$H NMR (400 MHz, Chloroform-d) δ 7.80 (d, J = 7.4 Hz, 1H), 7.56-7.45 (m, 2H), 7.43-7.31 (m, 2H), 7.23-7.13 (m, 2H), 5.75 (br s, 1H), 5.12 (d, J = 12.5 Hz, 1H), 4.80-4.60 (m, 1H), 4.48-4.32 (m, 1H), 3.67-3.48 (m, 1H), 2.76 (s, 3H), 1.78 (s, 3H).

**Example 23**

**(R)-(4-Chlorophenyl)(8-methyl-3-(4-methylbenzo[d]thiazol-2-yl)-5,6-dihydro-[ 1,2,4]triazolo[4,3-a]pyrazin-7(8H)-yl)methanone**

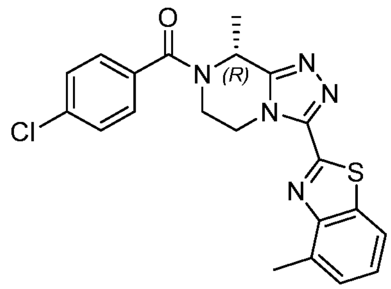

The preparation of Example **23** referred to Step 5 of Example **1.**

MS m/z (ESI): 423.8 $[M+H]^+$.

$^1H$ NMR (400 MHz, Chloroform-d) δ 7.82 (d, J = 7.3 Hz, 1H), 7.51-7.46 (m, 3H), 7.46-7.40 (m, 2H), 7.39-7.36 (m, 1H), 5.95-5.87 (m, 1H), 5.24-5.17 (m, 1H), 4.74-4.65 (m, 1H), 4.52-4.45 (m, 1H), 3.69-3.63 (m, 1H), 2.77 (s, 3H), 2.12 (m, 3H).

**Example 24**

**(R)-(4-Fluorophenyl)(8-methyl-3-(6-methylbenzo[d]thiazol-2-yl)-5,6-dihydro-[ 1,2,4]triazolo[4,3-a]pyrazin-7(8H)-yl)methanone**

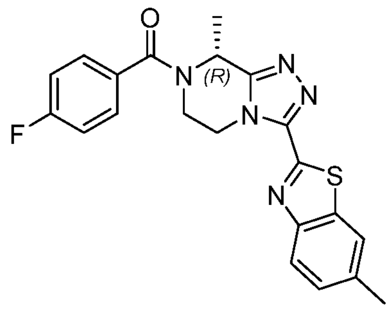

The preparation of Example **24** referred to Example **17.**

MS m/z (ESI): 408.1$[M+H]^+$.

$^1H$ NMR (400 MHz, Chloroform-d) δ 7.99-7.88 (m, 1H), 7.82-7.73 (m, 1H), 7.62-7.42 (m, 2H), 7.40-7.31 (m, 1H), 7.23-7.07 (m, 2H), 5.98-5.61 (m, 1H), 5.27-5.00 (m, 1H), 4.79-4.52 (m, 1H), 4.48-4.29 (m, 1H), 3.74-3.39 (m, 1H), 2.53 (s, 3H), 1.98-1.85 (m, 3H).

**Example 25**

(**R)-(4-Chlorophenyl)(8-methyl-3-(6-methylbenzo[d]thiazol-2-yl)-5,6-dihydro-[ 1,2,4]triazolo[4,3-a]pyrazin-7(8H)-yl)methanone**

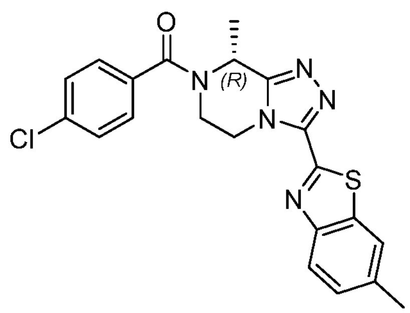

The preparation of Example **25** referred to Step 5 of Example **1.**
MS m/z (ESI): 424.1[M+H]$^+$.
$^1$H NMR (400 MHz, Chloroform-d) δ 7.95-7.86 (m, 1H), 7.79-7.70 (m, 1H), 7.51-7.37 (m, 4H), 7.37-7.29 (m, 1H), 6.01-5.48 (m, 1H), 5.15-5.00 (m, 1H), 4.84-4.44 (m, 1H), 4.44-4.19 (m, 1H), 3.66-3.41 (m, 1H), 2.52 (s, 3H), 1.75 (d, J = 6.8 Hz, 3H).

**Example 26**

**(R)-(3,4-Dichlorophenyl)(8-methyl-3-(6-methylbenzo[d]thiazol-2-yl)-5,6-dihyd ro-[1,2,4]triazolo[4,3-a]pyrazin-7(8H)-yl)methanone**

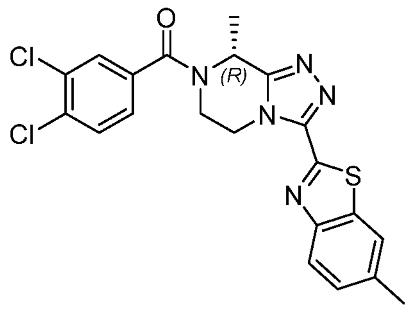

The preparation of Example **26** referred to Step 5 of Example **1.**
MS m/z (ESI): 458.0[M+H]$^+$.
$^1$H NMR (400 MHz, Chloroform-d) δ 7.96-7.85 (m, 1H), 7.80-7.69 (m, 1H), 7.64-7.51 (m, 2H), 7.41-7.28 (m, 2H), 6.04-5.51 (m, 1H), 5.23-5.00 (m, 1H), 4.84-4.42 (m, 1H), 4.43-4.23 (m, 1H), 3.74-3.44 (m, 1H), 2.52 (s, 3H), 1.82-1.75 (m, 3H).

**Example 27**

**(R)-(4-Fluorophenyl)(8-methyl-3-(7-methylbenzo[d]thiazol-2-yl)-5,6-dihydro-[ 1,2,4]triazolo[4,3-a]pyrazin-7(8H)-yl)methanone**

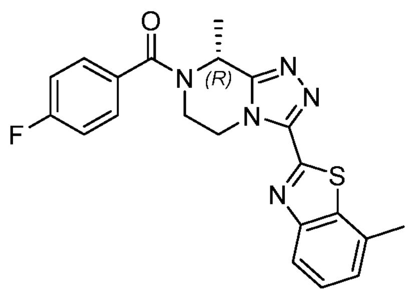

The preparation of Example **27** referred to Example **17.**
MS m/z (ESI): 408.1 [M+H]$^+$.
$^1$H NMR (400 MHz, $CDCl_3$) δ 7.88 (d, J = 8.4 Hz, 1H), 7.44 (t, J = 8.4 Hz, 3H), 7.32 (t, J = 8.4 Hz, 1H), 7.18 (d, J = 8.4 Hz, 2H), 5.98-5.66 (br, 1H), 5.19-5.04 (m, 1H), 4.80-4.45 (br, 1H), 4.45-4.31 (m, 1H), 3.66-3.49 (m, 1H), 2.63(s, 3H), 1.78 (d, J = 6.4 Hz, 3H).

**Example 28**

**(R)-(4-Chlorophenyl)(8-methyl-3-(7-methylbenzo[d]thiazol-2-yl)-5,6-dihydro-[ 1,2,4]triazolo[4,3-a]pyrazin-7(8H)-yl)methanone**

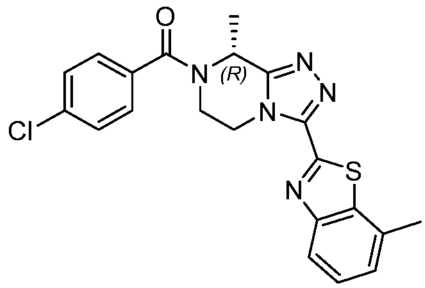

The preparation of Example **28** referred to Step 5 of Example **1.**

MS m/z (ESI): 424.0 $[M+H]^+$.

$^1H$ NMR (400 MHz, $CDCl_3$) δ 7.83 (d, J = 8.4 Hz, 2H), 7.46 (d, J = 8.4 Hz, 2H), 7.42 (d, J = 8.4 Hz, 2H), 7.31 (d, J = 8.4 Hz, 1H), 6.04-5.58 (br, 1H), 5.14-5.08 (m, 1H), 4.92-4.51 (br, 1H), 4.43-4.31 (m, 1H), 3.63-3.48 (br, 1H), 2.53(s, 3H), 1.76 (d, J = 6.4 Hz, 3H).

**Example 29**

**(R)-(4-Fluorophenyl)(3-(6-methoxybenzo[d]thiazol-2-yl)-8-methyl-5,6-dihydro -[1,2,4]triazolo[4,3-a]pyrazin-7(8H)-yl)methanone**

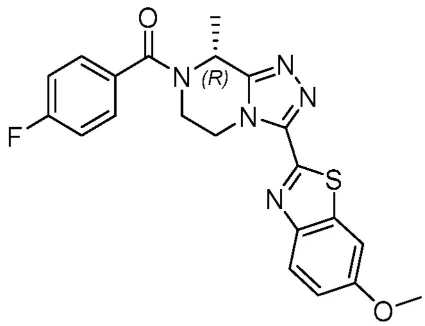

The preparation of Example **29** referred to Example **12.**

MS m/z (ESI): 424.1$[M+H]^+$.

$^1H$ NMR (400 MHz, Chloroform-d) δ 7.97-7.87 (m, 1H), 7.58-7.43 (m, 2H), 7.42-7.34 (m, 1H), 7.22-7.06 (m, 3H), 5.92-5.67 (m, 1H), 5.17-4.96 (m, 1H), 4.73-4.47 (m, 1H), 4.46-4.26 (m, 1H), 3.91 (s, 3H), 3.67-3.42 (m, 1H), 1.85-1.70 (m, 3H).

**Example 30**

**(R)-(4-Chlorophenyl)(3-(6-methoxybenzo[d]thiazol-2-yl)-8-methyl-5,6-dihydro -[1,2,4]triazolo[4,3-a]pyrazin-7(8H)-yl)methanone**

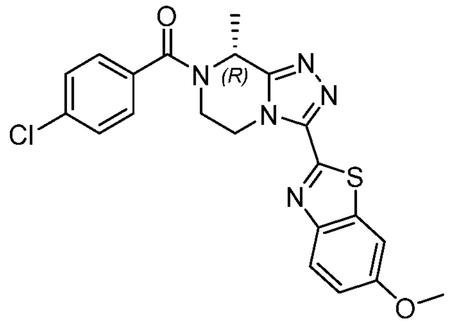

The preparation of Example **30** referred to Step 5 of Example **1.**

MS m/z (ESI): 440.1[M+H]+.

$^1$H NMR (400 MHz, Chloroform-d) δ 7.96-7.85 (m, 1H), 7.53-7.32 (m, 5H), 7.18-7.06 (m, 1H), 5.98-5.60 (m, 1H), 5.14-4.98 (m, 1H), 4.82-4.44 (m, 1H), 4.42-4.25 (m, 1H), 3.91 (s, 3H), 3.66-3.44 (m, 1H), 1.75 (d, J = 6.9 Hz, 3H).

**Example 31**

**(R)-(3,4-Dichlorophenyl)(3-(6-methoxybenzo[d]thiazol-2-yl)-8-methyl-5,6-dihy dro-[1,2,4]triazolo[4,3-a]pyrazin-7(8H)-yl)methanone**

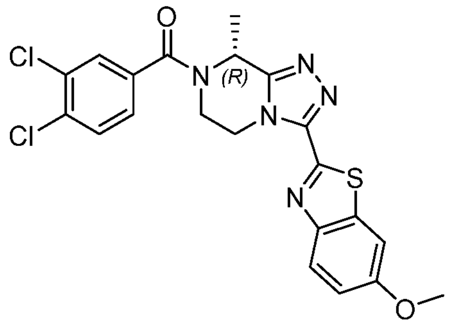

The preparation of Example **31** referred to Step 5 of Example **1.**

MS m/z (ESI): 474.1[M+H]+.

$^1$H NMR (400 MHz, Chloroform-d) δ 7.95-7.86 (m, 1H), 7.64-7.49 (m, 2H), 7.42-7.36 (m, 1H), 7.35-7.28 (m, 1H), 7.19-7.05 (m, 1H), 5.95-5.60 (m, 1H), 5.20-4.98 (m, 1H), 4.75-4.41 (m, 1H), 4.44-4.19 (m, 1H), 3.99-3.79 (m, 3H), 3.69-3.37 (m, 1H), 1.94-1.80 (m, 3H).

**Example 32**

**(R)-(4Fluorophenyl)(8-methyl-3-(thiazolo [5,4-b] pyridin-2-yl)-5,6-dihydro-[1,2, 4]triazolo[4,3-a]pyrazin-7(8H)-yl)methanone**

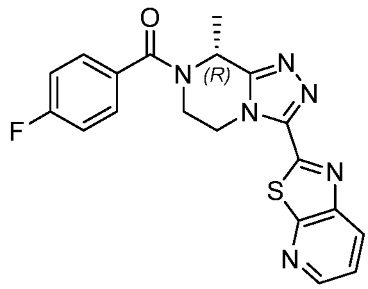

The preparation of Example **32** referred to Example **17.**

MS m/z (ESI): 395.0[M+H]$^+$.

$^1$H NMR (400 MHz, DMSO-$d_6$) δ 8.66 (m, 1H), 8.43 (d, J = 8.0 Hz, 1H), 7.63-7.60 (m, 1H), 7.55 (m, 2H), 7.28 (t, J = 8.4 Hz, 2H), 5.84-5.51 (br, 1H), 4.78 (d, J = 12.8 Hz , 1H), 4.37-4.31 (m, 1H), 4.05-3.87 (br, 1H), 3.66-3.58 (m, 1H), 1.57 (d, J = 6.4 Hz, 3H).

**Example 33**

**(R)-(4-Chlorophenyl)(8-methyl-3-(thiazolo[5,4-b]pyridin-2-yl)-5,6-dihydro-[1, 2,4]triazolo[4,3-a]pyrazin-7(8H)-yl)methanone**

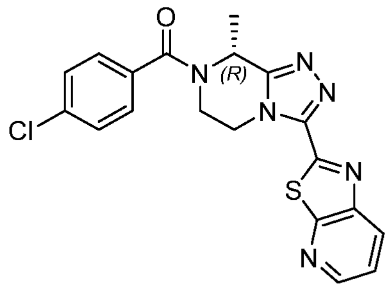

The preparation of Example **33** referred to Step 5 of Example **1.**

MS m/z (ESI): 411.0 [M+H]$^+$.

$^1$H NMR (400 MHz, DMSO-$d_6$) δ 8.75-8.70 (m, 1H), 8.50 (d, J = 8.4 Hz, 1H), 7.71-7.68 (m, 1H), 7.59 ( s, 4H), 5.93-5.58 (br, 1H), 4.82 (d, J = 14.8 Hz , 1H), 4.48-4.34 (m, 1H), 4.13-3.88 (br, 1H), 3.78-3.68 (br, 1H), 1.65 (d, J = 6.8 Hz, 3H).

**Example 34**

**(R)-(4-Fluorophenyl)(8-methyl-3-(thiazolo[4,5-c]pyridin-2-yl)-5,6-dihydro-[1,2 ,4]triazolo[4,3-a]pyrazin-7(8H)-yl)methanone**

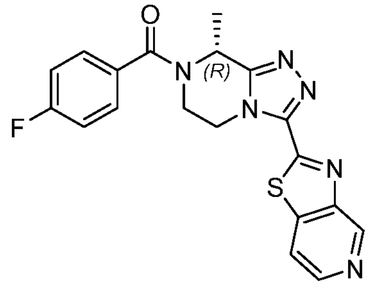

The preparation of Example **34** referred to Example **17.**

MS m/z (ESI): 395.1[M+H]$^+$.

**Example 35**

**(R)-(4-Chlorophenyl)(8-methyl-3-(thiazolo[4,5-c]pyridin-2-yl)-5,6-dihydro- [1, 2,4] triazolo[4,3-a]pyrazin-7(8H)-yl)methanone**

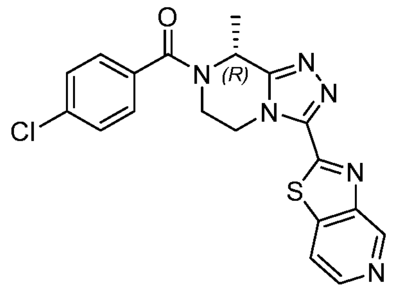

The preparation of Example **35** referred to Step 5 of Example **1.**

MS m/z (ESI): 411.1[M+H]$^+$.

$^1$H NMR (400 MHz, Chloroform-d) δ 9.39 (s, 1H), 8.72-8.44 (m, 1H), 8.11-7.83 (m, 1H), 7.64-7.33 (m, 4H), 6.01-5.51 (m, 1H), 5.19-4.94 (m, 1H), 4.80-4.43 (m, 1H), 4.45-4.24 (m, 1H), 3.70-3.40 (m, 1H), 1.78 (d, J = 6.9 Hz, 3H).

**Example 36**

**(R)-(3,4-Dichlorophenyl)(8-methyl-3-(thiazolo[4,5-c]pyridin-2-yl)-5,6-dihydro-[1,2,4]triazolo[4,3-a]pyrazin-7(8H)-yl)methanone**

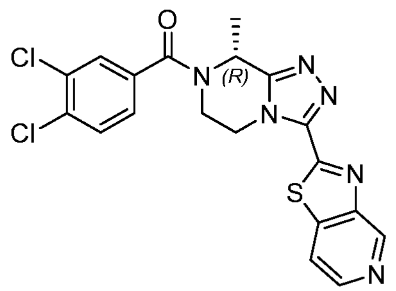

The preparation of Example **36** referred to Step 5 of Example **1.**

MS m/z (ESI): 445.0[M+H]$^+$.

$^1$H NMR (400 MHz, Chloroform-d) δ 9.40 (s, 1H), 8.72-8.57 (m, 1H), 8.16-8.01 (m, 1H), 7.68-7.52 (m, 2H), 7.41-7.29 (m, 1H), 6.01-5.51 (m, 1H), 5.19-4.94 (m, 1H), 4.80-4.43 (m, 1H), 4.45-4.24 (m, 1H), 3.70-3.40 (m, 1H), 1.78 (d, J = 6.9 Hz, 3H).

**Example 37**

**(R)-(3-(6-Chlorothiazolo[4,5-c]pyridin-2-yl)-8-methyl-5,6-dihydro-[1,2,4]triaz olo[4,3-a]pyrazin-7(8H)-yl)(4-fluorophenyl)methanone**

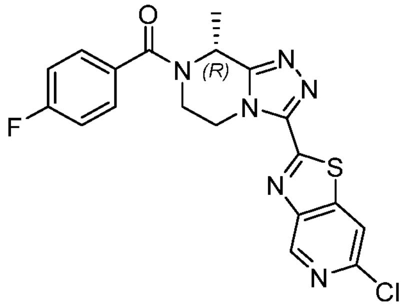

**Step 1: Ethyl 2-((4,6-dichloropyridin-3-yl)amino)-2-oxoacetate**

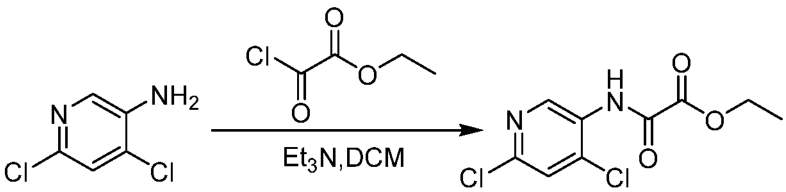


4,6-Dichloropyridine-3-amine (4.5 g, 27.6 mmol) and triethylamine (7.7 mL, 56.2 mmol) were dissolved in dichloromethane (150 mL), and ethyl 2-chloro-2-oxoacetate (4.5 g, 33.1 mmol) was added under a nitrogen atmosphere. The mixture was stirred at 0°C for 20 minutes, the reaction was quenched with saturated brine (100 mL), and the mixture was separated into two phases. The organic phase was washed with saturated brine (100 mL×3), dried over anhydrous sodium sulfate and concentrated under reduced pressure to obtain the target product ethyl 2-((4,6-dichloropyridin-3-yl)amino)-2-oxoacetate (4.5 g, yield: 62%), which was used directly in the next step.

MS m/z (ESI): 263.0[M+H]$^+$.

**Step 2: Ethyl 6-chlorothiazolo[4,5-c]pyridine-2-carboxylate**

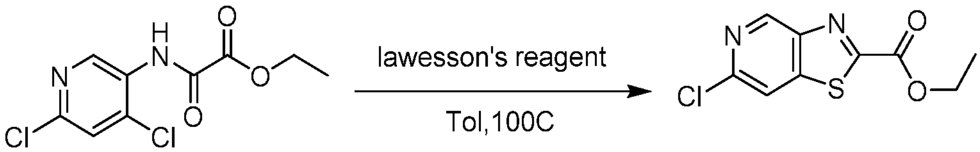


Ethyl 2-((4,6-dichloropyridin-3-yl)amino)-2-oxoacetate (2.2 g, 8.4 mmol) was dissolved in toluene (50 mL), and Lawesson's reagent (2.2 g, 5.0 mmol) was added under a nitrogen atmosphere. The mixture was stirred at 110°C for 3 hours, and then the reaction solution was evaporated until dry. The residue was purified by silica gel flash chromatography (elution: petroleum ether: ethyl acetate = 5:1) to obtain the target product ethyl 6-chlorothiazolo[4,5-c]pyridine-2-carboxylate (1 g, yield: 49%).

MS m/z (ESI): 243.0[M+H]$^+$.

**Step 3: 6-Chlorothiazolo[4,5-c]pyridine-2-carbohydrazide**

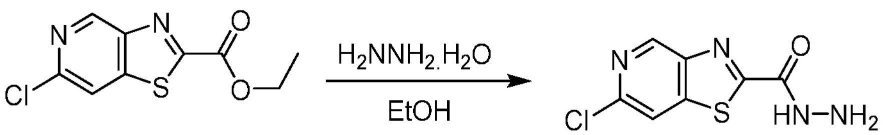

Ethyl 6-chlorothiazolo[4,5-c]pyridine-2-carboxylate (0.95 g, 3.9 mmol) was dissolved in ethanol (30 mL), and hydrazine hydrate (346 mg, 5.9 mmol, 85% aqueous solution) was added. The mixture was stirred at 25°C for 1 hour. The reaction solution was filtered to obtain the solid crude product 6-chlorothiazolo[4,5-c]pyridine-2-carbohydrazide (0.5 g, yield: 69%), which was used directly in the next step.

MS m/z (ESI): 229.0[M+H]$^+$.

**Step 4: (SR)-4-[(2,4-Dimethoxyphenyl)methyl]-6-ethoxy-5-methyl-3,5-dihydro-2H-pyrazin e**

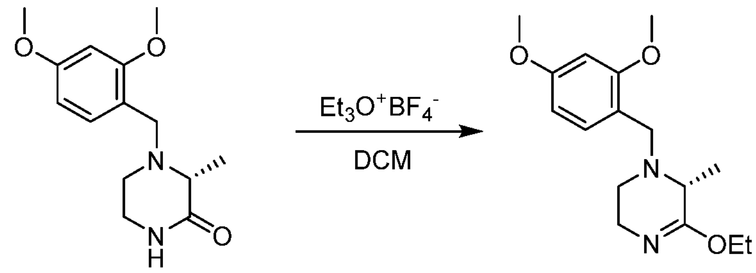


(3R)-4-[(2,4-Dimethoxyphenyl)methyl]-3-methyl-piperazin-2-one (0.25 g, 0.95 mmol) was dissolved in dichloromethane (3 mL), and triethyloxonium tetrafluoroborate (0.45 g, 2.38 mmol) was added at 0°C in three batches with an interval of 10 minutes. The mixture was stirred at 25°C for 3 hours. The reaction solution was added to a cold aqueous sodium hydroxide solution (2 M, 10 ml). The mixture was separated into two phases, and the aqueous phase was extracted with dichloromethane (10 mL×2). The organic phases were combined, dried over anhydrous sodium sulfate, and concentrated under reduced pressure to obtain the target product (5R)-4-[(2,4-dimethoxyphenyl)methyl]-6-ethoxy-5-methyl-3,5-dihydro-2H-pyrazine (0.27 g) as a colorless oil. The crude product was used directly in the next step.

MS m/z (ESI): 293.2[M+H]$^+$.

**Step 5: (R)-6-Chloro-2-(7-(2,4-dimethoxybenzyl)-8-methyl-5,6,7,8-tetrahydro-[1,2,4]triazol o[4,3-a]pyrazol-3-yl)thiazolo[4,5-c]pyridine**

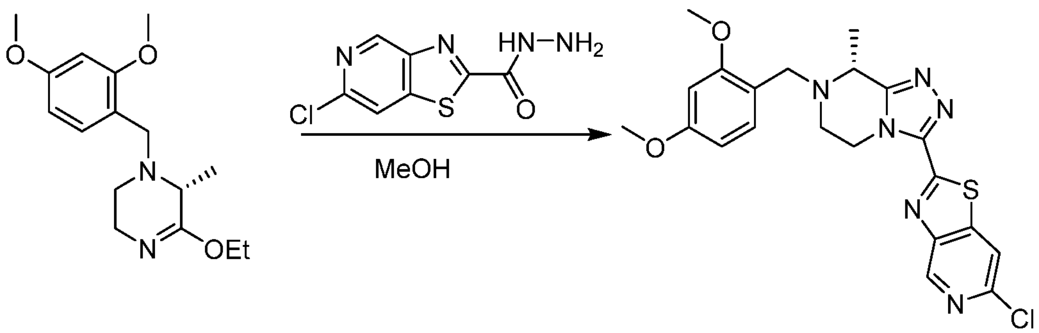


(5R)-4-[(2,4-Dimethoxyphenyl)methyl]-6-ethoxy-5-methyl-3,5-dihydro-2H-pyrazi ne (0.27 g, 0.95 mmol) and 6-chlorothiazolo[4,5-c]pyridine-2-carbohydrazide (184 mg, 0.8 mmol) were dissolved in MeOH (10 mL). The mixture was stirred at 80°C for 14 hours under a nitrogen atmosphere. The reaction solution was concentrated under reduced pressure. The residue was purified by silica gel flash chromatography (elution: dichloromethane: methanol = 100:0 to 95:5) to obtain the target product (R)-6-chloro-2-(7-(2,4-dimethoxybenzyl)-8-methyl-5,6,7,8-tetrahydro-[1,2,4]triazolo[4, 3-a]pyrazin-3-yl)thiazolo[4,5-c]pyridine (0.4 g, yield: 85%) as a light yellow solid.

MS m/z (ESI): 457.1[M+H]$^+$.

**Step 6: (R)-6-Chloro-2-(8-methyl-5,6,7,8-tetrahydro-[1,2,4]triazolo[4,3-a]pyrazol-3-yl)thia zolo[4,5-c]pyridine**

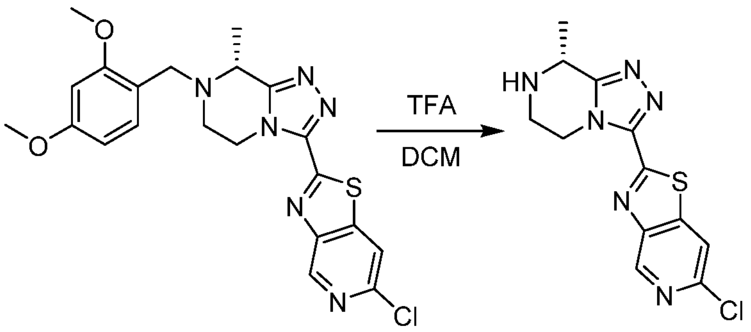


(R)-6-Chloro-2-(7-(2,4-dimethoxybenzyl)-8-methyl-5,6,7,8-tetrahydro-[1,2,4]triaz olo[4,3-a]pyrazin-3-yl)thiazol[4,5-c]pyridine (0.4 g, 0.88 mmol) was dissolved in dichloromethane (6 mL), and trifluoroacetic acid (6 mL) was added. The mixture was stirred at 25°C for 1 hour. The reaction solution was evaporated under reduced pressure until dry, and then water (10 ml) was added. The mixture was stirred at room temperature for 10 minutes, and then filtered to remove solids. The liquid phase was lyophilized to obtain the product (R)-6-chloro-2-(8-methyl-5,6,7,8-tetrahydro-[1,2,4]triazolo[4,3-a]pyrazol-3-yl)thiazolo[ 4,5-c]pyridine trifluoroacetate (180 mg, yield: 67%).

MS m/z (ESI): 307.0[M+H]$^+$.

**Step 7: (R)-(3-(6-Chlorothiazolo [4,5-c] pyridin-2-yl)-8-methyl-5,6-dihydro- [1,2,4]triazolo [4, 3-a] pyrazin-7(8H)-yl)(4-fluorophenyl)methanone**

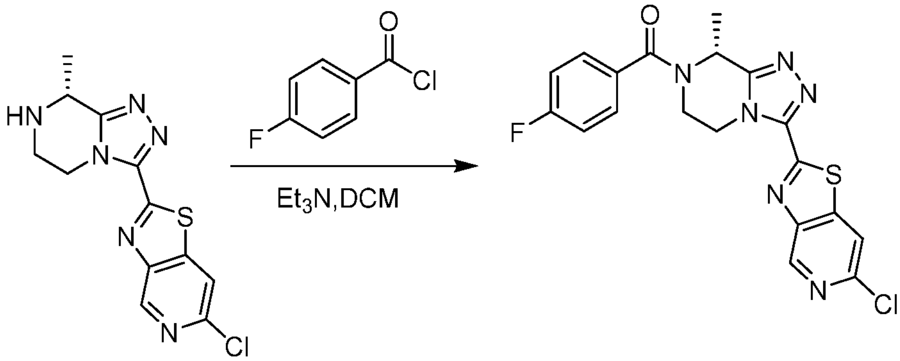


(R)-6-Chloro-2-(8-methyl-5,6,7,8-tetrahydro-[1,2,4]triazolo[4,3-a]pyrazol-3-yl)thia zolo[4,5-c]pyridine trifluoroacetate (100 mg, 0.32 mmol) and triethylamine (66 mg, 0.65 mmol) were dissolved in dichloromethane (10 mL), and a p-fluorobenzoyl chloride (78 mg, 0.49 mmol) solution was added. The mixture was stirred at 25°C for 20 min. The reaction solution was evaporated until dry. The crude product was separated by Prep-HPLC to obtain the product (R)-(3-(6-chlorothiazolo[4,5-c]pyridin-2-yl)-8-methyl-5,6-dihydro-[1,2,4]triazolo[4,3-a] pyrazin-7(8H)-yl)(4-fluorophenyl)methanone (70 mg, yield: 50%).

MS m/z (ESI): 429.1[M+H]$^+$.

$^1$H NMR (400 MHz, Chloroform-d) δ 9.11 (s, 1H), 7.95 (s, 1H), 7.55-7.44 (m, 2H), 7.23-7.14 (m, 2H), 6.02-5.63 (m, 1H), 5.12-4.99 (m, 1H), 4.81-4.46 (m, 1H), 4.46-4.32 (m, 1H), 3.68-3.49 (m, 1H), 1.78 (d, J = 6.9 Hz, 3H).

**Example 38**

**(R)-(3-(5-Chlorothiazolo[5,4-b]pyridin-2-yl)-8-methyl-5,6-dihydro-[1,2,4]triaz olo[4,3-a]pyrazin-7(8H)-yl)(4-fluorophenyl)methanone**

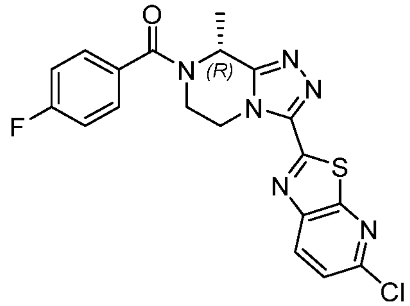

The preparation of Example **38** referred to Example **37.**

MS m/z (ESI): 429.2 $[M+H]^+$.

$^1H$ NMR (400 MHz, $CDCl_3$) δ 8.21 (d, J = 8.0 Hz, 1H), 7.51-7.48 (m, 3H), 7.20-7.16 (m, 2H), 5.79-5.75 (m, 1H), 5.06-5.01 (m, 1H), 4.63-4.34 (m, 2H), 3.59-3.53 (m, 1H), 1.77 (d, J = 8.0 Hz, 3H).

**Example 39**

**(R)-(4-Fluorophenyl)(8-methyl-3-(thiazolo[4,5-d]pyridazin-2-yl)-5,6-dihydro-[ 1,2,4]triazolo[4,3-a]pyrazin-7(8H)-yl)methanone**

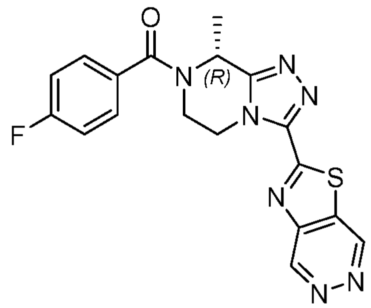

**Step 1: Preparation of ethyl 2-((5-chloropyridazin-4-yl)amino)-2-oxoacetate**

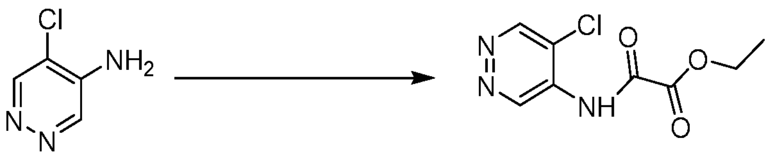

4-Amino-5-chloropyridazine (0.63 g, 4.86 mmol) was dissolved in dichloromethane (10 mL) in an ice bath, then triethylamine (0.74 g, 7.29 mmol) was added to the reaction system, and then ethyl oxalyl chloride (0.73 g, 5.35 mmol) was added dropwise. The reaction was carried out at room temperature for 3 hours, and LCMS indicated the completion of the reaction. The reaction solution was diluted with dichloromethane (20 mL) and then washed with saturated brine (10 mL×2). The organic phase was dried over anhydrous sodium sulfate, filtered and subjected to rotatory evaporation until dry to obtain the crude product ethyl 2-((5-chloropyridazin-4-yl)amino)-2-oxoacetate (1.20 g, yellow solid, crude product).

MS m/z (ESI): 230.0 $[M+H]^+$

**Step 2: Preparation of ethyl thiazolo[4,5-d]pyridazine-2-carboxylate**

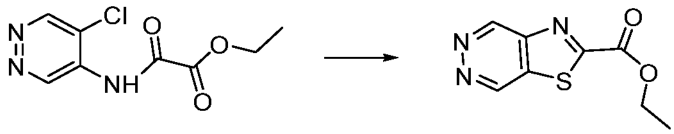

Ethyl 2-((5-chloropyridazin-4-yl)amino)-2-oxoacetate (1.20 g, yellow solid, crude product) was dissolved in toluene (20 mL) at room temperature, and then Lawesson's reagent (1.27 g, 3.14 mmol) was added to the reaction system. The reaction solution was heated to 90°C and kept for three hours. LCMS indicated the completion of the reaction. The reaction solution was cooled to room temperature and subjected to rotatory evaporation until dry. The crude product was separated by flash column chromatography (petroleum ether: ethyl acetate = 1:1) to obtain ethyl thiazolo[4,5-d]pyridazine-2-carboxylate (0.30 g, yellow solid, yield: 29.4%).

MS m/z (ESI):210.0 $[M+H]^+$

$^1H$ NMR (400 M, $CDCl_3$) δ 9.99 (s, 2H), 4.63 (q, J = 7.2 Hz, 2H), 1.53 (t, J = 7.2 Hz, 3H).

**Step 3: Preparation of thiazolo[4,5-d]pyridazine-2-carbohydrazide**

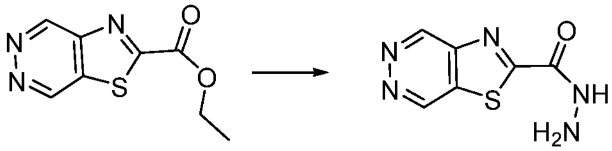

Ethyl thiazolo[4,5-d]pyridazine-2-carboxylate (0.30 g, 0.62 mmol) was dissolved in ethanol (5 mL) at room temperature, and then hydrazine hydrated (98%, 0.093 g, 1.86 mmol) was added to the reaction system. The reaction was carried out at room temperature overnight. LCMS indicated the completion of the reaction. The reaction solution was filtered. The solids were washed with petroleum ether and subjected to rotatory evaporation until dry to obtain thiazolo[4,5-d]pyridazine-2-carbohydrazide (0.06 g, yellow solid, yield: 49%).

MS m/z (ESI): 196.0 $[M+H]^+$.

**Step 4: Preparation of (R)-1-(2,4-dimethoxybenzyl)-5-ethoxy-6-methyl-1,2,3,6-tetrahydropyrazine**

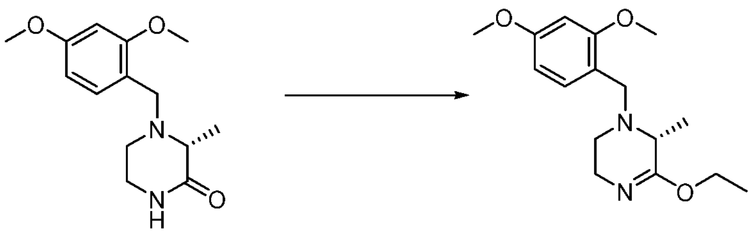

(R)-4-(2,4-Dimethoxybenzyl)-3-methylpiperazin-2-one (0.11 g, 0.42 mmol) was dissolved in dichloromethane (5 mL) in an ice bath, and then triethyloxonium tetrafluoroborate (0.32 g, 1.66 mmol) was added to the reaction system in batches. The reaction solution was stirred at room temperature for three hours. TLC showed the completion of the reaction. The reaction solution was added dropwise to an aqueous sodium hydroxide solution (2 N, 10 mL) in an ice bath. The reaction solution was stirred in an ice bath for ten minutes and separated into two phases. The organic phase was washed with saturated brine (10 mL×2), dried over anhydrous sodium sulfate, filtered and subjected to rotatory evaporation at a low temperature until dry to obtain the crude product (R)-1-(2,4-dimethoxybenzyl)-5-ethoxy-6-methyl-1,2,3,6-tetrahydropyrazine (0.17 g, colorless oil, crude product), which was used directly in the next step.

MS m/z (ESI): 293.1 $[M+H]^+$, 311.1$[M+18+H]^+$.

**Step 5: Preparation of (R)-2-(7-(2,4-dimethoxybenzyl)-8-methyl-5,6,7,8-tetrahydro-[1,2,4]triazolo[4,3-a]p yrazin-3-yl)thiazolo[4,5-d]pyridazine**

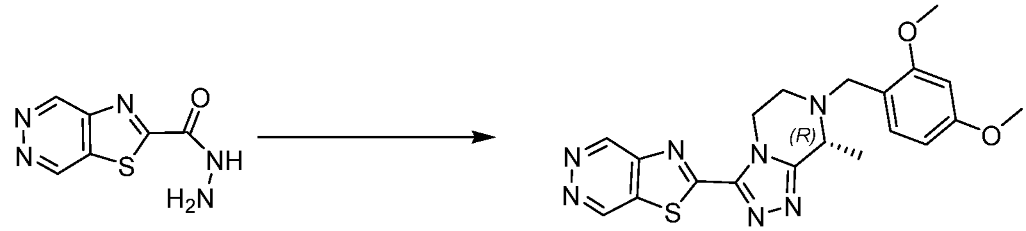

Thiazolo[4,5-d]pyridazine-2-carbohydrazide (0.06 g, 0.31 mmol) was dissolved in methanol (5 mL) at room temperature, and then a solution of (R)-1-(2,4-dimethoxybenzyl)-5-ethoxy-6-methyl-1,2,3,6-tetrahydropyrazine in methanol (0.17 g, 0.581 mmol, 2 mL methanol) was added dropwise to the reaction system. The reaction solution was heated to 70°C and stirred overnight. LCMS showed the completion of the reaction. The reaction solution was subjected to rotatory evaporation until dry. The crude product was separated by preparative thin layer chromatography (dichloromethane:methanol = 15:1) to obtain (R)-2-(7-(2,4-dimethoxybenzyl)-8-methyl-5,6,7,8-tetrahydro-[1,2,4]triazolo[4,3-a]pyraz in-3-yl)thiazolo[4,5-d]pyridazine (0.055 g, yellow solid, yield: 42%).

MS m/z (ESI): 424.1 $[M+H]^+$.

**Step 6: (R)-2-(8-Methyl-5,6,7,8-tetrahydro-[1,2,4]triazolo[4,3-a]pyrazin-3-yl)thiazolo[4,5-d ]pyridazine**

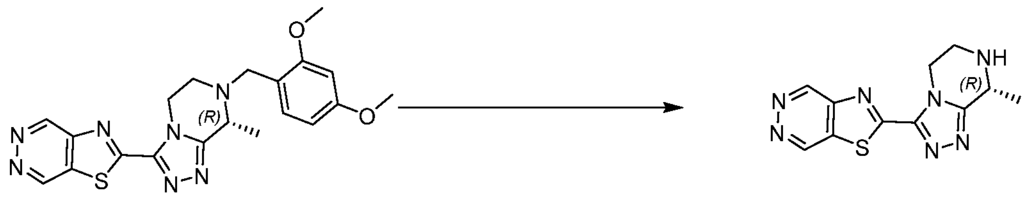

(R)-2-(7-(2,4-Dimethoxybenzyl)-8-methyl-5,6,7,8-tetrahydro-[1,2,4]triazolo[4,3-a] pyrazin-3-yl)thiazol[4,5-d]pyridazine (0.055 g, 0.13 mmol) was dissolved in dichloromethane (3 mL) at room temperature, and then trifluoroacetic acid (0.5 mL) was added to the reaction system. The reaction solution was stirred at room temperature for one hour. LCMS showed the completion of the reaction. The reaction was quenched with saturated brine (10 mL), and the reaction solution was stirred for 10 minutes at room temperature until the precipitation of white solids, and then filtered. The solids were washed with water. The pH of the solution was adjusted to 10-12 with an aqueous sodium hydroxide solution (3 M). Then the aqueous phase was extracted with dichloromethane (10 mL×2) to remove organic impurities, and the resulting aqueous solution was directly used in the next reaction.

MS m/z (ESI): 273.8 $[M+H]^+$.

**Step 7: Preparation of (R)-(4-fluorophenyl)(8-methyl-3-(thiazolo [4,5-d] pyridazin-2-yl)-5,6-dihydro-[1,2,4] triazolo [4,3-a] pyrazin-7(8H)-yl)methanone**

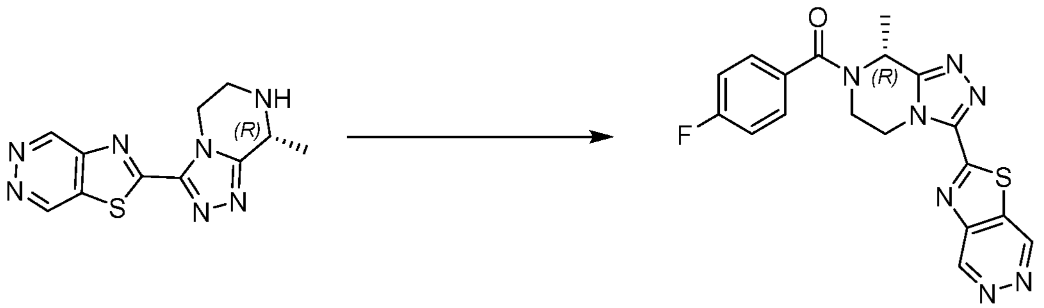

The resulting aqueous solution in the previous step was stirred at room temperature, then a saturated aqueous sodium bicarbonate solution (2 mL) was added, and then 4-fluorobenzoyl chloride (24.7 mg, 0.156 mmol) was added dropwise. The reaction solution was stirred at room temperature for one hour. LCMS indicated the completion of the reaction. The reaction solution was extracted with dichloromethane (10 mL×2). The organic phases were combined, dried over anhydrous sodium sulfate, filtered and subjected to rotatory evaporation until dry. The residue was separated by preparative high performance liquid chromatography and lyophilized to obtain (R)-2-(8-methyl-5,6,7,8-tetrahydro-[1,2,4]triazolo[4,3-a]pyrazin-3-yl)thiazolo[4,5-d]pyr idazine (9 mg, white solid, yield: 17.5%).
MS m/z (ESI): 396.0[M+H]+.
$^1$H NMR (400 MHz, MeOD) δ 10.00 (s, 1H), 9.85 (s, 1H), 7.64-7.60 (m, 2H), 7.30-7.26 (m, 2H), 5.99-5.76 (br, 1H), 5.07-5.03 (m, 1H), 4.53-4.45 (m, 1H), 4.40-4.22 (br, 1H), 3.82-3.69 (m, 1H), 1.76 (d, J = 7.2 Hz, 3H).

**Example 40**

**(R)-(4-Fluorophenyl)(8-methyl-3-(thieno[2,3-d]thiazol-2-yl)-5,6-dihydro-[1,2,4 ]triazolo[4,3-a]pyrazin-7(8H)-yl)methanone**

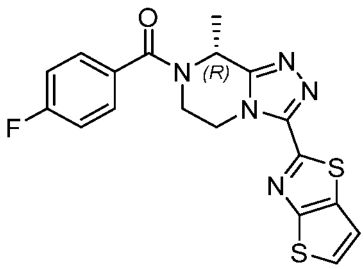

The preparation of Example **40** referred to Example **1.**
MS m/z (ESI): 400.2 [M+H]+.
$^1$H NMR (400 MHz, $CDCl_3$) δ 7.53-7.49 (m, 3H), 7.32-7.31 (m, 1H), 7.19-7.15 (m, 2H), 5.82-5.77 (m, 1H), 5.00-4.97 (m, 1H), 4.56-4.33 (m, 2H), 3.57-3.54 (m, 1H), 1.77-1.75 (m, 3H).

**Example 41**

**(R)-(4-Chlorophenyl)(8-methyl-3-(thieno[2,3-d]thiazol-2-yl)-5,6-dihydro-[1,2,4 ]triazolo[4,3-a]pyrazin-7(8H)-yl)methanone**

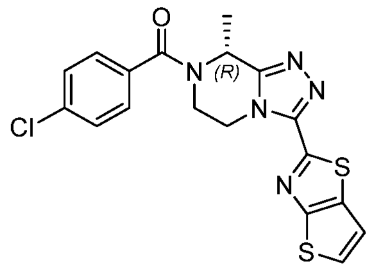

The preparation of Example **41** referred to Step 5 of Example **1.**

MS m/z (ESI): 416.2 $[M+H]^+$.

$^1H$ NMR (400 MHz, $CDCl_3$) δ 7.53-7.41 (m, 5H), 7.32-7.30 (m, 1H), 5.80-5.75 (m, 1H), 5.00-4.96 (m, 1H), 4.60-4.32 (m, 2H), 3.57-3.53 (m, 1H), 1.76 (d, J = 4.0 Hz, 3H).

**Example 42**

**(R)-(4-Fluorophenyl)(8-methyl-3-(thieno[3,2-d]thiazol-2-yl)-5,6-dihydro-[1,2,4 ]triazolo[4,3-a]pyrazin-7(8H)-yl)methanone**

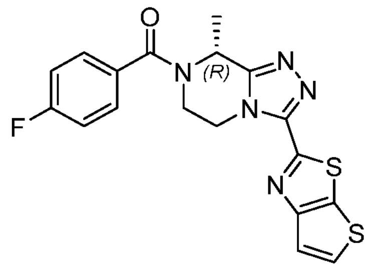

The preparation of Example **42** referred to Example **1.**

MS m/z (ESI): 400.0 $[M+H]^+$.

$^1H$ NMR (400 MHz, $CDCl_3$) δ 7.53-7.48 (m, 4H), 7.17 (t, J = 8.0 Hz , 2H), 6.03-5.50 (br, 1H), 4.98 (d, J = 12.8 Hz , 1H), 4.69-4.44 (br, 1H), 4.35-4.30 (m, 1H), 3.59-3.52 (m, 1H), 1.76 (d, J = 6.8 Hz, 3H).

**Example 43**

**(R)-(4-Chlorophenyl)(8-methyl-3-(thieno[3,2-d]thiazol-2-yl)-5,6-dihydro-[1,2,4 ]triazolo[4,3-a]pyrazin-7(8H)-yl)methanone**

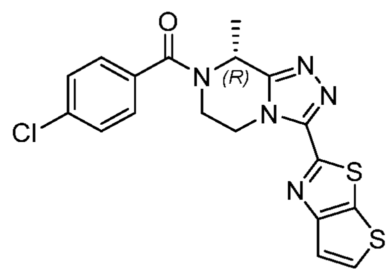

The preparation of Example **43** referred to Step 5 of Example **1.**

MS m/z (ESI): 415.9 $[M+H]^+$.

$^1H$ NMR (400 MHz, $CDCl_3$) δ 7.52 (t, J = 4.8 Hz , 1H), 7.48-7.41 (m, 5H), 6.04-5.51 (br, 1H), 5.01 (d, J = 13.6 Hz , 1H), 4.85-4.44 (br, 1H), 4.35-4.28 (m, 1H), 3.58-3.52 (m, 1H), 1.76 (d, J = 6.4 Hz, 3H).

**Example 44**

**(R)-(3,4-Dichlorophenyl)(8-methyl-3-(thieno[3,2-d]thiazol-2-yl)-5,6-dihydro-[1 ,2,4]triazolo[4,3-a]pyrazin-7(8H)-yl)methanone**

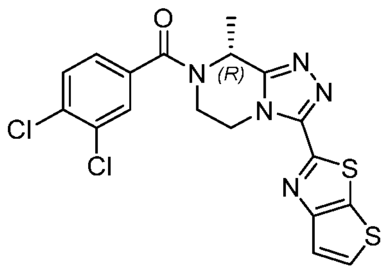

The preparation of Example **44** referred to Step 5 of Example **1.**

MS m/z (ESI): 449.9 $[M+H]^+$.

$^1$H NMR (400 MHz, $CDCl_3$) δ 7.59-7.53 (m, 3H), 7.48 (t, J = 4.8 Hz, 1H), 7.31 (t, J = 7.6 Hz , 1H), 6.05-5.61 (br, 1H), 5.03 (d, J = 12.0 Hz , 1H), 4.76-4.44 (br, 1H), 4.36-4.28 (m, 1H), 3.65-3.51 (br, 1H), 1.77 (d, J = 6.0 Hz, 3H).

**Example 45**

**(R)-(4-Chlorophenyl)(8-methyl-3-(pyrazolo[1,5-a]pyridin-2-yl)-5,6-dihydro-[l, 2,4]triazolo[4,3-a]pyrazin-7(8H)-yl)methanone**

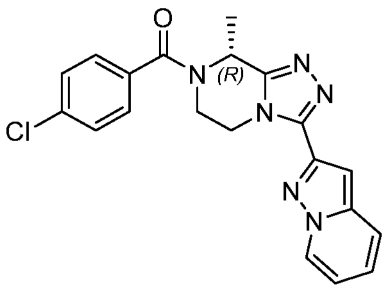

The preparation of Example **45** referred to Example **1.**

MS m/z (ESI): 393.2 $[M+H]^+$.

$^1$H NMR (400 MHz, Chloroform-d) δ 8.42 (d, J = 7.1 Hz, 1H), 7.61 (d, J = 8.9 Hz, 1H), 7.47-7.41 (m, 4H), 7.23 (s, 1H), 7.20-7.17 (m, 1H), 6.89-6.81 (m, 1H), 5.86-5.61 (m, 1H), 4.95 (d, J = 13.8 Hz, 1H), 4.78-4.49 (m, 1H), 4.36-4.24 (m, 1H), 3.60-3.48 (m, 1H), 1.75 (d, J = 5.7 Hz, 3H).

**Example 46**

**(R)-(3,4-Dichlorophenyl)(8-methyl-3-(pyrazolo[1,5-a]pyridin-2-yl)-5,6-dihydro -[1,2,4]triazolo[4,3-a]pyrazin-7(8H)-yl)methanone**

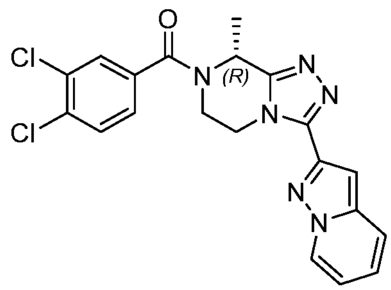

The preparation of Example **46** referred to Step 5 of Example **1.**

MS m/z (ESI): 427.2 $[M+H]^+$.

$^1H$ NMR (400 MHz, Chloroform-d) δ 8.42 (d, J = 7.1 Hz, 1H), 7.63-7.56 (m, 3H), 7.31 (d, J = 8.3 Hz, 2H), 7.22-7.15 (m, 1H), 6.89-6.81 (m, 1H), 5.75 (br s, 1H), 4.97 (d, J = 14.2 Hz, 1H), 4.86-4.70 (m, 1H), 4.36-4.24 (m, 1H), 3.62-3.50 (m, 1H), 1.76 (d, J = 6.2 Hz, 3H).

**Example 47**

**(R)-(4-Fluorophenyl)(8-methyl-3-(7-methylpyrazolo[1,5-a]pyridin-2-yl)-5,6-di hydro-[1,2,4]triazolo[4,3-a]pyrazin-7(8H)-yl)methanone**

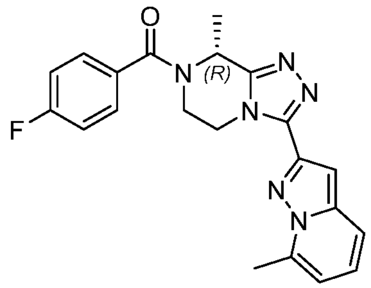

The preparation of Example **47** referred to Example **1.**

MS m/z (ESI): 391.1 $[M+H]^+$.

$^1H$ NMR (400 MHz, $CDCl_3$) δ 7.55-7.48 (m , 3H), 7.25 (s, 1H), 7.19-7.13 (m, 3H), 6.71 (t, J = 6.4 Hz, 1H), 5.84-5.51 (br, 1H), 5.00 (d, J = 14.0 Hz , 1H), 4.75-4.46 (br, 1H), 4.42-4.28 (m, 1H), 3.61-3.46 (m, 1H) ,2.75 (s, 3H), 1.77 (d, J = 6.4 Hz, 3H).

**Example 48**

**(R)-(4-Chlorophenyl)(8-methyl-3-(7-methylpyrazolo[1,5-a]pyridin-2-yl)-5,6-di hydro-[1,2,4]triazolo[4,3-a]pyrazin-7(8H)-yl)methanone**

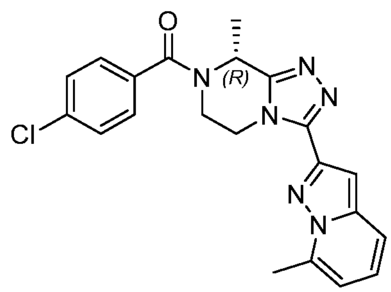

The preparation of Example 48 referred to Step 5 of Example 1.

MS m/z (ESI): 407.1 [M+H]$^+$.

$^1$H NMR (400 MHz, $CDCl_3$) δ 7.53 (d, J = 8.8 Hz, 1H), 7.45 (q, J = 8.0 Hz, 4H), 7.31 (s, 1H), 7.15 (d, J = 8.0 Hz, 1H), 6.72 (d, J = 6.4 Hz, 1H), 5.88-5.49 (br, 1H), 5.00 (d, J = 12.4 Hz , 1H), 4.86-4.50 (br, 1H), 4.42-4.27 (m, 1H), 3.63-3.44 (m, 1H) ,2.75 (s, 3H), 1.76 (d, J = 6.4 Hz, 3H).

**Example 49**

**(R)-(3,4-Dichlorophenyl)(8-methyl-3-(7-methylpyrazolo[1,5-a]pyridin-2-yl)-5, 6-dihydro-[1,2,4]triazolo[4,3-a]pyrazin-7(8H)-yl)methanone**

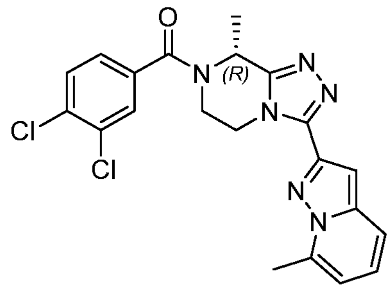

The preparation of Example **49** referred to Step 5 of Example **1.**

MS m/z (ESI): 441.0 [M+H]$^+$.

$^1$H NMR (400 MHz, DMSO-$d_6$) δ 7.85 (s, 1H), 7.77 (d, J = 8.0 Hz, 1H), 7.70 (d, J = 8.8 Hz, 1H), 7.53 (d, J = 8.0 Hz, 1H), 7.28 (t, J = 8.0 Hz, 1H), 7.17 (s, 1H), 6.93 (d, J = 6.4 Hz, 1H), 5.90-5.70 (br, 1H), 4.78 (d, J = 14.4 Hz , 1H), 4.32-4.25 ( m, 1H), 3.93-3.79 (br, 1H), 3.70-3.58 (br, 1H), 2.72(s, 3H), 1.62 (d, J = 6.4 Hz, 3H).

**Example 50**

**(R)-(4-Fluorophenyl-2,3,5,6-d4)(8-methyl-3-(3-(methyl-d3)-1,2,4-thiadiazol-5-yl)-5,6-dihydro-[1,2,4]triazolo[4,3-a]pyrazin-7(8H)-yl)methanone**

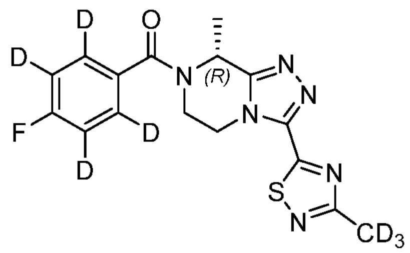

The preparation of Example **50** referred to Step 5 of Example **1.**

MS m/z (ESI): 366.1[M+H]$^+$.

$^1$H NMR (400 MHz, Chloroform-d) δ 5.97-5.56 (m, 1H), 5.11-4.79 (m, 1H), 4.73-4.147(m, 1H), 4.41-4.12 (m, 1H), 3.73-3.37 (m, 1H), 1.90-1.69 (m, 3H).

**Example 51**

**(R)-(4-Chlorophenyl)(8-methyl-3-(3-(methyl-d3)-1,2,4-thiadiazol-5-yl)-5,6-dih ydro-[1,2,4]triazolo[4,3-a]pyrazin-7(8H)-yl)methanone**

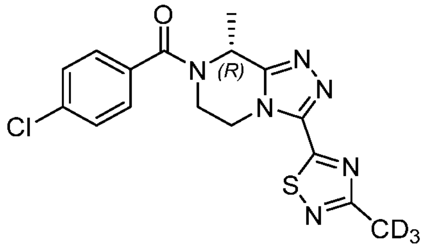

The preparation of Example **51** referred to Step 5 of Example **1.**

MS m/z (ESI): 378.2 $[M+H]^+$.

$^1H$ NMR (400 MHz, Chloroform-d) δ 7.50-7.38 (m, 4H), 5.94-5.70 (m, 1H), 4.94 (d, J = 13.6 Hz, 1H), 4.72-4.47 (m, 1H), 4.33-4.20 (m, 1H), 3.63-3.48 (m, 1H), 1.76 (d, J = 6.3 Hz, 3H).

**Example 52**

**(R)-(3,4-Dichlorophenyl)(8-methyl-3-(3-(methyl-d3)-1,2,4-thiadiazol-5-yl)-5,6-dihydro-[1,2,4]triazolo[4,3-a]pyrazin-7(8H)-yl)methanone**

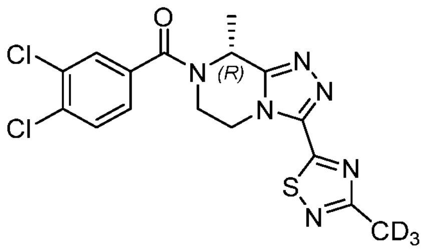

The preparation of Example **52** referred to Step 5 of Example **1.**

MS m/z (ESI): 412.2 $[M+H]^+$.

$^1H$ NMR (400 MHz, Chloroform-d) δ 7.58 (d, J = 9.3 Hz, 2H), 7.32 (d, J = 8.4 Hz, 1H), 5.81 (br s, 1H), 4.97 (d, J = 13.0 Hz, 1H), 4.65-4.44 (m, 1H), 4.35-4.23 (m, 1H), 3.64-3.50 (m, 1H), 1.79 (d, J = 6.7 Hz, 3H).

**Example 53**

**(R)-(8-Methyl-3-(3-(methyl-d3)-1,2,4-thiadiazol-5-yl)-5,6-dihydro-[1,2,4]triazo lo[4,3-a]pyrazin-7(8H)-yl)(4-(thien-2-yl)phenyl)methanone**

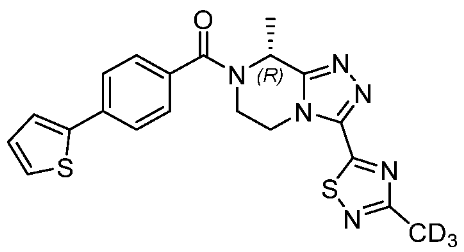

The preparation of Example **53** referred to Step 5 of Example **1.**

MS m/z (ESI): 426.2 $[M+H]^+$.

$^1H$ NMR (400 MHz, Chloroform-d) δ 7.71 (d, J = 7.8 Hz, 2H), 7.49 (d, J = 7.9 Hz, 2H), 7.43-7.33 (m, 2H), 7.13 (s, 1H), 5.93 (br s, 1H), 4.96-4.93 (m, 1H), 4.73-4.55 (m, 1H), 4.38-4.25 (m, 1H), 3.65-3.50 (m, 1H), 1.80 (d, J = 6.9 Hz, 3H).

**Example 54**

**(R)-(3-(3-Chloro-1,2,4-thiadiazol-5-yl)-8-methyl-5,6-dihydro-[1,2,4]triazolo[4, 3-a]pyrazin-7(8H)-yl)(4-fluorophenyl)methanone**

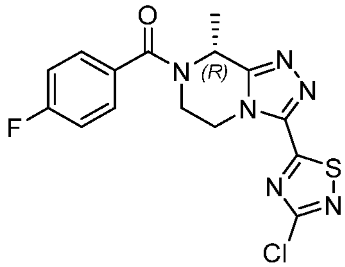

**Step 1: 3-Chloro-5-(1-ethoxyvinyl)-1,2,4-thiadiazole**

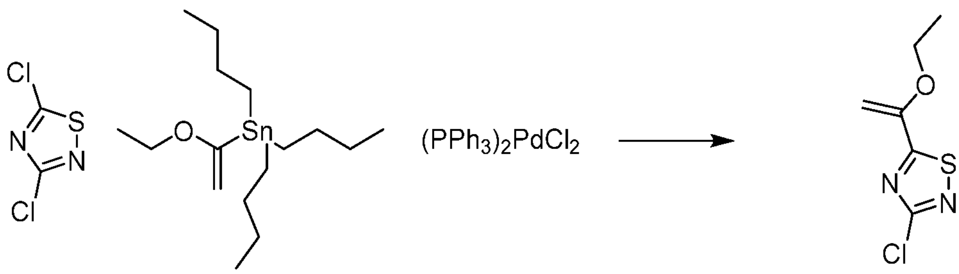


Bis(triphenylphosphine)palladium dichloride (1.13 g, 1.61 mmol) was added to a solution of 3,5-dichloro-1,2,4-thiadiazole (5 g, 32.26 mmol) and tributyl(1-ethoxyvinyl)stannane (10.48 g, 29.03 mmol) in anhydrous DMF (20 ml). The reaction solution was heated to 75°C for 14 hours under a nitrogen atmosphere and cooled. The reaction was quenched with an aqueous potassium fluoride solution (50 ml), and the reaction solution was filtered to remove the solids. The liquid phase was extracted with ethyl acetate (75 ml×2). The organic phase was washed with saturated brine (20 ml×6) and evaporated until dry to obtain the crude product. The crude product was separated by column (petroleum ether: ethyl acetate=99:1) to obtain the product 3-chloro-5-(1-ethoxyvinyl)-1,2,4-thiadiazole (4.3 g, yield: 70%).

$^1$H NMR (400 MHz, Chloroform-d) δ 5.52 (d, J = 3.1 Hz, 1H), 4.58 (d, J = 3.1 Hz, 1H), 4.02 (q, J = 7.0 Hz, 2H), 1.43 (t, J = 7.0 Hz, 3H).

**Step 2: Ethyl 3-chloro-1,2,4-thiadiazole-5-carboxylate**

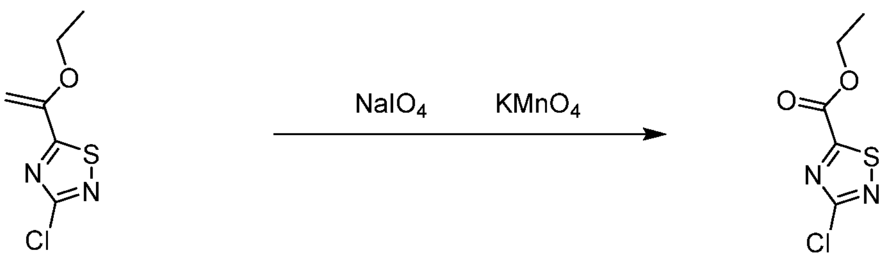


A solution of sodium periodate (8.30 g, 38.81 mmol) in water (35 ml) was added to a solution of 3-chloro-5-(1-ethoxyvinyl)-1,2,4-thiadiazole (3.7 g, 19.41 mmol) in dioxane (70 ml). The reaction solution was stirred for 2 minutes, and then potassium permanganate (460.05 mg, 2.91 mmol) was added to the reaction solution. The reaction solution

was stirred at room temperature overnight and extracted with ethyl acetate (100 mL×3). The organic phases were combined, washed with saturated brine (50 mL×2), dried over anhydrous sodium sulfate and concentrated under reduced pressure. The residue was purified by silica gel flash chromatography (elution: petroleum ether: ethyl acetate = 100:0 to 95:5) to obtain the target product ethyl 3-chloro-1,2,4-thiadiazole-5-carboxylate (1 g, yield: 27.03%).

$^1H$ NMR (400 MHz, Chloroform-d) δ 4.54 (q, J = 7.1 Hz, 2H), 1.47 (t, J = 7.2 Hz, 3H).

Step 3: 3-Chloro-1,2,4-thiadiazole-5-carbohydrazide

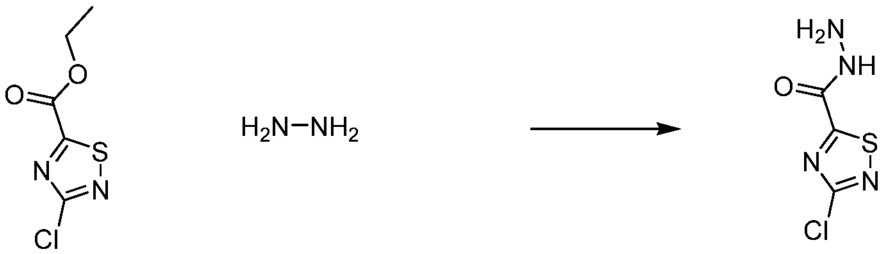

Hydrazine hydrate (229 mg, 3.89 mmol, 85% aqueous solution) was added to a solution of ethyl 3-chloro-1,2,4-thiadiazole-5-carboxylate (0.5 g, 2.6 mmol) in ethanol (10 ml). The reaction solution was stirred at room temperature for 1 hour and filtered to obtain the solid product 3-chloro-1,2,4-thiadiazole-5-carbohydrazide (440 mg, yield: 95%).

MS m/z (ESI):179.0 $[M+H]^+$.

**Step 4: (SR)-4-[(2,4-Dimethoxyphenyl)methyl]-6-ethoxy-5-methyl-3,5-dihydro-2H-pyrazin e**

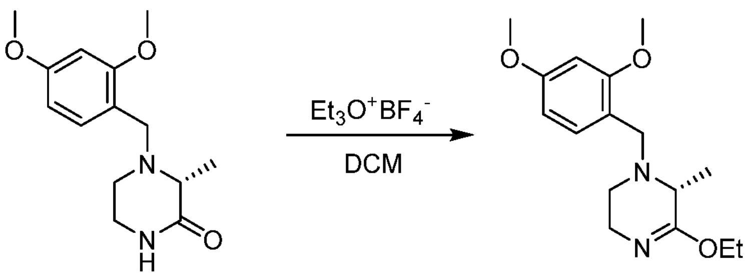


(3R)-4-[(2,4-Dimethoxyphenyl)methyl]-3-methyl-piperazin-2-one (0.6 g, 2.27 mmol) was dissolved in dichloromethane (7 mL), and triethyloxonium tetrafluoroborate (1.08 g, 5.67 mmol) was added at 0°C in three batches with an interval of 10 minutes. The mixture was stirred at 25°C for 3 hours. The reaction solution was added to a cold aqueous sodium hydroxide solution (2 M, 10 ml). The mixture was separated into two phases, and the aqueous phase was extracted with dichloromethane (10 mL×2). The organic phases were combined, dried over anhydrous sodium sulfate and concentrated under reduced pressure to obtain the target product (5R)-4-[(2,4-dimethoxyphenyl)methyl]-6-ethoxy-5-methyl-3,5-dihydro-2H-pyrazine (0.65 g) as a colorless oil. The crude product was used directly in the next step.

MS m/z (ESI): 293.2$[M+H]^+$.

**Step 5: 3-Chloro-5-[(8R)-7-[(2,4-dimethoxyphenyl)methyl]-8-methyl-6,8-dihydro-5H-[1,2,4 ]triazolo[4,3-a]pyrazin-3-yl]-1,2,4-thiadiazole**

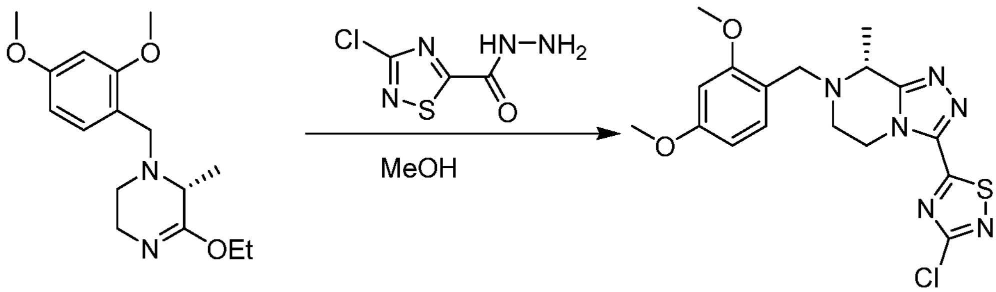


(5R)-4-[(2,4-Dimethoxyphenyl)methyl]-6-ethoxy-5-methyl-3,5-dihydro-2H-pyrazi ne (0.66 g, 2.26 mmol) and 3-chloro-1,2,4-thiadiazole-5-carbohydrazide (322.54 mg, 1.81 mmol) were dissolved in MeOH (10 mL). The mixture was stirred at 80°C for 14 hours under a nitrogen atmosphere. The reaction solution was concentrated under reduced pressure. The residue was purified by silica gel flash chromatography (elution: dichloromethane:methanol=100:0 to 95:5) to obtain the target product 3-chloro-5-[(8R)-7-[(2,4-dimethoxyphenyl)methyl]-8-methyl-6,8-dihydro-5H-[1,2,4]tri azolo[4,3-a]pyrazin-3-yl]-1,2,4-thiadiazole (0.22 g, yield: 23.95%) as a light yellow solid.
MS m/z (ESI): 406.8[M+H]$^+$.

**Step 6: 3-Chloro-5-[(8R)-8-methyl-5,6,7,8-tetrahydro-[1,2,4]triazolo[4,3-a]pyrazin-3-yl]-1, 2,4-thiadiazole**

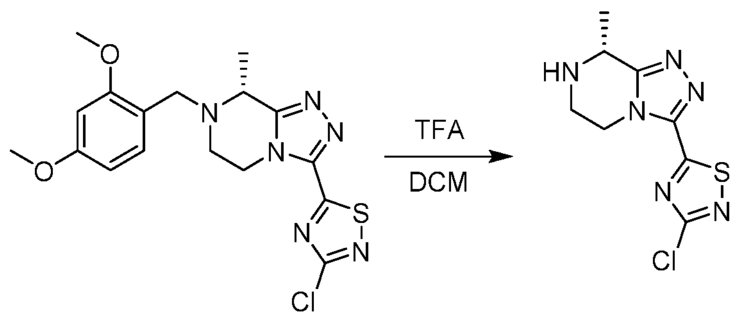


3-Chloro-5-[(8R)-7-[(2,4-dimethoxyphenyl)methyl]-8-methyl-6,8-dihydro-5H-[1,2 ,4]triazolo[4,3-a]pyrazin-3-yl]-1,2,4-thiadiazole (0.22 g, 540.69 μmol) was dissolved in dichloromethane (1.5 mL), and trifluoroacetic acid (3 mL) was added. The mixture was stirred at 25°C for 1 hour. The reaction solution was evaporated under reduced pressure until dry, and then water (7 ml) was added. The mixture was stirred at room temperature for 10 minutes, and then filtered to remove solids. The liquid phase was lyophilized to obtain the product 3-chloro-5-[(8R)-8-methyl-5,6,7,8-tetrahydro-[1,2,4]triazolo[4,3-a]pyrazin-3-yl]-1,2,4-t hiadiazole trifluoroacetate (0.2 g, yield: 99.77%).
MS m/z (ESI): 257.0[M+H]$^+$.

**Step 7: The preparation of Example 54 referred to Step 5 of Example 1.**

MS m/z (ESI): 379.0[M+H]$^+$.
$^1$H NMR (400 MHz, Chloroform-d) δ 7.54-7.43 (m, 2H), 7.23-7.12 (m, 2H), 5.95-5.69 (m, 1H), 4.95-4.80 (m, 1H), 4.73-4.44 (m, 1H), 4.36-4.18 (m, 1H), 3.64-3.45 (m, 1H), 1.76 (d, J = 6.8 Hz, 3H).

**Example 55**

**(R)-(3-(3-Chloro-1,2,4-thiadiazol-5-yl)-8-methyl-5,6-dihydro-[1,2,4]triazolo [4, 3-a]pyrazin-7(8H)-yl)(4-fluorophenyl-2,3,5,6-d4)methanone**

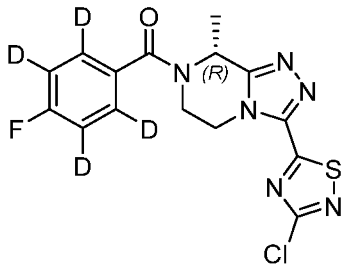

**Step 1: 3-Chloro-5-(1-ethoxyvinyl)-1,2,4-thiadiazole**

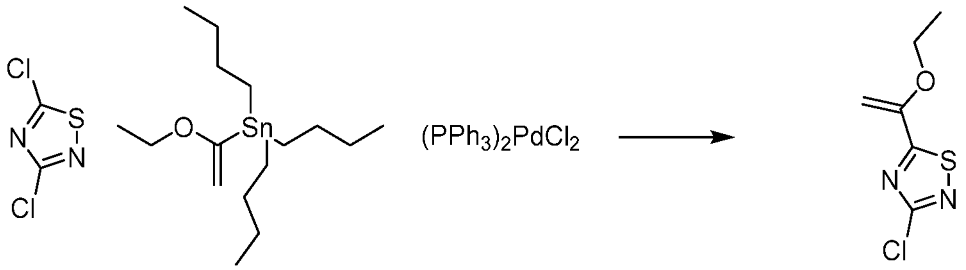


Bis(triphenylphosphine)palladium dichloride (1.13 g, 1.61 mmol) was added to a solution of 3,5-dichloro-1,2,4-thiadiazole (5 g, 32.26 mmol) and tributyl(1-ethoxyvinyl)stannane (10.48 g, 29.03 mmol) in anhydrous DMF (20 ml). The reaction solution was heated to 75°C for 14 hours under a nitrogen atmosphere and cooled. The reaction was quenched with an aqueous potassium fluoride solution (50 ml), and the reaction solution was filtered to remove solids. The liquid phase was extracted with ethyl acetate (75 ml×2). The organic phase was washed with saturated brine (20 ml×6) and evaporated until dry to obtain the crude product. The crude product was separated by column (petroleum ether: ethyl acetate=99:1) to obtain the product 3-chloro-5-(1-ethoxyvinyl)-1,2,4-thiadiazole (4.3 g, yield: 70%).

$^1$H NMR (400 MHz, Chloroform-d) δ 5.52 (d, J = 3.1 Hz, 1H), 4.58 (d, J = 3.1 Hz, 1H), 4.02 (q, J = 7.0 Hz, 2H), 1.43 (t, J = 7.0 Hz, 3H).

**Step 2: Ethyl 3-chloro-1,2,4-thiadiazole-5-carboxylate**

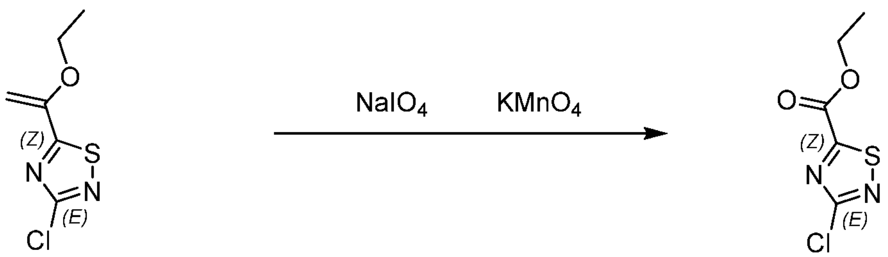


A solution of sodium periodate (8.30 g, 38.81 mmol) in water (35 ml) was added to a solution of 3-chloro-5-(1-ethoxyvinyl)-1,2,4-thiadiazole (3.7 g, 19.41 mmol) in dioxane (70 ml). The reaction solution was stirred for 2 minutes, and then potassium permanganate (460.05 mg, 2.91 mmol) was added to the reaction solution. The reaction solution was stirred at room temperature overnight and extracted with ethyl acetate (100 mL×3). The organic phases were combined, washed with saturated brine (50 mL×2), dried over anhydrous sodium sulfate and concentrated under reduced pressure. The residue was purified by silica gel flash chromatography (elution: petroleum ether: ethyl acetate = 100:0 to 95:5) to obtain the target product ethyl 3-chloro-1,2,4-thiadiazole-5-carboxylate (1 g, yield: 27.03%).

$^1$H NMR (400 MHz, Chloroform-d) δ 4.54 (q, J = 7.1 Hz, 2H), 1.47 (t, J = 7.2 Hz, 3H).

**Step 3: 3-Chloro-1,2,4-thiadiazole-5-carbohydrazide**

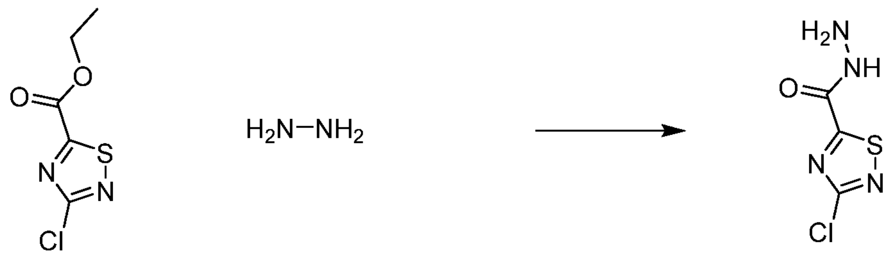

Hydrazine hydrate (229 mg, 3.89 mmol, 85% aqueous solution) was added to a solution of ethyl 3-chloro-1,2,4-thiadiazole-5-carboxylate (0.5 g, 2.6 mmol) in ethanol (10 ml). The reaction solution was stirred at room temperature for 1 hour and filtered to obtain the solid product 3-chloro-1,2,4-thiadiazole-5-carbohydrazide (440 mg, yield: 95%).

MS m/z (ESI):179.0 $[M+H]^+$.

**Step 4: (SR)-4-[(2,4-Dimethoxyphenyl)methyl]-6-ethoxy-5-methyl-3,5-dihydro-2H-pyrazin e**

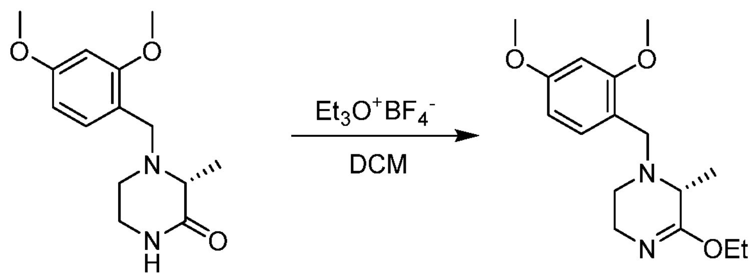


(3R)-4-[(2,4-Dimethoxyphenyl)methyl]-3-methyl-piperazin-2-one (0.6 g, 2.27 mmol) was dissolved in dichloromethane (7 mL), and triethyloxonium tetrafluoroborate (1.08 g, 5.67 mmol) was added at 0°C in three batches with an interval of 10 minutes. The mixture was stirred at 25°C for 3 hours. The reaction solution was added to a cold aqueous sodium hydroxide solution (2 M, 10 ml). The mixture was separated into two phases, and the aqueous phase was extracted with dichloromethane (10 mL×2). The organic phases were combined, dried over anhydrous sodium sulfate and concentrated under reduced pressure to obtain the target product (5R)-4-[(2,4-dimethoxyphenyl)methyl]-6-ethoxy-5-methyl-3,5-dihydro-2H-pyrazine (0.65 g) as a colorless oil. The crude product was directly used in the next step.

MS m/z (ESI): 293.2$[M+H]^+$.

**Step 5: 3-Chloro-5-[(8R)-7-[(2,4-dimethoxyphenyl)methyl]-8-methyl-6,8-dihydro-5H-[1,2,4 ]triazolo[4,3-a]pyrazin-3-yl]-1,2,4-thiadiazole**

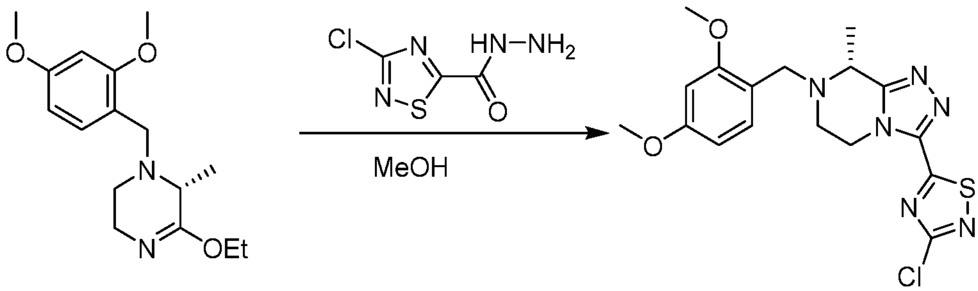

(5R)-4-[(2,4-Dimethoxyphenyl)methyl]-6-ethoxy-5-methyl-3,5-dihydro-2H-pyrazi ne (0.66 g, 2.26 mmol) and 3-chloro-1,2,4-thiadiazole-5-carbohydrazide (322.54 mg, 1.81 mmol) were dissolved in MeOH (10 mL). The mixture was stirred at 80°C for 14 hours under a nitrogen atmosphere. The reaction solution was concentrated under reduced pressure. The residue was purified by silica gel flash chromatography (elution: dichloromethane:methanol= 100:0 to 95:5) to obtain the target product 3-chloro-5-[(8R)-7-[(2,4-dimethoxyphenyl)methyl]-8-methyl-6,8-dihydro-5H-[1,2,4]tri azolo[4,3-a]pyrazin-3-yl]-1,2,4-thiadiazole (0.22 g, yield: 23.95%) as a light yellow solid.

MS m/z (ESI): 406.8[M+H]+.

**Step 6: 3-Chloro-5-[(8R)-8-methyl-5,6,7,8-tetrahydro-[1,2,4]triazolo[4,3-a]pyrazin-3-yl]-1, 2,4-thiadiazole**

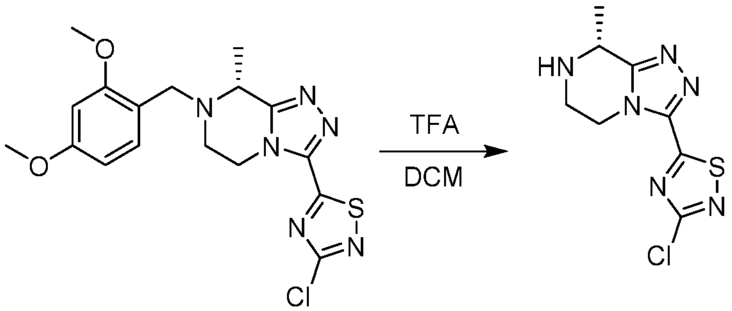


3-Chloro-5-[(8R)-7-[(2,4-dimethoxyphenyl)methyl]-8-methyl-6,8-dihydro-5H-[1,2 ,4]triazolo[4,3-a]pyrazin-3-yl]-1,2,4-thiadiazole (0.22 g, 540.69 μmol) was dissolved in dichloromethane (1.5 mL), and trifluoroacetic acid (3 mL) was added. The mixture was stirred at 25°C for 1 hour. The reaction solution was evaporated under reduced pressure until dry, and then water (7 ml) was added. The mixture was stirred at room temperature for 10 minutes and then filtered to remove solids. The liquid phase was lyophilized to obtain the product 3-chloro-5-[(8R)-8-methyl-5,6,7,8-tetrahydro-[1,2,4]triazolo[4,3-a]pyrazin-3-yl]-1,2,4-t hiadiazole trifluoroacetate (0.2 g, yield: 99.77%).

MS m/z (ESI): 257.0[M+H]+.

**Step 7: 4-Fluoro-2,3,5,6-d4 benzoic acid**

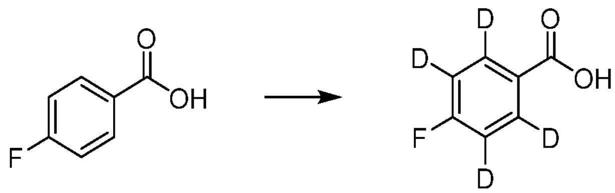

10% Pd/C (0.73 g, containing 50% w/w of water) was added to a solution of p-fluorobenzoic acid (1.8 g, 12.9 mmol) in isopropanol (50 mL) and deuterium water (100 mL) under a nitrogen atmosphere. The reaction solution was stirred at 100°C for 3 days, cooled, and extracted with ethyl acetate (30 mL×3). The organic phase was dried over anhydrous sodium sulfate and concentrated under reduced pressure to obtain the target product 4-fluoro-2,3,5,6-d4 benzoic acid (1.5 g, yield: 81%).

MS m/z (ESI): 143.0[M-H]-

**Step 8: (R)-(3-Chloro-1,2,4-thiadiazol-5-yl)-8-methyl-5,6-dihydro-[1,2,4]triazolo[4,3-a]pyr azin-7(8H)-yl)(4-fluorophenyl-2,3,5,6-d4)methanone**

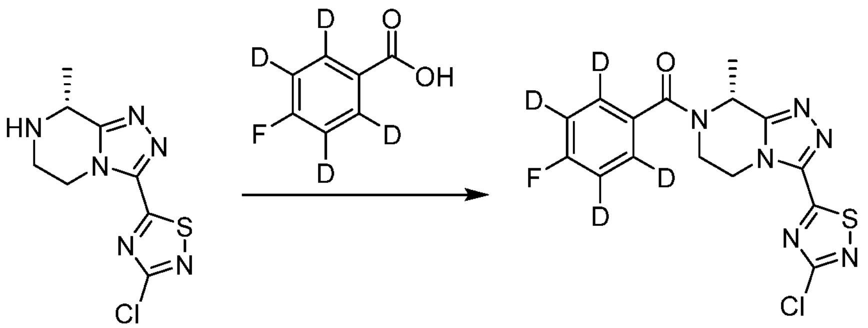

2-(7-Azabenzotriazol-1-yl)-N,N,N',N'-tetramethyluronium hexafluorophosphate (32 mg, 84 μmol) was added to a solution of 3-chloro-5-[(8R)-8-methyl-5,6,7,8-tetrahydro-[1,2,4]triazolo[4,3-a]pyrazin-3-yl]-1,2,4-t hiadiazole trifluoroacetate (20 mg, 56 μmol), 4-fluoro-2,3,5,6-d4 benzoic acid (10 mg, 68 μmol) and N,N-diisopropylethylamine (22 mg, 169 μmol) in N,N-dimethylformamide (1.5 ml). The reaction solution was stirred at room temperature for 16 hours. The crude product was separated directly by Prep-HPLC to obtain the product (R)-(3-(3-chloro-1,2,4-thiadiazol-5-yl)-8-methyl-5,6-dihydro-[1,2,4]triazolo[4,3-a]pyraz in-7(8H)-yl)(4-fluorophenyl-2,3,5,6-d4)methanone (10 mg, yield: 46%).

MS m/z (ESI): 383.1[M+H]+.

$^1$H NMR (400 MHz, Chloroform-d) δ 5.97-5.68 (m, 1H), 4.94-4.81 (m, 1H), 4.74-4.39 (m, 1H), 4.37-4.20 (m, 1H), 3.64-3.46 (m, 1H), 1.76 (d, J = 6.8 Hz, 3H).

**Example 56**

**(R)-(3-(3-Chloro-1,2,4-thiadiazol-5-yl)-8-methyl-5,6-dihydro-[1,2,4]triazolo[4,3-a]p yrazin-7(8H)-yl)(4-fluorophenyl-3-d)methanone**

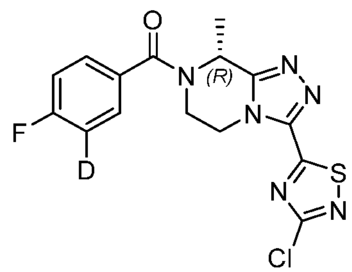

**Synthesis of the intermediate 3-chloro-5-[(8R)-8-methyl-5,6,7,8-tetrahydro-[1,2,4]triazolo[4,3-a]pyrazin-3-yl]-1,2 ,4-thiadiazole:**

**Step 1: 3-Chloro-5-(1-ethoxyvinyl)-1,2,4-thiadiazole**

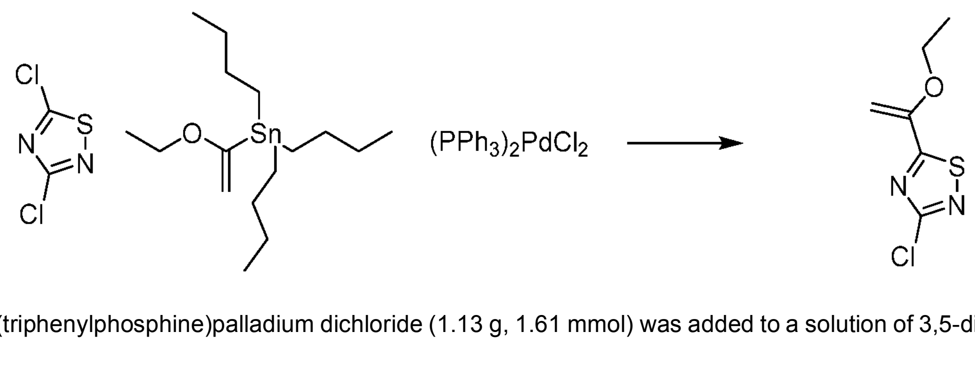


Bis(triphenylphosphine)palladium dichloride (1.13 g, 1.61 mmol) was added to a solution of 3,5-dichloro-1,2,4-thiadiazole (5 g, 32.26 mmol) and tributyl(1-ethoxyvinyl)stannane (10.48 g, 29.03 mmol) in anhydrous DMF (20 ml). The reaction solution was heated to 75°C and kept for 14 hours under a nitrogen atmosphere and then cooled. The reaction was quenched with an aqueous potassium fluoride solution (50 ml), and the reaction solution was filtered to remove the solids. The liquid phase was extracted with ethyl acetate (75 ml×2). The organic phase was washed with saturated brine (20 ml$\times_6$) and subjected to rotatory evaporation until dry to obtain the crude product. The crude product was separated by column (petroleum ether: ethyl acetate=99:1) to obtain the product 3-chloro-5-(1-ethoxyvinyl)-1,2,4-thiadiazole (4.3 g, yield: 70%).

$^1$H NMR (400 MHz, Chloroform-d) δ 5.52 (d, J = 3.1 Hz, 1H), 4.58 (d, J = 3.1 Hz, 1H), 4.02 (q, J = 7.0 Hz, 2H), 1.43 (t, J = 7.0 Hz, 3H).

**Step 2: Ethyl 3-chloro-1,2,4-thiadiazole-5-carboxylate**

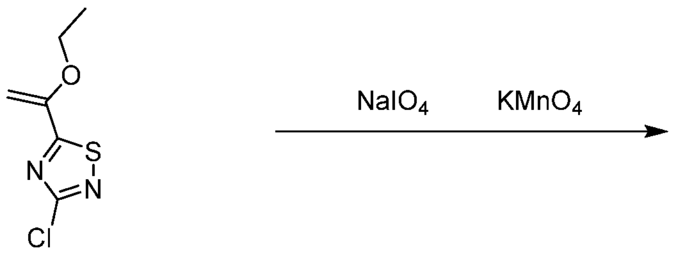


A solution of sodium periodate (8.30 g, 38.81 mmol) in water (35 ml) was added to a solution of 3-chloro-5-(1-ethoxyvinyl)-1,2,4-thiadiazole (3.7 g, 19.41 mmol) in dioxane (70 ml). The reaction solution was stirred for 2 minutes, and then potassium permanganate (460.05 mg, 2.91 mmol) was added to the reaction solution. The reaction solution was stirred at room temperature overnight and extracted with ethyl acetate (100 mL×3). The organic phases were combined, washed with saturated brine (50 mL×2), dried over anhydrous sodium sulfate and concentrated under reduced pressure. The residue was purified by silica gel flash chromatography (elution: petroleum ether: ethyl acetate = 100:0 to 95:5) to obtain the target product ethyl 3-chloro-1,2,4-thiadiazole-5-carboxylate (1 g, yield: 27.03%).

$^1$H NMR (400 MHz, Chloroform-d) δ 4.54 (q, J = 7.1 Hz, 2H), 1.47 (t, J = 7.2 Hz, 3H).

**Step 3: 3-Chloro-1,2,4-thiadiazole-5-carbohydrazide**

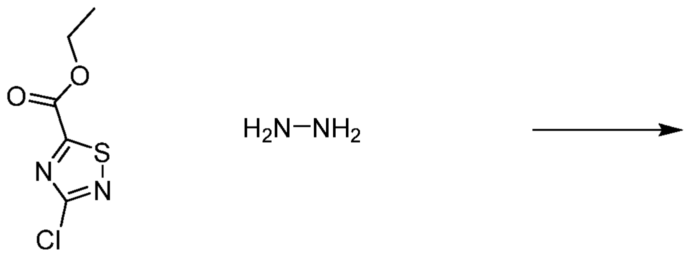


Hydrazine hydrate (229 mg, 3.89 mmol, 85% aqueous solution) was added to a solution of ethyl 3-chloro-1,2,4-thiadiazole-5-carboxylate (0.5 g, 2.6 mmol) in ethanol (10 ml). The reaction solution was stirred at room temperature for 1 hour and filtered to obtain the solid product 3-chloro-1,2,4-thiadiazole-5-carbohydrazide (440 mg, yield: 95%).

MS m/z (ESI):179.0 [M+H]$^+$.

**Step 4: (SR)-4-[(2,4-Dimethoxyphenyl)methyl]-6-ethoxy-5-methyl-3,5-dihydro-2H-pyrazin e**

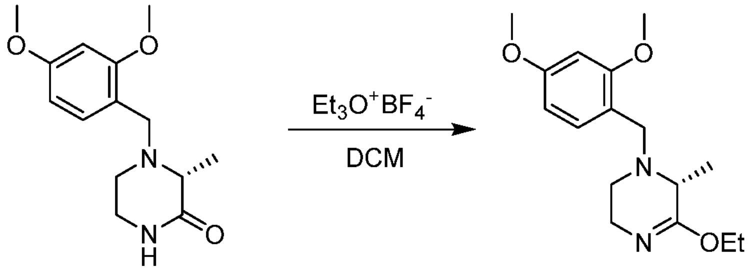


(3R)-4-[(2,4-Dimethoxyphenyl)methyl]-3-methyl-piperazin-2-one (0.6 g, 2.27 mmol) was dissolved in dichloromethane (7 mL), and triethyloxonium tetrafluoroborate (1.08 g, 5.67 mmol) was added at 0°C in three batches with an interval of 10 minutes. The mixture was stirred at 25°C for 3 hours. The reaction solution was added to a cold aqueous sodium hydroxide solution (2 M, 10 ml). The mixture was separated into two phases, and the aqueous phase was extracted with dichloromethane (10 mL×2). The organic phases were combined, dried over anhydrous sodium sulfate and concentrated under reduced pressure to obtain the target product (5R)-4-[(2,4-dimethoxyphenyl)methyl]-6-ethoxy-5-methyl-3,5-dihydro-2H-pyrazine (0.65 g) as a colorless oil. The crude product was directly used in the next step.

MS m/z (ESI): 293.2[M+H]$^+$.

**Step 5: 3-Chloro-5-[(8R)-7-[(2,4-dimethoxyphenyl)methyl]-8-methyl-6,8-dihydro-5H-[1,2,4 ]triazolo[4,3-a]pyrazin-3-yl]-1,2,4-thiadiazole**

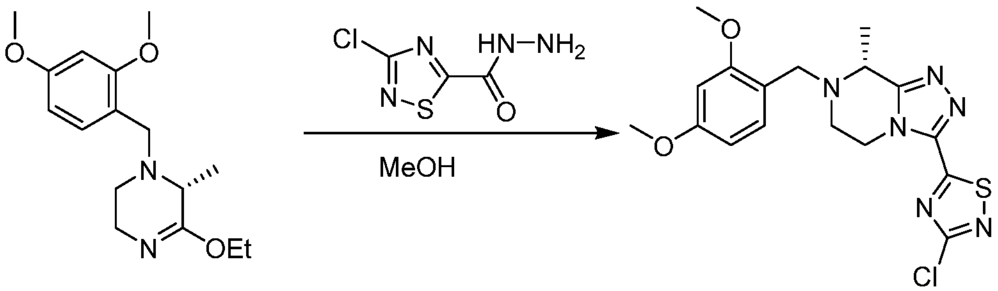


(5R)-4-[(2,4-Dimethoxyphenyl)methyl]-6-ethoxy-5-methyl-3,5-dihydro-2H-pyrazi ne (0.66 g, 2.26 mmol) and 3-chloro-1,2,4-thiadiazole-5-carbohydrazide (322.54 mg, 1.81 mmol) were dissolved in MeOH (10 mL). The mixture was stirred at 80°C for 14 hours under a nitrogen atmosphere and concentrated under reduced pressure. The residue was purified by silica gel flash chromatography (elution: dichloromethane: methanol= 100:0 to 95:5) to obtain the target product 3-chloro-5-[(8R)-7-[(2,4-dimethoxyphenyl)methyl]-8-methyl-6,8-dihydro-5H-[1,2,4]tri azolo[4,3-a]pyrazin-3-yl]-1,2,4-thiadiazole (0.22 g, yield: 23.95%) as a light yellow solid.

MS m/z (ESI): 406.8[M+H]$^+$.

**Step 6: 3-Chloro-5-[(8R)-8-methyl-5,6,7,8-tetrahydro-[1,2,4]triazolo[4,3-a]pyrazin-3-yl]-1, 2,4-thiadiazole**

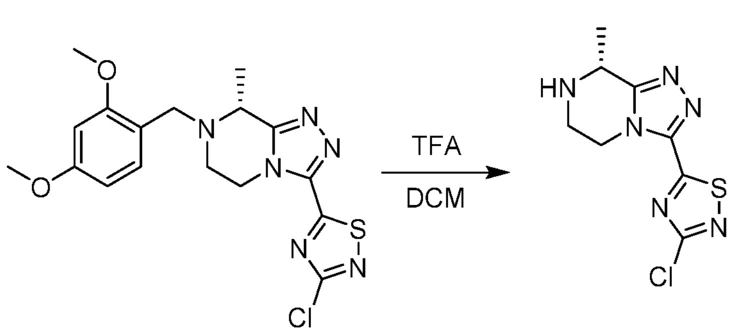

3-Chloro-5-[(8R)-7-[(2,4-dimethoxyphenyl)methyl]-8-methyl-6,8-dihydro-5H-[1,2 ,4]triazolo[4,3-a]pyrazin-3-yl]-1,2,4-thiadiazole (0.22 g, 540.69 μmol) was dissolved in dichloromethane (1.5 mL), and trifluoroacetic acid (3 mL) was added. The mixture was stirred at 25°C for 1 hour. The reaction solution was evaporated under reduced pressure until dry and then water (7 ml) was added. The mixture was stirred at room temperature for 10 minutes and then filtered to remove solids. The liquid phase was lyophilized to obtain the product 3-chloro-5-[(8R)-8-methyl-5,6,7,8-tetrahydro-[1,2,4]triazolo[4,3-a]pyrazin-3-yl]-1,2,4-t hiadiazole trifluoroacetate (0.2 g, yield: 99.77%).

MS m/z (ESI): 257.0$[M+H]^+$.

**Synthesis of the intermediate 3-deuterium-4-fluorobenzoyl chloride:**

**Step 1: Preparation of 3-deuterium-4-fluorobenzoic acid**

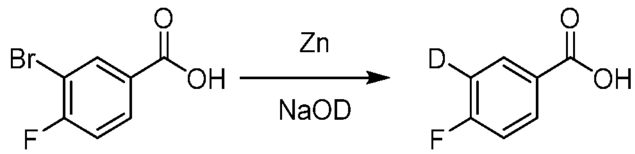


3-Bromo-4-fluorobenzoic acid (5 g, 22.83 mmol) was dissolved in a sodium deuteroxide solution (30 mL, 2 M in $D_2O$), and zinc powder (5.97 g, 91.32 mmol) was added. The mixture was stirred at 25°C for 24 hours. The reaction solution was filtered to remove solids. The pH of the aqueous phase was adjusted to 1 with 1 N HCl, and the aqueous phase was extracted with ethyl acetate (50 mL×3). The organic phases were combined, dried over anhydrous sodium sulfate and concentrated under reduced pressure to obtain the product 3-deuterium-4-fluorobenzoic acid (3 g, yield: 93.12%).

MS m/z (ESI): 139.8$[M-H]^-$

**Step 2: Preparation of 3-deuterium-4-fluorobenzoyl chloride**

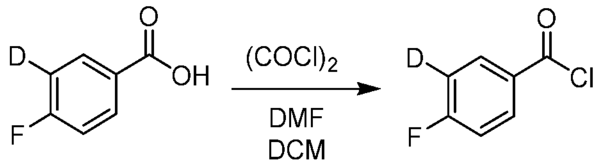


3-Deuterium-4-fluorobenzoic acid (200 mg, 1.42 mmol) was dissolved in dichloromethane (5 mL), oxalyl chloride (359.77 mg, 2.83 mmol) was added, and then N,N-dimethylformamide (0.05 mL) was added. The mixture was stirred at 25°C for 2 hours. The reaction solution was concentrated under reduced pressure. The residue 3-deuterium-4-fluorobenzoyl chloride (0.22 g) was used directly in the next step.

**Synthesis of the target product (R)-(3-chloro-1,2,4-thiadiazol-5-yl)-8-methyl-5,6-dihydro-[1,2,4]triazolo [4,3-a] pyra zin-7(8H)-yl)(4-fluorophenyl-3-d)methanone:**

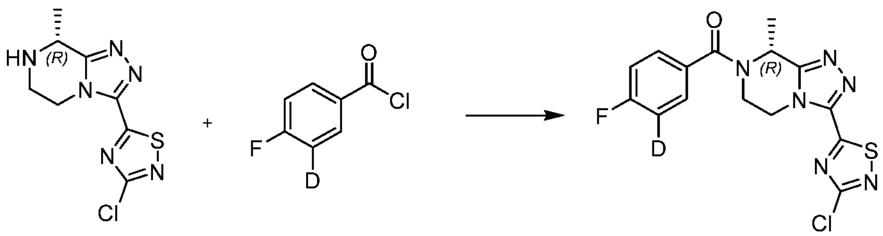

3-Chloro-5-[(8R)-8-methyl-5,6,7,8-tetrahydro-[1,2,4]triazolo[4,3-a]pyrazin-3-yl]-1 ,2,4-thiadiazole trifluoroacetate (0.35 g, 0.94 mmol) and triethylamine (286.59 mg, 2.83 mmol) were dissolved in dichloromethane (5 mL), and 3-deuterium-4-fluorobenzoyl chloride (210.89 mg, 1.32 mmol) was added. The mixture was stirred at 25°C for 20 minites. The reaction was quenched with saturated brine (5 mL), and the mixture was separated into two phases. The organic phase was dried over anhydrous sodium sulfate and concentrated under reduced pressure to obtain the crude product. The crude product was separated by preparative high performance liquid chromatography to obtain the product (R)-(3-(3-chloro-1,2,4-thiadiazol-5-yl)-8-methyl-5,6-dihydro-[1,2,4]triazolo[4,3-a]pyraz in-7(8H)-yl)(4-fluorophenyl-3-d)methanone (66 mg, 172.03 μmol, yield: 18.22%) as a white solid.

MS m/z (ESI): 379.8 $[M+H]^+$.

$^1$H NMR (400 MHz, Chloroform-d) δ 7.58-7.40 (m, 2H), 7.22-7.10 (m, 1H), 5.99-5.61 (m, 1H), 5.01-4.80 (m, 1H), 4.80-4.43 (m, 1H), 4.38-4.16 (m, 1H), 3.68-3.45 (m, 1H), 1.88-1.68 (m, 3H).

**Example 57**

**(R)-(3-(3-Chloro-1,2,4-thiadiazol-5-yl)-8-methyl-5,6-dihydro-[1,2,4]triazolo[4, 3-a]pyrazin-7(8H)-yl)(4-chlorophenyl)methanone**

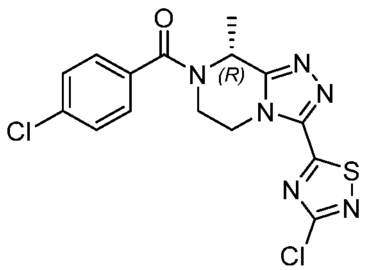

The preparation of Example **57** referred to Step 5 of Example **1.**

MS m/z (ESI): 395.0$[M+H]^+$.

$^1$H NMR (400 MHz, Chloroform-d) δ 7.50-7.44 (m, 2H), 7.44-7.37 (m, 2H), 5.97-5.60 (m, 1H), 4.92-4.81 (m, 1H), 4.76-4.40 (m, 1H), 4.36-4.20 (m, 1H), 3.63-3.42 (m, 1H), 1.76 (d, J = 6.8 Hz, 3H).

**Example 58**

**(R)-(3-(3-Chloro-1,2,4-thiadiazol-5-yl)-8-methyl-5,6-dihydro-[1,2,4]triazolo[4, 3-a]pyrazin-7(8H)-yl)(3,4,5-trifluorophenyl)methanone**

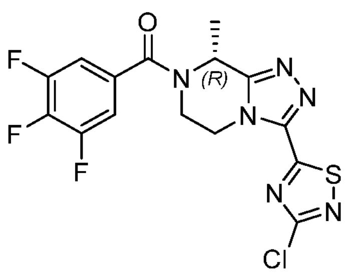

The preparation of Example **58** referred to Step 5 of Example **1.**

MS m/z (ESI): 415.0$[M+H]^+$.

$^1$H NMR (400 MHz, Chloroform-d) δ 7.21-7.08 (m, 2H), 5.98-5.50 (m, 1H), 5.00-4.82 (m, 1H), 4.70-4.36 (m, 1H), 4.36-4.20 (m, 1H), 3.68-3.45 (m, 1H), 1.77 (d, J = 6.8 Hz, 3H).

**Example 59**

**(R)-(4-Fluorophenyl)(3-(3-(methoxy-d3)-1,2,4-thiadiazol-5-yl)-8-methyl-5,6-di hydro-[1,2,4]triazolo[4,3-a]pyrazin-7(8H)-yl)methanone**

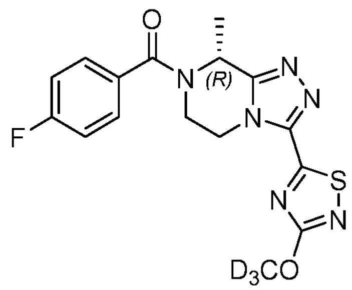

The preparation of Example **59** referred to Step 5 of Example **1.**
MS m/z (ESI): 377.8 $[M+H]^+$.
$^1$H NMR (400 MHz, Chloroform-d) δ 7.56-7.41 (m, 2H), 7.22-7.11 (m, 2H), 5.97-5.65 (m, 1H), 4.95-4.78 (m, 1H), 4.65-4.41 (m, 1H), 4.34-4.21 (m, 1H), 3.62-3.43 (m, 1H), 1.76 (s, 3H).

**Example 60**

**(R)-(3-(3-(Dimethylamino)-1,2,4-thiadiazol-5-yl)-8-methyl-5,6-dihydro-[1,2,4]t riazolo[4,3-a]pyrazin-7(8H)-yl)(4-fluorophenyl)methanone**

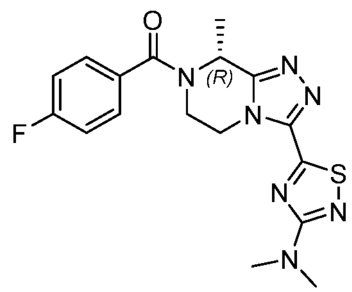

The preparation of Example **60** referred to Step 5 of Example **1.**
MS m/z (ESI): 388.1$[M+H]^+$.
$^1$H NMR (400 MHz, Chloroform-d) δ 7.55-7.42 (m, 2H), 7.23-7.09 (m, 2H), 5.97-5.50 (m, 1H), 4.99-4.80 (m, 1H), 4.80-4.48 (m, 1H), 4.40-4.10 (m, 1H), 3.64-3.42 (m, 1H), 3.24 (s, 6H), 1.76 (d, J = 6.6 Hz, 3H).

**Example 61**

**(R)-(3-(6-Fluorobenzo[d]thiazol-2-yl)-8-methyl-5,6-dihydro-[1,2,4]triazolo[4,3-a]pyrazin-7(8H)-yl)(4-fluorophenyl-3-d)methanone**

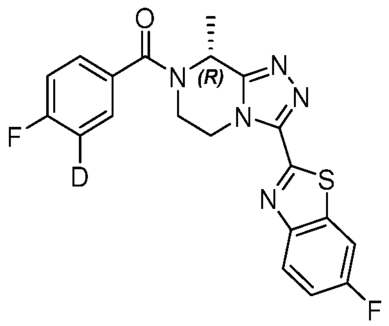

The preparation of Example **61** referred to Step 5 of Example **1.**

MS m/z (ESI): 412.9[M+H]$^+$.

$^1$H NMR (400 MHz, Chloroform-d) loroform-dHz, 2.9[M+H]rm-d) δ 7.55-7.427.52-7.50 (m, 2H), 7.30-7.28 (m, 1H), 7.19-7.18 (m, 1H), 5.72-5.68 (m, 1H), 5.18-5.12 (m, 1H), 4.64-4.60 (m, 1H), 4.34-4.30 (m, 1H), 3.58-3.55 (m, 1H), 1.84-1.80 (m, 3H).

**Example 62**

**(R)-(3-(5,6-Difluorobenzo[d]thiazol-2-yl)-8-methyl-5,6-dihydro-[1,2,4]triazolo[ 4,3-a]pyrazin-7(8H)-yl)(4-fluorophenyl-3-d)methanone**

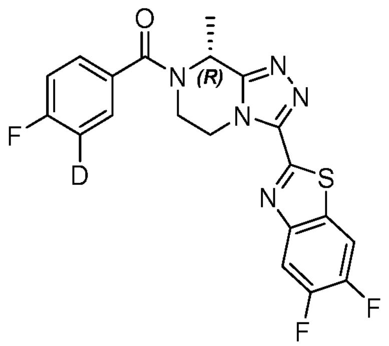

The preparation of Example **62** referred to Step 5 of Example **1.**

MS m/z (ESI): 431.0[M+H]$^+$.

$^1$H NMR (400 MHz, Chloroform-d) δ 7.93-7.78 (m, 1H), 7.78-7.66 (m, 1H), 7.60-7.38 (m, 2H), 7.24-7.08 (m, 1H), 6.14-5.42 (m, 1H), 5.30-4.88 (m, 1H), 4.88-4.13 (m, 2H), 3.92-3.16 (m, 1H), 1.70-1.85 (m, 3H).

**Example 63**

**(R)-6-Fluoro-2-(7-(4-fluorobenzoyl-3-d)-8-methyl-5,6,7,8-tetrahydro-[1,2,4]tri azolo[4,3-a]pyrazin-3-yl)benzo[d]thiazole-5-carbonitrile**

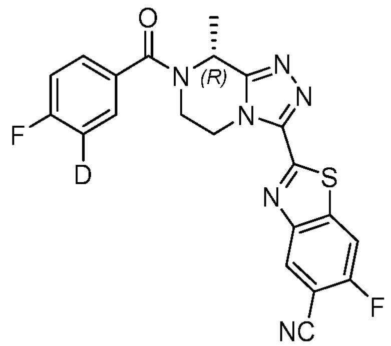

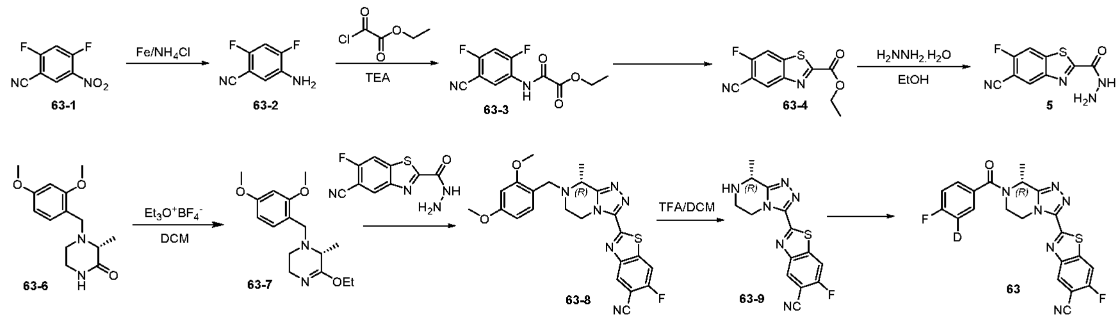

63-1 63-2 63-3 63-4 5
63-6 63-7 63-8 63-9 63

**Step 1: Preparation of 5-amino-2,4-difluorobenzonitrile**

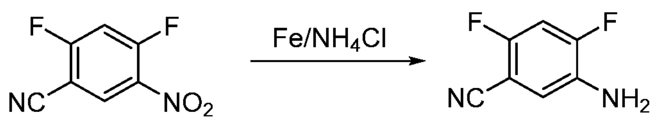


2,4-Difluoro-5-nitrobenzonitrile (5 g, 27.16 mmol) was dissolved in ethanol (30 mL) and water (10 mL), and iron powder (7.58 g, 135.80 mmol) and ammonium chloride (7.26 g, 135.80 mmol) were added. The reaction was carried out at 80°C for 2 hours. The reaction solution was filtered and extracted with EA (30 mL×2). The organic phases were combined, washed with saturated brine (30 mL), dried over anhydrous sodium sulfate and concentrated under reduced pressure. The residues was purified by silica gel flash chromatography (elution: PE:EA=10:1 to 5:1) to obtain the product 5-amino-2,4-difluorobenzonitrile (3 g, yield: 71.67%) as a yellow solid.

MS m/z (ESI): 155.0 $[M+H]^+$.

**Steps 2 to 8 referred to Example 37.**

MS m/z (ESI): 437.9 $[M+H]^+$.

$^1H$ NMR (400 MHz, Chloroform-d) δ 8.31 (d, J = 5.4 Hz, 1H), 7.82 (d, J = 7.9 Hz, 1H), 7.50 (s, 2H), 7.22-7.15 (m, 1H), 5.91-5.68 (m, 1H), 5.11-4.99 (m, 1H), 4.76-4.58 (m, 1H), 4.43-4.31 (m, 1H), 3.66-3.52 (m, 1H), 1.79 (s, 3H).

**Example 64**

**(R)-6-Chloro-2-(7-(4-fluorobenzoyl-3-d)-8-methyl-5,6,7,8-tetrahydro-[1,2,4]tri azolo[4,3-a]pyrazin-3-yl)benzo[d]thiazole-5-carbonitrile**

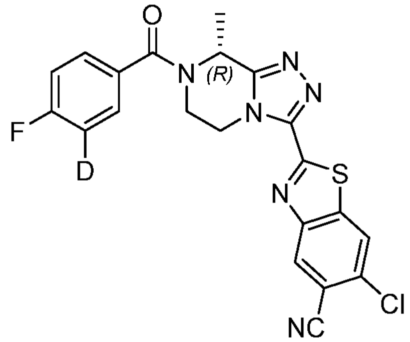

The preparation of Example 64 referred to Example 17.
MS m/z (ESI): 454.0 [M+H]$^+$.

**Example 65**

**(R)-(3-(6-Aminobenzo[d]thiazol-2-yl)-8-methyl-5,6-dihydro-[1,2,4]triazolo[4,3-a]pyrazin-7(8H)-yl)(4-fluorophenyl-3-d)methanone**

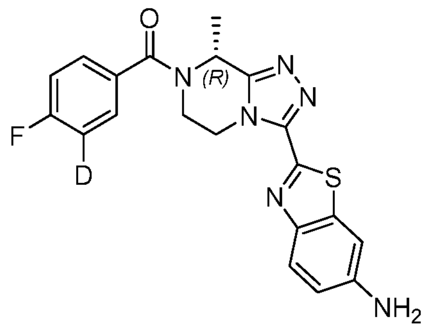

The preparation of Example **65** referred to Example **21.**
MS m/z (ESI): 409.8 [M+H]$^+$.

**Example 66**

**(R)-(3-(6-Dimethylaminobenzo[d]thiazol-2-yl)-8-methyl-5,6-dihydro-[1,2,4]tri azolo[4,3-a]pyrazin-7(8H)-yl)(4-fluorophenyl-3-d)methanone**

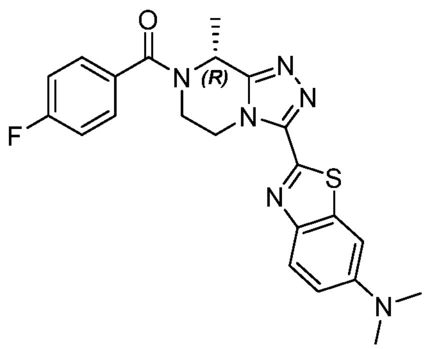

The preparation of Example **66** referred to Example 21.

MS m/z (ESI): 437.1 $[M+H]^+$.

**Example 67**

**(R)-(3-(6-Aminothiazolo[4,5-c]pyridin-2-yl)-8-methyl-5,6-dihydro-[1,2,4]triazo lo[4,3-a]pyrazin-7(8H)-yl)(4-fluorophenyl)methanone**

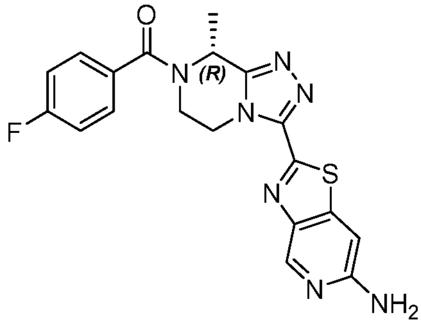

The preparation of Example **67** referred to Example **21.**
MS m/z (ESI): 410.0 $[M+H]^+$.

**Example 68**

**(R)-(4-Fluorophenyl)(8-methyl-3-(thiazolo[5,4-d]pyrimidin-2-yl)-5,6-dihydro-[ 1,2,4]triazolo[4,3-a]pyrazin-7(8H)-yl)methanone**

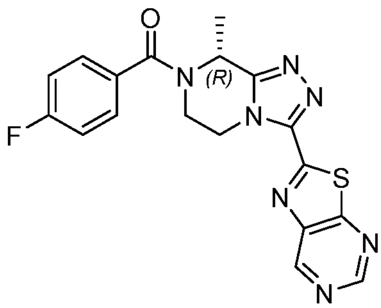

**Step 1: Preparation of ethyl 2-((4-chloropyrimidin-5-yl)amino)-2-oxoacetate**

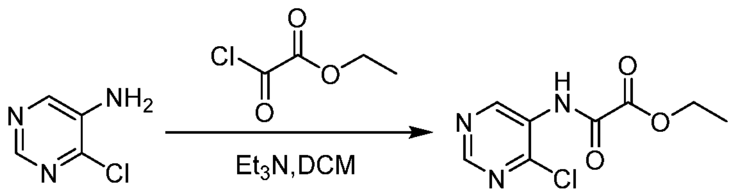


4-Chloropyrimidin-5-amine (5.0 g, 38.6 mmol) was dissolved in dichloromethane (50 mL) and cooled to 0°C. Triethylamine (9.8 g, 96.5 mmol) was added, and then ethyl 2-chloro-2-oxoacetate (6.3 g, 46 mmol) was added dropwise. The reaction solution was stirred at room temperature for 2 hours, water (100 mL) was added, and the aqueous phase was extracted with dichloromethane (100 mL×2). The organic phases were combined, dried over anhydrous sodium

sulfate, filtered and concentrated to obtain ethyl 2-((4-chloropyrimidin-5-yl)amino)-2-oxoacetate (3.5 g, 39%), which was used directly in the next step.

MS m/z (ESI): 230.2 $[M+H]^+$.

**Step 2: Preparation of ethyl thiazolo[5,4-d]pyrimidine-2-carboxylate**

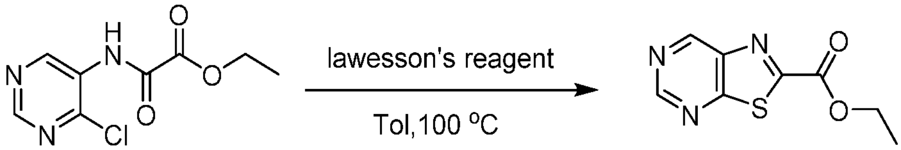


Ethyl 2-((4-chloropyrimidin-5-yl)amino)-2-oxoacetate (3.5 g, 15 mmol) was dissolved in toluene (30 mL), and Lawesson's reagent (6.2 g, 15 mmol) was added. The reaction solution was stirred at 100°C for 18 hours, subjected to rotatory evaporation to remove the solvent, and EA (50 mL) was added. The organic phase was washed with water (50 mL), dried over anhydrous sodium sulfate and concentrated under reduced pressure. The residue was separated by column chromatography (PE/EA = 10/1 ~ 3/1) to obtain ethyl thiazolo[5,4-d]pyrimidine-2-carboxylate (1.8 g, yield: 54%) as a orange solid.

MS m/z (ESI): 210.2 $[M+H]^+$.

**Step 3: Preparation of thiazolo[5,4-d]pyrimidine-2-carbohydrazide**

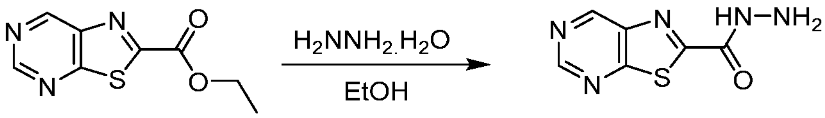


Ethyl thiazolo[5,4-d]pyrimidine-2-carboxylate (1.8 g, 8.6 mmol) was dissolved in anhydrous methanol (10 mL), and 85% hydrazine hydrate (0.5 g, 10 mmol) was added. The reaction solution was stirred at 25°C for 2 hours until the precipitation of solids, and then filtered. The solids were dried to obtain thiazolo[5,4-d]pyrimidine-2-carbohydrazide (0.9 g, yield: 54%) as a white solid.

MS m/z (ESI): 196.2 $[M+H]^+$.

**Step 4: Preparation of (R)-1-(2,4-dimethoxybenzyl)-5-ethoxy-6-methyl-1,2,3,6-tetrahydropyrazine**

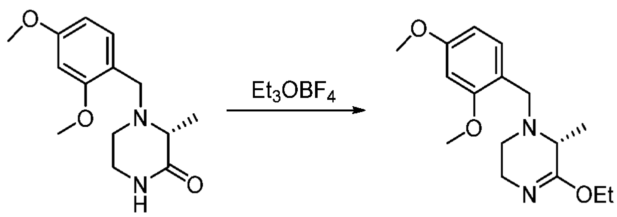


(R)-4-(2,4-Dimethoxybenzyl)-3-methylpiperazin-2-one (0.5 g, 1.1 mmol) was dissolved in dichloromethane (10 mL), cooled to 0°C, and triethyloxidanium tetrafluoroborate (1.44 g, 7.6 mmol) was added. The reaction solution was stirred at room temperature for 2 hours. TLC (DCM/MeOH = 10/1) showed the completion of the reaction. The reaction was quenched with a 2 M cold aqueous sodium hydroxide solution (30 mL), and the aqueous phase was extracted with dichloromethane (30 mL×2). The organic phases were combined, dried over anhydrous sodium sulfate, filtered and concentrated to obtain (R)-1-(2,4-dimethoxybenzyl)-5-ethoxy-6-methyl-1,2,3,6-tetrahydropyrazine (0.5 g, 90% crude product), which was directly used in the next step.

**Step 5: Preparation of (R)-2-(7-(2,4-dimethoxybenzyl)-8-methyl-5,6,7,8-tetrahydro-[1,2,4]triazolo[4,3-a]p yrazol-3-yl)thiazolo[5,4-d]pyrimidine**

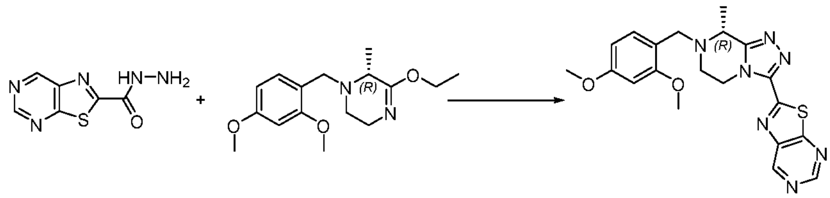

(R)-1-(2,4-Dimethoxybenzyl)-5-ethoxy-6-(methoxymethyl)-1,2,3,6-tetrahydropyra zine (500 mg, 1.7 mmol) was dissolved in methanol (20 mL), and thiazolo[5,4-d]pyrimidine-2-carbohydrazide (154 mg, 0.78 mmol) was added. The reaction solution was stirred at 65°C overnight and subjected to rotatory evaporation until dry. The residue was separated by column chromatography (PE/EA = 10/1 ~ 3/1) to obtain (R)-2-(7-(2,4-dimethoxybenzyl)-8-methyl-5,6,7,8-tetrahydro-[1,2,4]triazolo[4,3-a]pyraz ol-3-yl)thiazolo[5,4-d]pyrimidine (240 mg, yield: 72%) as a colorless colloid.

MS m/z (ESI): 424.2 $[M+H]^+$.

**Step 6: Preparation of (R)-2-(8-methyl-5,6,7,8-tetrahydro- [1,2,4] triazolo [4,3-a] pyrazol-3-yl)thiazolo [5,4-d] pyrimidine**

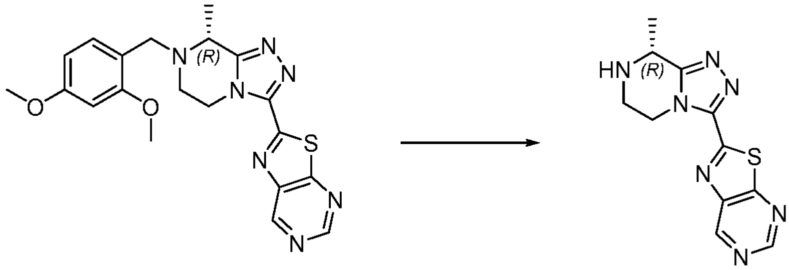

(R)-2-(7-(2,4-Dimethoxybenzyl)-8-methyl-5,6,7,8-tetrahydro-[1,2,4]triazolo[4,3-a] pyrazin-3-yl)thiazolo[5,4-d]pyrimidine (240 mg, 0.6 mmol) was dissolved in dichloromethane (10 mL), and trifluoroacetic acid (3 mL) was added. The reaction solution was stirred at room temperature for 1 hour, water (10 mL) was added, and the reaction solution was stirred for half an hour until the precipitation of solids, and then filtered. The pH of the filtrate was adjusted to >14 with a 4 M sodium hydroxide solution, and the filtrate was extracted with dichloromethane (20 mL×2). The organic phase was dried over anhydrous sodium sulfate, filtered and concentrated to obtain (R)-2-(8-methyl-5,6,7,8-tetrahydro-[1,2,4]triazolo[4,3-a]pyrazol-3-yl)thiazolo[5,4-d]pyr imidine (110 mg, yield: 71%).

MS m/z (ESI): 274.2 $[M+H]^+$.

**Step 7: Preparation of (R)-(4-fluorophenyl)(8-methyl-3-(thiazolo [5,4-d] pyrimidin-2-yl)-5,6-dihydro-[1,2,4] triazolo[4,3-a]pyrazin-7(8H)-yl)methanone**

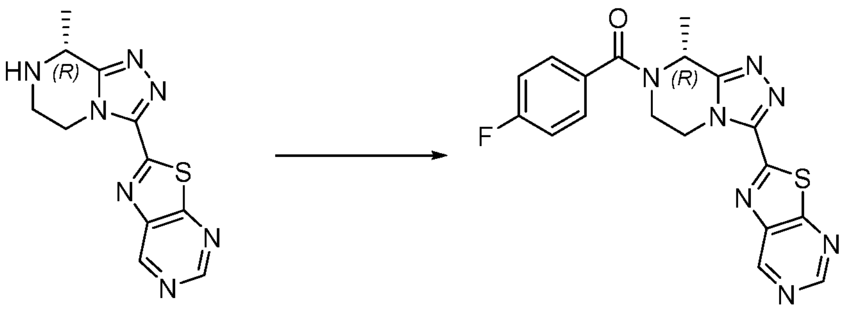

In accordance with Step 5 of Example **1,** (R)-(4-fluorophenyl)(8-methyl-3-(thiazolo[5,4-d]pyrimidin-2-yl)-5,6-dihydro-[1,2,4]tria zolo[4,3-a]pyrazin-7(8H)-yl)methanone (79 mg, yield: 50%) was obtained as a white solid.
MS m/z (ESI): 396.2 [M+H]+.
$^1$H NMR (400 MHz, $CDCl_3$) δ 9.36 (s, 1H), 9.20 (s, 1H), 7.52-7.49 (m, 2H), 7.21-7.16 (m, 2H), 5.83-5.79 (m, 1H), 5.07-5.04 (m, 1H), 4.66-4.37 (m, 2H), 3.62-3.56 (m, 1H), 1.79 (d, J = 4.0 Hz, 3H).

**Example 69**

**(R)-(4-Fluorophenyl)(8-methyl-3-(thiazolo[4,5-d]pyrimidin-2-yl)-5,6-dihydro-[ 1,2,4]triazolo[4,3-a]pyrazin-7(8H)-yl)methanone**

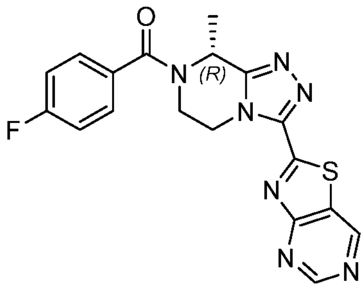

The preparation of Example **69** referred to Example **17.**
MS m/z (ESI): 396.2 [M+H]+.

**Example 70**

**(R)-(4-Fluorophenyl)(8-methyl-3-(thiazolo[4,5-b]pyrazin-2-yl)-5,6-dihydro-[1, 2,4]triazolo[4,3-a]pyrazin-7(8H)-yl)methanone**

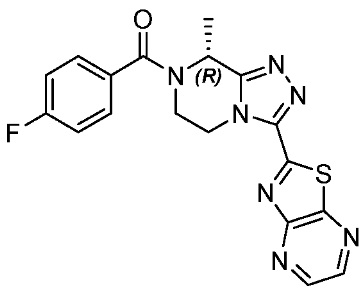

The preparation of Example **70** referred to Example **68.**
MS m/z (ESI): 396.1 [M+H]+.

**Example 71**

**(R)-(4-Fluorophenyl)(8-methyl-3-(thiazolo[5,4-c]pyridazin-6-yl)-5,6-dihydro-[ 1,2,4]triazolo[4,3-a]pyrazin-7(8H)-yl)methanone**

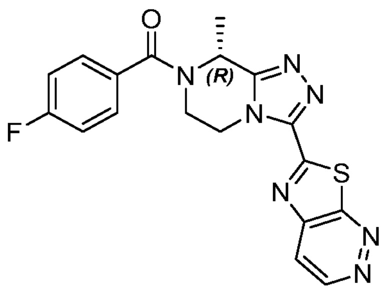

The preparation of Example **71** referred to Example **68.**

MS m/z (ESI): 396.1 $[M+H]^+$.

**Example 72**

**(R)-(4-Fluorophenyl)(8-methyl-3-(2-methyl-2H-pyrazolo[4,3-d]thiazol-3-yl)-5, 6-dihydro-[1,2,4]triazolo[4,3-a]pyrazin-7(8H)-yl)methanone**

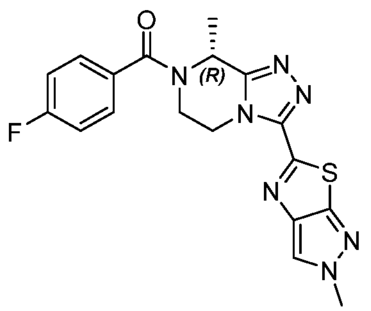

The preparation of Example **72** referred to Example **17.**

MS m/z (ESI): 398.0 $[M+H]^+$.

**Example 73**

**(R)-(3-(3-Chloro-1,2,4-thiadiazol-5-yl)-8-methyl-5,6-dihydro-[1,2,4]triazolo[4, 3-a]pyrazin-7(8H)-yl)(4-chlorophenyl-3-d)methanone**

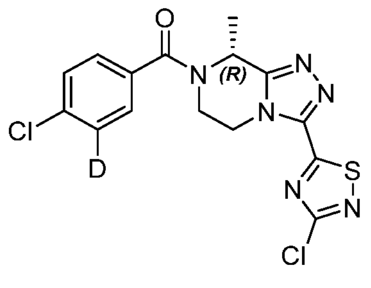

**Step 1: Preparation of 3-deuterium-4-chlorobenzoic acid**

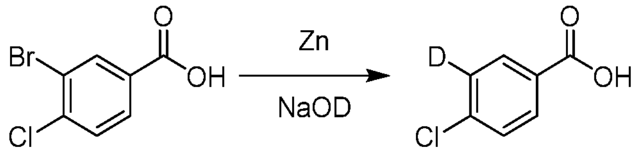


3-Bromo-4-chlorobenzoic acid (5 g, 21.23 mmol) was dissolved in a 2 M NaOD/$D_2O$ solution (15 mL), and zinc powder (5.55 g, 84.94 mmol) was added. The mixture was stirred at 25°C for 96 hours. The reaction solution was filtered to remove solids. The pH of the aqueous phase was adjusted to 1 with 1 N HCl, and the aqueous phase was extracted with ethyl acetate (50 mL×3). The organic phases were combined, dried over anhydrous sodium sulfate and concentrated under reduced pressure to obtain the crude product. The crude product was separated by preparative high performance liquid chromatography to obtain the product 3-deuterium-4-chlorobenzoic acid (1.3 g, yield: 38.85%).
MS m/z (ESI): 156.0[M-H]$^-$

**Step 2: Preparation of 3-deuterium-4-chlorobenzoyl chloride**

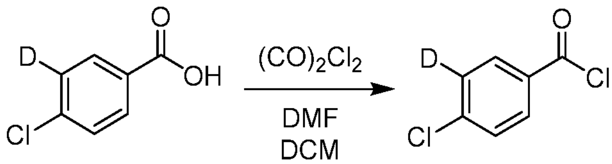


3-Deuterium-4-chlorobenzoic acid (0.1 g, 634.63 μmol) was dissolved in DCM (5 mL), oxalyl chloride (161.10 mg, 1.27 mmol) was added, and then DMF (0.05 mL) was added. The mixture was stirred at 25°C for 2 hours. The reaction solution was concentrated under reduced pressure to obtain the residue 3-deuterium-4-chlorobenzoyl chloride (110 mg), which was directly used in the next step.

**Step 3: The preparation of Example 73 referred to Example 56.**

MS m/z (ESI): 395.9 [M+H]$^+$.
$^1$H NMR (400 MHz, Chloroform-d) δ 7.51-7.44 (m, 1H), 7.44-7.36 (m, 2H), 6.12-5.57 (m, 1H), 4.99-4.79 (m, 1H), 4.78-4.35 (m, 1H), 4.35-4.15 (m, 1H), 3.67-3.42 (m, 1H), 1.76 (d, J = 6.9 Hz, 3H).

**Example 74**

**(R)-(3-(3-Fluoro-1,2,4-thiadiazol-5-yl)-8-methyl-5,6-dihydro-[1,2,4]triazolo[4,3 -a]pyrazin-7(8H)-yl)(4-fluorophenyl-3-d)methanone**

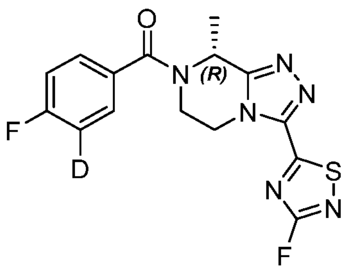

The preparation of Example **74** referred to Example **56.**

MS m/z (ESI): 364.1 $[M+H]^+$.

**Example 75**

**(R)-(4-Fluorophenyl)(3-(3-methoxy-1,2,4-thiadiazol-5-yl)-8-methyl-5,6-dihydr o-[1,2,4]triazolo[4,3-a]pyrazin-7(8H)-yl)methanone**

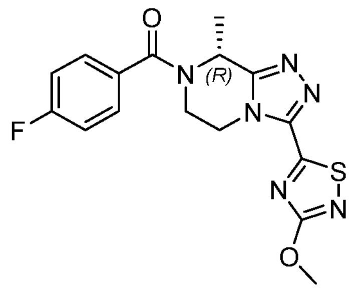

**Step 1: Preparation of (R)-5-(7-(2,4-dimethoxybenzyl)-8-methyl-5,6,7,8-tetrahydro-[1,2,4]triazolo[4,3-a]p yrazin-3-yl)-3-methoxy-1,2,4-thiadiazole**

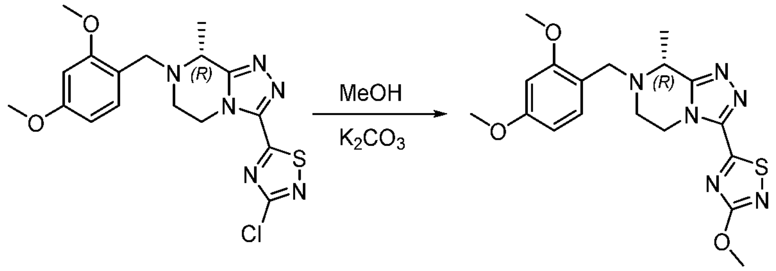


(R)-3-Chloro-5-(7-(2,4-dimethoxybenzyl)-8-methyl-5,6,7,8-tetrahydro-[1,2,4]triaz olo[4,3-a]pyrazin-3-yl)-1,2,4-thiadiazole (100 mg, 0.25 mmol) was dissolved in methanol (5 mL), and andanhydrous potassium carbonate (104 mg, 0.75 mmol) was added. The reaction was carried out at 65°C for 16 hours, and water (10 mL) was added. The reaction solution was extracted with EA (10 mL×2). The organic phases were combined, washed with saturated brine (20 mL), dried over anhydrous sodium sulfate and concentrated under reduced pressure. The residue was purified by pre-TLC (PE:EA=1:1) to obtain the product (R)-5-(7-(2,4-dimethoxybenzyl)-8-methyl-5,6,7,8-tetrahydro-[1,2,4]triazolo[4,3-a]pyraz in-3-yl)-3-methoxy-1,2,4-thiadiazole (70 mg, yield: 71%).
MS m/z (ESI): 402.8 $[M+H]^+$.

**Step 2: Preparation of (R)-3-methoxy-5-(8-methyl-5,6,7,8-tetrahydro-[1,2,4]triazolo[4,3-a]pyrazin-3-yl)-1, 2,4-thiadiazole**

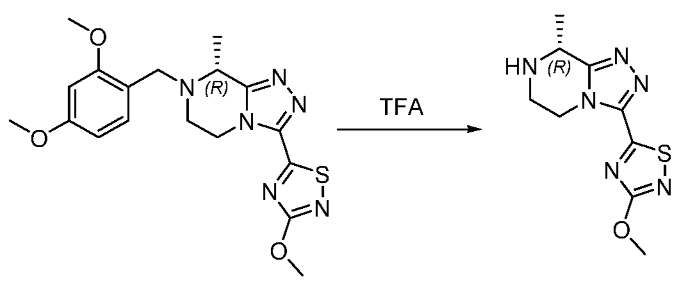

(R)-5-(7-(2,4-Dimethoxybenzyl)-8-methyl-5,6,7,8-tetrahydro-[1,2,4]triazolo[4,3-a] pyrazin-3-yl)-3-methoxy-1,2,4-thiadiazole (70 mg, 0.17 mmol) was dissolved in dichloromethane (2 mL), and trifluoroacetic acid (1 mL) was added. The reaction was carried out at room temperature for 1 hour, and water (5 mL) was added. The reaction solution was stirred for 5 minutes and filtered. The pH of the filtrate was adjusted to >7, and the filtrate was extracted with DCM (5 mL×2). The organic phases were combined, washed with saturated brine (10 mL), dried over anhydrous sodium sulfate and concentrated under reduced pressure to obtain the product (R)-3-methoxy-5-(8-methyl-5,6,7,8-tetrahydro-[1,2,4]triazolo[4,3-a]pyrazin-3-yl)-1,2,4-thiadiazole (30 mg, yield: 70%).

MS m/z (ESI): 253.0 $[M+H]^+$.

**Step 3: Preparation of (R)-(4-fluorophenyl)(3-(3-methoxy-1,2,4-thiadiazol-5-yl)-8-methyl-5,6-dihydro-[1,2, 4]triazolo[4,3-a]pyrazin-7(8H)-yl)methanone**

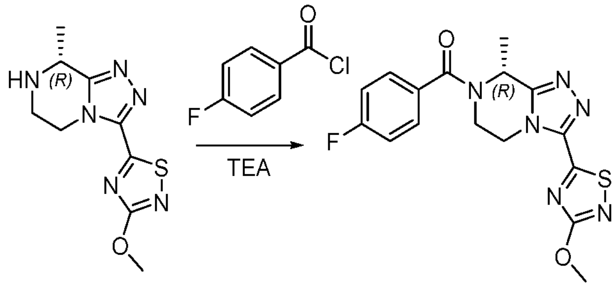


(R)-3-Methoxy-5-(8-methyl-5,6,7,8-tetrahydro-[1,2,4]triazolo[4,3-a]pyrazin-3-yl)-1,2,4-thiadiazole (30 mg, 0.12 mmol) was dissolved in dichloromethane (2 mL), and triethylamine (18 mg, 0.18 mmol) and p-fluorobenzoyl chloride (23 mg, 0.14 mmol) were added. The reaction was carried out at room temperature for 1 hour. The reaction solution was subjected to rotatory evaporation to remove the solvent, and purified by pre-HPLC to obtain the product (R)-(4-fluorophenyl)(3-(3-methoxy-1,2,4-thiadiazol-5-yl)-8-methyl-5,6-dihydro-[1,2,4]t riazolo[4,3-a]pyrazin-7(8H)-yl)methanone (26 mg, yield: 58%).

MS m/z (ESI): 374.8 $[M+H]^+$.

$^1$H NMR (400 MHz, Chloroform-d) δ 7.52-7.43 (m, 2H), 7.21-7.12 (m, 2H), 5.95-5.67 (m, 1H), 4.86 (dd, J = 13.6, 3.8 Hz, 1H), 4.67-4.39 (m, 1H), 4.27 (td, J = 12.9, 4.4 Hz, 1H), 4.17 (s, 3H), 3.60-3.45 (m, 1H), 1.75 (d, J = 6.9 Hz, 3H).

**Example 76**

**(R)-(3-(3-Amino-1,2,4-thiadiazol-5-yl)-8-methyl-5,6-dihydro-[1,2,4]triazolo[4,3-a]p yrazin-7(8H)-yl)(4-fluorophenyl)methanone**

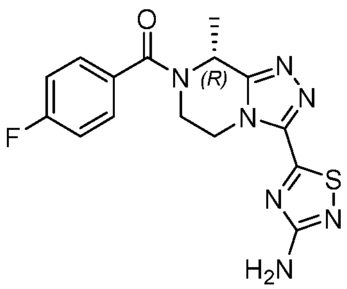

**Step 1: Preparation of 3-bromo-5-(1-ethoxyvinyl)-1,2,4-thiadiazole**

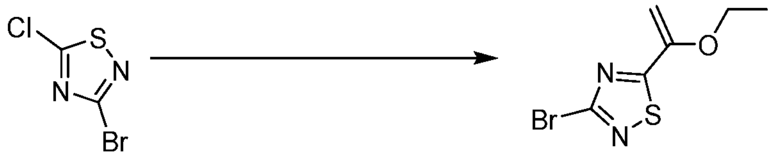

3-Bromo-5-chloro-1,2,4-thiadiazole (4.0 g, 19.65 mmol) and tributyl(1-ethoxyvinyl)stannane (6.39 g, 17.69 mmol) were dissolved in anhydrous N,N-dimethylformamide (20 mL) at room temperature, and then bis(triphenylphosphine)palladium dichloride (0.70 mg, 0.98 mmol) was added. The reaction system was purged with nitrogen, and the reaction solution was heated to 80°C and kept for 14 hours. LCMS indicated the completion of the reaction. The reaction solution was cooled to room temperature, and the reaction was quenched with a saturated potassium fluoride solution (40 mL). The reaction solution was stirred at room temperature for ten minutes and filtered. The solids were washed with an ethyl acetate solution (40 mL×3). The organic phase was washed with saturated brine (20 mL×5), dried over anhydrous sodium sulfate, filtered and subjected to rotatory evaporation until dry. The residue was purified by silica gel flash chromatography (elution: petroleum ether: ethyl acetate = 100:0 to 98:2) to obtain 3-bromo-5-(1-ethoxyvinyl)-1,2,4-thiadiazole (2.64 g, white solid, yield: 57%).

$^1H$ NMR (400 M, $CDCl_3$) δ 5.53 (d, J = 3.2 Hz, 1H), 4.58 (d, J = 3.2 Hz, 1H), 4.02 (q, J = 7.2 Hz, 2H), 1.43 (t, J = 7.2 Hz, 3H).

**Step 2: Preparation of ethyl 3-bromo-1,2,4-thiadiazole-5-carboxylate**

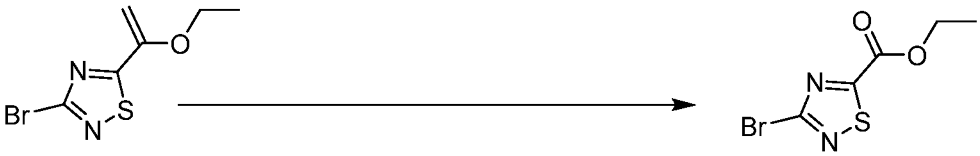

3-Bromo-5-(1-ethoxyvinyl)-1,2,4-thiadiazole (1.10 g, 4.68 mmol) was dissolved in 1,4-dioxane (15 mL) in an ice bath, and then a solution of sodium periodate (3.00 g, 14.04 mmol) in water (7.5 mL) (subjected to ultrasonic oscillation so that sodium periodate was completely dissolved in water) was added, and then potassium permanganate (295.77 mg, 1.87 mmol) was added. The reaction solution was stirred at room temperature for 14 hours. LCMS indicated that the reaction was not complete, thus additional sodium periodate (1.50 g, 7.02 mmol) and potassium permanganate (295.77 mg, 1.87 mmol) were added. The reaction was continued for 14 hours, and LCMS indicated the completion of the reaction. The reaction solution was filtered, and the solids were washed with ethyl acetate (20 mL×3). The organic phases were combined, washed successively with a saturated sodium sulfite solution (30 mL×3) and saturated brine (20 mL×2), dried over anhydrous sodium sulfate, filtered and subjected to rotatory evaporation until dry. The crude product was separated by silica gel flash chromatography (elution: PE:EA=100:0 to 98:2) to obtain ethyl 3-bromo-1,2,4-thiadiazole-5-carboxylate (0.28 g, colorless oil, yield: 25.24%).

$^1H$ NMR (400 MHz, $CDCl_3$) δ 4.53 (q, J = 7.2 Hz, 2H), 1.47 (t, J = 7.2 Hz, 3H).

**Step 3: Preparation of 3-bromo-1,2,4-thiadiazole-5-carbohydrazide**

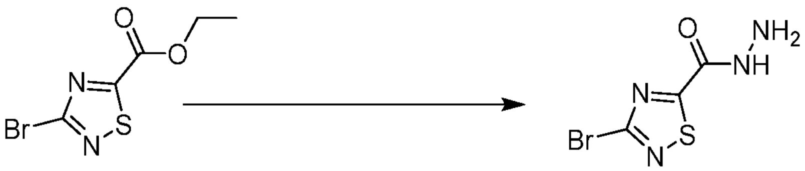

Ethyl 3-bromo-1,2,4-thiadiazole-5-carboxylate (0.62 g, 2.62 mmol) was dissolved in ethanol (8 mL) at room temperature, and then hydrazine hydrate (146.95 mg, 2.88 mmol, 98% purity) was added dropwise. The reaction solution was stirred at room temperature overnight, and LCMS indicated the completion of the reaction. The reaction solution was subjected to rotatory evaporation until dry to obtain 3-bromo-1,2,4-thiadiazole-5-carbohydrazide (0.55 g, bright yellow solid, yield: 94%).

**Step 4: Preparation of (R)-1-(2,4-dimethoxybenzyl)-5-ethoxy-6-methyl-1,2,3,6-tetrahydropyrazine**

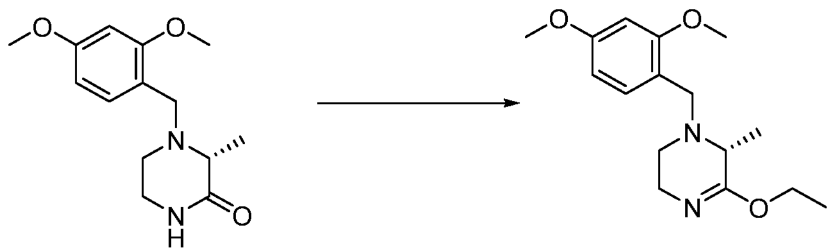

(R)-4-(2,4-Dimethoxybenzyl)-3-methylpiperazin-2-one (1.0 g, 3.78 mmol) was dissolved in dichloromethane (10 mL) in an ice bath, and triethyloxonium tetrafluoroborate (2.16 g, 11.35 mmol) was added to the reaction system in batches. The reaction solution was stirred at room temperature for three hours, and TLC showed the completion of the reaction. The reaction solution was added to an aqueous sodium hydroxide solution (2 N, 10 mL) in an ice bath, stirred for ten minutes in an ice bath and separated into two phases. The organic phase was washed with saturated brine (10 mL×2), dried over anhydrous sodium sulfate, filtered and subjected to rotatory evaporation at low temperature until dry to obtain the crude product (R)-1-(2,4-dimethoxybenzyl)-5-ethoxy-6-methyl-1,2,3,6-tetrahydropyrazine (1.36 g, colorless oil, crude product), which was directly used in the next step.

MS m/z (ESI): 293.1 $[M+H]^+$,311.1$[M+18+H]^+$

**Step 5: Preparation of (R)-3-bromo-5-(7-(2,4-dimethoxybenzyl)-8-methyl-5,6,7,8-tetrahydro-[1,2,4]triazol o[4,3-a]pyrazin-3-yl)-1,2,4-thiadiazole**

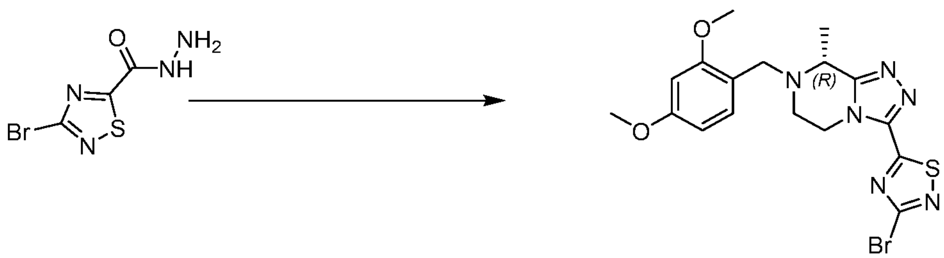

3-Bromo-1,2,4-thiadiazole-5-carbohydrazide (550 mg, 2.47 mmol) was dissolved in methanol (10 mL) at room temperature, and then a solution of (R)-1-(2,4-dimethoxybenzyl)-5-ethoxy-6-methyl-1,2,3,6-tetrahydropyrazine (1.36 g, 4.65 mmol) in methanol (3 mL) was added dropwise. The reaction solution was heated to 70°C and kept for 14 hours, and then cooled to room temperature. LCMS indicated the completion of the reaction. The reaction solution was subjected to rotatory evaporation until dry, and the residue was purified by silica gel flash chromatography (elution: dichloromethane: methanol = 100:0 to 98:2) to obtain (R)-3-bromo-5-(7-(2,4-dimethoxybenzyl)-8-methyl-5,6,7,8-tetrahydro-[1,2,4]triazolo[4, 3-a]pyrazin-3-yl)-1,2,4-thiadiazole (0.60 g, yellow solid, yield: 53.9%).

MS m/z (ESI): 450.9,453.0 $[M+H]^+$.

**Step 6: Preparation of (R)-3-bromo-5-(8-methyl-5,6,7,8-tetrahydro-[1,2,4]triazolo[4,3-a]pyrazin-3-yl)-1,2, 4-thiadiazole**

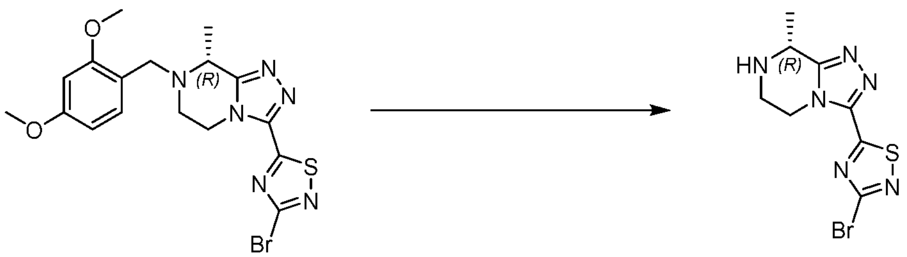

(R)-3-Bromo-5-(7-(2,4-dimethoxybenzyl)-8-methyl-5,6,7,8-tetrahydro-[1,2,4]triaz olo[4,3-a]pyrazin-3-yl)-1,2,4-thiadiazole (0.41 g, 0.91 mmol) was dissolved in dichloromethane (4 mL) at room temperature, and then trifluoroacetic acid (2 mL) was added dropwise. The reaction solution was stirred at room temperature for 1.5 hours. LCMS indicated the completion of the reaction. The reaction was quenched with saturated brine. The reaction solution was stirred at room temperature for ten minutes until the precipitation of solids, and then filtered. The solids were washed with water. The reaction solution was neutralized with an aqueous sodium hydroxide solution (2 N), the pH was adjusted to 10-12, and then the reaction solution was extracted with dichloromethane (15 mL×3). The organic phases were combined, dried over anhydrous sodium sulfate, filtered, and subjected to rotatory evaporation until dry to obtain (R)-3-bromo-5-(8-methyl-5,6,7,8-tetrahydro-[1,2,4]triazolo[4,3-a]pyrazin-3-yl)-1,2,4-th iadiazole (0.225 g, yellow solid, yield): 82%).

MS m/z (ESI): 300.9,302.9 $[M+H]^+$.

**Step 7: Preparation of (R)-(3-bromo-1,2,4-thiadiazol-5-yl)-8-methyl-5,6-dihydro-[1,2,4]triazolo [4,3-a] pyra zin-7(8H)-yl)(4-fluorophenyl)methanone**

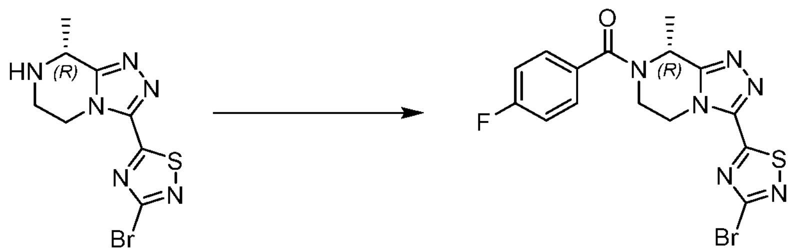

(R)-3-Bromo-5-(8-methyl-5,6,7,8-tetrahydro-[1,2,4]triazolo[4,3-a]pyrazin-3-yl)-1, 2,4-thiadiazole (0.225 g, 0.747 mmol) was dissolved in dichloromethane (6 mL) at room temperature, then a saturated aqueous sodium bicarbonate solution (2 mL) was added, and then 4-fluorobenzoyl chloride (0.14 g, 0.897 mmol) was added dropwise. The reaction solution was stirred at room temperature for 1 hour. LCMS indicates the completion of the reaction. The reaction solution was diluted with dichloromethane (10 mL), and then washed successively with a saturated sodium bicarbonate solution (10 mL×2) and saturated brine (10 mL×2). The organic phase was dried over anhydrous sodium sulfate, filtered and subjected to rotatory evaporation until dry. The residue was separated by preparative thin layer chromatography (PE:EA=1:1) to obtain (R)-(3-bromo-1,2,4-thiadiazol-5-yl)-8-methyl-5,6-dihydro-[1,2,4]triazolo[4,3-a]pyrazin-7(8H)-yl)(4-fluorophenyl)methanone (0.25 g, yellow solid, yield: 79%).

MS m/z (ESI): 422.9,424.9 $[M+H]^+$.

**Step 8: Preparation of tert-butyl (R)-(5-(7-(4-fluorobenzoyl)-8-methyl-5,6,7,8-tetrahydro-[1,2,4]triazolo[4,3-a]pyrazi n-3-yl)-1,2,4-thiadiazol-3-yl)carbamate**

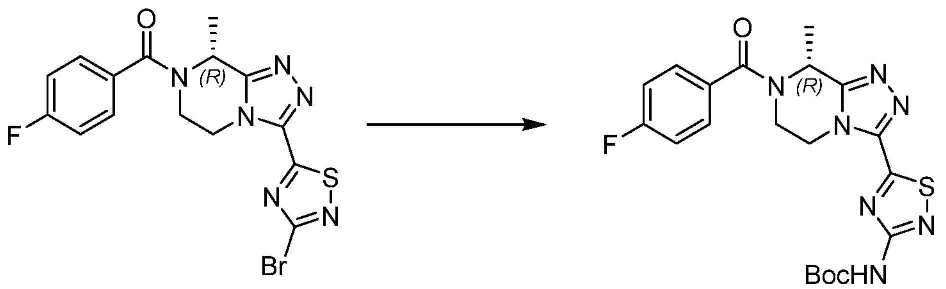

(R)-(3-(3-Bromo-1,2,4-thiadiazol-5-yl)-8-methyl-5,6-dihydro-[1,2,4]triazolo[4,3-a] pyrazin-7(8H)-yl)(4-fluorophenyl)methanone (0.075 g, 0.177 mmol), tert-butylcarbamate (0.042 g, 0.354 mmol), tris(dibenzylideneacetone)dipalladium (0.032 g, 0.035 mmol), sodium tert-butoxide (0.034 g, 0.354 mmol) and (1-diphenylphosphanyl-3,4-dimethyl-9H-xanthenyl-2-yl)-diphenyl-phosphane (0.041 g, 0.071 mmol) were dissolved in toluene (4 mL). The reaction system was purged with nitrogen, and the reaction solution was heated to 100°C and kept overnight, and then cooled to room temperature. LCMS indicated the completion of the reaction. The reaction solution was subjected to rotatory evaporation until dry. The residue was dissolved in ethyl acetate (25 mL), and then washed with saturated brine (15 mL×2). The organic phase was dried over anhydrous sodium sulfate, filtered and subjected to rotatory evaporation until dry. The residue was separated by preparative thin layer chromatography ($CH_2Cl_2$: MeOH=15: 1) to obtain tert-butyl (R)-(5-(7-(4-fluorobenzoyl)-8-methyl-5,6,7,8-tetrahydro-[1,2,4]triazolo[4,3-a]pyrazin-3 -yl)-1,2,4-thiadiazol-3-yl)carbamate (30 mg, yellow oil, yield: 38%).

MS m/z (ESI): 460.1 $[M+H]^+$.

**Step 9: Preparation of (R)-(3-(3-amino-1,2,4-thiadiazol-5-yl)-8-methyl-5,6-dihydro- [1,2,4] triazolo [4,3-a] py razin-7(8H)-yl)(4-fluorophenyl)methanone**

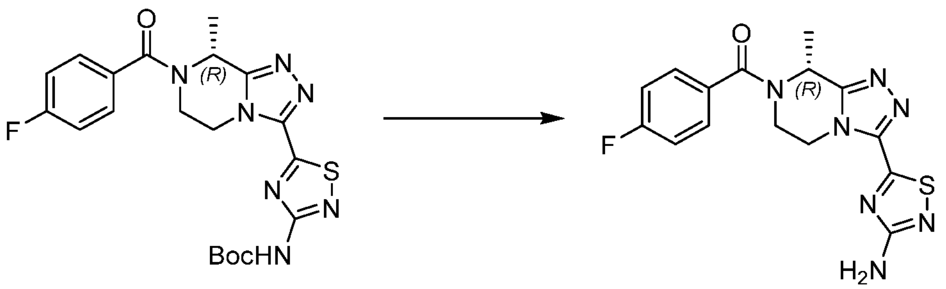

Tert-butyl (R)-(5-(7-(4-fluorobenzoyl)-8-methyl-5,6,7,8-tetrahydro-[1,2,4]triazolo[4,3-a]pyrazin-3 -yl)-1,2,4-thiadiazol-3-yl)carbamate (30 mg, 0.067 mmol) was dissolved in dichloromethane (2 mL) at room temperature, and then trifluoroacetic acid (0.5 mL) was added dropwise. The reaction solution was stirred at room temperature for 2 hours. LCMS indicated the completion of the reaction. The reaction solution was subjected to rotatory evaporation until dry. The residue was dissolved in ethyl acetate (20 mL) and then washed with a saturated sodium bicarbonate solution (10 mL×2) and saturated brine (10 mL×2). The organic phase was dried over anhydrous sodium sulfate, filtered and subjected to rotatory evaporation until dry. The residue was separated by preparative high performance liquid chromatography and lyophilized to obtain (R)-(3-(3-amino-1,2,4-thiadiazol-5-yl)-8-methyl-5,6-dihydro-[1,2,4]triazolo[4,3-a]pyraz in-7(8H)-yl)(4-fluorophenyl)methanone (15 mg, white solid, yield: 61.8%).

MS m/z (ESI): 360.1 $[M+H]^+$.

$^1H$ NMR (400 MHz, $CDCl_3$) δ 7.50-7.47 (m, 2H), 7.17 (d, J = 8.4 Hz, 2H), 5.88-5.65 (br, 1H), 5.20-5.15 (br, 2H), 4.94-4.87 (m, 1H), 4.83-4.72 (br, 1H), 4.27-4.21 (m, 1H), 3.55-3.48 (m, 1H), 1.75 (d, J = 6.8 Hz, 3H).

**Example 77**

**(R)-(4-Fluorophenyl)(8-methyl-3-(3-(methylamino)-1,2,4-thiadiazol-5-yl)-5,6-d ihydro-[1,2,4]triazolo[4,3-a]pyrazin-7(8H)-yl)methanone**

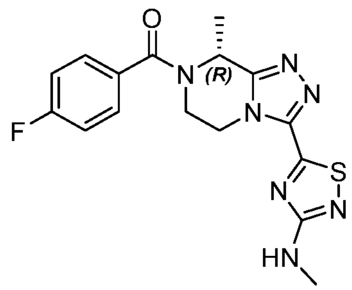

The preparation of Example 77 referred to Example 76.

MS m/z (ESI): 373.9 $[M+H]^+$.

**Example 78**

**(R)-(4-Fluorophenyl)(8-methyl-3-(3-(methylthio)-1,2,4-thiadiazol-5-yl)-5,6-dih ydro-[1,2,4]triazolo[4,3-a]pyrazin-7(8H)-yl)methanone**

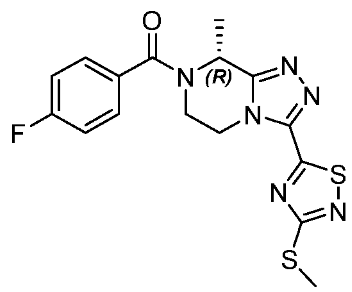

The preparation of Example **78** referred to Example **75.**

MS m/z (ESI): 390.0 $[M+H]^+$.

**Example 79**

**(R)-(3-Ethynyl-1,2,4-thiadiazol-5-yl)-8-methyl-5,6-dihydro-[1,2,4]triazolo[4,3-a]pyrazin-7(8H)-yl)(4-fluorophenyl)methanone**

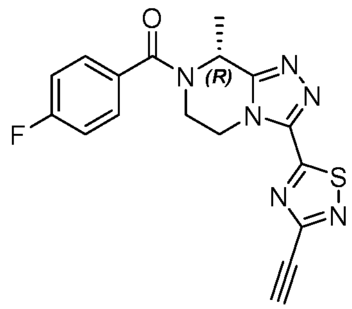

**Step 1: Preparation of (E)-5-(2-chlorovinyl)-1,3,4-oxathiazol-2-one**

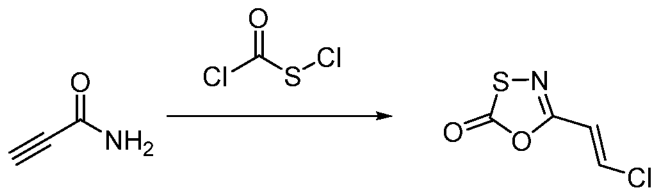

Propiolamide (1.0 g, 14.48 mmol) was dissolved in 1,2-dichloroethane (20 mL) at room temperature, and then carbonochloridic hypochlorous thioanhydride (2.28 g, 17.38 mmol) was added. The reaction solution was heated to 85°C and kept overnight, and then cooled to room temperature. TLC indicated the completion of the reaction. The reaction solution was subjected to rotatory evaporation to remove the solvent to obtain the crude product (E)-5-(2-chlorovinyl)-1,3,4-oxathiazol-2-one (2.0 g, yellow oil), which was directly used in the next step.

**Step 2: Preparation of ethyl (E)-3-(2-chlorovinyl)-1,2,4-thiadiazole-5-carboxylate**

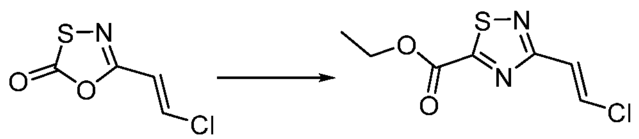

(E)-5-(2-Chlorovinyl)-1,3,4-oxathiazol-2-one (2.0 g, yellow oil) was dissolved in xylene (20 mL) at room temperature, and then ethyl cyanoformate (3.63 g, 36.68 mmol) was added. The reaction solution was heated to 140°C for 4 hours. LCMS indicated that the reaction was not complete. The reaction solution was cooled to room temperature, and additional ethyl cyanoformate (1.82 g, 18.34 mmol) was added. Then the reaction solution was heated to 140°C for 14 hours, and cooled to room temperature. LCMS indicated the completion of the reaction. Xylene was removed by pumping vacuum with an oil pump, and the residue was purified by silica gel flash chromatography (elution: petroleum ether: ethyl acetate = 100:0 to 97:3) to obtain the target product ethyl (E)-3-(2-chlorovinyl)-1,2,4-thiadiazole-5-carboxylate (1.30 g, yellow solid, total yield of two steps: 41%).

MS m/z (ESI): 218.8 $[M+H]^+$.

$^1H$ NMR (400 MHz, Chloroform-d) δ 7.60 (d, J = 13.6 Hz, 1H), 7.06 (d, J = 13.6 Hz, 1H), 4.50 (q, J = 7.2 Hz, 2H), 1.46 (t, J = 7.2 Hz, 3H).

**Step 3: Preparation of (E)-3-(2-chlorovinyl)-1,2,4-thiadiazole-5-carbohydrazide**

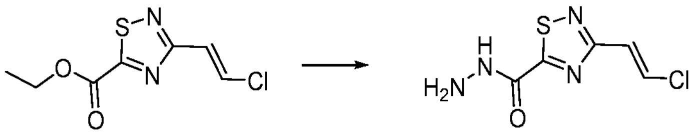

Ethyl (E)-3-(2-chlorovinyl)-1,2,4-thiadiazole-5-carboxylate (0.22 g, 1.01 mmol) was dissolved in ethanol (5 mL) at room temperature, and then hydrazine hydrate (0.06 g, 1.21 mmol) was added dropwise. The reaction solution was stirred at room temperature for 3 hour. LCMS indicated the completion of the reaction. Solids were precipitated, and the reaction solution was filtered to collect the solids. The solids were dried to obtain (E)-3-(2-chlorovinyl)-1,2,4-thiadiazole-5-carbohydrazide (0.19 g, yellow solid, yield: 92%).

**Step 4: Preparation of (R)-1-(2,4-dimethoxybenzyl)-5-ethoxy-6-methyl-1,2,3,6-tetrahydropyrazine**

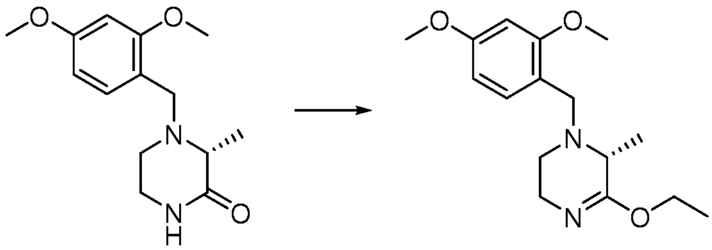

(R)-4-(2,4-Dimethoxybenzyl)-3-methylpiperazin-2-one (0.38 g, 1.44 mmol) was dissolved in dichloromethane (5 mL) in an ice bath, and triethyloxonium tetrafluoroborate (0.68 g, 3.59 mmol) was added to the reaction system in batches. The reaction solution was stirred at room temperature for three hours. TLC showed the completion of the reaction. The reaction solution was added to an aqueous sodium hydroxide solution (2 N, 10 mL) in an ice bath, stirred for ten minutes in an ice bath, and separated into two phases. The organic phase was washed with saturated brine (10 mL×2), dried over anhydrous sodium sulfate, filtered and subjected to rotatory evaporation at low temperature until dry to obtain the crude product (R)-1-(2,4-dimethoxybenzyl)-5-ethoxy-6-methyl-1,2,3,6-tetrahydropyrazine (0.45 g, colorless oil, crude product), which was directly used in the next step.

MS m/z (ESI): 293.1 $[M+H]^+$, 311.1$[M+18+H]^+$

**Step 5: Preparation of (R,E)-3-(2-chlorovinyl)-5-(7-(2,4-dimethoxybenzyl)-8-methyl-5,6,7,8-tetrahydro-[1, 2,4]triazolo[4,3-a]pyrazin-3-yl)-1,2,4-thiadiazole**

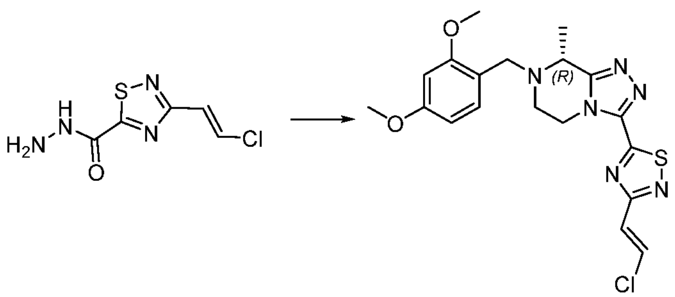

(E)-3-(2-Chlorovinyl)-1,2,4-thiadiazole-5-carbohydrazide (0.19 g, 0.93 mmol) was dissolved in methanol (10 mL) at room temperature, and then a solution of (R)-1-(2,4-dimethoxybenzyl)-5-ethoxy-6-methyl-1,2,3,6-tetrahydropyrazine (0.45 g, 1.54 mmol) in methanol (2 mL) was added. The reaction solution was heated to 65°C and kept overnight, and then cooled to room temperature. LCMS indicated the completion of the reaction. The reaction solution was subjected to rotatory evaporation until dry. The residue was separated by preparative thin layer chromatography (dichloromethane: methanol = 20:1) to obtain (R,E)-3-(2-chlorovinyl)-5-(7-(2,4-dimethoxybenzyl)-8-methyl-5,6,7,8-tetrahydro-[1,2,4 ]triazolo[4,3-a]pyrazin-3-yl)-1,2,4-thiadiazole (0.29 g, yellow oil, yield: 72.5%).

MS m/z (ESI): 433.1 $[M+H]^+$

**Step 6: Preparation of (R)-5-(7-(2,4-dimethoxybenzyl)-8-methyl-5,6,7,8-tetrahydro-[1,2,4]triazolo[4,3-a]p yrazin-3-yl)-3-ethynyl-1,2,4-thiadiazole**

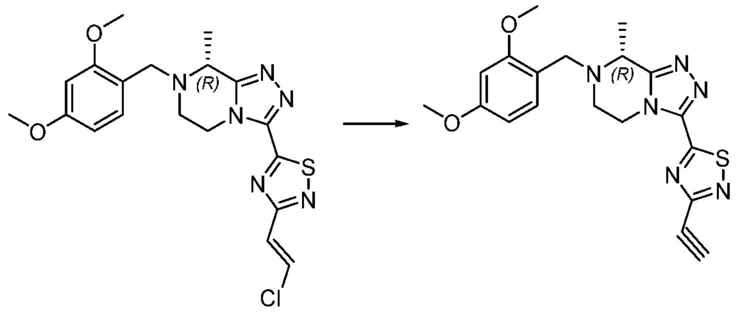

(R,E)-3-(2-Chlorovinyl)-5-(7-(2,4-dimethoxybenzyl)-8-methyl-5,6,7,8-tetrahydro-[ 1,2,4]triazolo[4,3-a]pyrazin-3-yl)-1,2,4-thiadiazole (0.13 g, 0.3 mmol) was dissolved in tetrahydrofuran (5 mL), and the reaction system was purged with nitrogen. Sodium hexamethyldisilazide (1.0 M in THF, 0.9 mL, 0.9 mmol) was added dropwise under a nitrogen atmosphere. The reaction solution was stirred overnight at room temperature. LCMS indicated the formation of the target product. The reaction was quenched with a saturated ammonium chloride solution (15 mL), and solids were precipitated. The reaction solution was filtered, the solids were washed with ethyl acetate (20 mL), and the mixture was separated into two phases. The organic phase was washed with saturated brine (10 mL×2), dried over anhydrous sodium sulfate, filtered and subjected to rotatory evaporation until dry to obtain (R)-5-(7-(2,4-dimethoxybenzyl)-8-methyl-5,6,7,8-tetrahydro-[1,2,4]triazolo[4,3-a]pyraz in-3-yl)-3-ethynyl-1,2,4-thiadiazole (0.09 g, brown oil, crude product). The crude product was directly used in the next step.

MS m/z (ESI): 397.0 $[M+H]^+$.

**Step 7: Preparation of (R)-3-ethynyl-5-(8-methyl-5,6,7,8-tetrahydro-[1,2,4]triazolo[4,3-a]pyrazin-3-yl)-1,2, 4-thiadiazole**

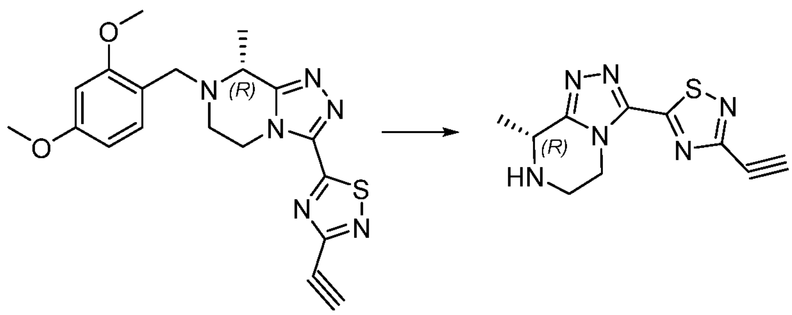

(R)-5-(7-(2,4-Dimethoxybenzyl)-8-methyl-5,6,7,8-tetrahydro-[1,2,4]triazolo[4,3-a] pyrazin-3-yl)-3-ethynyl-1,2,4-thiadiazole (0.09 g, 0.23 mmol) was dissolved in dichloromethane (5 mL) in an ice bath, and then trifluoroacetic acid (1 mL) was added dropwise. The reaction solution was stirred at room temperature for 1 hour. LCMS indicated the completion of the reaction. The reaction was quenched with saturated brine. The reaction solution was stirred for ten minutes at room temperature until the precipitation of solids, and then filtered. The solids were washed with water. The reaction solution was neutralized with an aqueous sodium hydroxide solution (2 N), the pH was adjusted to 10-12, and then the reaction solution was extracted with dichloromethane (15 mL×2). The organic phases were combined, dried over anhydrous sodium sulfate, filtered, and subjected to rotatory evaporation until dry to obtain (R)-3-ethynyl-5-(8-methyl-5,6,7,8-tetrahydro-[1,2,4]triazolo[4,3-a]pyrazin-3-yl)-1,2,4-t hiadiazole (0.025 g, yellow oil, crude product). The crude product was directly used in the next step.

MS m/z (ESI): 247.0 $[M+H]^+$

**Step 8: (R)-(3-Ethynyl-1,2,4-thiadiazol-5-yl)-8-methyl-5,6-dihydro-[1,2,4]triazolo[4,3-a]pyr azin-7(8H)-yl)(4-fluorophenyl)methanone**

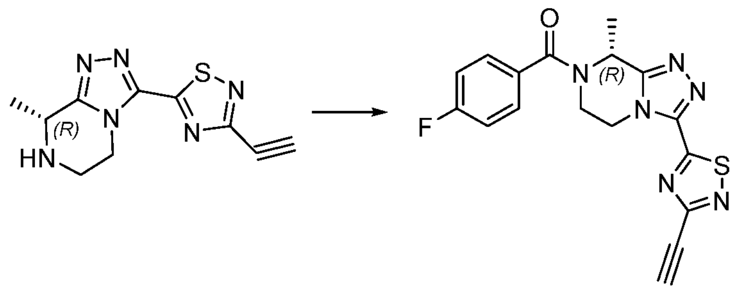

(R)-3-Ethynyl-5-(8-methyl-5,6,7,8-tetrahydro-[1,2,4]triazolo[4,3-a]pyrazin-3-yl)-1, 2,4-thiadiazole (0.025 g, 0.101 mmol) was dissolved in dichloromethane (5 mL) at room temperature, then a saturated aqueous sodium bicarbonate solution (2 mL) was added, and then a solution of 4-fluorobenzoyl chloride (0.019 g, 0.121 mmol) in dichloromethane (1 mL) was added dropwise. The reaction solution was stirred at room temperature for 1 hour. LCMS indicated the completion of the reaction. The reaction solution was diluted with methylene chloride (10 mL), then washed with a saturated sodium bicarbonate solution (10 mL×2) and washed with saturated brine (10 mL×2). The organic phase was dried over anhydrous sodium sulfate, filtered and subjected to rotatory evaporation until dry. The residue was separated by preparative high performance liquid chromatography and lyophilized to obtain (R)-(3-ethynyl-1,2,4-thiadiazol-5-yl)-8-methyl-5,6-dihydro-[1,2,4]triazolo[4,3-a]pyrazin -7(8H)-yl)(4-fluorophenyl)methanone (4 mg, white solid, total yield of three steps: 3.6%).

MS m/z (ESI): 369.1 $[M+H]^+$.

$^1H$ NMR (400 MHz, Chloroform-d) δ7.46-7.50 (m, 2H), 7.17 (t, J = 8.4 Hz, 2H), 5.59-5.98 (br, 1H), 4.90-4.94 (m, 1H), 4.35-4.78 (br, 1H), 4.26-4.30(m, 1H), 3.50-3.70 (m, 1H), 3.22 (s, 1H), 1.77 (d, J = 6.8 Hz, 3H).

**Example 80**

**(R)-(3-(5-Fluoro-4-methylthiazol-2-yl)-8-methyl-5,6-dihydro-[1,2,4]triazolo[4, 3-a]pyrazin-7(8H)-yl)(4-fluorophenyl-3-d)methanone**

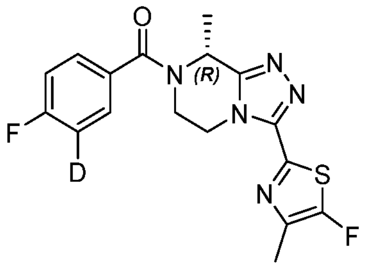

The preparation of Example **80** referred to Step 5 of Example **1.**

MS m/z (ESI): 377.0 $[M+H]^+$.

**Example 81**

**(R)-(4-Fluorophenyl)(3-(4-methoxythiazol-2-yl)-8-methyl-5,6-dihydro-[1,2,4]tr iazolo[4,3-a]pyrazin-7(8H)-yl)methanone**

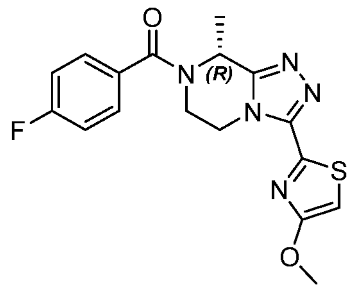

The preparation of Example **81** referred to Step 5 of Example **1.** MS m/z (ESI): 373.8 $[M+H]^+$.

**Example 82**

**(R)-(3-(5-Fluoro-4-methoxythiazol-2-yl)-8-methyl-5,6-dihydro-[1,2,4]triazolo[ 4,3-a]pyrazin-7(8H)-yl)(4-fluorophenyl)methanone**

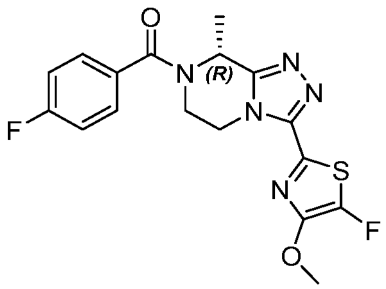

The preparation of Example **82** referred to Step 5 of Example **1.**

MS m/z (ESI): 392.1 $[M+H]^+$.

**Example 83**

**(R)-(3-(4-Dimethylamino)thiazol-2-yl)-8-methyl-5,6-dihydro-[1,2,4]triazolo[4,3 -a]pyrazin-7(8H)-yl)(4-fluorophenyl)methanone**

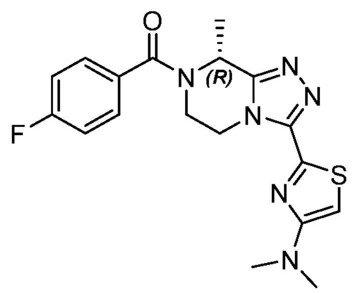

The preparation of Example **83** referred to Step 5 of Example **1.**

MS m/z (ESI): 386.9 $[M+H]^+$.

$^1$H NMR (400 MHz, Chloroform-d) δ7.45-7.52 (m, 2H), 7.14-7.18 (m, 2H), 5.84(s, 1H), 5.32-5.40 (m, 2H), 4.88-4.93 (m, 1H), 4.20-4.31 (m, 1H), 3.45-3.56 (m, 1H), 3.00(s, 6H), 1.74 (d, $J$ = 6.8 Hz, 3H).

**Example 84**

**(R)-(4-Fluorophenyl)(8-methyl-3-(5-methyl-4,5,6,7-tetrahydrothiazolo[5,4-c]p yridin-2-yl)-5,6-dihydro-[1,2,4]triazolo[4,3-a]pyrazin-7(8H)-yl)methanone**

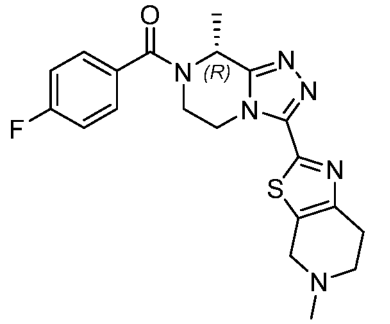

The preparation of Example **84** referred to Step 5 of Example **1.**

MS m/z (ESI): 413.0 $[M+H]^+$.

$^1H$ NMR (400 MHz, $CDCl_3$) δ 7.47 (t, J = 6.0 Hz, 2H), 7.16 (t, J = 8.0 Hz, 2H), 5.84-5.63 (br, 1H), 4.88 (d, J = 14.4 Hz , 1H), 4.65-4.45 ( br, 1H), 4.22 (m, 1H), 3.97-3.81 (br, 2H), 3.55-3.45 (m, 1H), 3.13-2.93 (br, 4H), 2.65 (s, 3H), 1.73 (d, J = 6.4 Hz, 3H).

**Example 85**

**(R)-(4-Chlorophenyl)(8-methyl-3-(5-methyl-4,5,6,7-tetrahydrothiazolo[5,4-c]p yridin-2-yl)-5,6-dihydro-[1,2,4]triazolo[4,3-a]pyrazin-7(8H)-yl)methanone**

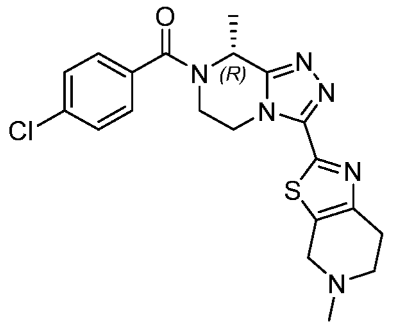

The preparation of Example **85** referred to Step 5 of Example **1.**

MS m/z (ESI): 429.0 $[M+H]^+$.

$^1H$ NMR (400 MHz, $CDCl_3$) δ 7.44 (d, J = 8.0 Hz, 2H), 7.41 (d, J = 8.0 Hz, 2H), 5.94-5.53 (br, 1H), 4.88 (d, J = 12.4 Hz , 1H), 4.58-4.48 ( m, 1H), 4.24-4.18 (m, 1H), 3.90-3.80 (m, 2H), 3.54-3.48 (m, 1H), 3.01-2.95 ( m, 4H), 2.62 (s, 3H), 1.72 (d, J = 6.4 Hz, 3H).

**Example 86**

**(R)-(4-Fluorophenyl)(3-(imidazo[2,1-b]thiazol-6-yl)-8-methyl-5,6-dihydro-[1,2, 4]triazolo[4,3-a]pyrazin-7(8H)-yl)methanone**

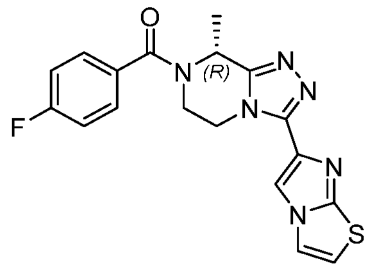

The preparation of Example **86** referred to Step 5 of Example **1.**

MS m/z (ESI): 383.2 $[M+H]^+$.

$^1H$ NMR (400 MHz, $CDCl_3$) δ 8.42 (s, 1H), 7.54 (d, J = 4.0 Hz, 1H), 7.51-7.47 (m , 2H), 7.16 (d, J = 8.0 Hz, 2H), 6.97 (d, J = 4.0 Hz, 1H),5.94-5.63 (br, 1H), 4.98 (d, J = 12.8 Hz , 1H), 4.69-4.40 (br, 1H), 4.32-4.25 (m, 1H), 3.58-3.51 (m, 1H) , 1.73 (d, J = 6.8 Hz, 3H).

**Example 87**

**(R)-(4-Chlorophenyl)(3-(6,7-dihydro-4H-pyrano[4,3-d]thiazol-2-yl)-8-methyl-5,6-dihydro-[1,2,4]triazolo[4,3-a]pyrazin-7(8H)-yl)methanone**

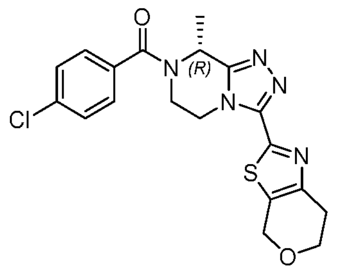

The preparation of Example 87 referred to Step 5 of Example 1.

MS m/z (ESI): 416.2 $[M+H]^+$.

$^1H$ NMR (400 MHz, Chloroform-d) δ 7.46-7.40 (m, 4H), 5.89-5.63 (m, 1H), 4.95-4.90 (m, 3H), 4.70-4.38 (m, 1H), 4.30-4.19 (m, 1H), 4.08-4.06 (m, 2H), 3.61-3.45 (m, 1H), 2.95 (s, 2H), 1.75 (d, J = 6.8 Hz, 3H).

**Example 88**

**(R)-(4-Fluorophenyl)(8-methyl-3-(pyrazolo[1,5-a]pyridin-2-yl)-5,6-dihydro-[1, 2,4]triazolo[4,3-a]pyrazin-7(8H)-yl)methanone**

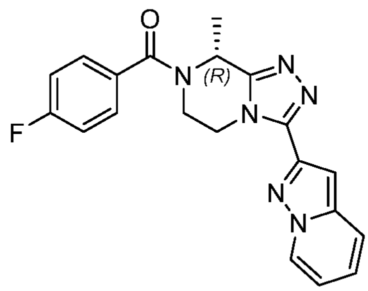

The preparation of Example **88** referred to Step 5 of Example **1.**

MS m/z (ESI): 377.2 $[M+H]^+$.

$^1H$ NMR (400 MHz, Chloroform-d) δ 8.42 (d, J = 6.8 Hz, 1H), 7.61 (d, J = 9.0 Hz, 1H), 7.56-7.43 (m, 2H), 7.26-7.14 (m, 4H), 6.86-6.82 (m, 1H), 5.75 (br s, 1H), 4.94 (d, J = 15.1 Hz, 1H), 4.76-4.50 (m, 1H), 4.38-4.24 (m, 1H), 3.62-3.47 (m, 1H), 1.75 (d, J = 6.9 Hz, 3H).

**Example 89**

**(R)-(3-(1,3-Dimethyl-1H-thieno[2,3-c]pyrazol-5-yl)-8-methyl-5,6-dihydro-[1,2, 4]triazolo[4,3-a]pyrazin-7(8H)-yl)(4-fluorophenyl)methanone**

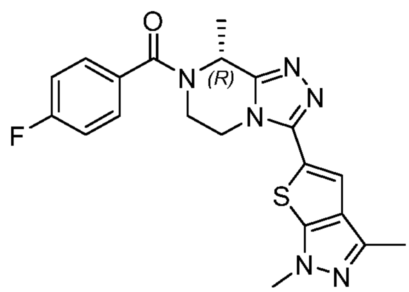

The preparation of Example **89** referred to Step 5 of Example **1.**
MS m/z (ESI): 411.2 $[M+H]^+$.
$^1H$ NMR (400 MHz, Chloroform-d) δ 7.65-7.58 (m, 1H), 7.58-7.49 (m, 2H), 7.20-7.15 (m, 2H), 5.80-5.66 (m, 1H), 4.71-4.57 (m, 1H), 4.47-4.34 (m, 2H), 3.92 (s, 3H), 3.79-3.71 (m, 1H), 2.43 (s, 3H), 1.82 (d, J = 6.8 Hz, 3H).

**Example 90**

**(R)-(4-Chlorophenyl)(3-(1,3-dimethyl-1H-thieno[2,3-c]pyrazol-5-yl)-8-methyl-5,6-dihydro-[1,2,4]triazolo[4,3-a]pyrazin-7(8H)-yl)methanone**

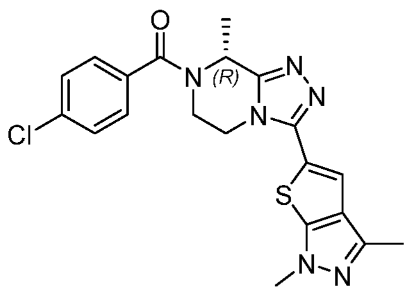

The preparation of Example **90** referred to Step 5 of Example **1.**
MS m/z (ESI): 427.2 $[M+H]^+$.
$^1H$ NMR (400 MHz, Chloroform-d) δ 7.52-7.43 (m, 4H), 7.39-7.34 (m, 1H), 5.64 (br s, 1H), 4.78-4.60 (m, 1H), 4.39-4.26 (m, 2H), 3.93 (s, 3H), 3.68-3.56 (m, 1H), 2.45 (s, 3H), 1.77 (d, J = 6.8 Hz, 3H).

**Example 91**

**(R)-(3,4-Dichlorophenyl)(3-(1,3-dimethyl-1H-thieno[2,3-c]pyrazol-5-yl)-8-met hyl-5,6-dihydro-[1,2,4]triazolo[4,3-a]pyrazin-7(8H)-yl)methanone**

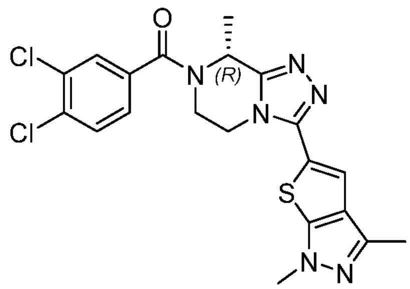

The preparation of Example **91** referred to Step 5 of Example **1.**

MS m/z (ESI): 461.2 $[M+H]^+$.

$^1H$ NMR (400 MHz, Chloroform-d) δ 7.62 (s, 1H), 7.57 (d, J = 8.1 Hz, 1H), 7.43-7.33 (m, 2H), 5.67 (br s, 1H), 4.73-4.57 (m, 1H), 4.39-4.28 (m, 2H), 3.93 (s, 3H), 3.71-3.59 (m, 1H), 2.44 (s, 3H), 1.78 (d, J = 6.5 Hz, 3H).

**Example 92**

**(R)-(4-Fluorophenyl)(3-(3-fluoropyrazolo [1,5-a] pyridin-2-yl)-8-methyl-5,6-dih ydro-[1,2,4]triazolo[4,3-a]pyrazin-7(8H)-yl)methanone**

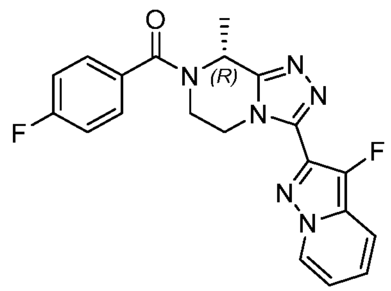

The preparation of Example **92** referred to Step 5 of Example **1.**

MS m/z (ESI): 394.8 $[M+H]^+$.

$^1H$ NMR (400 MHz, Chloroform-d) δ 8.28 (d, J = 7.1 Hz, 1H), 7.63 (d, J = 9.0 Hz, 1H), 7.55-7.45 (m, 2H), 7.22-7.11 (m, 3H), 6.88-6.86 (m, 1H), 5.88-5.66 (m, 1H), 4.87-4.78 (m, 1H), 4.70-4.50 (m, 1H), 4.38-4.26 (m, 1H), 3.55 (s, 1H), 1.80 (d, J = 5.2 Hz, 3H).

**Example 93**

**(R)-(4-Fluorophenyl)(3-(imidazo[1,2-a]pyridin-2-yl)-8-methyl-5,6-dihydro-[1,2 ,4]triazolo[4,3-a]pyrazin-7(8H)-yl)methanone**

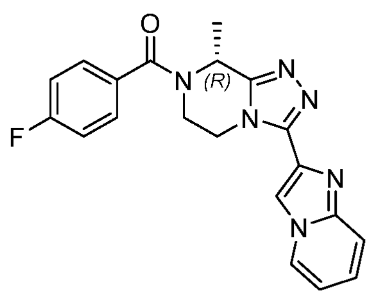

The preparation of Example **93** referred to Step 5 of Example **1.**

MS m/z (ESI): 377.2 [M+H]+.

$^1$H NMR (400 MHz, $CDCl_3$) δ 8.64-8.62 (m, 1H), 8.28-8.26 (m, 1H), 7.67 (d, J = 8.0 Hz, 1H), 7.52-7.49 (m, 2H), 7.34-7.32 (m, 1H), 7.19-7.15 (m, 2H), 6.96-6.94 (m, 1H), 5.85-5.80 (m, 1H), 5.20-5.15 (m, 1H), 4.54-4.40 (m, 2H), 3.63-3.57 (m, 1H), 1.75-1.74 (m, 3H).

**Example 94**

**(R)-(4-Chlorophenyl)(3-(imidazo[1,2-a]pyridin-2-yl)-8-methyl-5,6-dihydro-[1, 2,4]triazolo[4,3-a]pyrazin-7(8H)-yl)methanone**

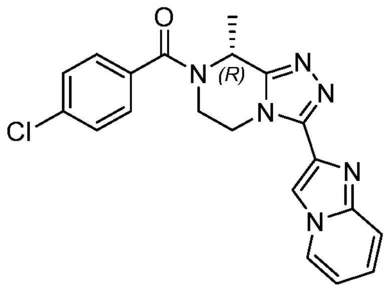

The preparation of Example **94** referred to Step 5 of Example **1.**

MS m/z (ESI): 393.2 [M+H]+.

$^1$H NMR (400 MHz, $CDCl_3$) δ 8.45-8.42 (m, 1H), 8.25-8.23 (m, 1H), 7.63 (d, J = 8.0 Hz, 1H), 7.47-7.43 (m, 3H), 7.31-7.28 (m, 2H), 6.95-6.90 (m, 1H), 5.79-5.72 (m, 1H), 5.15-5.08 (m, 1H), 4.56-4.34 (m, 2H), 3.58-3.54 (m, 1H), 1.75 (d, J = 4.0 Hz, 3H).

**Example 95**

**(R)-(3-(4,5-Dimethylthiazol-2-yl)-8-methyl-5,6-dihydro-[1,2,4]triazolo[4,3-a]py razin-7(8H)-yl)(4-fluorophenyl)methanone**

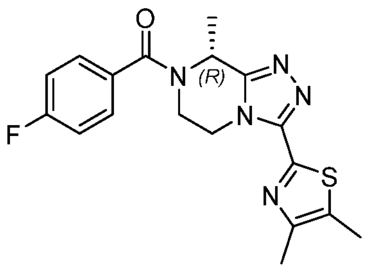

The preparation of Example **95** referred to Step 5 of Example **1.**

MS m/z (ESI): 372.2 [M+H]+.

$^1$H NMR (400 MHz, $CDCl_3$) δ 7.50-7.45 (m, 2H), 7.18-7.15 (m, 2H), 5.78-5.75 (m, 1H), 4.96-4.92 (m, 1H), 4.57-4.25 (m, 2H), 3.55-3.50 (m, 1H), 2.43 (s, 3H), 2.37 (s, 3H), 1.77-1.75 (m, 3H).

**Example 96**

**(R)-(4-Fluorophenyl)(8-methyl-3-(5-methylthiazol-2-yl)-5,6-dihydro-[1,2,4]tria zolo[4,3-a]pyrazin-7(8H)-yl)methanone**

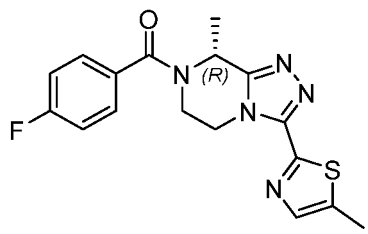

The preparation of Example **96** referred to Step 5 of Example **1.**

MS m/z (ESI): 358.1[M+H]$^+$.

$^1$H NMR (400 MHz, Chloroform-d) δ 7.55 (s, 1H), 7.52-7.42 (m, 2H), 7.21-7.09 (m, 2H), 5.87-5.58 (m, 1H), 4.98-4.82 (m, 1H), 4.71-4.35 (m, 1H), 4.32-4.13 (m, 1H), 3.59-3.42 (m, 1H), 2.55 (s, 3H), 1.74 (d, J = 6.7 Hz, 3H).

**Example 97**

**(R)-(4-Chlorophenyl)(3-(6-chlorothiazolo[4,5-c]pyridin-2-yl)-8-methyl-5,6-dihydro-[1,2,4]triazolo[4,3-a]pyrazin-7(8H)-yl)methanone**

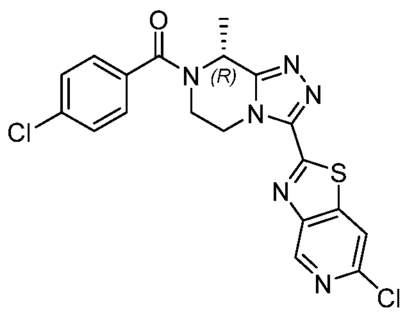

The preparation of Example **97** referred to Step 7 of Example **37.**

MS m/z (ESI): 444.9 [M+H]$^+$.

$^1$H NMR (400 MHz, Chloroform-d) δ 7.55 (s, 1H), 7.52-7.42 (m, 2H), 7.21-7.05.85-5.80 (m, 1H), 5.07-5.05 (m, 1H), 4.66-4.39 (m, 2H), 3.61-3.57 (m, 1H), 1.80-1.77 (m, 3H).

**Example 98**

**(R)-(3-(3-Bromo-1,2,4-thiadiazol-5-yl)-8-methyl-5,6-dihydro-[1,2,4]triazolo[4,3 -a]pyrazin-7(8H)-yl)(4-fluorophenyl)methanone**

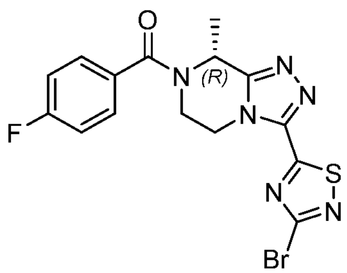

The preparation of Example **98** referred to Step 7 of Example **76.**

MS m/z (ESI): 422.9, 424.9 [M+H]$^+$.

$^1$H NMR (400 MHz, Chloroform-d) δ 7.44-7.53 (m, 2H), 7.16-7.20 (m, 2H), 5.78-5.98 (br, 1H), 4.82-4.95 (br, 1H), 4.48-4.67 (br, 1H), 4.20-4.38 (br, 1H), 3.47-3.65 (br, 1H), 1.75 (d, J = 5.6 Hz, 3H).

**Example 99**

**(R)-(4-Chlorophenyl-3-d)(3-(5,6-difluorobenzo[d]thiazol-2-yl)-8-methyl-5,6-di hydro-[1,2,4]triazolo[4,3-a]pyrazin-7(8H)-yl)methanone**

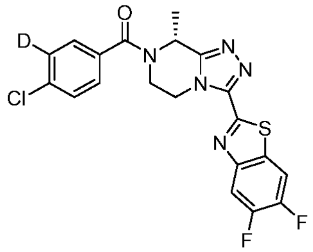

The preparation of Example 99 referred to Step 8 of Example 17.

MS m/z (ESI): 447.1[M+H]$^+$.

$^1$H NMR (400 MHz, Chloroform-d) δ 7.92-7.78 (m, 1H), 7.78-7.66 (m, 1H), 7.58-7.32 (m, 3H), 6.10-5.51 (m, 1H), 5.25-4.91 (m, 1H), 4.83-4.14 (m, 2H), 3.77-3.20 (m, 1H), 1.65-1.85 (m, 3H).

**Example 100**

**(R)-(3-(6-Chlorothiazolo[4,5-b]pyridin-2-yl)-8-methyl-5,6-dihydro-[1,2,4]triaz olo[4,3-a]pyrazin-7(8H)-yl)(4-fluorophenyl)methanone**

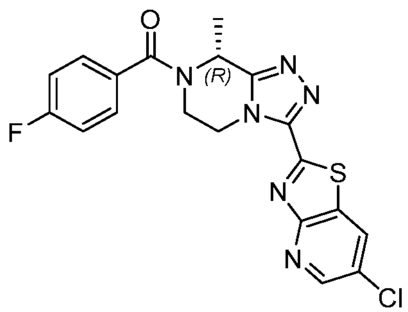

The preparation of Example **100** referred to Example **37.**

MS m/z (ESI): 429.0 [M+H]$^+$.

$^1$H NMR (400 MHz, Chloroform-d) δ 8.72 (d, J = 2.4 Hz, 1H), 8.33 (d, J = 2.4 Hz, 1H), 7.47-7.51 (m, 2H), 7.16-7.20 (m, 2H), 5.77-6.02 (m, 1H), 5.10-5.15 (m, 1H), 4.48-4.48 (m, 2H), 3.54-3.62 (m, 1H), 1.77 (d, J = 6.8 Hz, 3H).

**Example 101**

**(R)-(4-Chlorophenyl-3-d)(3-(6-fluorobenzo[d]thiazol-2-yl)-8-methyl-5,6-dihyd ro-[1,2,4]triazolo[4,3-a]pyrazin-7(8H)-yl)methanone**

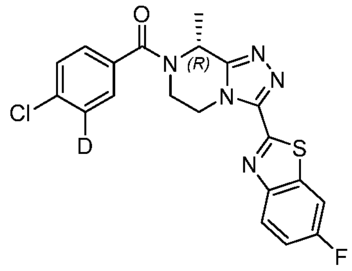

The preparation of Example **101** referred to Step 9 of Example 12.

MS m/z (ESI): 429.0 $[M+H]^+$.

$^1H$ NMR (400 MHz, Chloroform-d) δ 8.01-7.98 (m, 1H), 7.66-7.63 (m, 1H), 7.48-7.43 (m, 3H), 7.30-7.25 (m, 1H), 5.81-5.77 (m, 1H), 5.08-5.05 (m, 1H), 4.59-4.35 (m, 2H), 3.58-3.56 (m, 1H), 1.76 (d, J = 8.0 Hz, 3H).

**Example 102**

**(R)-(3-(6-Chlorobenzo[d]thiazol-2-yl)-8-methyl-5,6-dihydro-[1,2,4]triazolo[4,3 -a]pyrazin-7(8H)-yl)(4-chlorophenyl-3-d)methanone**

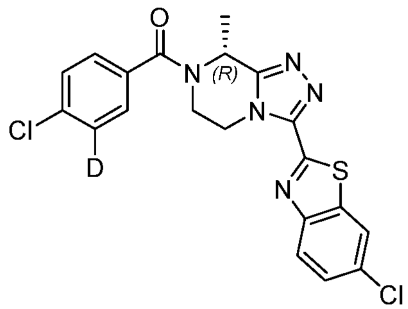

The preparation of Example 102 referred to Example 17.

MS m/z (ESI): 445.0 $[M+H]^+$.

$^1H$ NMR (400 MHz, Chloroform-d) δ 8.01-7.92 (m, 2H), 7.55-7.39 (m, 4H), 5.90-5.75 (m, 1H), 5.13-5.05 (m, 1H), 4.68-4.54 (m, 1H), 4.42-4.34 (m, 1H), 3.63-3.54 (m, 1H), 1.79 (d, J = 6.7 Hz, 3H).

**Example 103**

**(R)-(3-(6-Chlorobenzo[d]thiazol-2-yl)-8-methyl-5,6-dihydro-[1,2,4]triazolo[4,3 -a]pyrazin-7(8H)-yl)(4-fluorophenyl-3-d)methanone**

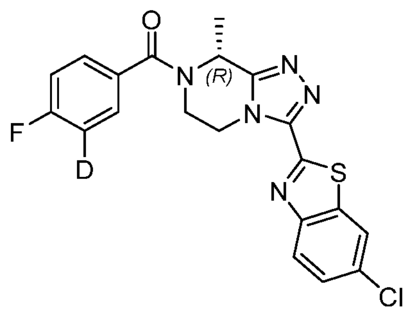

The preparation of Example 103 referred to Example 17.

MS m/z (ESI): 428.8 $[M+H]^+$.

$^1$H NMR (400 MHz, Chloroform-d) δ 8.00-7.91 (m, 2H), 7.55-7.45 (m, 3H), 7.21-7.12 (m, 1H), 5.79 (s, 1H), 5.07 (dd, J = 13.6, 3.8 Hz, 1H), 4.73-4.52 (m, 1H), 4.43-4.31 (m, 1H), 3.65-3.50 (m, 1H), 1.77 (d, J = 6.9 Hz, 3H).

**Example 104**

**(R)-(4-Fluorophenyl)(3-(6-fluorothiazolo[4,5-b]pyridin-2-yl)-8-methyl-5,6-dih ydro-[1,2,4]triazolo[4,3-a]pyrazin-7(8H)-yl)methanone**

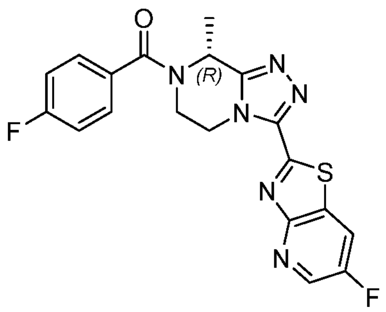

The preparation of Example 104 referred to Example 17.
MS m/z (ESI): 413.1 [M+H]$^+$.
$^1$H NMR (400 MHz, Chloroform-d) δ 8.65 (s, 1H), 8.06 (d, J = 7.1 Hz, 1H), 7.55-7.44 (m, 2H), 7.22-7.13 (m, 2H), 5.87 (s, 1H), 5.19-5.07 (m, 1H), 4.60-4.35 (m, 2H), 3.65-3.51 (m, 1H), 1.78 (br, 3H).

**Example 105**

**(R)-(3-(5-Chloro-6-fluorobenzo[d]thiazol-2-yl)-8-methyl-5,6-dihydro-[1,2,4]tri azolo[4,3-a]pyrazin-7(8H)-yl)(4-fluorophenyl-3-d)methanone**

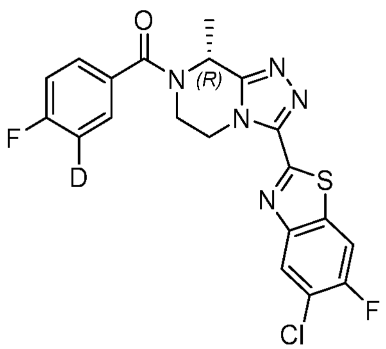

The preparation of Example 105 referred to Example 17.
MS m/z (ESI): 447.1 [M+H]$^+$.
$^1$H NMR (400 MHz, Chloroform-d) δ 8.11 (d, J = 6.5 Hz, 1H), 7.74 (d, J = 8.0 Hz, 1H), 7.53-7.46 (m, 2H), 7.21-7.15 (m, 1H), 5.81 (s, 1H), 5.09-5.01 (m, 1H), 4.69-4.57 (m, 1H), 4.41-4.33 (m, 1H), 3.63-3.54 (m, 1H), 1.78 (d, J = 6.9 Hz, 3H).

**Example 106**

**(R)-(3-(3-(Bis(methyl-d3)amino)-1,2,4-thiadiazol-5-yl)-8-methyl-5,6-dihydro-[ 1,2,4]triazolo[4,3-a]pyrazin-7(8H)-yl)(4-fluorophenyl)methanone**

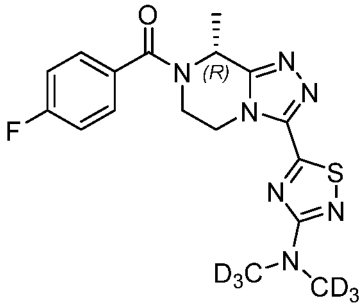

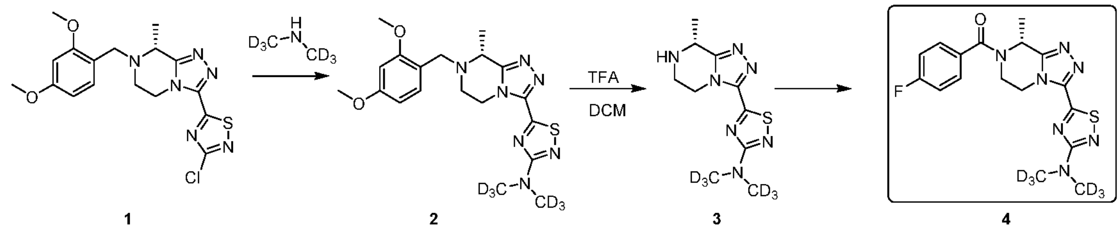


1 2 3 4

Step 1: Preparation of (R)-5-(7-(2,4-dimethoxybenzyl)-8-methyl-5,6,7,8-tetrahydro-[1,2,4]triazolo[4,3-a]pyraz in-3-yl)-N,N-bis(methyl-d3)-1,2,4-thiadiazol-3-amine

3-Chloro-5-[(8R)-7-[(2,4-dimethoxyphenyl)methyl]-8-methyl-6,8-dihydro-5H-[1,2 ,4]triazolo[4,3-a]pyrazin-3-yl]-1,2,4-thiadiazole (250 mg, 614.42 μmol) was dissolved in DMF (5 mL), and triethylamine (248.69 mg, 2.46 mmol, 342.79 μL) and bis(methyl-d3)amine hydrochloride (161.44 mg, 1.84 mmol) was added. The reaction solution was kept at 100°C in microwave for 1 hour, concentrated under reduced pressure, and then purified by silica gel flash chromatography (elution: PE:EA=1:1 to 1:4) to obtain the product (R)-5-(7-(2,4-dimethoxybenzyl)-8-methyl-5,6,7,8-tetrahydro-[1,2,4]triazolo[4,3-a]pyraz in-3-yl)-N,N-bis(methyl-d3)-1,2,4-thiadiazol-3-amine (200 mg, 474.44 μmol, yield: 77.22%) as a light yellow solid.
MS m/z (ESI): 422.0 $[M+H]^+$.

Steps 2 to 3 referred to Steps 4 to 5 of Example 1.

MS m/z (ESI): 394.0 $[M+H]^+$.
$^1H$ NMR (400 MHz, Chloroform-d) δ 7.53-7.44 (m, 2H), 7.21-7.13 (m, 2H), 5.71 (s, 1H), 4.86 (dd, J = 13.5, 3.8 Hz, 1H), 4.76-4.47 (m, 1H), 4.33-4.19 (m, 1H), 3.60-3.45 (m, 1H), 1.75 (d, J = 6.9 Hz, 3H).

**Example 107**

**(R)-(3-(3-(Bis(methyl-d3)amino)-1,2,4-thiadiazol-5-yl)-8-methyl-5,6-dihydro-[ 1,2,4]triazolo[4,3-a]pyrazin-7(8H)-yl)(4-chlorophenyl)methanone**

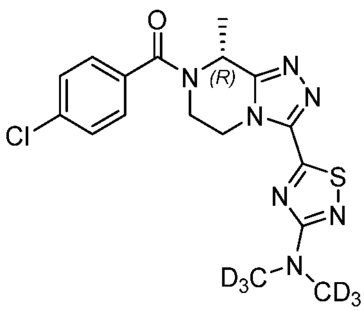

The preparation of Example 107 referred to Example 106.
MS m/z (ESI): 410.1$[M+H]^+$.

$^1H$ NMR (400 MHz, Chloroform-d) δ 7.49-7.37 (m, 4H), 5.71 (s, 1H), 4.86 (dd, J = 13.5, 3.8 Hz, 1H), 4.77-4.44 (m, 1H), 4.32-4.14 (m, 1H), 3.60-3.44 (m, 1H), 1.74 (d, J = 6.9 Hz, 3H).

**Example 108**

**(R)-(4-Fluorophenyl)(3-(6-fluorothiazolo[4,5-c]pyridin-2-yl)-8-methyl-5,6-dihy dro-[1,2,4]triazolo[4,3-a]pyrazin-7(8H)-yl)methanone**

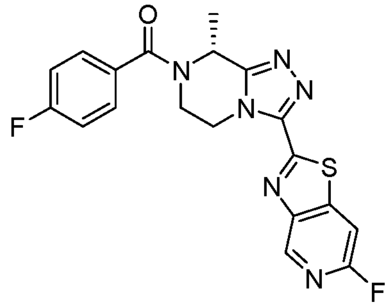

Step 1: 4,6-Difluoro-3-aminopyridine

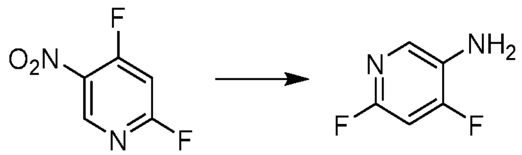

2,4-Difluoro-5-nitropyridine (1 g, 6.25 mmol) was dissolved in ethyl acetate (30 mL), and 10% palladium on carbon (0.1 g, water content 50%) was added under a nitrogen atmosphere. The mixture was hydrogenated at room temperature (1 atm) for 14 hours. The reaction solution was filtered to obtain the organic phase containing the crude product 4,6-difluoro-3-aminopyridine (0.81 g, 6.23 mmol). The organic phase was directly used in the next step.
MS m/z (ESI): 131.0[M+H]$^+$.

Step 2: Ethyl 2-((4,6-difluoropyridin-3-yl)amino)-2-oxoacetate

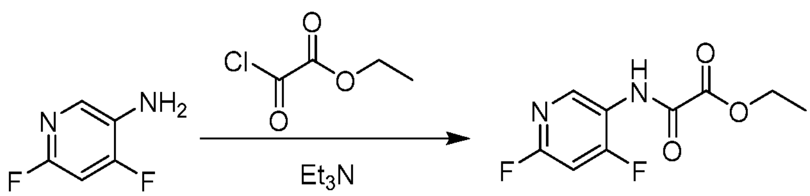


Ethyl oxalyl chloride (1.28 g, 9.34 mmol) was added to a solution of 4,6-difluoro-3-aminopyridine (0.81 g, 6.23 mmol) and triethylamine (2.6 mL, 18.7 mmol) in ethyl acetate (30 mL) at 0°C under a nitrogen atmosphere. The mixture was stirred at 0°C for 30 min. The reaction was quenched with saturated brine (100 mL). The mixture was separated into two phases, and the aqueous phase was extracted with ethyl acetate (20 mL×3). The organic phases were combined, washed with saturated brine (50 mL), dried overanhydrous sodium sulfate and concentrated under reduced pressure to obtain the target product ethyl 2-((4,6-difluoropyridin-3-yl)amino)-2-oxoacetate (1.16 g, 5.04 mmol, yield: 80.94%) as a colorless oil.
MS m/z (ESI): 231.0[M+H]$^+$.

Step 3: Ethyl 6-fluorothiazolo[4,5-c]pyridine-2-carboxylate

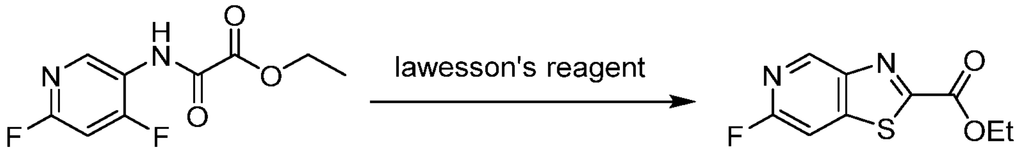


Ethyl 2-((4,6-difluoropyridin-3-yl)amino)-2-oxoacetate (1.16 g, 5.04 mmol) was dissolved in toluene (30 mL), and Lawesson's reagent (1.22 g, 3.02 mmol) was added under a nitrogen atmosphere. The mixture was stirred at 110°C for 2 hours. The reaction solution was cooled to room temperature and concentrated under reduced pressure. The residue was purified by silica gel flash chromatography (elution: petroleum ether: ethyl acetate = 5:1) to obtain the target product ethyl 6-fluorothiazolo[4,5-c]pyridine-2-carboxylate (0.43 g, 1.90 mmol, yield: 37.71%) as a white solid.

MS m/z (ESI): 227.0[M+H]$^+$.

Step 4: 6-Fluorothiazolo[4,5-c]pyridine-2-carbohydrazide

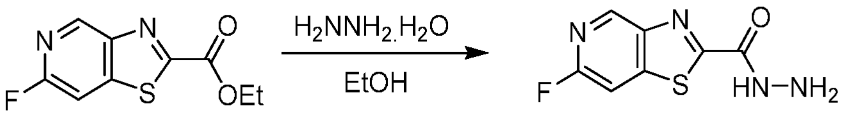


Ethyl 6-fluorothiazolo[4,5-c]pyridine-2-carboxylate (0.43 g, 1.90 mmol) was dissolved in ethanol (15 mL), and a 85% hydrazine hydrate (167.64 mg, 2.85 mmol) solution was added. The mixture was stirred at 25°C for 1 hr. The reaction solution was filtered to obtain the solid product 6-fluorothiazolo[4,5-c]pyridine-2-carbohydrazide (0.33 g, 1.56 mmol, yield: 81.82%) as a light yellow solid.

MS m/z (ESI): 213.0[M+H]$^+$.

The subsequent steps referred to the corresponding steps of Example 1, and (R)-(4-fluorophenyl)(3-(6-fluorothiazolo[4,5-c]pyridin-2-yl)-8-methyl-5,6-dihydro-[1,2, 4]triazolo[4,3-a]pyrazin-7(8H)-yl)methanone was obtained.

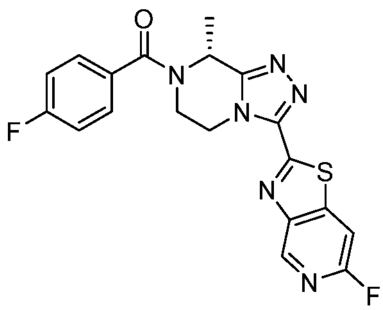

MS m/z (ESI): 413.1[M+H]$^+$.

$^1$H NMR (400 MHz, Chloroform-d) δ 8.95 (s, 1H), 7.56-7.44 (m, 3H), 7.24-7.09 (m, 2H), 6.07-5.58 (m, 1H), 5.19-4.94 (m, 1H), 4.80-4.46 (m, 1H), 4.46-4.26 (m, 1H), 3.78-3.35 (m, 1H), 1.78 (d, J = 6.9 Hz, 3H).

## Example 109

**(R)-5-(7-(4-Fluorobenzoyl)-8-methyl-5,6,7,8-tetrahydro-[1,2,4]triazolo[4,3-a]p yrazin-3-yl)-1,2,4-thiadiazole-3-carbonitrile**

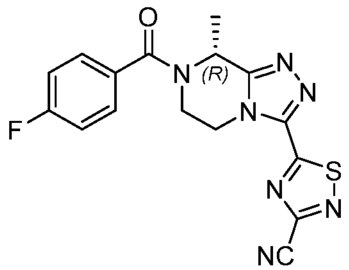

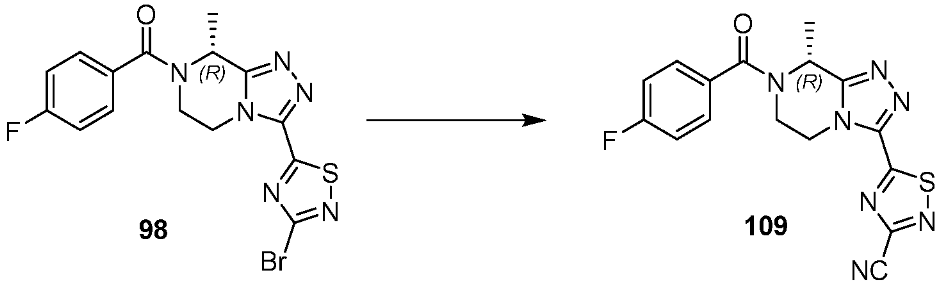

98

109

(R)-(3-Bromo-1,2,4-thiadiazol-5-yl)-8-methyl-5,6-dihydro-[1,2,4]triazolo[4,3-a]py razin-7(8H)-yl)(4-fluorophenyl)methanone (Example 98, 0.05 g, 0.118 mmol), tris(dibenzylideneacetone)dipalladium (0.011 g, 0.012 mmol), 4,5-bis(diphenylphosphino)-9,9-dimethylxanthene ( 0.014 g, 0.024 mmol), zinc cyanide (0.042 g, 0.35 mmol) and zinc powder (0.015 g, 0.24 mmol) were dissolved in N,N-dimethylformamide (4 mL). The reaction system was purged with nitrogen, and the reaction solution was heated to 100°C and kept overnight, and then cooled to room temperature. LCMS indicated the completion of the reaction. The reaction solution was diluted with ethyl acetate (25 mL), filtered, and the solids were washed with ethyl acetate. The organic phases were combined, washed with saturated brine (10 mL×5), dried over anhydrous sodium sulfate, filtered and subjected to rotatory evaporation until dry. The residue was separated by preparative thin layer chromatography (dichloromethane: methanol = 20:1). Then the crude product was separated by preparative high performance liquid chromatography and lyophilized to obtain (R)-5-(7-(4-fluorobenzoyl)-8-methyl-5,6,7,8-tetrahydro-[1,2,4]triazolo[4,3-a]pyrazin-3-yl)-1,2,4-thiadiazole-3-carbonitrile (4 mg, white solid, yield): 9.2%).

MS m/z (ESI): 370.0 $[M+H]^+$.

$^1$H NMR (400 MHz, Chloroform-d) δ7.47-7.51 (m, 2H), 7.16-7.21 (m, 2H), 5.76-5.97 (br, 1H),4.86-4.90(m, 1H), 4.53-4.69 (br, 1H), 4.28-4.53 (m, 1H), 3.53-3.61 (m, 1H), 1.77 (d, J = 6.8 Hz, 3H).

**Example 110**

**(R)-(3-(6-Chlorothiazolo[5,4-c]pyridin-2-yl)-8-methyl-5,6-dihydro-[1,2,4]triaz olo[4,3-a]pyrazin-7(8H)-yl)(4-fluorophenyl)methanone**

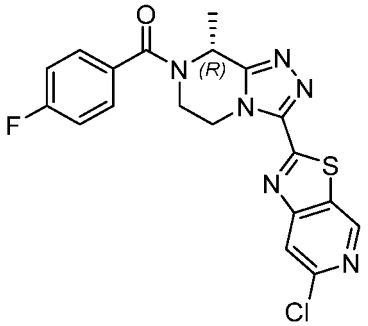

The preparation of Example 110 referred to Example 37.

MS m/z (ESI): 429.0 $[M+H]^+$.

$^1$H NMR (400 MHz, Chloroform-d) δ 9.07 (s, 1H), 7.98 (s, 1H), 7.52-7.49 (m, 2H), 7.21-7.13 (m, 2H), 5.86-5.82 (m, 1H), 5.07-5.04 (m, 1H), 4.63-4.39 (m, 2H), 3.60-3.57 (m, 1H), 1.80-1.79 (m, 3H).

**Example 111**

**(R)-(4-Fluorophenyl)(8-methyl-3-(6-methylthiazolo[4,5-c]pyridin-2-yl)-5,6-dih ydro-[1,2,4]triazolo[4,3-a]pyrazin-7(8H)-yl)methanone**

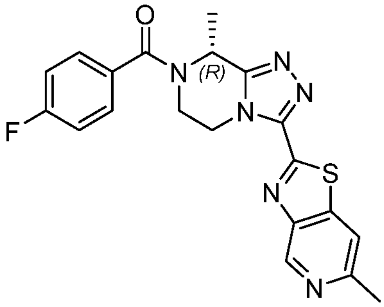

The preparation of Example 111 referred to Example 37.

MS m/z (ESI): 409.0 $[M+H]^+$.

$^1$H NMR (400 MHz, Chloroform-d) δ 9.27 (s, 1H), 7.85 (s, 1H), 7.51-7.49 (m, 2H), 7.18-7.16 (m, 2H), 5.84-5.81 (m, 1H), 5.09-5.06 (m, 1H), 4.62-4.39 (m, 2H), 3.60-3.58 (m, 1H), 2.81 (s, 3H), 1.78 (d, J = 8.0 Hz, 3H).

**Example 112**

**(R)-(4-Fluorophenyl)(3-(3-(isopropyl(methyl)amino)-1,2,4-thiadiazol-5-yl)-8-m ethyl-5,6-dihydro-[1,2,4]triazolo[4,3-a]pyrazin-7(8H)-yl)methanone**

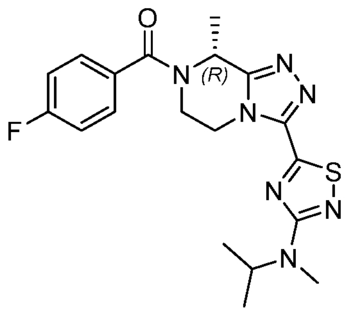

The preparation of Example 112 referred to Example 106.

MS m/z (ESI): 416.0 $[M+H]^+$.

$^1$H NMR (400 MHz, Chloroform-d) δ 7.52-7.45 (m, 2H), 7.21-7.14 (m, 2H), 5.71 (s, 1H), 4.89-4.82 (m, 1H), 4.78-4.60 (m, 2H), 4.34-4.21 (m, 1H), 3.58-3.48 (m, 1H), 3.06 (s, 3H), 1.77 (d, J = 6.9 Hz, 3H), 1.24 (d, J = 6.7 Hz, 6H).

**Example 113**

**(R)-(3-(3-(3,3-Difluoroazetidin-1-yl)-1,2,4-thiadiazol-5-yl)-8-methyl-5,6-dihydr o-[1,2,4]triazolo[4,3-a]pyrazin-7(8H)-yl)(4-fluorophenyl-3-d)methanone**

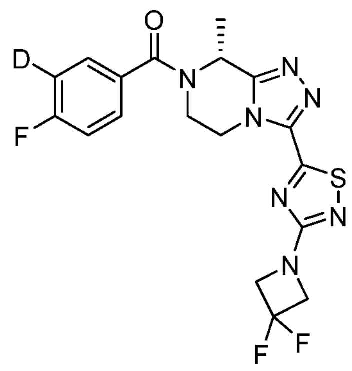

**Step 1: (R)-3-(3,3-Difluoroazetidin-1-yl)-5-(7-(2,4-dimethoxybenzyl)-8-methyl-5,6,7,8-tetra hydro-[1,2,4]triazolo[4,3-a]pyrazin-3-yl)-1,2,4-thiadiazole**

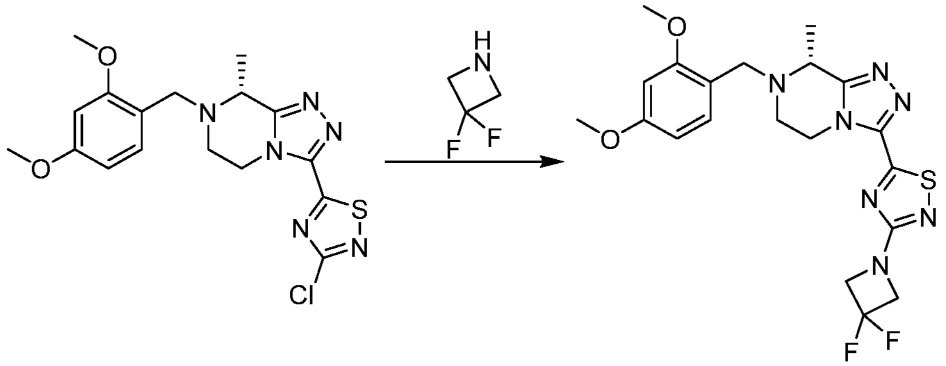

(R)-3-chloro-5-(7-(2,4-dimethoxybenzyl)-8-methyl-5,6,7,8-tetrahydro-[1,2,4]triazo lo[4,3-a]pyrazin-3-yl)-1,2,4-thiadiazole (75 mg, 184.33 μmol), 3,3-difluoroazetidine hydrochloride (238.77 mg, 1.84 mmol) and N,N-diisopropylethylamine (476.46 mg, 3.69 mmol, 642.12 μL) were dissolved in N,N-dimethylformamide (5 mL), and cesium carbonate (180.17 mg, 552.98 μmol) was added. The mixture was kept at 110°C in microwave for 1 hour. The reaction solution was concentrated under reduced pressure. The residue was purified by silica gel flash chromatography (dichloromethane: methanol=20:1) to obtain the target product (R)-3-(3,3-difluoroazetidin-1-yl)-5-(7-(2,4-dimethoxybenzyl)-8-methyl-5,6,7,8-tetrahyd ro-[1,2,4]triazolo[4,3-a]pyrazin-3-yl)-1,2,4-thiadiazole (60 mg, 129.45 μmol, yield: 70.23%).
MS m/z (ESI): 464.2[M+H]$^+$.
The subsequent steps referred to the corresponding steps of Example 1, and (R)-(3-(3-(3,3-difluoroazetidin-1-yl)-1,2,4-thiadiazol-5-yl)-8-methyl-5,6-dihydro-[1,2,4] triazolo[4,3-a]pyrazin-7(8H)-yl)(4-fluorophenyl-3-d)methanone was obtained.

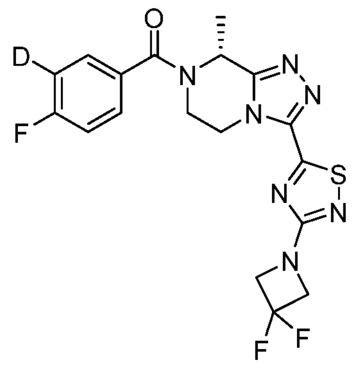

MS m/z (ESI): 437.1[M+H]$^+$.
$^1$H NMR (400 MHz, Chloroform-d) δ 7.55-7.41 (m, 2H), 7.23-7.11 (m, 1H), 5.97-5.51 (m, 1H), 4.90-4.76 (m,

1H), 4.71-4.44 (m, 5H), 4.35-4.12 (m, 1H), 3.62-3.38 (m, 1H), 1.75 (d, J = 6.9 Hz, 3H).

**Example 114**

**(R)-(4-Fluorophenyl-3-d)(8-methyl-3-(3-(pyrrolidin-1-yl)-1,2,4-thiadiazol-5-yl) -5,6-dihydro-[1,2,4]triazolo[4,3-a]pyrazin-7(8H)-yl)methanone**

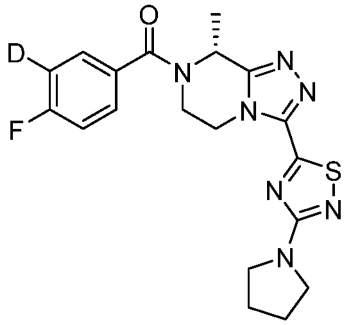

The preparation of Example 114 referred to Example 113.
MS m/z (ESI): 415.2[M+H]$^+$.
$^1$H NMR (400 MHz, Chloroform-d) δ 7.55-7.42 (m, 2H), 7.21-7.10 (m, 1H), 5.91-5.47 (m, 1H), 4.97-4.79 (m, 1H), 4.79-4.34 (m, 1H), 4.33-4.13 (m, 1H), 3.74-3.58 (m, 4H), 3.58-3.42 (m, 1H), 2.13-1.95 (m, 4H), 1.75 (d, J = 6.9 Hz, 3H).

**Example 115**

**(R)-(3-(3-(Cyclopropyl(methyl)amino)-1,2,4-thiadiazol-5-yl)-8-methyl-5,6-dihy dro-[1,2,4]triazolo[4,3-a]pyrazin-7(8H)-yl)(4-fluorophenyl)methanone**

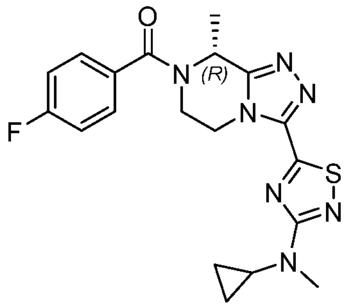

The preparation of Example 115 referred to Example 106.
MS m/z (ESI): 414.0 [M+H]$^+$.
$^1$H NMR (400 MHz, Chloroform-d) δ 7.53-7.42 (m, 2H), 7.19-7.15 (m, 2H), 5.70 (s, 1H), 4.90 (dd, J = 13.6, 3.8 Hz, 1H), 4.81-4.49 (m, 1H), 4.32-4.19 (m, 1H), 3.59-3.45 (m, 1H), 3.22 (s, 3H), 2.82-2.74 (m, 1H), 1.75 (d, J = 6.9 Hz, 3H), 0.91-0.84 (m, 2H), 0.79-0.71 (m, 2H).

**Example 116**

**(R)-(4-Fluorophenyl-3-d)(8-methyl-3-(4-(pyrrolidin-1-yl)thiazol-2-yl)-5,6-dihy dro-[1,2,4]triazolo[4,3-a]pyrazin-7(8H)-yl)methanone**

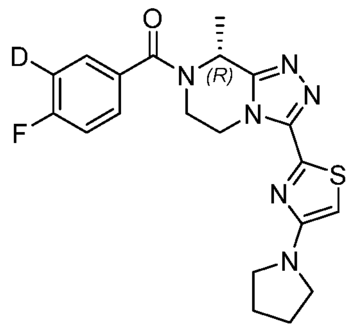

The preparation of Example 116 referred to Example 60.

MS m/z (ESI): 414.1 $[M+H]^+$.

$^1H$ NMR (400 MHz, Chloroform-d) δ7.44-7.52 (m, 2H), 7.17 (t, J = 6.8 Hz, 1H), 6.60-6.81 (br, 1H), 5.63-5.80 (br, 1H), 4.85-4.89 (m, 1H), 4.54-4.76 (br, 1H), 4.21-4.38 (m, 1H), 3.45-3.61 (br, 5H), 2.10-2.21 (br, 4H), 1.77 (d, J = 6.8 Hz, 3H).

**Example 117**

**(R)-(3-(4-(3-Fluoroazetidin-1-yl)thiazol-2-yl)-8-methyl-5,6-dihydro-[1,2,4]triaz olo[4,3-a]pyrazin-7(8H)-yl)(4-fluorophenyl-3-d)methanone**

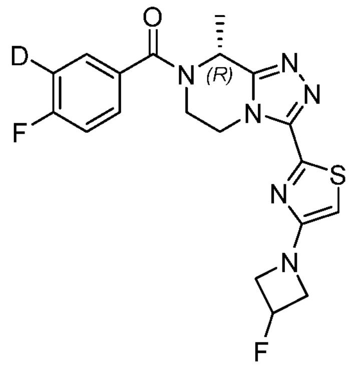

The preparation of Example 117 referred to Example 60.

MS m/z (ESI): 418.0 $[M+H]^+$.

$^1H$ NMR (400 MHz, Chloroform-d) δ7.43-7.52 (m, 2H), 7.14-7.19 (m, 1H), 6.01(s, 1H), 5.66-5.83 (br, 1H), 5.33-5.54 (m, 1H),4.85-4.89 (m, 1H), 4.50-4.70 (br, 1H), 4.19-4.36 (m, 3H), 4.04-4.13 (m, 2H), 3.43-3.59 (m, 1H),1.77 (d, J = 6.8 Hz, 3H).

**Example 118**

**(R)-(3-(3-Fluoroazetidin-1-yl)-1,2,4-thiadiazol-5-yl)-8-methyl-5,6-dihydro-[1,2, 4]triazolo[4,3-a]pyrazin-7(8H)-yl)(4-fluorophenyl-3-d)methanone**

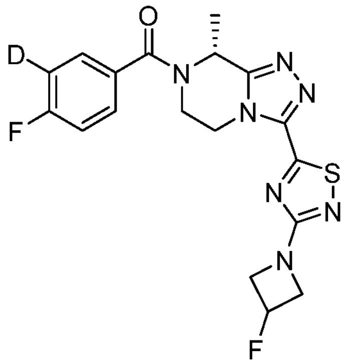

The preparation of Example 118 referred to Example 113.

MS m/z (ESI): 419.1[M+H]+.

$^1$H NMR (400 MHz, Chloroform-d) δ 7.58-7.39 (m, 2H), 7.23-7.12 (m, 1H), 5.92-5.62 (m, 1H), 5.58 -5.33 (m, 1H), 4.90-4.76 (m, 1H), 4.72-4.03 (m, 6H), 3.63-3.40 (m, 1H), 1.75 (d, J = 6.9 Hz, 3H).

**Example 119**

**(R)-(3-(3-(Azidin-1-yl)-1,2,4-thiadiazol-5-yl)-8-methyl-5,6-dihydro-[1,2,4]triaz olo[4,3-a]pyrazin-7(8H)-yl)(4-fluorophenyl-3-d)methanone**

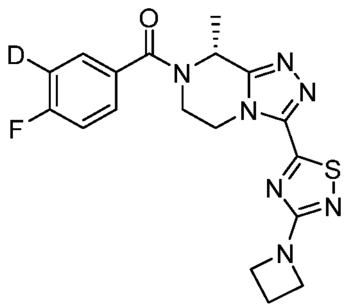

The preparation of Example 119 referred to Example 113.

MS m/z (ESI):401.1 [M+H]+.

$^1$H NMR (400 MHz, Chloroform-d) δ 7.55-7.40 (m, 2H), 7.23-7.12 (m, 1H), 5.85-5.62 (m, 1H), 4.91-4.78 (m, 1H), 4.75-4.52 (m, 1H), 4.35-4.12 (m, 4H), 3.74-3.59 (m, 1H), 3.59-3.43 (m, 1H), 2.54-2.38 (m, 2H), 1.75 (d, J = 6.9 Hz, 3H).

**Example 120**

**(R)-(4-Fluorophenyl)(8-methyl-3-(6-methylthiazolo[5,4-c]pyridin-2-yl)-5,6-dih ydro-[1,2,4]triazolo[4,3-a]pyrazin-7(8H)-yl)methanone**

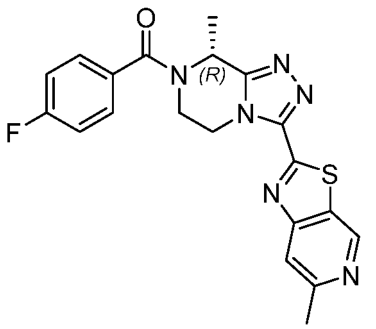

(R)-(3-(6-Chlorothiazolo[5,4-c]pyridin-2-yl)-8-methyl-5,6-dihydro-[1,2,4]triazol o[4,3-a]pyrazin-7(8H)-yl)(4-fluorophenyl)methanone (20 mg, 46.63 $\mu$mol), dimethylzinc (1.0 M, 233.17 $\mu$L) and Pd(dppf)$Cl_2$ (6.82 mg, 9.33 $\mu$mol) were dissolved in 1,4-dioxane (3 mL). The reaction system was purged with nitrogen, and and the reaction solution was stirred at 100°C for 5 hours. LCMS showed the formation of the product. The reaction was quenched with saturated brine (5 mL), and the aqueous phase was extracted with DCM (5 mL×3). The organic phases were combined, dried over anhydrous sodium sulfate and concentrated under reduced pressure. The residue was purified with pre-HPLC to obtain (R)-(4-fluorophenyl)(8-methyl-3-(6-methylthiazolo[5,4-c]pyridin-2-yl)-5,6-dihydro-[1,2 ,4]triazolo[4,3-a]pyrazin-7(8H)-yl)methanone (1.2 mg, 2.94 $\mu$mol, yield: 6.30%) as a light pink solid.
MS m/z (ESI): 409.0 $[M+H]^+$.

**Example 121**

**(R)-(3-(4-Chlorothiazol-2-yl)-8-methyl-5,6-dihydro-[1,2,4]triazolo[4,3-a]pyrazi n-7(8H)-yl)(4-fluorophenyl-3-d)methanone**

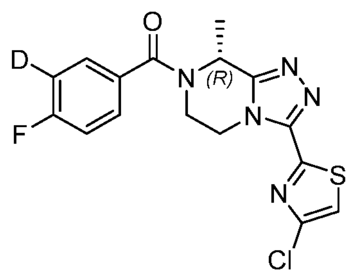

The preparation of Example 121 referred to Example 55.
MS m/z (ESI): 379.0 $[M+H]^+$.
$^1$H NMR (400 MHz, Chloroform-d) $\delta$7.45-7.52 (m, 2H), 7.24 (s, 1H), 7.17 (t, J = 8.4 Hz, 1H), 5.69-5.99 (br, 1H), 4.89-4.92 (m, 1H), 4.52-4.70 (br, 1H), 4.19-4.37 (br, 1H), 3.45-3.67 (m, 1H), 1.77 (d, J = 6.0 Hz, 3H).

**Example 122**

**(R)-(3-(3-Chloro-1,2,4-thiadiazol-5-yl)-8-methyl-5,6-dihydro-[1,2,4]triazolo[4, 3-a]pyrazin-7(8H)-yl)(4-fluorophenyl-2-d)methanone**

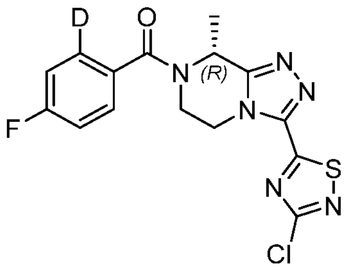

The preparation of Example **122** referred to Example **56.**
MS m/z (ESI): 380.0 $[M+H]^+$.

## BIOLOGICAL ASSAYS AND EVALUATION

The present invention is further described with reference to the following test examples, but these examples should not be considered as limiting the scope of the present invention.

### I. Cell function experiments

### Test Example 1. Determination of the effect of the compounds of the present invention on calcium ion fluidity in cells with stable expression of NK3 receptors

Experimental objective: The objective of this test example was to determine the inhibitory effect of the compounds on NK3 receptors.
Experimental instruments:

384-well plate (Corning, 3712);
Pipettes (Axygen);
FLIPR (Molecular Devices).

Experimental reagents:

DMEM (Invitrogen, 11965);
Fetal bovine serum (Biowest, S1810-500);
Dialyzed serum (S-FBS-AU-065, Serana);
Penicillin and streptomycin (Biowest, L0022-100);
Hygromycin B (CABIOCHEM, 400052);
Matrigel (BD, 354230);
DMSO (Sigma, D2650);
HBSS (Invitrogen, 14065);
HEPES (Invitrogen, 15630080);
Probenecid (Sigma, P8761);
BSA (renview, FA016);
Trypsin (HDB, 0458).

Experimental methods:

1. Buffer preparation: 1× HBSS, 20 mM HEPES, 2.5 mM probenecid (probenecid was 400 mM stock in 1 M NaOH), 0.1% BSA. Probenecid and BSA were freshly added on the day of the experiment. The buffers for the experiments included dye buffer, compound dilution buffer and the like.
2. The cells were digested with trypsin and then seeded in a 384-well plate at a density of $1\times10^4$ cells/well and incubated for 16-24 hours (at least overnight).
3. The culture medium was discarded, and 20 μL of dye was added. The cells were incubated at 37°C in the dark for 60 min, and then the calcium signal was read.
4. The antagonist was prepared before the experiments. 5× concentration of the antagonist compound was added

to the 384-well plate at 5 μL/well, and the cells were incubated at room temperature in the dark for 15 min. The plate was transferred to FLIPR, and 6× concentration of the agonist compound was added at 5 μL/well. The values were read and the data were saved by using FLIPR. The total assay volume was 30 μL, comprising 20 μL/well of dye buffer, 5 μL/well of 5× concentration of the experimental compound, and 5 μL/well of 6× concentration of the agonist compound.

Experimental data processing procedures:
The calcium signal value was read by FLIPR. The output of each sampling time point in the experiment was calculated as the ratio of the signals at wavelengths of 340/510 nm to 380/510 nm. The maximum minus minimum value was calculated from the ratio signal curve. The $IC_{50}$ value of the compound was calculated by fitting the data of inhibition rate percentage and ten concentrations to the parametric nonlinear logistic equation by using GraphPad prism.
Experimental results:

Table 1. The $IC_{50}$ values of the compounds on the calcium ion fluidity in cells with stable expression of NK3 receptors

| Example No. | NK3R $IC_{50}$ (nM) FLIPR |
|---|---|
| Fezolinetant | 904.32 |
| 1 | 76.23 |
| 2 | 58.23 |
| 3 | 30.53 |
| 4 | 61.32 |
| 5 | 158.92 |
| 9 | 33.30 |
| 10 | 51.39 |
| 11 | 47.49 |
| 12 | 37.05 |
| 13 | 71.61 |
| 14 | 97.62 |
| 17 | 6.81 |
| 18 | 129.61 |
| 19 | 10.92 |
| 20 | 60.99 |
| 21 | 122.79 |
| 22 | 73.33 |
| 23 | 97.76 |
| 24 | 45.57 |
| 25 | 58.44 |
| 26 | 96.72 |
| 29 | 65.78 |
| 30 | 47.38 |
| 31 | 86.70 |
| 37 | 48.90 |
| 38 | 246.62 |
| 40 | 297.93 |

(continued)

| Example No. | NK3R $IC_{50}$ (nM) FLIPR |
|---|---|
| 41 | 223.61 |
| 44 | 255.21 |
| 46 | 107.44 |
| 49 | 86.69 |
| 50 | 146.99 |
| 53 | 43.22 |
| 55 | 219.07 |
| 56 | 154.85 |
| 57 | 60.98 |
| 58 | 182.87 |
| 60 | 204.90 |
| 61 | 78.92 |
| 62 | 73.63 |
| 73 | 266.65 |
| 97 | 156.36 |
| 98 | 78.36 |
| 99 | 56.16 |
| 101 | 86.65 |
| 102 | 32.50 |
| 103 | 27.07 |
| 105 | 268.44 |
| 107 | 183.95 |

Experimental conclusion:
It can be seen from the data in the table that the compounds of the examples of the present invention showed good inhibitory activity in the experiment on calcium flow in cells with stable expression of NK3.

**Test Example 2. Determination of the effect of the compounds of the present invention on calcium ion fluidity in cells with stable expression of NK1/NK2 receptors**

Experimental objective: The objective of this test example was to determine the inhibitory effect of the compounds on NK1/NK2 receptors.

Experimental instruments:

384-well plate (Corning, 3712);
Pipettes (Axygen);
FLIPR (Molecular Devices).

Experimental reagents:

DMEM (Invitrogen, 11965);
Fetal bovine serum (Biowest, S1810-500);
Dialyzed serum (S-FBS-AU-065, Serana);
Penicillin and streptomycin (Biowest, L0022-100);

Hygromycin B (CABIOCHEM, 400052);
Matrigel (BD, 354230);
DMSO (Sigma, D2650);
HBSS (Invitrogen, 14065);
HEPES (Invitrogen, 15630080);
Probenecid (Sigma, P8761);
BSA (renview, FA016);
Trypsin (HDB, 0458).

Experimental methods:

1. Buffer preparation: 1× HBSS, 20 mM HEPES, 2.5 mM probenecid (probenecid was 400 mM stock in 1 M NaOH), 0.1% BSA. Probenecid and BSA were freshly added on the day of the experiment. The buffers for the experiments included dye buffer, compound dilution buffer and the like.
2. The cells were digested with trypsin and then seeded in a 384-well plate at a density of $1\times10^4$ cells/well and incubated for 16-24 hours (at least overnight).
3. The culture medium was discarded, and 20 μL of dye was added. The cells were incubated at 37°C in the dark for 60 min, and then the calcium signal was read.
4. The antagonist was prepared before the experiments. Mode of the antagonist: 5× concentration of the antagonist compound was added to the 384-well plate at 5 μL/well, and the cells were incubated at room temperature in the dark for 15 min. The plate was transferred to FLIPR, and 6× concentration of the agonist compound was added at 5 μL/well. The values were read and the data were saved by using FLIPR. The total assay volume was 30 μL, comprising 20 μL/well of dye buffer, 5 μL/well of 5× concentration of the experimental compound, and 5 μL/well of 6× concentration of the agonist compound.

Experimental data processing procedures:
The calcium signal value was read by FLIPR. The output of each sampling time point in the experiment was calculated as the ratio of the signals at wavelengths of 340/510 nm to 380/510 nm. The maximum minus minimum value was calculated from the ratio signal curve. The $IC_{50}$ value of the compound was calculated by fitting the data of inhibition rate percentage and ten concentrations to the parametric nonlinear logistic equation by using GraphPad prism.
Experimental results:
The compounds of the examples of the present invention exhibited both NK1R $IC_{50}$ (nM) and NK2R $IC_{50}$ (nM) values of >10,000 on calcium ion fluidity in cells with stable expression of NK1/NK2 receptors.
Experimental conclusion:
The compounds of the examples of the present invention showed good selectivity in the experiment on calcium flow in cells with stable expression of NK3 and NK1/NK2 receptors.

**Test Example 3. Determination of the effect of the compounds of the present invention on IP1 in HEK293 cells with stable expression of NK3**

1. Experimental objective:
The objective of this test example was to determine the antagonism effect of the compounds on the activity of HEK293-NK3 cells.
2. Experimental instruments and reagents:

2.1 Experimental instruments and consumables:

384-well cell culture plate (Corning, 3824);
384-well Echo compound plate (Labcyte, LP-0200);
Bravo Tips (Agilent, 10734-202);
EnVision Multilabel Reader (PE, 2104-0010);
Bravo (Agilent) and ECHO 550 (LABCYTE) pipetting stations;
Liquid dispensers (Multidrop Combi).

2.2 Experimental reagents:

DMEM, high glucose (Gibco, 12100);
Fetal bovine serum (Biosera, FB-1058/500);
P/S (Biosera, XC-A4122);
5X Matrigel (Corning, 354230);
IP-ONE Gq Kit (Cisbio, 62IPAPEJ);
The agonist Senktide (MCE, HY-P0187);
The positive control compound Talnetant (MCE, HY-14552);
Complete medium: DMEM + 10% FBS + 1× P/S; 1X Matrigel: 5× Matrigel diluted with DMEM;
Cell line: HDB HEK293-NK3, HD Biosciences (Shanghai) Co., Ltd.

3. Experimental methods:

1. 150 mm Petri dishes were coated with 1× Matrigel at room temperature for 10 minutes.
2. HEK293-NK3 cell line was cultured in complete medium and seeded in the coated dishes at 37°C, 5% $CO_2$ until 70%~90% confluence.
3. Preparation of the positive control compound and the compounds to be tested:

1) The compound was diluted to 11 concentrations on a 384-well Echo compound plate (LABCYTE, LP-0200) by using the Bravo instrument;
2) Then 10 nL of compound per well (at storage concentration of the compound, for example, the highest concentration point 10 mM) was transferred to a 384-well cell culture plate (Corning, 3824) by using the ECHO instrument, and the plate was kept at room temperature for use.

4. The cells were digested and resuspended in 1× Stimulation Buffer (IP-ONE Gq Kit), seeded into the 384-well cell culture plate (which contained the compound to be tested) at 5,000 cells/well/5 μL by using Multidrop Combi. The culture plate was centrifuged at 300 rpm at room temperature for 60 seconds, and then incubated at 37°C, 5% $CO_2$ for 15 minutes.
5. The cell culture plate was taken out from the $CO_2$ incubator. 5 μL of 2× $EC_{80}$ (to reach a final concentration of 3 nM) was added by using Multidrop Combi. The culture plate was centrifuged at 300 rpm at room temperature for 60 seconds, and then incubated at 37°C, 5% $CO_2$ for 2 hours.
6. The cell culture plate was taken out from the $CO_2$ incubator. 5 μL of IP1 d2 reagent and then 5 μL of IP1 Tb Cryptate Antibody reagent were added by using Multidrop Combi. The culture plate was centrifuged at room temperature at 300 rpm for 60 seconds, and left to stand at room temperature for 1 hour.
7. The plate was read by using EnVision Multilabel Reader, and data were collected at the same time.

Experimental data processing procedures:

The fluorescence signal ratio (Ratio) was read and collected by EnVision Multilabel Reader. According to the readings of the Low control (DMSO control) and High control (positive compound) experimental groups, the antagonism percentage data were calculated as {% antagonism = (Ratio sample - Ratio low control) / (Ratio high control - Ratio low control)×100}. The 11 concentrations of the compound to be tested after being diluted 3-fold by the reaction system were 10 uM to 0.17 nM. The $IC_{50}$ value of the compound was calculated by fitting the data of antagonism percentage and 11 concentrations to the parametric nonlinear logistic equation by using XLFit.

4. Experimental results:

Table 2. The $IC_{50}$ values of the compounds on the antagonism of IP1 in cells with stable expression of NK3 receptors

| Example No. | NK3R $IC_{50}$ (nM) IP1 |
|---|---|
| 2 | 171.36 |
| 3 | 68.67 |
| 4 | 94.30 |
| 9 | 45.95 |
| 10 | 172.45 |

(continued)

| Example No. | NK3R $IC_{50}$ (nM) IP1 |
|---|---|
| 11 | 176.03 |
| 12 | 39.35 |
| 13 | 130.51 |
| 14 | 41.88 |
| 16 | 145.81 |
| 17 | 15.18 |
| 19 | 15.60 |
| 20 | 27.26 |
| 21 | 28.22 |
| 22 | 56.96 |
| 23 | 43.59 |
| 24 | 49.46 |
| 25 | 47.76 |
| 26 | 36.08 |
| 29 | 29.04 |
| 30 | 187.13 |
| 31 | 43.15 |
| 37 | 56.50 |
| 49 | 135.10 |
| 53 | 178.76 |
| 55 | 88.32 |
| 56 | 75.99 |
| 57 | 28.86 |
| 58 | 84.06 |
| 60 | 189.71 |
| 61 | 108.43 |
| 62 | 92.32 |
| 97 | 172.32 |
| 98 | 100.17 |
| 99 | 23.29 |
| 101 | 60.96 |
| 102 | 16.43 |
| 103 | 15.98 |
| 105 | 108.31 |
| 106 | 129.04 |
| 107 | 80.48 |
| 115 | 76.62 |
| 117 | 116.17 |

(continued)

| Example No. | NK3R $IC_{50}$ (nM) IP1 |
|---|---|
| 121 | 132.05 |

5. Experimental conclusion:

It can be seen from the data in the table that the compounds of the examples of the present invention showed good antagonism activity in the experiment on IP1 in cells with stable expression of NK3.

**Test Example 4. Pharmacokinetic assays in SD rats**

1. Experimental objective:
SD rats were used as test animals. The *in vivo* (plasma and brain tissue) pharmacokinetic behavior of the compounds of the present invention was investigated by oral administration of the compound in rats at a dose of 5 mg/kg.
2. Experimental protocols:
2.1 Experimental drugs:
The compounds of the examples of the present invention, prepared by the inventors.
2.2 Experimental animals:
Male SD rats (21 animals per group),purchased from Shanghai JieSiJie Laboratory Animal Co., Ltd., under the Animal Production License Number of SCXK (Shanghai) 2013-0006 No. 311620400001794.
2.3 Formulation of the preparation:
The experimental drug was dissolved in 0.5% CMC-Na (1% Tween 80) by sonication to formulate a clear solution or a homogeneous suspension.
2.4 Drug administration:
After an overnight fast, male SD rats (21 animals per group) were administered p.o. with the experimental drug at an administration dose of 5 mg/kg and the administration volume of 10 mL/kg.
2.5 Sample collection:
Rats were sacrificed with $CO_2$ at 0, 0.5, 1, 2, 4, 8 and 24 hours before and after the drug administration. 8 mL of blood was collected by cardiac puncture, placed in EDTA-$K_2$ tubes, and centrifuged at 4°C at 6,000 rpm for 6 min. Plasma was separated and stored at -80°C. The whole brain tissue was dissected and weighed, and then placed in a 2 mL centrifuge tube and stored at -80°C.
2.6 Sample processing:

1) 160 μL of acetonitrile was added to 40 μL of plasma sample for precipitation. After mixing, the sample was centrifuged at 3500×g for 5-20 minutes.
2) 90 μL of acetonitrile containing internal standard (100 ng/mL) was added to 30 μL of plasma or brain homogenate sample for precipitation. After mixting, the sample was centrifuged at 13,000 rpm for 8 minutes.
3) 70 μL of water was added to 70 μL of treated supernatant, and mixed by vortex for 10 minutes. Then 20 μL of the sample was analyzed by LC/MS/MS to determine the concentration of the compound to be tested. The LC/MS/MS instrument for analysis was AB Sciex API 4000 Qtrap.

2.7 Liquid chromatography analysis:
• Liquid chromatography conditions: Shimadzu LC-20AD pump
• Chromatographic column: Agilent ZORBAX XDB-C18 (50×2.1 mm, 3.5 μm) Mobile phase: Eluent A was 0.1% formic acid in water, and Eluent B was acetonitrile.
• Flow rate: 0.4 mL/min
• Elution time: 0-4.0 minutes, the eluate was as follows:

| Time/minute | Solution A | Solution B |
|---|---|---|
| 0.01 | 90% | 10% |
| 0.5 | 90% | 10% |
| 0.8 | 5% | 95% |

(continued)

| Time/minute | Solution A | Solution B |
|---|---|---|
| 2.4 | 5% | 95% |
| 2.5 | 90% | 10% |
| 4.0 | Stop | |

3. Experimental results and analysis

The major pharmacokinetic parameters were calculated by using WinNonlin 6.1, and the results of the pharmacokinetic experiments in rats were shown in Table 3 below:

Table 3: Experimental results of pharmacokinetic assay in rats

| Example | Pharmacokinetic assay (5 mg/kg) | | | | |
|---|---|---|---|---|---|
| | Peak time | Plasma concentration | Area under curve | Half life | Mean residence time |
| | $t_{max}$ (h) | $C_{max}$ (ng/mL) | $AUC_{0-t}$ (ng/mL×h) | $t_{1/2}$ (h) | MRT (h) |
| 17 Plasma | 6.0 | 2303 | 46696 | NA | NA |
| 17 Brain | 4.0 | 1118 | 3226 | NA | NA |
| 37 Plasma | 8.0 | 4000 | 77786 | NA | NA |
| 37 Brain | 4.0 | 1636 | 4201 | NA | NA |
| 56 Plasma | 2.0 | 1170 | 6147 | 3.1 | 5.1 |
| 56 Brain | 1.0 | 798 | 2710 | NA | NA |
| 57 Plasma | 2.0 | 930 | 7126 | 3.2 | 4.8 |
| 57 Brain | 1.0 | 670 | 2337 | NA | NA |
| NA: Not assessed. | | | | | |

4. Experimental conclusion:

It can be seen from the experimental results of the pharmacokinetic assay in rats in the table that the compounds of the examples of the present invention showed good metabolic properties, and showed good results for the exposure AUC and the maximum plasma concentration $C_{max}$.

**Test Example 5. Effect of the compounds of the present invention on luteinizing hormone (LH) content in plasma of bilateral ovariectomy (OVX) rats**

1. Experimental objective:

The objective of this test example was to determine the effect of the compounds on luteinizing hormone (LH) in plasma of bilateral ovariectomy (OVX) rats.

2. Experimental instruments and reagents:

2.1 Experimental instruments:

Balance (PR2202ZH/E, OHAUS);
Centrifuge (5424R, Eppendorf);
Microplate reader (BioTek Synergy H1);
Plate washer (Thermo Scientific);
Pipettes (Eppendorf & Rainin);
Pure water system (Thermo Scientific);

2.2 Experimental reagents:

CMCNa (30036365, Sinopharm Chemical Reagent);
Tween 80 (30189828, Sinopharm Chemical Reagent);
Rat LH ELISA Kit (S-type) was purchased from Shibayagi, Japan, Catalog No. AKRLH-010S, and the kit comprised Assay buffer C, Sample dilution buffer G, Washing buffer, biotinylated anti-LHα antibody, HRP-conjugated streptavidin and Chromogen (TMB).

2.3 Experimental animals:
10-Week-old female bilateral ovariectomy SD rats were purchased from Beijing Vital River Laboratory Animal Technology Co., Ltd. The animals were housed in a SPF grade animal facility with 5 SD rats per cage. The cages, bedding, feed and water were disinfected at high temperatures before use. All animals had free access to food and water.

3. Experimental methods:
Drug administration by groups: 10-Week-old female SD rats were subjected to bilateral ovariectomy and used in experiments after 3 weeks. Each SD rat was fasted overnight before the experiment, and then randomly divided into groups (3 rats per group) before the drug administration. Different test compounds were administered orally to each drug administration group at a dose of 30 mg/kg and a volume of 10 mL/kg.

Sample collection: 0.2-0.3 mL of whole blood of the SD rats in each group was collected at 0, 0.5, 1, 2, 4, 8 hours before and after the drug administration, and placed in EDTA-K2 tubes. The tubes were inverted and mixed well, and centrifuged at 4°C at 5,000 rpm for 5 min. Then the plasma was separated and stored in an ultra-low temperature freezer (-80±10°C).

Sample detection: All reagents, samples and ELISA plates were equilibrated at room temperature (20-25°C) for at least 30 min. The sample was thawed, vortexed and centrifuged for use. The standard curve was prepared with Assay buffer C according to the concentration sequence of 10, 5, 2.5, 1.25, 0.625, 0.313 and 0. The sample was diluted by two folds with Sample dilution buffer G and left to stand at room temperature for 10 min. Then the sample from the previous step was diluted by 2.5 folds with Assay buffer C and placed at room temperature for use. 1× Washing buffer was added to a 96-well plate at 300 μL/well for plate washing, and this step was repeated for 4 times. According to the setting of standard curve wells and sample wells, 50 μL of the corresponding standard and sample was added to each well and mixed well. The plate was sealed and incubated at 20-25°C for 2 h. 1× Biotinylated anti-LHα antibody was added at 50 μL per well and mixed well. The plate was sealed and incubated at 20-25°C for 1 h. 1× Washing buffer was added at 300 μL/well for plate washing, and this step was repeated for 4 times. 1× HRP-conjugated streptavidin formulated was added at 50 μL per well and mixed well. The plate was sealed and incubated at 20-25°C for 0.5 h. 1× Washing buffer was added at 300 μL/well for plate washing, and this step was repeated for 4 times. Chromogen (TMB) was added at 50 μL per well and mixed well. The plate was incubated at 20-25°C for 20 min. Stop solution (1 M $H_2SO_4$) was added at 50 μL per well and mixed well. The OD values of each well was read at a wavelength of 450 nm with a microplate reader.

Experimental data processing procedures:

The standard curve was plotted by using Graphpad, and the concentration of the sample was calculated. If the sample was diluted upon measurement, then the value should be multiplied by the corresponding dilution fold in the final calculation to give the actual concentration of the sample.

5. Experimental results:

Table 4. Effect of the compounds on luteinizing hormone (LH) content in plasma of ovariectomy (OVX) rats.

| Example No. | ∑AUC(ΔLH/LH•h, Mean ± SD) |
|---|---|
| 37 | -0.52 ± 2.24<br>(1.37 ± 1.98) |
| 62 | -1.77 ± 0.42<br>(-1.84 ± 0.24) |

(continued)

| Example No. | ∑AUC(ΔLH/LH•h, Mean ± SD) |
|---|---|
| 73 | -2.04 ± 1.36<br>(-1.84 ± 0.24) |
| Note: The data in parentheses indicate that this example corresponds to the Vehicle group (i.e., the control group). | |

6. Experimental conclusion:

It can be seen from the data in the table that the compounds of the examples of the present invention could significantly reduce the LH content in plasma of ovariectomy (OVX) rats.

**Test Example 6. Pharmacodynamic study of the test compounds on tail skin temperature in a Senktide-induced bilateral ovariectomy rat model**

1. Experimental objective:
To evaluate the effect of the test compounds on tail skin temperature in Senktide-induced bilateral ovariectomy rats.
2. Experimental instruments and reagents:

2.1. Instruments:

Balance (BSA2202s-CW, Sartorius);
Thermometer (BAT-10, Physitemp);
Probe (SST-1, Physitemp).

2.2. Reagents

Senktide (106128-89-6, MCE);
NaCl (10019318, Sinopharm Chemical Reagent);
DMSO (D2650-100ML, Sigma);
CMCNa (30036365, Sinopharm Chemical Reagent);
Tween 80 (30189828, Sinopharm Chemical Reagent).

2.3. Experimental animals
Ten-Week-old female bilateral ovariectomy SD rats were purchased from Beijing Vital River Laboratory Animal Technology Co., Ltd. The animals were housed in a SPF grade animal facility with 5 SD rats per cage. The cages, bedding, feed and water were disinfected at high temperatures before use. All animals had free access to food and water.
2.4. Test compounds:
The compounds of the examples of the present invention, prepared by the inventors.

3. Experimental operations:
Ten-week-old female SD rats were subjected to bilateral ovariectomy and recovered for two weeks, and then randomly divided into negative control group, model group and drug administration group (8 rats per group), based on body weight. The probe was attached to the dorsal surface of the rat tail with medical tape, 1-2 cm away from the tailhead of the rat.

The vehicle was administered orally to the negative control group and the model group, and different test compounds were administered orally to each drug administration group at a dose of 30 mg/kg and a volume of 10 mL/kg.
Thirty minutes after the oral administration of the vehicle or test compound, the negative control group was injected subcutaneously with normal saline (at a volume of 5 mL/kg); the model group and each drug administration group were injected subcutaneously with 0.2 mg/mL of Senktide (at a volume of 5 mL/kg) to induce the hot flashes-like symptom. The tail skin temperature (TST) was measured and recorded at 0 minute before the injection, and every 5 minutes after the injection, for a total of 75 min.

4. Data processing:

The change in tail skin temperature (ΔTST) at each time point relative to time point 0 was calculated, the ΔTST-time curve of the change in tail skin temperature was plotted, and the area under curve ($AUC_{\Delta TST}$) was calculated. $\Delta TST=TST_n-TST_0$, $TST_0$ is the tail skin temperature at 0 minute before the subcutaneous injection of normal saline or Senktide; $TST_n$ is the tail skin temperature at the n minute after the subcutaneous injection.

$$AUC_{\Delta TST}=\sum (\Delta TST_n+\Delta TST_{n+5}) *5/2$$

5. Experimental results:

Table 5. Pharmacodynamic data of tail skin temperature in a OVX rat model

| Example No. | $AUC_{\Delta TST}$, °C•min |
|---|---|
| Fezolinetant | 116.7 ± 50.6<br>(150.2 ± 40.4) |
| 2 | 71.3 ± 25.5<br>(157.1 ± 41.0) |
| 3 | 81.3 ± 37.4<br>(157.1 ± 41.0) |
| 51 | 108.2±30.4<br>(164.3 ± 37.6) |
| 56 | 53.8 ± 19.8*<br>(150.2 ± 40.4) |
| Note: The data in parentheses indicate that this example corresponds to the Vehicle/Senktide group (i.e., the control group). | |

6. Experimental conclusion:

It can be seen from the data in the table that the compounds of the examples of the present invention could effectively inhibit the symptom of Senktide-induced hot flashes in ovariectomy rats.

## Claims

**1.** A compound of formula (I), a stereoisomer thereof or a pharmaceutically acceptable salt thereof,

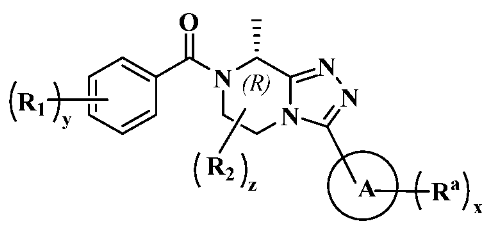

(I)

wherein:

ring A is selected from the group consisting of cycloalkyl, heterocyclyl, aryl and heteroaryl;
$R^a$ is selected from the group consisting of hydrogen, deuterium, alkyl, deuterated alkyl, haloalkyl, hydroxyalkyl, alkoxy, alkylthio, haloalkoxy, halogen, amino, nitro, hydroxy, cyano, alkenyl, alkynyl, cycloalkyl, heterocyclyl,

aryl, heteroaryl, $-(CH_2)_n-$, $-(CH_2)_nR_{aa}$, $-(CH_2)_nOR_{aa}$, $-(CH_2)_nSR_{aa}$, $-(CH_2)_nC(O)R_{aa}$, $-(CH_2)_nC(O)OR_{aa}$, $-(CH_2)_nS(O)_mR_{aa}$, $-(CH_2)_nNR_{aa}R_{bb}$, $-(CH_2)_nC(O)NR_{aa}R_{bb}$, $-(CH_2)_nNR_{aa}C(O)R_{bb}$ and $-(CH_2)_nNR_{aa}S(O)_mR_{bb}$, the amino, alkyl, deuterated alkyl, haloalkyl, hydroxyalkyl, alkoxy, alkylthio, haloalkoxy, alkenyl, alkynyl, cycloalkyl, heterocyclyl, aryl and heteroaryl may optionally be further substituted;

$R_1$ is selected from the group consisting of hydrogen, deuterium, alkyl, deuterated alkyl, haloalkyl, hydroxyalkyl, alkoxy, alkylthio, haloalkoxy, halogen, amino, nitro, hydroxy, cyano, alkenyl, alkynyl, cycloalkyl, heterocyclyl, aryl and heteroaryl, the amino, alkyl, deuterated alkyl, haloalkyl, hydroxyalkyl, alkoxy, alkylthio, haloalkoxy, alkenyl, alkynyl, cycloalkyl, heterocyclyl, aryl and heteroaryl may optionally be further substituted;

$R_2$ is selected from the group consisting of hydrogen, deuterium, alkyl, deuterated alkyl, haloalkyl, hydroxyalkyl, alkoxy, alkylthio, haloalkoxy, halogen, amino, nitro, hydroxy, cyano, oxo, alkenyl, alkynyl, cycloalkyl, heterocyclyl, aryl and heteroaryl, the amino, alkyl, deuterated alkyl, haloalkyl, hydroxyalkyl, alkoxy, alkylthio, haloalkoxy, alkenyl, alkynyl, cycloalkyl, heterocyclyl, aryl and heteroaryl may optionally be further substituted;

$R_{aa}$ and $R_{bb}$ are each independently selected from the group consisting of hydrogen, deuterium, alkyl, deuterated alkyl, haloalkyl, hydroxyalkyl, alkoxy, alkylthio, haloalkoxy, halogen, amino, nitro, hydroxy, cyano, alkenyl, alkynyl, cycloalkyl, heterocyclyl, aryl and heteroaryl, the amino, alkyl, deuterated alkyl, haloalkyl, hydroxyalkyl, alkoxy, alkylthio, haloalkoxy, alkenyl, alkynyl, cycloalkyl, heterocyclyl, aryl and heteroaryl may optionally be further substituted;

x is 0, 1, 2, 3, 4, 5 or 6;
y is 0, 1, 2, 3, 4 or 5;
z is 0, 1, 2, 3, 4 or 5;
m is 0, 1 or 2; and
n is 0, 1 or 2.

2. The compound of formula (I), the stereoisomer thereof or the pharmaceutically acceptable salt thereof according to claim 1, **characterized in that**, at least one deuterium atom is present, but (R)-(4-fluorophenyl)-(8-methyl-3-(3-(methyl-d3)-1,2,4-thiadiazol-5-yl)-5,6-dihydro-[1, 2,4]triazolo[4,3-a]pyrazin-7(8H)-yl)methanone is excluded.

3. The compound of formula (I), the stereoisomer thereof or the pharmaceutically acceptable salt thereof according to claim 1, **characterized in that**, at least one deuterium atom is present in $R_1$.

4. The compound of formula (I), the stereoisomer thereof or the pharmaceutically acceptable salt thereof according to any one of claims 1 to 3, **characterized in that**, ring A is selected from the group consisting of $C_{3-8}$ cycloalkyl, 3-12 membered heterocyclyl, $C_{6-14}$ aryl and 5-14 membered heteroaryl; preferably 5-10 membered heteroaryl containing 1-4 atoms selected from the group consisting of N, O and S; more preferably five membered heteroaryl, five membered fused five membered heteroaryl, or five membered fused six membered heteroaryl containing 1-4 atoms selected from the group consisting of N, O and S; further preferably

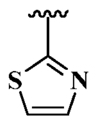 , 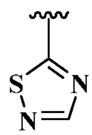 , 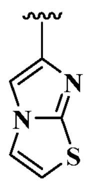 , 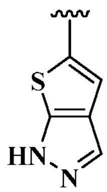 ,

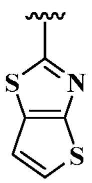 , 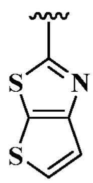 , 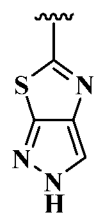 , 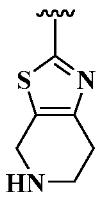 , 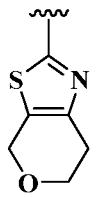 , 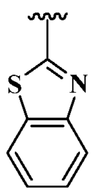 , 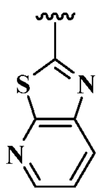 , 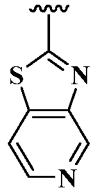 , 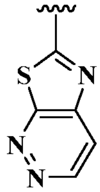 ,

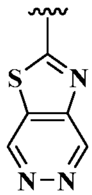, 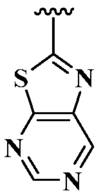, 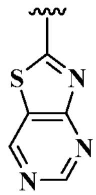, 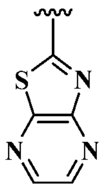, 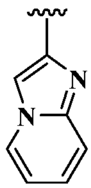 or 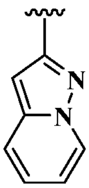.

5. The compound of formula (I), the stereoisomer thereof or the pharmaceutically acceptable salt thereof according to any one of claims 1 to 3, **characterized in that**, $R^a$ is selected from the group consisting of hydrogen, deuterium, halogen, amino, hydroxy, cyano, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-8}$ cycloalkyl, 3-12 membered heterocyclyl, $C_{6-14}$ aryl, 5-14 membered heteroaryl, $-(CH_2)_n-$, $-(CH_2)_nR_{aa}$, $-(CH_2)_nOR_{aa}$, $-(CH_2)_nSR_{aa}$, $-(CH_2)_nC(O)R_{aa}$, $-(CH_2)_nC(O)OR_{aa}$, $-(CH_2)_nS(O)_mR_{aa}$, $-(CH_2)_nNR_{aa}R_{bb}$, $-(CH_2)_nC(O)NR_{aa}R_{bb}$, $-(CH_2)_nNR_{aa}C(O)R_{bb}$ and $-(CH_2)_nNR_{aa}S(O)_mR_{bb}$, the $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-8}$ cycloalkyl, 3-12 membered heterocyclyl, $C_{6-14}$ aryl and 5-14 membered heteroaryl are each optionally further substituted by one or more substituents of deuterium, halogen, amino, hydroxy, cyano, $C_{1-6}$ aryl, $C_{2-6}$ alkenyl, $C_{2-6}$ alkynyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-8}$ cycloalkyl, 3-12 membered heterocyclyl, $C_{6-14}$ aryl and 5-14 membered heteroaryl;

preferably hydrogen, deuterium, halogen, cyano, amino, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ deuterated alkoxy, $-(CH_2)_{n1}OR_{aa}$, $-(CH_2)_{n1}SR_{aa}$ or $-(CH_2)_{n1}NR_{aa}R_{bb}$;
more preferably hydrogen, deuterium, fluorine, chlorine, bromine, cyano, amino, methyl, ethyl, isopropyl, deuterated methyl, ethynyl, $-OCH_3$, $-OCD_3$, $-NHCH_3$, $-N(CH_3)_2$ or $-SCH_3$.

6. The compound of formula (I), the stereoisomer thereof or the pharmaceutically acceptable salt thereof according to any one of claims 1 to 3, **characterized in that**, $R_1$ is selected from the group consisting of hydrogen, deuterium, halogen, amino, hydroxy, cyano, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-8}$ cycloalkyl, 3-12 membered heterocyclyl, $C_{6-14}$ aryl and 5-14 membered heteroaryl, the $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-8}$ cycloalkyl, 3-12 membered heterocyclyl, $C_{6-14}$ aryl and 5-14 membered heteroaryl are each optionally further substituted by one or more substituents of deuterium, halogen, amino, hydroxy, cyano, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-8}$ cycloalkyl, 3-12 membered heterocyclyl, $C_{6-14}$ aryl and 5-14 membered heteroaryl;

preferably hydrogen, deuterium, halogen or 5-6 membered heteroaryl;
more preferably hydrogen, deuterium, fluorine, chlorine, bromine or thienyl.

7. The compound of formula (I), the stereoisomer thereof or the pharmaceutically acceptable salt thereof according to any one of claims 1 to 3, **characterized in that**, $R_2$ is selected from the group consisting of hydrogen, deuterium, halogen, amino, hydroxy, cyano, oxo, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-8}$ cycloalkyl, 3-12 membered heterocyclyl, $C_{6-14}$ aryl and 5-14 membered heteroaryl.

8. The compound of formula (I), the stereoisomer thereof or the pharmaceutically acceptable salt thereof according to any one of claims 1 to 3, **characterized in that**, $R_{aa}$ and $R_{bb}$ are each independently selected from the group consisting of hydrogen, deuterium, halogen, amino, hydroxy, cyano, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-8}$ cycloalkyl, 3-12 membered heterocyclyl, $C_{6-14}$ aryl and 5-14 membered heteroaryl, the $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-8}$ cycloalkyl, 3-12 membered heterocyclyl, $C_{6-14}$ aryl and 5-14 membered heteroaryl are each optionally further substituted by one or more substituents of deuterium, halogen, amino, hydroxy, cyano, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-8}$ cycloalkyl, 3-12 membered heterocyclyl, $C_{6-14}$ aryl and 5-14 membered heteroaryl.

9. The compound of formula (I), the stereoisomer thereof or the pharmaceutically acceptable salt thereof according to

any one of claims 1 to 3, **characterized in that**, ring

A is selected from the group consisting of

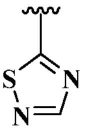

and

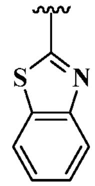

;

$R^a$ is selected from the group consisting of hydrogen, deuterium, halogen, cyano, amino, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ deuterated alkoxy, $-(CH_2)_{n1}OR_{aa}$, $-(CH_2)_{n1}SR_{aa}$ and $-(CH_2)_{n1}NR_{aa}R_{bb}$;
preferably hydrogen, deuterium, fluorine, chlorine, cyano, amino, methyl, deuterated methyl, ethynyl, $-OCH_3$, $-OCD_3$, $-NHCH_3$, $-N(CH_3)_2$ or $-SCH_3$.

**10.** The compound of formula (I), the stereoisomer thereof or the pharmaceutically acceptable salt thereof according to any one of claims 1 to 3, **characterized in that**, the formula (I) is further as shown in formula (II):

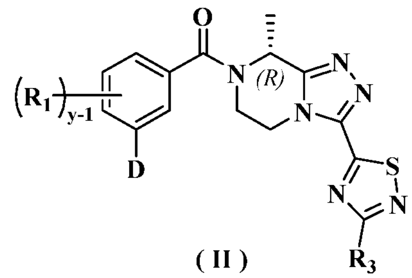

(II)

wherein:

$R_1$ is selected from the group consisting of hydrogen, deuterium and halogen; preferably hydrogen, deuterium, fluorine, chlorine or bromine;
$R_3$ is selected from the group consisting of hydrogen, deuterium, halogen, $C_{1-6}$ alkyl, $C_{1-6}$ deuterated alkyl, 3-6 membered heterocyclyl; the $C_{1-6}$ alkyl, $C_{1-6}$ deuterated alkyl and 3-6-membered heterocyclyl are each optionally further substituted by one or more substituents of deuterium and halogen; preferably hydrogen, deuterium, fluorine, chlorine, bromine, methyl, deuterated methyl, azetidinyl, tetrahydropyrrolyl,

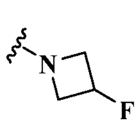

or

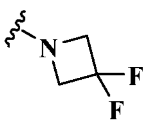

;

y is 1, 2, 3, 4 or 5.

**11.** The compound of formula (I), the stereoisomer thereof or the pharmaceutically acceptable salt thereof according to any one of claims 1 to 10, **characterized in that**, the specific structure of the compound is as follows:

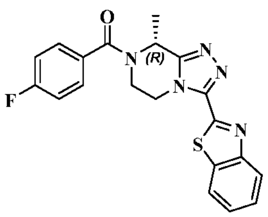

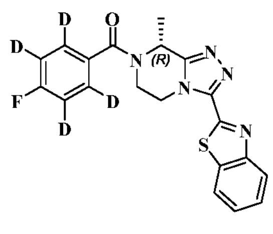

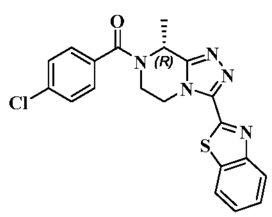

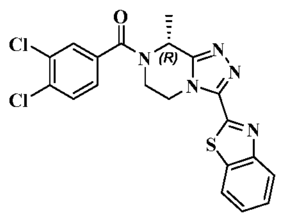

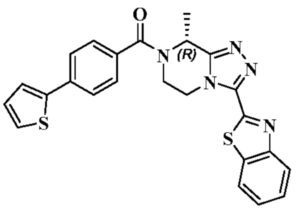
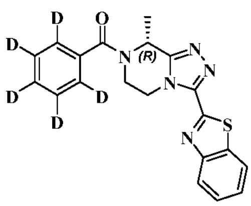
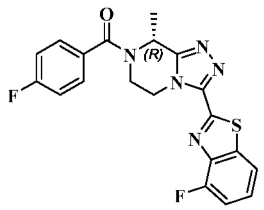
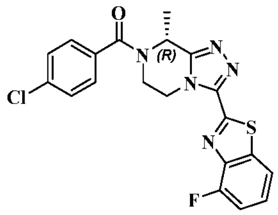
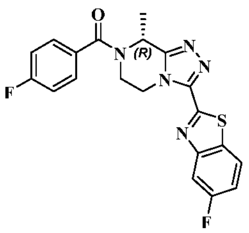
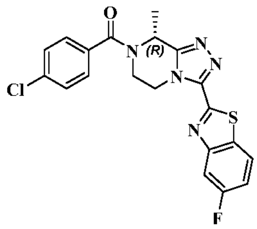
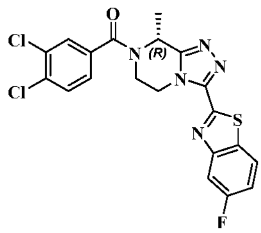
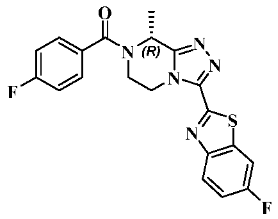
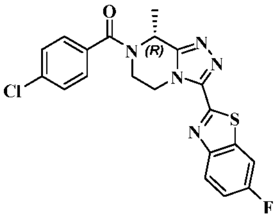
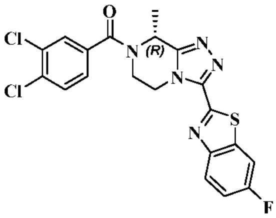
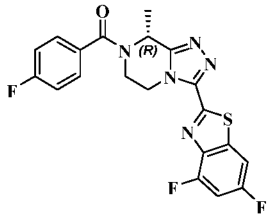
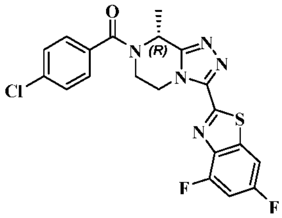
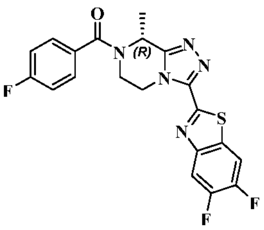
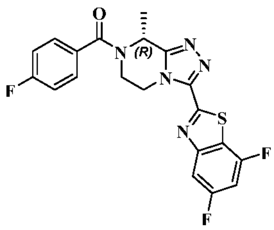
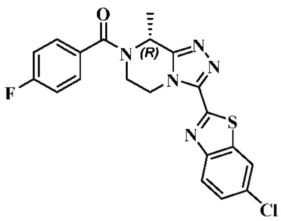
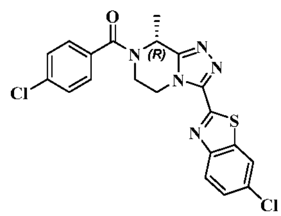
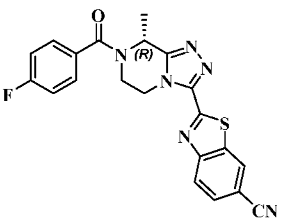
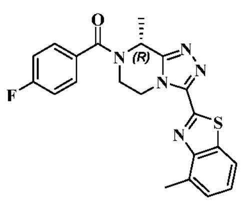
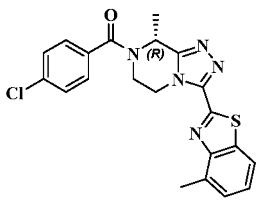
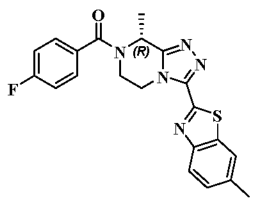
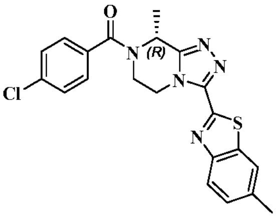
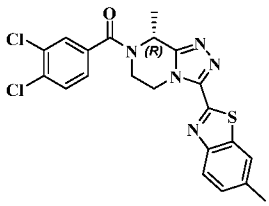
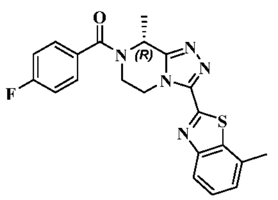
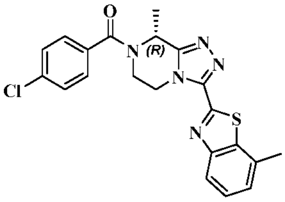

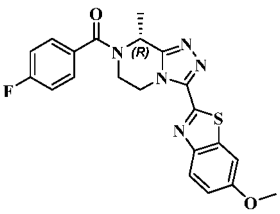
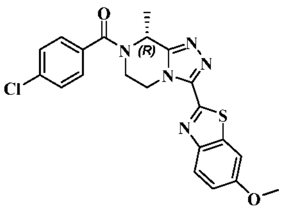
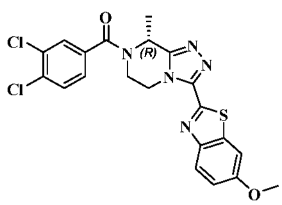
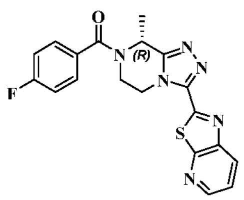

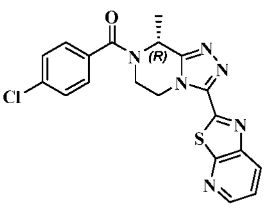
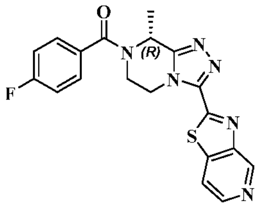
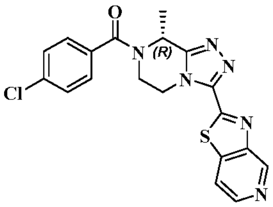
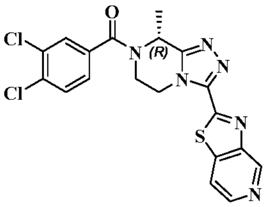

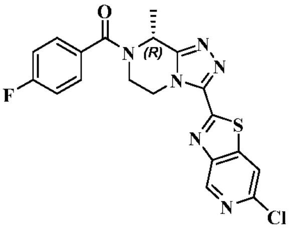
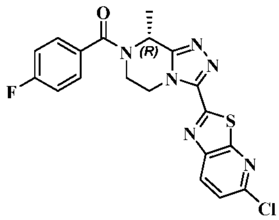
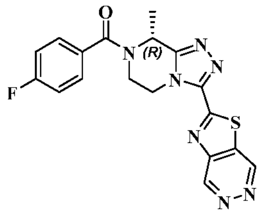
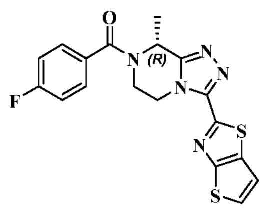

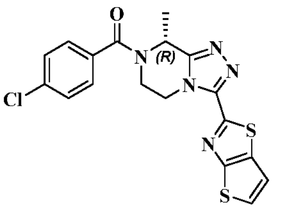
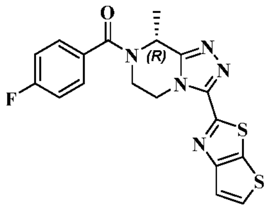
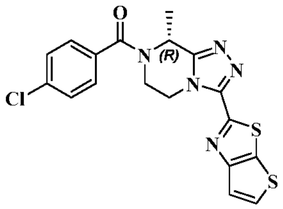
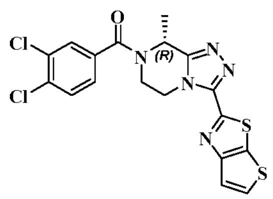

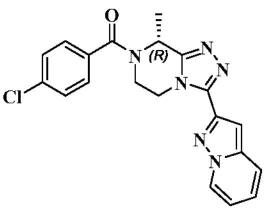
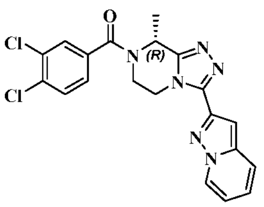
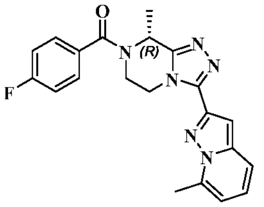
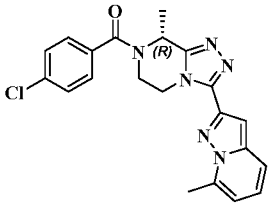

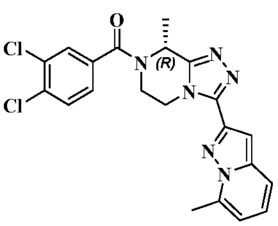
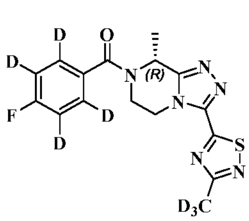
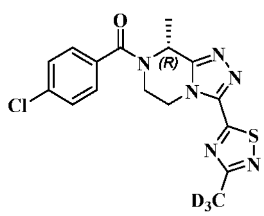
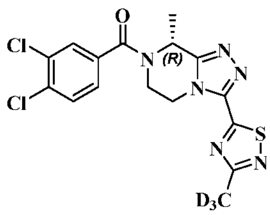

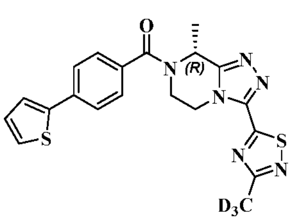
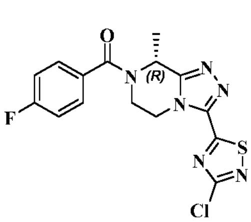
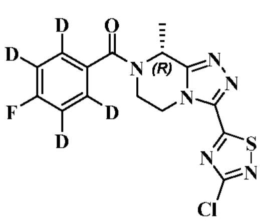
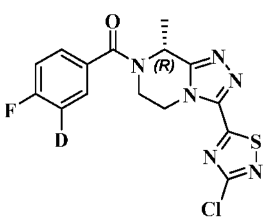

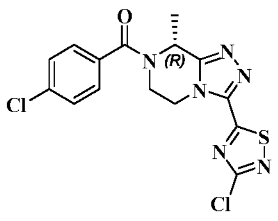
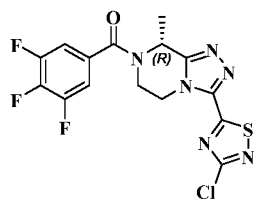
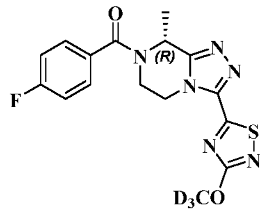
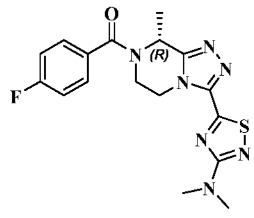
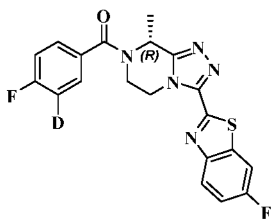
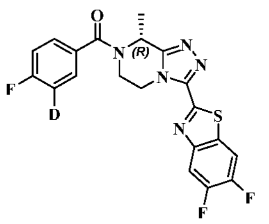
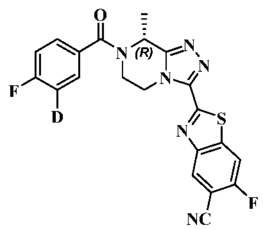
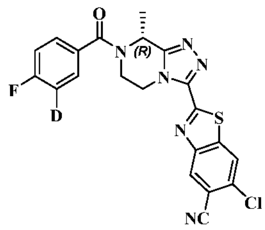
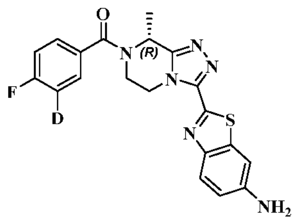
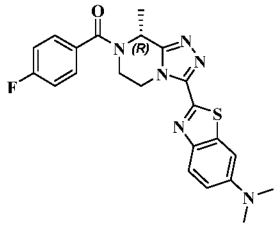
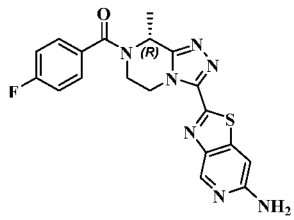
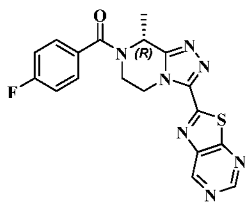
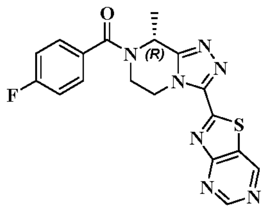
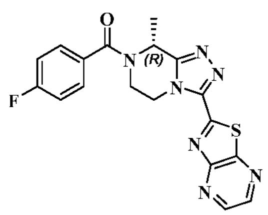
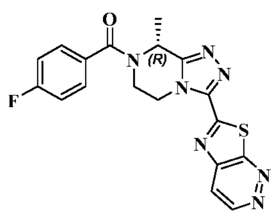
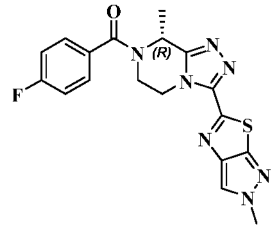
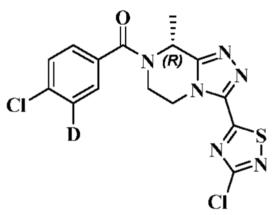
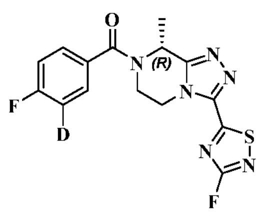
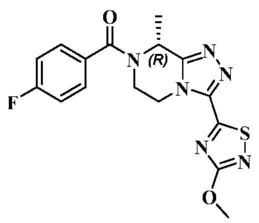
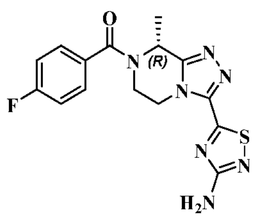
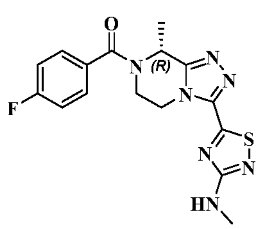
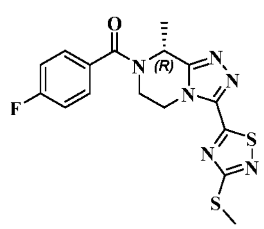
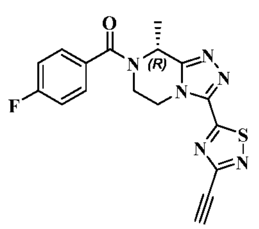
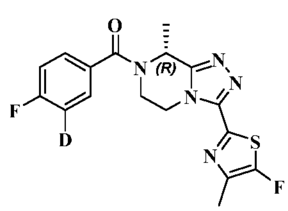
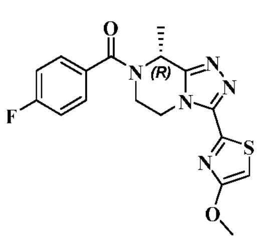
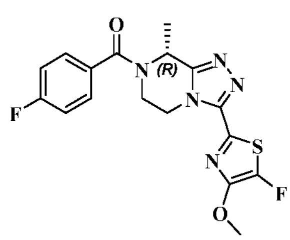
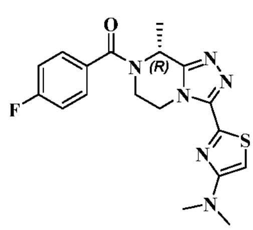
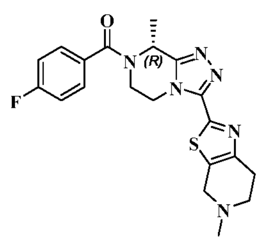

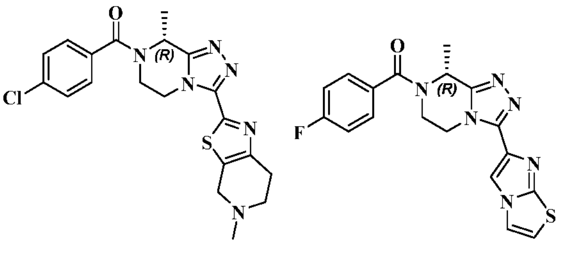
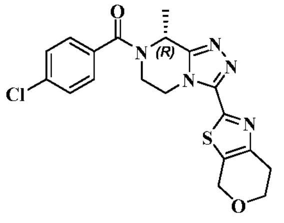
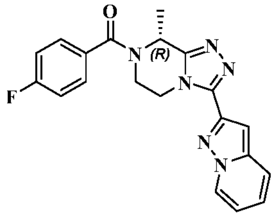

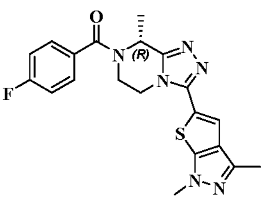
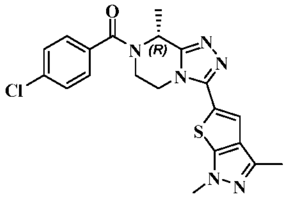
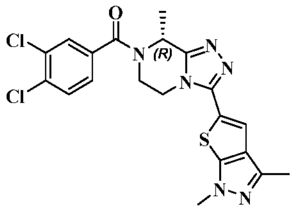
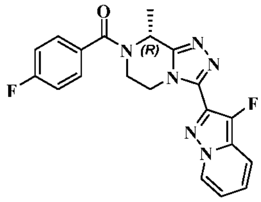

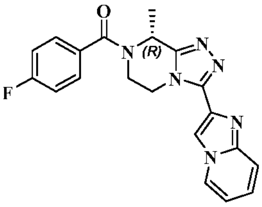
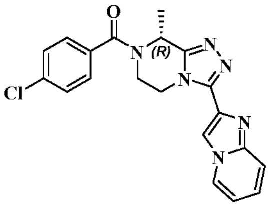
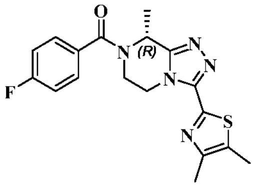
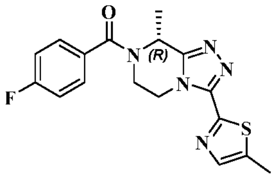

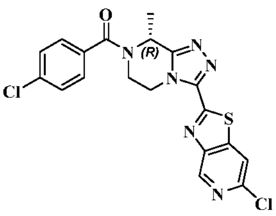
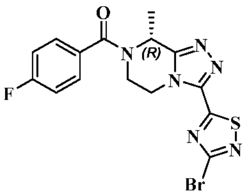
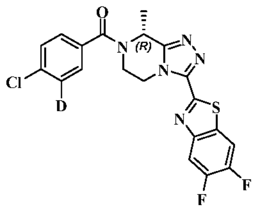
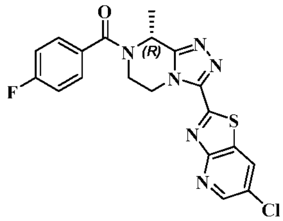

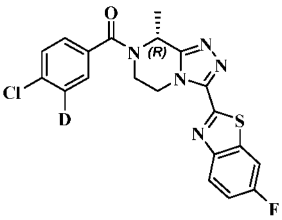
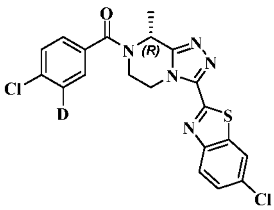
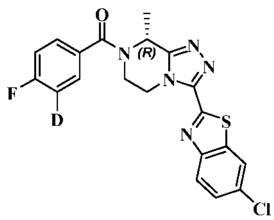
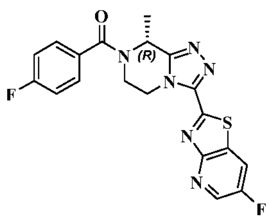

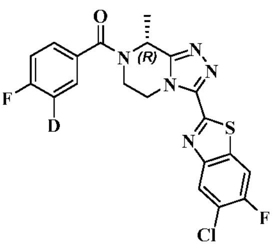
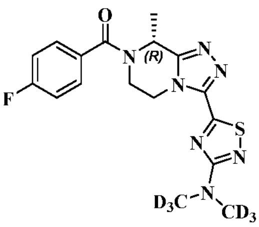
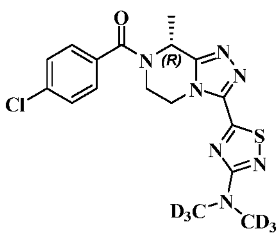
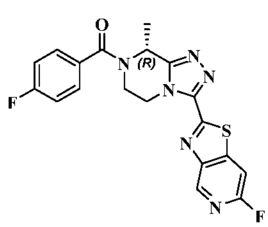

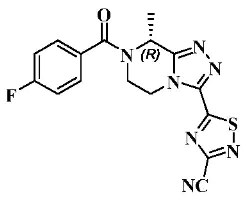
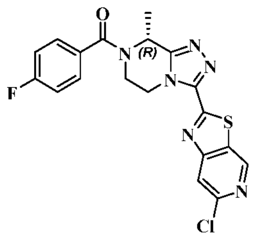
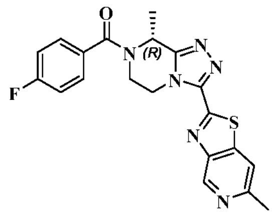
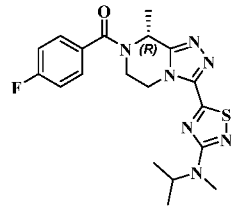
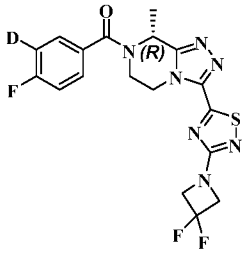
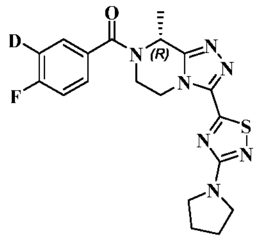
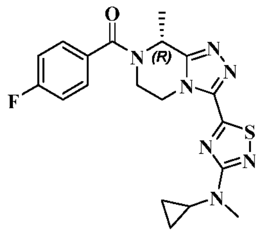
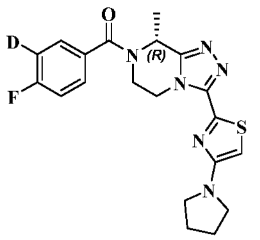
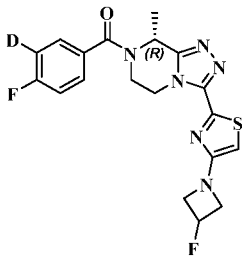
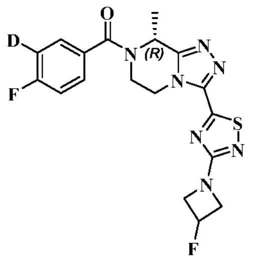
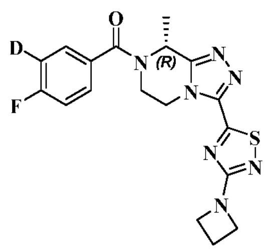
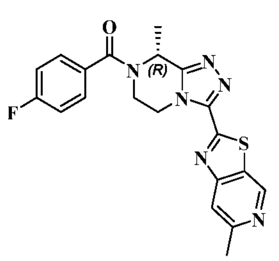
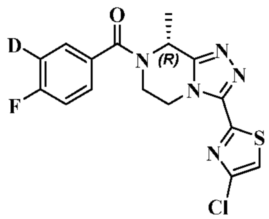
or
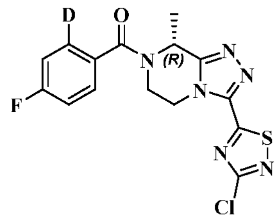
.

**12.** A method for preparing the compound of formula (I), the stereoisomer thereof or the pharmaceutically acceptable salt thereof according to claim 1, **characterized in that**, the method comprises the following steps of:

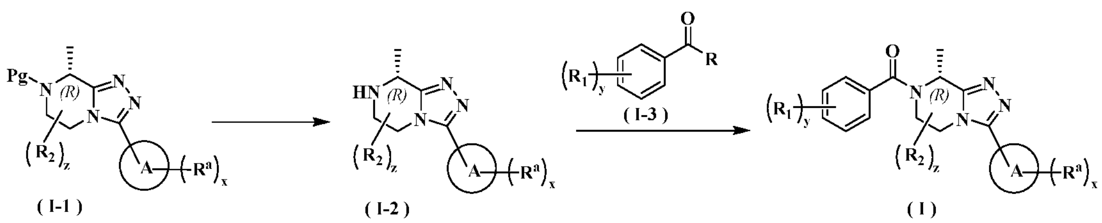

deprotecting a compound of formula (I-1) to obtain a compound of formula (I-2) or a stereoisomer thereof and a pharmaceutically acceptable salt thereof; then, subjecting the compound of formula (I-2) and a compound of formula (I-3) to a condensation reaction to obtain the compound of formula (I) or the stereoisomer thereof or the pharmaceutically acceptable salt thereof;

wherein,

Pg is an amino protecting group; preferably allyloxycarbonyl, trifluoroacetyl, 2,4-dimethoxybenzyl, nitrobenzenesulfonyl, triphenylmethyl, fluorenylmethyloxycarbonyl, p-toluenesulfonyl, formate, acetyl, benzyloxycarbonyl, tert-butoxycarbonyl, benzyl or p-methoxyphenyl; more preferably 2,4-dimethoxybenzyl;

R is selected from the group consisting of halogen, hydroxy and -C(O)OR$_A$, preferably fluorine, chlorine, bromine,

iodine or hydroxy; more preferably chlorine or hydroxy;
$R_A$ is $C_{1-6}$ alkyl.

**13.** A method for preparing the compound of formula (II), the stereoisomer thereof or the pharmaceutically acceptable salt thereof according to claim 1, **characterized in that**, the method comprises the following steps of:

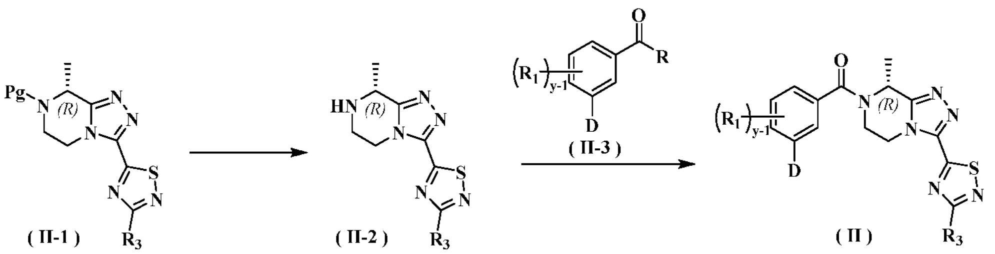

deprotecting a compound of formula (II-1) to obtain a compound of formula (II-2) or a stereoisomer thereof and a pharmaceutically acceptable salt thereof; then, subjecting the compound of formula (II-2) and a compound of formula (II-3) to a condensation reaction to obtain the compound of formula (II) or the stereoisomer thereof or the pharmaceutically acceptable salt thereof;
wherein, Pg and R are as defined in claim 12.

**14.** A pharmaceutical composition comprising a therapeutically effective amount of the compound, the stereoisomer thereof or the pharmaceutically acceptable salt thereof according to claims 1 to 11, and one or more pharmaceutically acceptable carriers or excipients.

**15.** Use of the compound, the stereoisomer thereof or the pharmaceutically acceptable salt thereof according to any one of claims 1 to 11, or the pharmaceutical composition according to claim 14 in the preparation of a NK inhibitor-related medicament, particularly in the preparation of a NK3 inhibitor-related medicament.

**16.** Use of the compound, the stereoisomer thereof or the pharmaceutically acceptable salt thereof according to any one of claims 1 to 11, or the pharmaceutical composition according to claim 14 in the preparation of a medicament for treating and/or preventing psychotic disorder, cognitive disorder, Parkinson's disease, Alzheimer's disease, attention-deficit hyperactivity disorder, pain, convulsion, obesity, inflammatory disease, vomiting, preeclampsia, airway-related disease, reproductive disorder, sex hormone-dependent disease or gynecological disease-related disease.

**17.** Use of the compound, the stereoisomer thereof or the pharmaceutically acceptable salt thereof according to any one of claims 1 to 11, or the pharmaceutical composition according to claim 14 in the preparation of a medicament for treating and/or preventing menopausal syndrome-related disease, wherein the menopausal syndrome includes symptoms of hot flashes, sweating, palpitations, vertigo and obesity.

## INTERNATIONAL SEARCH REPORT

International application No.
**PCT/CN2021/109577**

**A. CLASSIFICATION OF SUBJECT MATTER**

C07D 487/04(2006.01)i; A61K 31/495(2006.01)i; A61K 31/4985(2006.01)i; A61P 25/22(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

WPI, EPODOC, CNPAT, CNKI, REGISTRY, CAPLUS: NK-3, 受体, 拮抗+, [1, 2, 4]三唑并[4, 3-a]吡嗪, 氘, deuterium, triazolo, pyrazine, structure search

**C. DOCUMENTS CONSIDERED TO BE RELEVANT**

| Category* | Citation of document, with indication, where appropriate, of the relevant passages | Relevant to claim No. |
|---|---|---|
| X | CN 102906093 A (EUROSCREEN SA) 30 January 2013 (2013-01-30) description paragraphs [0074]-[0079], [0334]-[0354], [0627], [0833] | 1, 4-9, 11-12, 14-17 |
| X | CN 105175421 B (EUROSCREEN SA) 13 July 2018 (2018-07-13) description paragraphs [0037]-[0042], [0264]-[0272], [0427], [0549], [0785], [0793], [0802] | 1, 4-9, 11-12, 14-17 |
| X | CN 111212838 A (EUROSCREEN SA) 29 May 2020 (2020-05-29) description paragraphs [0014]-[0016], [0026]-[0027], [0054]-[0062], [0155] | 1-12, 14-17 |
| X | CN 105229008 A (EUROSCREEN SA) 06 January 2016 (2016-01-06) description paragraphs [0034]-[0056], [0163]-[0173], [0191]-[0217], [0249]-[0270], [0491]-[0497], [0508], [0518] | 1, 4-9, 11-12, 14-17 |
| PX | CN 111825677 A (MINGHUI PHARMACEUTICAL (HANGZHOU) CO., LTD. et al.) 27 October 2020 (2020-10-27) description paragraphs [0009] to [0039], [0058] to [0060], embodiments 8-9 | 1-17 |
| PY | CN 111825677 A (MINGHUI PHARMACEUTICAL (HANGZHOU) CO., LTD. et al.) 27 October 2020 (2020-10-27) description paragraphs [0009] to [0039], [0058] to [0060], embodiments 8-9 | 1-17 |

☑ Further documents are listed in the continuation of Box C.  ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **15 October 2021** | **04 November 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/109577**

**C. DOCUMENTS CONSIDERED TO BE RELEVANT**

| Category* | Citation of document, with indication, where appropriate, of the relevant passages | Relevant to claim No. |
|---|---|---|
| PX | WO 2020211798 A1 (SHANGHAI HANSOH BIOMEDICAL CO., LTD. et al.) 22 October 2020 (2020-10-22) description page 2 lines 1-23, page 26 lines 1-10, embodiment 170 | 1-17 |
| PY | WO 2020211798 A1 (SHANGHAI HANSOH BIOMEDICAL CO., LTD. et al.) 22 October 2020 (2020-10-22) description page 2 lines 1-23, page 26 lines 1-10, embodiment 170 | 1-17 |

Form PCT/ISA/210 (second sheet) (January 2015)

**INTERNATIONAL SEARCH REPORT**
**Information on patent family members**

International application No.
**PCT/CN2021/109577**

| Patent document cited in search report | Publication date (day/month/year) | Patent family member(s) | Publication date (day/month/year) |
|---|---|---|---|
| CN 102906093 A | 30 January 2013 | CA 2793313 A1 | 06 October 2011 |
| | | US 10544150 B2 | 28 January 2020 |
| | | US 9926325 B2 | 27 March 2018 |
| | | DK 2552920 T3 | 12 June 2017 |
| | | RS 55997 B1 | 29 September 2017 |
| | | AU 2011234398 A1 | 13 September 2012 |
| | | EP 3176171 A1 | 07 June 2017 |
| | | US 8871761 B2 | 28 October 2014 |
| | | SI 2552920 T1 | 31 July 2017 |
| | | AU 2011234398 A2 | 17 January 2013 |
| | | AU 2011234398 C1 | 26 February 2015 |
| | | AU 2011234398 B8 | 05 March 2015 |
| | | CN 102906093 B | 13 January 2016 |
| | | MX 2012011132 A | 08 March 2013 |
| | | WO 2011121137 A1 | 06 October 2011 |
| | | US 2014371218 A1 | 18 December 2014 |
| | | LT 2552920 T | 12 June 2017 |
| | | JP 5822911 B2 | 25 November 2015 |
| | | EP 2552920 A1 | 06 February 2013 |
| | | MX 342161 B | 19 September 2016 |
| | | CA 2793313 C | 23 January 2018 |
| | | KR 101878888 B1 | 17 August 2018 |
| | | ES 2627688 T3 | 31 July 2017 |
| | | JP 2013523697 A | 17 June 2013 |
| | | BR 112012025101 A2 | 20 June 2017 |
| | | HR P20170712 T1 | 28 July 2017 |
| | | HU E032770 T2 | 30 October 2017 |
| | | KR 20130021384 A | 05 March 2013 |
| | | AU 2011234398 A8 | 05 March 2015 |
| | | AU 2011234398 B2 | 23 October 2014 |
| | | EA 201290940 A1 | 29 March 2013 |
| | | SI EP2552920 T1 | 31 July 2017 |
| | | PL 2552920 T3 | 31 August 2017 |
| | | EA 023161 B1 | 29 April 2016 |
| | | US 2018194772 A1 | 12 July 2018 |
| | | PT 2552920 T | 07 June 2017 |
| | | EP 2552920 B1 | 15 March 2017 |
| | | US 2013023530 A1 | 24 January 2013 |
| CN 105175421 B | 13 July 2018 | US 10941151 B2 | 09 March 2021 |
| | | US 9475814 B2 | 25 October 2016 |
| | | US 2020283447 A1 | 10 September 2020 |
| | | SM T201600164 B | 01 July 2016 |
| | | SI EP2763992 T1 | 31 August 2016 |
| | | AU 2012320568 B2 | 06 July 2017 |
| | | PL 2763992 T3 | 31 August 2016 |
| | | EP 2763992 A1 | 13 August 2014 |
| | | BR 112014007652 A2 | 11 April 2017 |
| | | US 2017029429 A1 | 02 February 2017 |
| | | CN 105175421 A | 23 December 2015 |
| | | CA 2849751 C | 11 June 2019 |

Form PCT/ISA/210 (patent family annex) (January 2015)

**INTERNATIONAL SEARCH REPORT**
**Information on patent family members**

International application No.
**PCT/CN2021/109577**

| Patent document cited in search report | Publication date (day/month/year) | Patent family member(s) | Publication date (day/month/year) |
|---|---|---|---|
| | | MX 367774 B | 04 September 2019 |
| | | JP 6294918 B2 | 14 March 2018 |
| | | JP 2014531464 A | 27 November 2014 |
| | | JP 2017019803 A | 26 January 2017 |
| | | CY 1117773 T1 | 17 May 2017 |
| | | EP 2763992 B9 | 24 August 2016 |
| | | DK 2763992 T3 | 25 April 2016 |
| | | AU 2012320568 A1 | 10 April 2014 |
| | | SI 2763992 T1 | 31 August 2016 |
| | | EA 029340 B1 | 30 March 2018 |
| | | CN 103906750 B | 24 August 2016 |
| | | AU 2012320568 C1 | 19 July 2018 |
| | | EP 3029042 A1 | 08 June 2016 |
| | | US 2014275097 A1 | 18 September 2014 |
| | | KR 20140072906 A | 13 June 2014 |
| | | CN 103906750 A | 02 July 2014 |
| | | EA 030586 B1 | 31 August 2018 |
| | | HU E028858 T2 | 30 January 2017 |
| | | RS 54833 B1 | 31 October 2016 |
| | | HK 1197676 A1 | 06 February 2015 |
| | | KR 102049534 B1 | 27 November 2019 |
| | | BR 112014007652 A8 | 23 January 2018 |
| | | EP 2763992 B1 | 06 April 2016 |
| | | MX 2014004057 A | 22 August 2014 |
| | | EA 201490579 A1 | 30 July 2014 |
| | | WO 2013050424 A1 | 11 April 2013 |
| | | EA 201591204 A2 | 29 January 2016 |
| | | JP 6023814 B2 | 09 November 2016 |
| | | CA 2849751 A1 | 11 April 2013 |
| | | US 2019023711 A1 | 24 January 2019 |
| | | US 10683295 B2 | 16 June 2020 |
| | | HR P20160396 T1 | 15 July 2016 |
| | | EA 201591204 A3 | 29 February 2016 |
| | | US 10065961 B2 | 04 September 2018 |
| | | ES 2572604 T3 | 01 June 2016 |
| | | CN 103906750 B9 | 12 October 2016 |
| CN 111212838 A | 29 May 2020 | WO 2019012033 A1 | 17 January 2019 |
| | | US 2020270254 A1 | 27 August 2020 |
| | | EP 3428168 A1 | 16 January 2019 |
| CN 105229008 A | 06 January 2016 | HU E035870 T2 | 28 May 2018 |
| | | US 2015232471 A1 | 20 August 2015 |
| | | HK 1214816 A1 | 05 August 2016 |
| | | KR 20210021406 A | 25 February 2021 |
| | | HK 1244273 A1 | 03 August 2018 |
| | | US 2017240551 A9 | 24 August 2017 |
| | | NZ 712802 A | 28 August 2020 |
| | | WO 2014154895 A1 | 02 October 2014 |
| | | JP 2016515565 A | 30 May 2016 |
| | | PT 2948455 T | 15 November 2017 |
| | | MX 2015013711 A | 26 February 2016 |

Form PCT/ISA/210 (patent family annex) (January 2015)

**INTERNATIONAL SEARCH REPORT**
**Information on patent family members**

International application No.
**PCT/CN2021/109577**

| Patent document cited in search report | Publication date (day/month/year) | Patent family member(s) | Publication date (day/month/year) |
|---|---|---|---|
| | | RS 56501 B1 | 28 February 2018 |
| | | EP 3219715 A1 | 20 September 2017 |
| | | EA 027570 B1 | 31 August 2017 |
| | | JP 6316932 B2 | 25 April 2018 |
| | | HR P20171688 T1 | 29 December 2017 |
| | | AU 2014242906 A1 | 22 October 2015 |
| | | AU 2014242906 A2 | 05 November 2015 |
| | | US 9422299 B2 | 23 August 2016 |
| | | ZA 201506234 B | 30 January 2019 |
| | | EP 2948455 A1 | 02 December 2015 |
| | | US 9987274 B2 | 05 June 2018 |
| | | CA 2907809 C | 04 May 2021 |
| | | IL 241473 A | 31 March 2019 |
| | | ES 2646488 T3 | 14 December 2017 |
| | | US 2017095472 A1 | 06 April 2017 |
| | | SI 2948455 T1 | 29 December 2017 |
| | | CN 105229008 B | 15 December 2017 |
| | | NO 2948455 T3 | 24 February 2018 |
| | | DK 2948455 T3 | 20 November 2017 |
| | | LT 2948455 T | 27 November 2017 |
| | | EA 201591689 A1 | 29 January 2016 |
| | | US 10030025 B2 | 24 July 2018 |
| | | CY 1119576 T1 | 07 March 2018 |
| | | JP 2018118988 A | 02 August 2018 |
| | | DK 2948455 T5 | 19 March 2018 |
| | | US 2016318941 A1 | 03 November 2016 |
| | | BR 112015024907 A2 | 18 July 2017 |
| | | KR 20160007515 A | 20 January 2016 |
| | | EP 2948455 B1 | 27 September 2017 |
| | | MX 370000 B | 28 November 2019 |
| | | KR 102218621 B1 | 22 February 2021 |
| | | SG 11201508005 X A | 29 October 2015 |
| | | IL 241473 D0 | 30 November 2015 |
| | | PL 2948455 T3 | 31 January 2018 |
| | | AU 2014242906 B2 | 26 July 2018 |
| | | CA 2907809 A1 | 02 October 2014 |
| CN 111825677 A | 27 October 2020 | None | |
| WO 2020211798 A1 | 22 October 2020 | CN 112272670 A | 26 January 2021 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014154895 A
- CN 103906750
- CN 105229008 B
- CN 102906093 B